(19) Europäisches Patentamt
European Patent Office
Office européen des brevets

(11) EP 2 261 258 A1

(12) **EUROPÄISCHE PATENTANMELDUNG**

(43) Veröffentlichungstag:
**15.12.2010 Patentblatt 2010/50**

(51) Int Cl.:
***C07K 16/30*** *(2006.01)* ***A61K 39/395*** *(2006.01)*
***A61P 35/00*** *(2006.01)* ***C07K 16/46*** *(2006.01)*

(21) Anmeldenummer: **09162200.1**

(22) Anmeldetag: **08.06.2009**

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**
Benannte Erstreckungsstaaten:
**AL BA RS**

(71) Anmelder: **Schuler, Gerold**
**91080 Spardorf (DE)**

(72) Erfinder:
- **Schuler, Gerold Prof. Dr.**
  **91080 Spardorf (DE)**
- **Schwenkert, Michael**
  **90471 Nürnberg (DE)**
- **Dörrie, Jan**
  **90409 Nürnberg (DE)**
- **Schaft, Niels**
  **91074 Herzogenaurach (DE)**

(74) Vertreter: **von Kreisler Selting Werner**
**Deichmannhaus am Dom**
**Bahnhofsvorplatz 1**
**50667 Köln (DE)**

(54) **MCSP-gerichtete scFv-Antikörperfragmente und deren Verwendung**

(57) Die Erfindung betrifft gegen das Melanom-assoziierte Chondroitinsulfat Proteoglykan (MCSP)-gerichtete scFv-Antikörperfragmente mit spezifischer Anordnung der variablen Domänen und spezielle Fusionsproteine dieser scFv-Fragmente, deren Herstellung und Verwendung zur behandlung von Tumorerkrankungen.

EP 2 261 258 A1

## Beschreibung

Die Erfindung betrifft gegen das Melanom-assoziierte Chondroitinsulfat Proteoglykan (MCSP)-gerichtete scFv-Antikörperfragmente mit spezifischer Anordnung der variablen Domänen und spezielle Fusionsproteine dieser scFv-Fragmente, deren Herstellung und Verwendung zur behandlung von Tumorerkrankungen.

## Hintergrund der Erfindung

Maligne Melanome stellen die häufigste Form tödlich verlaufender Hautkrebserkrankungen dar und ihr Auftreten hat sich in den letzten 25 Jahren mehr als verdreifacht. Die Zehn-Jahres-Überlebensrate bei Patienten ohne Metastasierung liegt bei 95% und verschlechtert sich nach Metastasierung auf unter 10%. Im Fall von Fernmetastasen ist das Fortschreiten der Erkrankung meist schneller und die durchschnittliche Lebenserwartung beträgt gewöhnlich nur sechs bis neun Monate. Chemo- und Strahlentherapie werden meist nur unter palliativem Gesichtspunkt angewandt und die Behandlungserfolge des metastasierten Melanoms sind nicht zufriedenstellend. Daher besteht ein dringender Bedarf an neuartigen Therapieoptionen. Antikörper-abgeleitete Immuntherapeutika gegen Melanom-assoziierte Antigene bieten eine vielversprechende Alternative bzw. Ergänzung zu den herkömmlichen Therapieschemata.

Jährlich erkranken in Deutschland etwa 430.000 Menschen an Krebs. Mit 210.000 Todesfällen pro Jahr sind Krebserkrankungen die zweithäufigste Todesursache nach dem Versagen des Herz-Kreislauf-Systems. Die häufigsten Krebserkrankungen bei Männern sind Prostatakrebs (25,4%), Darmkrebs (16,2%) und Lungenkrebs (14,3%). Bei Frauen überwiegen die malignen Erkrankungen der Brustdrüsen (27,8%) vor Darmkrebs (17,5%) und Lungenkrebs (6,4%). Das mittlere Erkrankungsalter liegt unabhängig vom Geschlecht bei 69 Jahren, das mittlere krebsbedingte Sterbealter liegt bei Frauen bei 71 und bei Männern bei 75 Jahren. Die Fünf-Jahres-Überlebensrate gemittelt über alle Krebserkrankungen beträgt lediglich 60% bei Frauen und 53% bei Männern.

Das Maligne Melanom (MM), auch bekannt als schwarzer Hautkrebs, ist ein bösartiger Tumor, der von melanozytären Zellen ausgeht und sich überwiegend an der Haut manifestiert. An einem MM erkrankten im Jahr 2004 in Deutschland rund 8.400 Frauen und 6.500 Männer, was einer Inzidenz von 16,5 bzw. 13,5 Fällen pro 100.000 Einwohner entspricht. Somit stellt diese Krebsform bei Frauen 4,1% und bei Männern 2,8% aller malignen Neoplasien dar und verursacht etwa 1% aller Krebstodesfälle. In den letzten 30 Jahren hat sich die Zahl der Erkrankungen mehr als verdreifacht, was vor allem durch die verstärkte Strahlenexposition hellhäutiger Bevölkerungsgruppen erklärt wird (Armstrong, BK. & Kricker, A., Cancer Surv., 19-20:219-240 (1994); Garbe, C. & Blum, A., Skin Pharmacol. Appl. Skin Physiol. 14:280-290 (2001)). Diesen hohen Einfluss der Strahlenbelastung zeigt auch die weltweit höchste Inzidenz des MM in Australien mit 50 - 60 Fällen pro 100.000 Einwohner pro Jahr.

Mit 63 Jahren bei Männern und 57 Jahren bei Frauen ist das Durchschnittsalter bei der Diagnose eines MM rund 10 Jahre geringer als beim gemittelten Diagnosealter aller Krebsarten. Nach Brustkrebs und Hodenkrebs stellt das MM die zweithäufigste Krebserkrankung im Alter von 20 bis 40 Jahren dar und relevante Erkrankungsraten finden sich bereits ab dem 20. Lebensjahr (Krebs in Deutschland 2003-2004, Robert Koch Institut, 2008). Trotz des erhöhten Auftretens der Krankheit ist die melanombedingte Sterberate in den letzten 30 Jahren auf einem konstanten Niveau geblieben. Hierfür werden eine erhöhte Aufklärung der Bevölkerung sowie eine Sensibilisierung der Ärzteschaft verantwortlich gemacht. Durch die Lokalisation auf der Körperoberfläche können MM in sehr frühen, klinisch günstigen Stadien diagnostiziert werden.

Alle malignen Entartungen der verschiedenen Zelltypen der Haut werden als Hautkrebs zusammengefasst. Dabei treten am häufigsten nicht-melanozytären Formen auf, wie Basalzell- (80%) oder Plattenepithelkarzinome (19%). Allerdings gibt es vergleichsweise wenige Todesfälle durch diese Hautkrebsformen, da sie in der Regel nicht metastasieren. In dieser Eigenschaft besteht der gravierendste Unterschied zum MM. Letzteres weist im Vergleich zu anderen Tumoren eine, im Verhältnis zur Tumormasse, extrem frühe Tendenz zur Metastasierung auf, was in vielen Fällen zu einer sehr ungünstigen Prognose führt. Somit ist das MM für etwa 90% der durch Hautkrebs bedingten Mortalität verantwortlich.

Die Zehn-Jahres-Überlebensrate (10-JÜ) nach Diagnose eines MM beträgt im Mittel 75 - 80%. Bei etwa 90% aller erstmalig diagnostizierten Melanome handelt es sich ausschließlich um den Primärtumor ohne erkennbare Metastasierung, was zu einer sehr günstigen Prognose führt. Neben dem Invasionslevel nach Clark, sowie der Tumorlokalisation und dem Geschlecht, ist der wichtigste prognostische Faktor im Primärtumorstadium die vertikale Tumordicke am histologischen Präparat nach Breslow (Breslow, A., Ann. Surg. 172:902-908 (1970)). So beträgt die 10-JÜ bei dünnen Primärtumoren mit einer Tumordicke < 1 mm 88 - 95%, während sie für Tumordicken > 4 mm auf 52 - 54% sinkt. Nach stattgefundener Metastasierung verschlechtert sich die Prognose drastisch. Im Fall von klinisch manifesten regionären Lymphknotenmetastasen sterben 60 - 80% der Patienten innerhalb von 10 Jahren, und beim Auftreten von viszeralen Metastasen liegt die 10-JÜ unter 10%. Meistens nimmt die Erkrankung im Fall von Fernmetastasen einen rapideren Verlauf mit einer durchschnittlichen Lebenserwartung von lediglich sechs bis neun Monaten (Garbe, C., Recent Results Cancer Res. 160:205-215 (2002)). Entsprechend dem Durchmesser des Primärtumors sowie der Existenz regionärer

Lymphknoten- bzw. Fernmetastasen erfolgt die Einteilung in unterschiedliche klinische Stadien (Balch, CM. et al., J. Clin. Oncol. 19:3635-3648 (2001)), die für den Therapieverlauf eine große Rolle spielen.

Zum Einsatz systemischer Chemotherapie kommt es bei inoperablen Rezidivtumoren sowie Fernmetastasen. Allerdings zeichnen sich Melanomzellen durch eine ausgeprägte Resistenz gegenüber Chemotherapeutika aus, wodurch diese Therapieoption geringe Erfolgsquoten verzeichnet (Helmbach, H. et al., Recent Results Cancer Res. 161:93-110 (2003)). Daher erfolgt eine Behandlung überwiegend unter palliativem Gesichtspunkt und führt meist nicht zu einer Verlängerung des Gesamtüberlebens. Weltweiter Therapiestandard und einziges in Deutschland zugelassenes Chemotherapeutikum ist Dacarbazin (DTIC). Die Ansprechraten werden in den meisten Studien mit 10 - 20% angegeben, wobei laut neueren Studien die Raten eher im Bereich von 7% liegen (Avril, MF. et al., J. Clin. Oncol. 22:1118-1125 (2004)). Es kommt äußerst selten zu kompletten Remissionen und die mittlere Remissionsdauer liegt dann bei nur sechs Monaten. Eine Alternative zu DTIC stellt Temozoloid dar, das durch seine Liquorgängigkeit auch bei Hirnmetastasen Wirkung zeigt und etwa die gleichen Ansprechraten wie DTIC aufweist, ohne dabei lebensverlängernde Wirkung zu zeigen (Quirt, I. et al., Oncologist 12:1114-1123 (2007)).

Die Therapie des MM ist bei ausbleibender Metastasierung relativ unkompliziert und erfolgsversprechend. Abgesehen von Ausnahmefällen, wie der operativen Entfernung an ungünstigen Körperregionen, ist die Exzision der malignen Läsion weitestgehend komplikationsfrei. Im Gegensatz dazu sind beim fortgeschrittenen MM die Behandlungserfolge unbefriedigend. Dabei zeichnen sich die angewandten Methoden durch eine Vielzahl von Limitierungen aus. Sowohl die Strahlentherapie als auch die Chemotherapie werden vor allem unter palliativem Gesichtspunkt angewandt. Hier ist gerade im Fall der Chemotherapie eine erhöhte Begleittoxizität zu beobachten. Die alkylierenden Verbindungen DTIC und Themozoloid übertragen Alkylgruppen auf die DNA und verhindern so die DNA-Replikation. Ihre Wirkung ist nicht Tumor-spezifisch sondern betrifft schnell proliferierende Zellen. Im Fall von DTIC und Themozoloid kommt es zu Knochenmarkschädigungen mit daraus folgender Thrombozytopenie und Neutropenie, zu Übelkeit und Erbrechen, sowie Hepatotoxizität und Neurotoxizität (Atallah, E. & Flaherty, L., Curr. Treat Options Oncol. 6:185-193 (2005)). Bei der Kombination unterschiedlicher Chemotherapeutika kommen neben der Erhöhung der Toxizität weitere unangenehme Begleiterscheinungen hinzu. So bewirkt beispielsweise die Kombination aus DTIC, Vindesin und Cisplatin neben den durch DTIC ausgelösten Begleiterscheinungen zusätzlich Haarausfall und Nierenschädigung.

Die unspezifische Toxizität von Chemotherapeutika ist ein großer Nachteil bei der Behandlung maligner Erkrankungen. Dabei liegen die für den Körper tolerierbaren Dosen meist im suboptimalen Bereich ihrer Wirksamkeit, was zu unbefriedigenden Therapieresultaten führt (Allen, TM., Nat. Rev. Cancer 2:750-763 (2002)). Wünschenswert wäre eine spezifischere Therapie, um Tumorzellen gezielt zu eliminieren und gleichzeitig gesundes Gewebe nicht zu schädigen. Neben dem Einsatz von sogenannten kleinen inhibitorischen Molekülen (*small inhibitory molecules*), die gezielt intrazelluläre Signalwege der Tumorzellen beeinflussen, sind Antikörper für diese Zielsetzung besonders gut geeignet. Sie besitzen eine enorm hohe Bindevariabilität und sind damit in der Lage unterschiedlichste Epitope, wie Peptide oder Kohlenhydrate spezifisch zu erkennen. Mit der Entwicklung der Hybridomtechnologie (Kohler, G. & Milstein, C., Nature 256:495-497 (1975)) war die Möglichkeit gegeben, monoklonale Antikörper gegen humane Epitope in großem Maßstab herzustellen und für verschiedenste Anwendungen einzusetzen. Fortan stand ein reichhaltiges Repertoire zur Bekämpfung maligner Erkrankungen zur Verfügung.

Membranständige Differenzierungsantigene stellen ein potentielles Ziel für Antikörper-basierte Therapapeutika dar, nicht zuletzt weil sie auf Tumorzellen oft stärker exprimiert werden, als auf gesunden Zellen des gleichen Zelltyps (Arteaga, CL., Breast Cancer Res. 5:96-100 (2003)). Mit Hilfe molekularbiologischer und biochemischer Methoden lassen sich Antikörper sowohl auf DNA- als auch auf Proteinebene so modifizieren und optimieren, dass sie unterschiedlichsten therapeutischen Voraussetzungen und Zielsetzungen gerecht werden. Mittlerweile existieren auf diesem Forschungsgebiet zahlreiche Antikörperformate, von denen einige bereits zur Behandlung maligner Erkrankungen eingesetzt werden.

Die Hybridomtechnologie ermöglicht die Herstellung von Antikörpern gegen humane Antigene. Allerdings lösen die aus der Maus oder dem Kaninchen stammenden Antikörper im Menschen Immunreaktionen aus, wodurch Sie nur begrenzt einsetzbar sind. So bilden Patienten nach Gabe eines murinen Antikörpers sogenannte humane-anti-Maus Antikörper (*human-anti-mouse antibodies,* HAMA), welche allergische Reaktionen auslösen und bei wiederholter Gabe den therapeutischen Antikörper neutralisieren (Mirick, GR. et al., Q. J. Nucl. Med. Mol. Imaging 48:251-257 (2004)). Um die Bildung von HAMA-Antikörpern so gering wie möglich zu halten, werden die murinen Regionen durch humane ersetzt. Chimäre Antikörper bestehen aus humanen konstanten und murinen variablen Regionen, während bei humanisierten Antikörpern nur noch die hypervariablen Bereiche (*complementarity determining regions;* CDRs) innerhalb der variablen Regionen murinen Ursprungs sind (Morrison, SL. et al., Proc. Natl. Acad. Sci. U S A 81:6851-6855 (1984); Jenes, PT. et al., Nature 321:522-525 (1986); Qu, Z. et al., Methods 36:84-95 (2005)). Durch *phage display* Technologie ist es möglich, humane Antikörperbanken nach Antikörperfragmenten gewünschter Spezifität zu durchsuchen, welche durch Fusion an eine konstante Region humanen Ursprungs zu ganzen humanen Antiköper werden (Marks, JD. et al., J. Mol. Biol. 222:581-597 (1991); Hoogenboom, HR., Nat. Biotechnol. 23:1105-1116 (2005)). Durch Immunisieren von Tieren, die transgen für humane Immunglobulingene sind, ist es ebenfalls möglich komplett humane Antikörper herzustellen (Jakobovits, 1995; Lonberg & Huszar, 1995).

Je nach Anwendungsbereich werden neben ganzen Antikörpern auch Antikörper-fragmente eingesetzt. So besteht die Möglichkeit, nur den Antigen-bindenden Antikörperteil in Form eines *Fragment (antigen-binding)*$_2$ (F(ab)$_2$), eines *Fragment antigen-binding* (Fab) oder eines *single-chain Fragment variable* (scFv) zu nutzen. Eine Kombination zweier Bindeköpfe, etwa im Format eines bispezifischen scFv-(bsscFv) Fragments, ermöglicht eine simultane Bindung zweier Epitope und kann beispielsweise dazu verwendet werden, einen Effektor des Immunsystems an die Tumorzellen zu rekrutieren und zu aktivieren. Figur 1 gibt eine Übersicht über einige grundlegende Formate therapeutischer Antikörper und Antikörperfragmente. Antikörper besitzen eine Vielzahl von Wirkmechanismen, die zu unterschiedlichsten therapeutischen Zwecken eingesetzt werden. Generell kann man dabei direkte und indirekte Wirkmechanismen unterscheiden (siehe Figur 2). Die direkte Wirkung eines Antikörpers beruht ausschließlich auf seiner spezifischen Bindefähigkeit. Der jeweilige Isotyp spielt hierbei keine Rolle, da es sich um Fc-Rezeptor unabhängige Mechanismen handelt. Auf diese Weise können beispielweise Interaktionen zwischen Wachstumsfaktor-Rezeptoren und deren Liganden blockiert werden, indem der Antikörper an den Rezeptor oder den Ligand bindet. Ein vergleichbarer Effekt wird erzielt, indem der Kontakt zweier Zellen durch die Bindung interagierender Oberflächenstrukturen inhibiert wird. Der blockierende Effekt monoklonaler Antikörper wird bei der Behandlung von Autoimmunerkrankungen klinisch angewandt. Adelimumab (*Humira*®) und Infliximab (*Remicade*®) sind gegen das Zytokin Tumor-Nekrose-Faktor $\alpha$(TNF$\alpha$) gerichtet und werden bei der Behandlung von Morbus Crohn und rheumatoider Arthritis eingesetzt. Durch die Unterdrückung der Interaktion zwischen TNF$\alpha$ und dem Rezeptor kommt es zu einer Verringerung von pro-inflammatorischen Signalen (Markham, A. & Lamb, HM., Drugs 59:1341-1359 (2000); Navarro-Sarabia, F. et al., J. Rheumatol. 33:1075-1081 (2006)). Auch in der Onkologie findet die blockierende Wirkung monoklonaler Antikörper Anwendung. Der Antikörper Bevecizumab (*Avastin*®) wirkt, indem er den, von Tumorzellen ausgeschütteten vaskulären endothelialen Wachstumsfaktor (*vascular endothelial growth factor,* VEGF) bindet. Dadurch kommt es zu einer verringerten Neubildung von Blutgefäßen im Tumorgewebe und der Tumor wird schlechter mit Nährstoffen versorgt (Ferrara, N., Oncology 69 Suppl. 3:11-16 (2005)). Die Wirkung des beim Mamma-Karzinom eingesetzten Antikörpers Trastuzumab (*Herceptin*®) beruht unter anderem auf einer Blockade zwischen einem Wachstumsfaktor und seinem Rezeptor. Anders als bei Bevecizumab wird hier nicht der Ligand, sondern der Rezeptor HER2/neu blockiert, wodurch der epitheliale Wachstumsfaktor (*epithelian growth factor,* EGF) nicht mehr binden kann. Das Blockieren dieses Proliferations-fördernden Zytokins führt zu verlangsamter Zellteilung und zu besserer Tumorkontrolle (Harries, M. & Smith, I., Endocr. Relat. Cancer, 9:75-85 (2002)). Ein weiteres direktes Wirkprinzip von Antikörpern ist die Induktion anti-proliferativer oder pro-apoptotischer Zellsignale durch Bindung und ggf. Kreuzvernetzung von Oberflächenantigenen. Die Eigenschaft durch Bindung apoptotische Signale auszulösen die die Tumorzelle eliminieren, wurde erstmals anhand eines Antikörpers gegen den Todesrezeptor CD95 gezeigt (Trauth, BC. et al., Science 245:301-305 (1989)). Bei der Therapie von *Non-Hodgkin* Lymphomen (NHL) stellt die Behandlung mit anti-Idiotyp Antikörpern, welche gegen die variablen Regionen des B-Zell Rezeptorkomplexes gerichtet sind, eine der effektivsten Therapieoptionen dar. Bei über 70 % der Patienten führt diese Behandlung zu einer kompletten Remission. Dieser Effekt wird durch die alleinige Bindung an den B-Zellrezeptor ausgelöst und ist unabhängig von den konstanten Regionen des verwendeten Antikörpers (Vuist, WM. et al., Blood 83:899-906 (1994); Davis, TA. et al., Blood 92:1184-1190 (1998)). Der gegen das B-lymphoide Oberflächenantigen CD20 gerichtete chimäre Antikörper Rituximab (*MabThera*®) zeigt großen Erfolg bei der Behandlung von NHL (Smith, MR., Oncogene 22:7359-7368 (2003)). Obwohl die Rekrutierung von Effektoren für de Wirksamkeit von Rituximab relevant ist (Voso, MT. et al., Haematologica 87: 918-925 (2002)), spielen die direkt durch diesen Antikörper ausgelösten Signale ebenfalls eine wichtige Rolle. Die Kreuzvernetzung von CD20 induziert Apoptose und der Grad der Kreuzvernetzung steht im direkten Zusammenhang mit der Stärke des apoptotischen Effekts (Shan, D. et al., Blood 91:1644-1652 (1998)). Neben CD20 sind noch weitere Oberflächenantigene bekannt, die nach Kreuzvernetzung antiproliferativ oder pro-apoptotisch wirken, wie z. B. CD19, CD22, CD33 oder HLAII (Benoit, NE. & Wade, WF., Immunopharmacology 35:129-139 (1996); Meng, R. et al., Clin. Cancer Res. 10:1274-1281 (2004); Vitale, C. et al., Proc. Natl. Acad. Sci. U S A 96:15091-15096 (1999); Dechant, M. et al., Semin. Oncol. 30:465-475 (2003)).

Die indirekten Wirkmechanismen therapeutischer Antikörper beruhen auf der Wechselwirkung ihrer konstanten Regionen mit Effektoren des Immunsystems. Neben der Antigenspezifität ist hier der Isotyp des Antikörpers in Form seines Fc-Anteils ausschlaggebend. Über ihn werden sowohl Zell-unabhängige Effektoren als auch Effektorzellen an die opsonisierte Zelle rekrutiert. Der Fc-Teil eines Antikörpers kann beispielsweise die Komplementkaskade aktivieren. Hierbei lagert sich der Komplement-Faktor C1q an die Fc-Region der Antikörper an. Als Folge der proteolytischen Komplementkaskade wird der sogenannte Membran-Angriffs Komplex (*membrane-attack complex,* MAC) gebildet, was zur Lyse der Zielzelle führt (*complement-dependent cytotoxicity,* CDC). Der therapeutische Erfolg von Rituximab wird neben den beschriebenen signalisierenden Eigenschaften auch der CDC zugeschrieben (Di Gaetano, N. et al., J. Immunol. 171:1581-1587 (2003)). Durch die Rekrutierung von Effektorzellen an die mit therapeutischen Antikörpern opsonisierte Tumorzelle kommt es zur Antikörper-vermittelten zellulären Zytotoxizität (*antibody-dependent cellular cytotoxicity,* ADCC). Rekrutierte Effektoren, wie beispielsweise Natürliche Killerzellen (NK-Zellen), schütten nach Bindung an die Fc-Rezeptoren Substanzen wie Perforine oder Granzyme aus, die die Tumorzelle lysieren. Nach der Aktivierung

von Makrophagen kommt es zur Phagozytose der Zielzelle. Diese Mechanismen sind stark vom Isotyp des Antikörpers abhängig. Der häufigste Isotyp therapeutischer Antikörper ist IgG1 mit drei unterschiedlichen Fc-Rezeptoren (FcRs), dem hoch affinen FcγRI (CD64) und die beiden niedrig affinen FcγRII (CD32) und FcγRIII (CD16). Die FcRs dienen hierbei nicht nur der Rekrutierung, sondern führen zur Aktivierung des zytotoxischen Potentials der Effektorzelle durch ihre Vernetzung auf der Oberfläche der Zielzelle. Wie die Signalisierung und die CDC spielt auch die ADCC bei Rituximab eine wichtige Rolle für dessen Behandlungserfolge in der Klinik (Clynes, RA. et al., Nat. Med. 6:443-446 (2000)).

Weitere therapeutische Anwendbarkeit finden Antikörper als Vehikel für toxische Substanzen. Das Prinzip besteht darin die Spezifität eines Antikörpers mit der unspezifischen Toxizität eines Effektors zu kombinieren, um ein spezifisches zytotoxisches Agens zu erhalten. Durch Kopplung zytotoxischer Agentien an Antikörper oder Antikörper-Fragmente ist es möglich diese Substanzen an den Tumor zu dirigieren und unspezifische Nebenwirkungen herabzusetzen. Auf dieser Grundlage basieren unterschiedliche Ansätze, die in Figur 3 zusammengestellt sind. Zur Veranschaulichung sind in dieser Figur ausschließlich ganze Antikörper verwendet. Jedoch erweisen sich für diese Anwendung Antikörperfragmente häufig vorteilhafter und finden daher auf diesem Gebiet verstärkten Einsatz.

In den 70er Jahren wurden erstmals Radionuklide an einen monoklonalen Antikörper gekoppelt und als Diagnosewerkzeug für das Carcinoembryonale Antigen (CEA) verwendet (Goldenberg, DM. et al., N. Engl. J. Med. 298: 1384-1386 (1978)). Heute stellen die Radioimmunkonjugate die aus klinischer Sicht am weitesten entwickelten Immunkonjugate dar. Sie erzielen ihre zytotoxische Wirkung durch ionisierende Strahlung unabhängig von der Internalisierung des gebundenen Antigens. Konjugate aus einem gegen CD20 gerichteten Antikörper und den radioaktiven Isotopen $^{90}$Yttrium (Ibritumomab tiuxetan; *Zevalin*®) und $^{131}$Iod (Tositumomab; *Bexxar*®) wurden 2002 bzw. 2003 von der FDA zugelassen und finden Einsatz bei der Behandlung rezidivierter NHL (Witzig, TE. et al., J. Clin. Oncol. 20: 2453-2463 (2002); Davis, TA. et al., Clin. Cancer Res. 10:7792-7798 (2004)). Gegen einige Oberflächenantigene, darunter CEA, HLA-DR10, CD22, MUC-1 und TAG-72 befinden sich Radioimmunkonjugate in fortgeschrittenen klinischen Testphasen (Milenic, DE. et al., Nat. Rev. Drug Discov. 3:488-499 (2004); Aarts, F. et al., Cancer Biother. Radiopharm. 23:92-107 (2008)). Eine Einschränkung der Radioimmunkonjugate ist die hohe Belastung des Knochenmarks durch die im Kreislauf zirkulierenden Moleküle, was oft zu schweren Knochenmarksdepressionen führt (Sharkey, RM. & Goldenberg, DM., CA. Cancer J. Clin. 56:226-243 (2006)). Durch die Strahlenbelastung wird auch ein vermehrtes Auftreten sekundärer akuter myeloischer Leukämien (AML) beschrieben (Bennett, JM. et al., Blood 105:4576-4582 (2005)).

Immunzytokine sind Konjugate aus einem Antikörper und einem oder mehreren Zytokinen. Dieser Ansatz beruht auf dem Konzept, dass tumorspezifische Antikörper die gekoppelten Zytokine zum Tumor leiten und dass die Zytokine dann gezielt im Tumorgewebe eine Anti-Tumorantwort stimulieren, die sich anschließend systemisch ausbreitet. Das Konzept der Immunzytokine ist vergleichsweise neu. Ein Konjugat eines CD20-gerichteten Antikörpers und IL2 zeigte erste Erfolge im Mausmodel gegen humane Lymphomzellen (Gillies, SD. et al., Blood 105:3972-3978 (2005)). Aktuell befinden sich zahlreiche Antikörper-IL2 Fusionen in klinischer Erprobung. Ein weiterer Ansatz ist die Fusion von Liganden der TNF Superfamilie an Antikörperfragmente. So zeigten Fusionen aus scFv-Fragmenten und dem TNF*related apoptosis-inducing ligand* (TRAIL) in vorklinischen Studien potente tumorzellspezifische Apoptose in soliden und hämatopoietischen Neoplasien (Bremer, E. et al., Int. J. Cancer 109:281-290 (2004); Bremer, E. et al., Cancer Res., 65:3380-3388 (2005)).

Auch Chemotherapeutika werden mit Hilfe von Antikörpern spezifisch an den Tumor dirigiert. Hierfür werden die Zytostatika chemisch an den Antikörper gekoppelt (Dubowchik, G. M. et al., Nat. Biotechnol. 23:1137-1146 (2005)). Diese Therapieform wird begünstigt, wenn das Zielantigen nach Bindung internalisiert, um das Zytostatikum verstärkt in die Zelle zu transportieren. In ersten Ansätzen wurden klassische Chemotherapeutika wie Doxorubicin oder Methotrexat gekoppelt. Ein Antikörper gegen das Kohlenhydrat-Antigen Lewis Y, gekoppelt an Doxorubicin zeigte Erfolge bei der Behandlung von Darm-, Lungen- und Brustkrebs im Tiermodell (Trail, PA. et al., Science 261:212-215 (1993)). Bei Tieren die das Immunkonjugat erhielten wurde in unmittelbarer Umgebung des Tumors eine wesentlich höhere Konzentration an Doxorubicin festgestellt als bei Tieren die Doxorubicin alleine verabreicht bekamen (Mosure, K. W. et al., Cancer Chemother. Pharmacol. 40:251-258 (1997)). Es waren vor allem dosislimitierende Begleittoxizitäten, die die Applikation dieses Moleküls im Menschen erschwerten und die Gabe effektiver Konzentrationen zur Tumorbehandlung verhinderten (Ajani, JA. et al., Cancer J. 6:78-81 (2000)).

Erfolgreicher sind Konjugate mit toxischeren Substanzen, wie dem aus Pilzen isolierten Zytostatikum Calicheamycin. Ein an diese Substanz gekoppelter, gegen CD33 gerichteter humanisierter Antikörper, Gemtuzumab ozogamicin (GO, *Mylotarg*®), ist in Amerika für die Therapie rezidivierter AML-Patienten über 65 Jahre zugelassen Bernstein, ID., Leukemia 14:474-475 (2000); Hamann, PR. et al., Bioconjug. Chem. 13:47-58 (2002)). Etwa 30% der Patienten sprechen auf GO an und die mittlere rückfallfreie Überlebenszeit beträgt 6,8 Monate (Bross, PF. et al., Clin. Cancer Res. 7:1490-1496 (2001)). Durch die klinische Anwendung von GO werden aber auch die Nachteile derartiger Immunkonjugate verdeutlicht. Die pHlabile Verknüpfung zwischen Antikörper und Toxin ließ schon vor dem Einsatz von GO vermuten, dass sich ein Teil des Calicheamycins im Organismus fernab des Tumors abspalten könnte. Diese Vermutungen haben sich zum Teil bestätigt. Im Serum von behandelten Patienten finden sich freie Metabolite von

Calicheamycin die für die Nebenwirkungen, wie der Lebervenen-Verschluss Erkrankung (*venoocclusive disease,* VOD) verantwortlich sein könnten. Da in der Leber keine CD33 Expression nachgewiesen ist (Favaloro, EJ., Immunol. Cell. Biol. 71 ( Pt 6):571-581 (1993)), wird von einer unspezifischen Wirkung der Toxinkomponente ausgegangen (Wadleigh, M. et al., Blood 102:1578-1582 (2003)). Man nimmt daher an, dass die instabile Verknüpfung und die resultierende vorzeitige Freisetzung von Calicheamycin die Hauptursache der Nebenwirkungen von GO sind (Jedema, I. et al., Leukemia 18:316-325 (2004); Schwemmlein, M. et al., Br. J. Haematol. 133:141-151 (2006)). Diese Erkenntnis verlangt die Konstruktion verbesserter therapeutisch wirksamer Immunkonjugate.

Obwohl Immuntoxine oft als Immunkonjugate angesehen werden, grenzen sie sich gegenüber den beschriebenen Konjugaten aus Antikörpern und zytotoxischen Effektoren dadurch ab, dass sie natürlich vorkommende Proteintoxine oder davon abgeleiteten Moleküle nutzen. Die Wirkung von Chemotherapeutika, meist kleinen niedermolekularen Substanzen, beruht häufig auf einer DNA-Schädigung, wobei relativ viele Moleküle erforderlich sind um eine Zelle zu eliminieren. Im Gegensatz zu dieser stöchiometrischen Wirkung basiert die Wirkungsweise der verwendeten Proteintoxine auf katalytischer Enzymaktivität, wodurch im Fall von Diphterietoxin nur ein einziges Molekül genügt um eine Zelle abzutöten (Yamaizumi, M. et al., Cell. 15:245-250 (1978)). Ein weiterer Vorteil dieser Moleküle ist, dass sie von Natur aus ein Freisetzungs- und Aktivierungsmechanismus innerhalb der Zelle besitzen, was die Verwendung säurelabiler chemischer *linker,* wie bei Calicheamycin überflüssig macht. Die Vorteile derartiger, in der Zelle spaltbarer Peptidbindungen im Vergleich zu der in GO verwendeten chemischen Hydrazon-Verknüpfung wurde für Immunkonjugate vergleichend gezeigt (Doronina, S. O. et al., Nat. Biotechnol. 21:778-784 (2003)).

Bei den Effektorkomponenten von Immuntoxinen handelt es sich meist um die Gruppe der sogenannten Ribosomen-inaktivierenden Proteine (*ribosome-inactivating proteins*; RIPs). Diese inhibieren enzymatische die Proteinbiosynthese was zum Tod der betroffenen Zelle führt. Verwendet werden die pflanzlichen Toxine Saporin aus dem gewöhnlichen Seifenkraut (*Saponaria officinalis*) und Rizin aus dem Wunderbaum (*Ricinus communis*), Gelonin aus der Himalaya Euphorbiacee (*Gelonium multiflorum*), das anti-virale Protein (*pokeweed antiviral protein,* PAP) aus der Amerikanischen Kermesbeere (*Phytolacca americana*) sowie die bakteriellen Toxine Diphterietoxin (DT) aus *Corynebacterium diphtheriae* und Exotoxin A (ETA) aus *Pseudomonas aeruginosa.* Obwohl diese Toxine die Proteinsynthese inhibieren, wirken sie z.T. unterschiedlich.

Um die Proteinsynthese zu inhibieren, müssen die Toxine ins Zytoplasma der Zelle gelangen. Alle Toxine besitzen daher eine Domäne, mit der sie an einen häufig vorkommenden eukaryotischen Oberflächenmarker binden. Das Lektin Rizin besteht aus 2 Polypeptiden (A und B), wobei eine Kette die Bindefunktion und die andere Kette die Effektorfunktion vermittelt. Mit der B-Kette erfolgt die Bindung an häufig auf eukaryotischen Zellen vorkommende Galaktosereste (Hartley, MR. & Lord, JM., Biochim. Biophys. Acta. 1701:1-14 (2004)). Für Saporin wurde bislang keine rezeptorbindende Domäne beschrieben. DT bindet an den *heparin-binding epidermal growth factor like* Vorläufer und ETA an den alpha2-Makroglobulin-Rezeptor (CD91; Naglich, JG. et al., Cell. 69:1051-1061 (1992); Kounnas, M. Z. et al., J. Biol. Chem. 267:12420-12423 (1992)). Nach der Bindung erfolgt die rezeptorvermittelte Endozytose des Toxin-Rezeptorkomplexes in frühe Endosomen. Bei DT genügt ein pH-Abfall im Vesikel um mit Hilfe einer Konformationsänderung die Endosomenmembran zu passieren und ins Cytosol zu gelangen (Lord, J. M. et al., Cell Microbiol. 1: 85-91 (1999)). ETA kann nicht in dieser Weise auf den pH-Abfall reagieren. Wie Rizin A gelangt ETA zunächst über den umgekehrten Weg sekretorischer Proteine ins endoplasmatische Retikulum, wo anschließend die Translokation ins Zytoplasma stattfindet. Auch im Zytoplasma unterscheiden sich die Wirkmechanismen. Die pflanzlichen Toxine Rizin, Saporin und PAP besitzen N-Glycosidase Aktivität, wodurch sie ein Adenin aus der 28S rRNA entfernen und somit die Proteinbiosythese am Ribosom inhibieren (Endo, Y. & Tsurugi, K., J. Biol. Chem. 263: 8735-8739 (1988); Ippoliti, R. et al., FEBS Lett. 298:145-148 (1992); Hudak, KA. et al., J. Biol. Chem. 274:3859-3864 (1999)). Die bakteriellen Toxine DT und ETA inhibieren den eukaryotischen Elongationsfaktor 2 (EF2). Dieses für die Translation wichtige Protein besitzt eine einzigartige, von Histidin abgeleitete Aminosäure namens Diphtamid. Die katalytischen Domänen von DT und ETA bewirken, dass ein ADP-Ribosyl Rest von Nicotinamidadenindinukleotid ($NAD^+$) an den Diphtamid-Rest des EF2 übertragen wird, wodurch der EF2 seine Funktionalität verliert und die Proteinsythese zum erliegen kommt (Van Ness, BG. et al., J. Biol. Chem. 255:10710-10716 (1980); Pastan, I. & FitzGerald, D., J. Biol. Chem. 264:15157-15160 (1989)).

*Pseudomonas aeruginosa* Exotoxin A (ETA) besteht aus einer einzelnen Polypeptidkette mit einer Länge von 613 Aminosäuren und einem Molekulargewicht von 66 kDa (Allured, VS. et al., Proc. Natl. Acad. Sci. U S A 83:1320-1324 (1986)). Strukturell lassen sich drei Domänen unterscheiden, die jeweils unterschiedliche Funktionen für die Wirksamkeit des Toxins übernehmen (Hwang, J. et al., Cell 48:129-136 (1987)).

Mit Domäne I erfolgt die Bindung an den alpha2-MakroglobulinRezeptor, auch bekannt als *low density lipoprotein-related protein 1* (LRP1) oder CD91, was zu einer rezeptorvermittelten Internalisierung des Toxin-Rezeptor Komplexes führt. Von den *Clathrin* Vesikeln (*clathrin coated pits*) gelangt das Protein in das Trans-Golgi Netzwerk (*trans-golgi network*; TGN). Dort wird es von der zelleigenen Protease Furin zwischen den Aminosäureresten 279 und 280 gespalten, was grob dem Übergang von Domäne I zu Domäne II entspricht (Gordon, VM. et al., Infect. Immun. 63: 82-87 (1995)). Allerdings bleiben die beiden Fragmente über eine Disulfidbrücke miteinander verbunden. Im TGN bindet das, der eukaryortischen KDEL-Sequenz ähnliche, C-terminale REDL-Motiv an den KDEL-Rezeptor. Dieses REDL-Motiv entsteht nach Abspaltung des Lysins aus REDLK durch eine Carboxypeptidase vor dem Eintritt in die Zelle (Hessler, JL. & Kreitman, RJ., Biochemistry 36:14577-14582 (1997)). Die natürliche Funktion des KDEL-Rezeptors besteht darin, versehentlich in den sekretorischen Weg gelangte Proteine des Endolasmatischen Retikulums (ER), wie z. B. Chaperone, zu binden und ins ER zurückzuführen (Munro, S. & Pelham, HR., Cell 48:899-907 (1987)). ETA nutzt diesen retrograden Transportmechanismus durch Nachahmen des natürlichen Signalpeptids um ins ER zu gelangen (Lord, JM. et al., Cell. Microbiol. 1:85-91 (1999)). Dort erfolgt die Reduktion der Disulfidbrücke, und die Domäne II bewirkt über ihre Translokationsfunktion einen Transport der Domänen II und III ins Zytoplasma (Hwang, J. et al., Cell 48: 129-136 (1987)). Anschließend katalysiert Domäne III unter Spaltung von $NAD^+$ eine Anheftung eines ADP-Riboserests an den Diphtamidrest des Elongationsfaktors 2, wodurch die Proteinsynthese der Zelle zum erliegen kommt. (Lord, JM. et al., Cell. Microbiol. 1:85-91 (1999)). Diese Inhibition führt zur Induktion der Apoptose, wobei mittlerweile sichergestellt ist, dass ETA zusätzlich durch weitere, bislang ungeklärte Mechanismen Apoptose auslöst (Andersson, Y. et al., Int. J. Cancer 112:475-483 (2004)).

Ähnlich den Zytostatika-Immunkonjugaten bestanden die ersten Immuntoxine aus murinen monoklonalen Antikörpern, an die die Proteintoxine chemisch fusioniert waren. Die Effektorkomponenten waren dabei Saporin, PAP, Gelonin, Rizin und ETA (Morland, BJ. et al., Br. J. Cancer 69:279-285 (1994); Myers, DE. et al., J. Immunol. Methods 136:221-237 (1991); Scott, CF. Jr., et al., J. Natl. Cancer Inst. 79:1163-1172 (1987); Krolick, KA. et al., J. Exp. Med. 155:1797-1809 (1982); FitzGerald, DJ. et al., J. Clin. Invest. 74:966-971 (1984)). Um die unspezifische Bindung des damals häufig verwendeten Rizins zu reduzieren, wurde nur die enzymatisch aktive A-Kette (Rizin A) verwendet. Doch auch Rizin A wurde wegen Glykosylierungsresten unspezifisch von Leberzellen aufgenommen (Thorpe, PE. et al., Eur. J. Biochem. 147:197-206 (1985)). Die Deglykosylierung von Rizin A brachte erste Erfolge bei der Reduktion der unspezifischen Zytotoxizität und Rizinbasierte Immuntoxine gegen CD22 wurden in klinische Studien untersucht (Amlot, PL. et al., Blood 82:2624-2633 (1993)). Allerdings blieben die Erfolge wegen dosislimitierender Begleittoxizitäten wie dem *vascular leak syndrom* (VLS) aus. Beim VLS werden die Blutgefäße durch Schädigung des Endothels durchlässig und es kommt zu einer Einblutung in umliegende Gewebe (Stone, MJ. et al., Blood 88:1188-1197 (1996); Messmann, RA. et al., Clin. Cancer Res. 6:1302-1313 (2000)). Als Ursache dieser Nebenwirkung wurden Aminosäuremotive gefunden, die die Bindung von Rizin an Endothelzellen bewirken. Ähnliche Motive finden sich auch bei ETA, wobei hier die Affinität zu Endothelzellen im Vergleich zu Rizin etwa um den Faktor $10^3$ reduziert ist (Baluna, R. & Vitetta, ES., J. Immunother. 22:41-47 (1999)). Das zweite große Problem bei der Behandlung mit klassischen Immuntoxinen der ersten Generation war die Induktion von HAMA-Antworten gegen die murinen Antikörper, wodurch ein Großteil der Immuntoxine im Körper neutralisiert wurde, noch bevor die Wirkung entfaltet werden konnte (Amlot, PL. et al., Blood 82:2624-2633 (1993)).

Die Einschränkungen klassischer Immuntoxine wurden mit Hilfe moderner molekularbiologischer Techniken verbessert. Die genetische Verknüpfung von Binde- und Effektordomäne ermöglichte die Verwendung von Antikörperfragmenten anstelle ganzer Antikörper. Mit scFv-Fragmenten oder über Disulfidbrücken zusammengehaltenen variablen Ig-Domänen (*disulfide-stabilized Fv,* dsFv) wurden Immuntoxine ohne den für die HAMA-Antworten maßgeblich verantwortlichen konstanten Antikörperteil entwickelt (Thorpe, SJ. et al., Scand. J. Immunol. 57:85-92 (2003)). Außerdem wurde so die Bindung und Aufnahme des Immuntoxins durch Fc-Rezeptor positive Zellen verhindert.

Immunkonjugate zeichenen sich zusätzlich durch eine aufwendige Produktion mit der chemischen Kopplung der Toxinkomponente und einer daraus resultierenden großen Heterogenität der therapeutisch eingesetzten Moleküle aus. Untersuchungen zeigten, dass die verabreichte Form von GO nur 50% Calicheamycin-gekoppelte Antikörper aufweist (Bross, PF. et al., Clin. Cancer Res. 7:1490-1496 (2001)). Dagegen ermöglicht die Anordnung von Binde- und Effektordomäne auf einer Polypeptidkette die Herstellung homogener Proteinfraktionen. Aufgrund ihrer reduzierten Größe sollten die fortgeschrittenen Immuntoxine der zweiten Generation ausserdem besser gewebegängig sein, was vor allem im Hinblick auf solide Tumore ein wichtiges Kriterium darstellt.

Während bei klassischen Immuntoxinen häufig Wildtyp Toxine zum Einsatz kommen, die mit ihrer natürlichen Bindedomäne in gesunde Zellen eindringen und diese schädigen, werden bei rekombinanten Toxinen mit ETA und DT verkürzte Varianten verwendet. Die natürliche Bindedomäne wird deletiert und alternative bindende Proteine kommen zum Einsatz. Neben Antikörperfragmenten werden natürliche Liganden von Rezeptoren, wie die Wachstumsfaktoren *granulocyte-macrophage colony stimulating factor* (GMCSF) und *transforming growth factor alpha* (TGF$\alpha$; Hall, PD. et al., Leukemia 13:629-633 (1999); Sampson, JH. et al., J. Neurooncol. 65:27-35 (2003)) oder Zytokine wie Interleukin 13 (IL13; Parney, IF. et al., J. Neurosurg. 102:267-275 (2005)) verwendet. Ein chimäres Fusionsprotein aus IL2 und DT findet sich in dem 1999 zugelassenen Zytotoxin Denileukin diftitox (*Ontak*®), das bei kutanen T-Zell-Lymphomen eingesetzt wird (Foss, F. et al., Blood 106:454-457 (2005)).

In vorklinischen Studien wurden bereits zahlreiche ETA'-abgeleitete Immuntoxine getestet. Im Fall von hämatopoietischen Neoplasien wurden u.a. die Oberflächenmarker CD7, CD19, CD25, CD30, CD33 und CD64 anvisiert (Peipp, M. et al., Cancer Res. 62:2848-2855 (2002); Schwemmlein, M. et al., Leukemia 21:1405-1412 (2007); Barth, S. et al., Int. J. Cancer 86:718-724 (2000b); Barth, S. et al., Blood 95:3909-3914 (2000c); Schwemmlein, M. et al., Br. J. Haematol. 133:141-151 (2006); Tur, MK. et al., Cancer Res. 63:8414-8419 (2003)). Bei soliden Tumoren wurden u.a. IL13, der EGF-Rezeptor, Ep-CAM und MUC1 zur zielgerichteten Therapie untersucht (Parney, IF. et al., J. Neurosurg.

102:267-275 (2005); Bruell, D. et al., Int. J. Mol. Med., 15:305-313 (2005); Di Paolo, C. et al., Clin. Cancer Res. 9: 2837-2848 (2003); Singh, R. et al., Mol. Cancer Ther. 6: 562-569 (2007)). In früher klinischer Erprobung befinden sich ein Lewis Y-scFv-ETA' Toxin gegen Karzinome (Posey, JA. et al., Clin. Cancer Res. 8:3092-3099 (2002)) und ein HER2/neu-scFv-ETA' Toxin gegen metastasierten Brust- und Prostatakrebs (Azemar, M. et al., Breast Cancer Res. Treat. 82:155-164 (2003); von Minckwitz, G. et al., Breast Cancer Res. 7:R617-626 (2005)). Die in klinischer Erprobung am weitesten fortgeschrittenen rekombinanten Moleküle dieses Formats sind ein CD25-scFv-ETA'- (LMB-2) und ein CD22-dsFv-ETA'- (BL22) Immuntoxin. In klinischer Phase I mit Chemotherapie-resistenten hämatologischen Neoplasien zeigte LMB2 bei Patienten mit refraktärer Haarzell-Leukämie (*hairy cell leukemia;* HCL) gute Ansprechraten (Kreitman, RJ. et al., Blood 94:3340-3348 (1999); Kreitman, RJ. et al., J. Clin. Oncol. 18:1622-1636 (2000)). BL22 war ebenfalls bei HCL-Patienten in einer klinischen Phase I sehr wirksam mit Ansprechraten von über 80%. Vereinzelte Nebenwirkungen waren erhöhte Leberwerte sowie VLS und hämolytisches uremisches Syndrom, ohne dabei dosislimitiernd zu sein (Kreitman, RJ. et al., J. Clin. Oncol. 23:6719-6729 (2005)).

Offensichtlich ist das Problem zu hoher Begleittoxizitäten der Immuntoxine der ersten Generation durch die Einführung rekombinanter Immuntoxine, wenn auch nicht behoben, so zumindest stark verbessert. Allerdings gibt die hohe Immunogenität bakterieller und pflanzlicher Toxine nach wie vor Anlass zu Diskussion. Bei der LMB-2 Studie wiesen nur 6 der 35 Patienten neutralisierende Antikörper im Blut auf, und bei der BL22-Studie waren es 11 von 46 Patienten und das vorwiegend erst nach wiederholter Gabe des Medikaments (Kreitman, RJ. et al., N. Engl. J. Med. 345:241-247 (2001); Kreitman, RJ. et al., J. Clin. Oncol. 23:6719-6729 (2005)). Diese relativ geringe Immunogenität lässt sich jedoch durch das geschwächte Immunsystem von Leukämiepatienten erklären, was sowohl durch die Leukämie selbst als auch durch die vorangegangene Chemotherapie-Behandlung erklärbar ist (Pastan, I. et al., Nat. Rev. Cancer 6:559-565 (2006)). Bei der Behandlung solider Tumoren ist trotzdem mit einer verstärkten Immunreaktion gegen die bakteriellen Epitope zu rechnen. Auf diesem Gebiet werden aktuell verschiedene Lösungsansätze untersucht. So zeigt die Punktmutation der B-Zell Epitope im ETA' eine deutlich reduzierte Produktion neutralisierender Antikörper im Mausmodell, ohne dabei die Funktionalität des Toxins zu beeinflussen (Onda, M. et al., J. Immunol. 177:8822-8834 (2006)). Alternativ denkbar wäre auch eine Kombinationstherapie mit immunsupprimierenden Agenzien mit denen man möglicherweise auch eine Steigerung des spezifischen zytotoxischen Potentials erreichen könnte (Ohno, N. et al., Leuk. Lymphoma. 43:885-888 (2002); Schwenkert, M. et al., Melanoma Res. 18:73-84 (2008)).

Ein vielversprechendes Oberflächenantigen zur Antikörper-basierten Therapie des MM ist das Melanom-assoziierte Chondroitinsulfat Proteoglykan (*melanoma-associated chondroitin sulfate proteoglycan,* MCSP) auch bekannt unter dem Namen *high molecular weight melanoma-associated antigen* (HMW-MAA). Es wurde durch die Immunisierung von Mäusen mit humanen Melanomzellen entdeckt. Bei der Analyse der erhaltenen Antikörper war MCSP eines der immundominanten Melanomassoziierten Antigene (*melanoma-asocciated antigens,* MAA; Wilson, BS. et al., Int. J. Cancer 28:293-300 (1981); Bumol, TF. & Reisfeld, RA., Proc. Natl. Acad. Sci. U S A 79:1245-1249 (1982); Reisfeld, RA. & Cheresh, DA., Adv. Immunol. 40:323-377 (1987)).

In der Neurologie wird häufig der Name *nerve/glial cell antigen 2* (NG2) anstelle von MCSP verwendet, was in der Literatur oftmals zu Verwirrungen führt, da NG2 als das Rattenhomolog des humanen MCSP angesehen wird (Campoli, MR. et al., Crit. Rev. Immunol. 24:267-296 (2004)). Das Homolog auf murinen Zellen bezeichnet man als AN2 (Schneider, S. et al., J. Neurosci. 21:920-933 (2001), Stegmuller, J. et al., J. Neurocytol. 31:497-505 (2002)). NG2 und AN2 teilen eine Aminosäure Sequenzhomologie von über 90% und zeigen gegenüber MCSP mit 80% Sequenzhomologie ebenfalls hohe Identität. MCSP, NG2 und AN2 bilden eine eigene Proteinfamilie, mit charakteristischen Strukturmerkmalen und Eigenschaften und weisen geringe Homologie zu anderen Proteinen auf.

Das Oberflächenantigen MCSP ist ein stark glykosyliertes, Membrandurchspannendes Chondroitinsulfat Proteoglykan mit einer Gesamtgröße von ca. 450 kDa (Wilson, BS. et al., Cancer Immunol. Immunother. 14:196-201 (1983)). Dabei besteht es aus einer etwa 230 kDa Polypeptidkette, die zunächst posttranslational zu einem 280 kDa Proteoglykan modifiziert wird. Auf der Zelloberfläche werden die restlichen Kohlenhydratreste in Form von Glykosaminoglykanen angefügt. Bei diesen N-glykosidisch angefügten Disaccharid Wiederholungen handelt es sich, neben einer einzelnen, für den Namen verantwortlichen Chondroitinsulfat-Kette aus N-Acetyl-Galaktosamin und Glucuronsäure, hauptsächlich um Hyaluronsäure, die aus N-Acetyl-Glucosamin und Glucuronsäure besteht. Hinweise deuten darauf hin, dass auf der Zelloberfläche nicht alle MCSP-Moleküle zum 450 kDa-Produkt modifiziert werden, sondern dass sowohl das 280 kDa als auch das 450 kDa Proteoglykan getrennt nebeneinander vorliegen (Campoli, MR. et al., Crit. Rev. Immunol. 24: 267-296 (2004)).

Das Gen *cspg4* mit einer Größe von 58,5 kbp liegt auf Chromosom 15 und codiert für eine 2322 Aminosäuren lange Polypeptidkette (Pluschke, G. et al., Proc. Natl. Acad. Sci. U S A 93:9710-9715 (1996)). Die große extrazelluläre Domäne enthält 15 potentielle N-Glykosylierungsstellen und lässt sich in drei Subdomänen unterteilen. Die globuläre N-terminale Domäne 1 wird über zwei bis drei Disulfidbrücken stabilisiert und besitzt zwei potentielle Laminin-G Bindestellen zur Interaktion mit zellulären Rezeptoren, Kohlenhydraten oder weiteren extrazellulären Liganden (Timpl, R. et al., Matrix Biol. 19:309-317 (2000)). Die flexible und stabähnliche Domäne 2 trägt zahlreiche sogenannte Chondroitinsulfat Proteoglykan (*chondroitin sulfate proteoglycan,* CSPG) Wiederholungen aus gehäuften Serinen und Glycinen.

Einer dieser Serinreste ist die Glykosylierungsstelle für die einzige Chondroitinsulfat-Kette (Stallcup, WB. & Dahlin-Huppe, K., J. Cell. Sci. 114:2315-2325 (2001)). Ausserdem befindet sich in der CSPG-Domäne eine Bindestelle für Kollagen V und Kollagen VI zur Wechselwirkung mit der extrazellulären Matrix. Die globuläre Domäne 3 schließt den extrazellulären Teil des Proteoglykans ab. Die Transmembrandomäne fällt durch die Existenz eines Cysteinrests auf, dessen Funktion unbekannt ist, und der auch bei anderen Oberflächenantigenen ähnlicher Funktion, wie CD44 oder Syndecan (CD138) gefunden wurde (Liu, D. & Sy, MS., J. Exp. Med. 183:1987-1994 (1996); Bernfield, M. et al., Annu. Rev. Biochem. 68:729-777 (1999)). Der kurze intrazelluläre Teil trägt drei Threoninreste, die als potentielle Phosphorylierungsstellen für die Proteinkinase C angesehen werden, ohne dass die Phosphorylierung bislang gezeigt wurde (Pluschke, G. et al., Proc. Natl. Acad. Sci. U S A 93:9710-9715 (1996)). Zusammen mit dem PDZ-Motiv zur Proteininteraktion (Barritt, DS. et al., J. Cell Biochem. 79:213-224 (2000)) werden so wahrscheinlich Signale ins Zellinnere weitergeleitet. Figur 4 zeigt schematisch die Struktur von MCSP.

Im Vergleich mit den Strukturmerkmalen der homologen Proteine AN2 und NG2 fehlt bei der humanen Form ein Cluster aus 6 Cystein-Resten in der extrazellulären Domäne 3, dessen Funktion bislang noch nicht aufgeklärt wurde. Vor allem die Existenz der charakteristischen CSPG Wiederholungen, die in keiner anderen Proteinfamilie zu finden sind (Staub, E. et al., FEBS Lett. 527:114-118 (2002)), lassen auf in der Evolution konservierte Funktionen dieses Proteins schließen. Allerdings zeigt das Ausschalten des AN2-Gens bislang keinen auffälligen Phänotyp in Mäusen, so dass davon ausgegangen werden kann, dass diese Funktionen weitestgehend durch andere Antigene ersetzbar sind (Grako, KA. et al., J. Cell Sci. 112 ( Pt 6):905-915 (1999)).

Auf nicht-pathologischem Gewebe wird MCSP auf Vorläufern der Haarfollikel- und Epidermiszellen, sowie auf Endothelzellen und auf aktivierten Perizyten, z. B. bei der Wundheilung, nicht jedoch auf ausgereifter Vaskulatur exprimiert (Ferrone, S. et al., Pharmacol. Ther. 57:259-290 (1993); Schlingemann, RO. et al., Am. J. Pathol. 136:1393-1405 (1990)). Außerdem wird MCSP auf Chondrozyten des Gelenkknorpels (Midwood, KS. & Salter, DM., Osteoarthritis Cartilage 6:297-305 (1998)), auf Zellen glatter Muskulatur (Tordsson, JM. et al., Cancer Immunol. Immunother. 48: 691-702 (2000)) und auf Zellen der neuromuskulären Synapse des postnatalen menschlichen Skelettmuskels (Petrini, S. et al., Mol. Cell. Neurosi. 23:219-231 (2003)) exprimiert. Während das Antigen auf fötalen Melanozyten exprimiert wird, ist es auf gesunden Melanozyten von Erwachsenen nicht zu finden (Challier, JC. et al., Cell Mol. Biol. (Noisy-le-grand) 47 Online Pub:OL79-87 (2001)). Betrachtet man pathologisches Gewebe, wird MCSP auf über 90% aller biopsierten Melanomgeweben mit sehr geringer intra- und interläsionärer Heterogenität gefunden (Natali, PG. et al., J. Cutan. Pathol. 10: 225-237 (1983)). Vergleicht man die Frequenz der MCSP-Expression bei den unterschiedlichen Melanomtypen, so ist sie beim SSM, LMM und NM etwa gleich, und beim ALM etwas geringer (Kageshita, T. et al., Cancer Res. 51:1726-1732 (1991)). Während bei den drei erstgenannten Typen die MCSP-Expression des Primärtumors und der Metastasen weitgehend identisch ist, zeigt sich für das ALM eine erhöhte Expression von MCSP auf metastasiertem Gewebe, und die Stärke der MCSP-Expression korreliert direkt mit einer schlechten Prognose (Kageshita, T. et al., Cancer Res. 53:2830-2833 (1993)). Neben der Expression auf maligne entarteten Melanozyten wird MCSP auf weiteren Neuralleisten-abgeleiteten Tumoren exprimiert. So findet sich MCSP auf Astrozytomen, Gliomen, Neuroblastomen und Sarkomen (Chekenya, M. et al., Int. J. Dev. Neurosci. 17:421-435 (1999); Shoshan, Y. et al., Proc. Natl. Acad. Sci. U S A 96:10361-10366 (1999); Godal *et al.,* 1986). Neben der Expression auf Basalzell-Karzinomen wird MCSP auf einigen akut lymphatischen und akut myeloischen Leukämien (ALL und AML) exprimiert (Behm, FG. et al., Blood 87: 1134-1139 (1996)). Während MCSP im gesunden hämatopoietischen System nicht zu finden ist, korreliert die Expression bei diesen Leukämien mit einer Translokation in das *mixed-lineage leukemia* (MLL) Gen auf der Chromosomenregion 11q23 und die MCSP-Expression bedeutet für die Patienten eine sehr schlechte Prognose (Smith, FO. et al., Blood 87:1123-1133 (1996)).

Viele Aspekte der Funktionen von MCSP wurden an Melanom-Zellen untersucht. Als Mitglied der Zelloberflächen-Proteoglykane besitzt MCSP zahlreiche Adhesionsrezeptoren. Sowohl die Kohlenhydratreste als auch der Proteinkern sind an der Wechselwirkung mit Chemokinen, Zytokinen, Wachstumsfaktoren und Teilen der extrazellulären Matrix (EZM) wie Kollagen und Fibrinogen, sowie an Zell-Zell Kontakten beteiligt (Bernfield, M. et al., Annu. Rev. Biochem. 68:729-777 (1999)). MCSP wird eine Funktion in der Zelladhäsion und Zellmigration zugesprochen, da MCSP-gerichtete Antikörper diese Prozesse *in vitro* unterdrücken (de Vries, JE. et al., Int. J. Cancer 38:465-473 (1986)).

Diese Funktionen deuten auf eine wichtige Rolle von MCSP bei der Metastasierung von Melanomzellen hin, was auch durch Experimente mit den homologen AN2 und NG2 unterstützt wird (Campoli, MR. et al., Crit. Rev. Immunol. 24:267-296 (2004)). So wurde gezeigt, dass durch die stabile Transfektion von MCSP cDNA die Invasivität von Zellen *in vitro* signifikant zunimmt (Campoli, MR. et al., Crit. Rev. Immunol. 24:267-296 (2004)). Eine Voraussetzung bei der Metastasierung ist die Invasion in endotheliale Zellen was mit Hilfe von Enzymen, die die EZM abbauen geschieht. MCSP kontaktiert und aktiviert derartige Enzyme, wie die Serinprotease Plasmin (Ranson, M. & Andronicos, NM., Front Biosci. 8:s294-304 (2003)) und Metalloproteinasen wie die *membran-type 3 matrix metalloproteinase* (MT3-MMP, Iida, J. et al., J. Biol. Chem. 276:18786-18794 (2001)). Die Rekrutierung der MT3-MMP führt dann zu deren Aktivierung und zu einem gesteigerten Invasionsverhalten durch den Abbau der EZM (Campoli, MR. et al., Crit. Rev. Immunol. 24: 267-296 (2004)).

MCSP ist wahrscheinlich in sogenannten *mikrospike* Domänen auf der Zelloberfläche organisiert (Garrigues, HJ. et al., J. Cell Biol. 103:1699-1710 (1986)). Diese aktinreichen Domänen bilden bei der Zellmigration über Filopodien Kontakte zur EZM aus. Allerdings wird hierfür eine dynamische Umstrukturierung des gesamten Aktinskeletts vorausgesetzt, was nur durch eine Kaskade intrazellulärer Signale ausgelöst werden kann. Es wurde gezeigt, dass der MCSP-EZM Kontakt den Signaltransduktionsweg der Rho GTPase Familie auslöst, und die *focal adhesion kinase* (FAK) verstärkt aktiviert (Yang, J. et al., J. Cell. Biol. 165:881-891 (2004)). Diese Signale bewirken schließlich über zahlreiche Zwischenstufen eine Reorganisation des Zytoskeletts und schließlich eine Zellmigration. Ähnlich wie bei dem verwandten Oberflächenmarker CD44 interagiert MCSP mit $\alpha_4\beta_1$ Integrin. Bei CD44 wurde gezeigt, dass diese Interaktion eine Rolle bei der Adhäsion und Proliferation von CML-Vorläufern spielt. Der EZM-MCSP-Kontakt führt außerdem auf einem bislang unbekannten Weg zu einer Aktivierung der *extracellular regulated kinase* (ERK), was zu gesteigerten onkogenen Eigenschaften in Form von erhöhter Zellproliferation, Zellinvasion und Zelladhäsion, sowie zu einem verlängerten Überleben der Zellen führt (Smalley, KS., Int. J. Cancer 104:527-532 (2003)). Diese Effekte des Kontakts EZM-MCSP können mit MCSP-gerichteten monoklonalen Antikörpern nachgestellt werden. So bewirkt der monoklonale Antikörper 9.2.27 eine Clusterbildung und verstärkt die $\alpha_4\beta_1$ Integrin Signalisierung, während der Antikörper 763.74 diese inhibiert. Zusätzlich inhibiert 9.2.27 die FAK Aktivierung, die Zelladhäsion und die Reorganisation des Zytoplasmas (Campoli, MR. et al., Crit. Rev. Immunol. 24:267-296 (2004)).
MCSP spielt eine wichtige Rolle bei der Angiogenese. Allerdings wurde die molekulare Ursache hierfürn noch nicht gegeklärt. Eine Theorie wäre, dass MCSP die Spaltung von Plasminogen in Plasmin und Angiostatin unterstützt. Der freigesetzte Aktivator Plasmin übernimmt seine Aufgabe und fördert die Neubildung der Vaskulatur, während der Inhibitor Angiostatin von MCSP sequestriert wird und so dessen unterdrückende Eigenschaften auf die Neovaskularisierung verhindert. Das weitgehend auf Tumorzellen beschränkte Expressionsprofil zeichnet MCSP als gut geeignetes Oberflächenantigen zur Antikörper-basierten Therapie Maligner Melanome aus (Campoli, MR. et al., Crit. Rev. Immunol. 24: 267-296 (2004)). Ein wichtiger Aspekt ist hierbei, dass die MCSP-Expression nicht durch Chemotherapie beeinflusst wird (Ferrone *et al.,* 2003), wodurch die kombinierte Anwendung MCSP-gerichteter Immuntherapeutika mit konventionellen Zytostatika nicht von vornherein ausgeschlossen wird.
MCSP wird als Zielantigen bei Tumorvakzinierungs-Ansätzen herangezogen. Das Problem ist zunächst, dass das Immunsystem das körpereigene Antigen (*selfantigen*) MCSP toleriert. Diese Ansätze zielen darauf ab, diese Selbsttoleranz durch Beeinflussung des Immunsystems zu brechen, um eine Immunantwort gegen den MCSP-positiven Tumor zu aktivieren. Mit Hilfe von MCSP-spezifischen T-Helferzellen aus gesunden Spendern ist es gelungen Tumor-spezifische T-Zellantworten in Melanompatienten zu erzeugen (Erfurt, C. et al., J. Immunol. 178:7703-7709 (2007)). Ein weiterer Ansatz, der die Lebenszeit von Melanompatienten der klinischen Phase IV verlängerte, war die Immunisierung mit dem anti-Idiotyp Antikörper MK2-23, der das Epitop des MCSP-bindenden Antikörpers 763.74 nachstellt (Ferrone, S. et al., Pharmacol. Ther. 57:259-290 (1993)). Mit Hilfe bispezifischer Antikörper mit der Spezifität [MCSPxCD28] soll über CD28 eine T-Zellen-Kostimulation erfolgen, die in ersten Versuchen erfolgreiche Resultate hervorbrachte (Grosse-Hovest, L. et al., Eur. J. Immunol. 33:1334-1340 (2003)). Allerdings hat der ausgelöste Zytokinsturm nach Applikation eines gegen CD28 gerichteten monoklonalen Antikörpers (TGN1412) bei 6 von 6 Personen die Anwendbarkeit CD28-gerichteter Moleküle erst einmal gebremst (Suntharalingam, G., et al., N. Engl. J. Med. 355:1018-1028 (2006)). Die bereits erwähnte Expression von MCSP auf aktivierten Perizyten hat mittlerweile ein großes Interesse in anti-Angiogenese basierten Therapieansätzen gefunden. Die Vaskularisierung ist ein essentieller Vorgang beim Wachstum solider Tumore, um eine konstante Versorgung des Tumorgewebes mit Nährstoffen und Sauerstoff zu sichern. Sowohl im xenotransplantierten Mausmodel mit humanen Melanomzellen als auch mit humanen Prostata-Karzinomzellen wurde durch gezieltes Auschschalten von MCSP eine abgeschwächte Neovaskularisierung des Tumors gezeigt (Ozerdem, U., Ophthalmic Res. 38:251-254 (2006a); Ozerdem, U., Prostate 66:294-304 (2006b)). Monoklonale Antikörper gegen MCSP wurden in den letzten 20 Jahren in unterschiedlichen Therapieansätzen zur Behandlung von Melanompatienten genutzt (Schroff *et al.,* 1985). Bis auf wenige Erfolge im Mausmodell (Bumol, TF. et al., Proc. Natl. Acad. Sci. U S A 80:529-533 (1983); Sivam, G. et al., Cancer Res. 47:3169-3173 (1987)) war das klinische Ansprechen sehr unbefriedigend, wodurch das Interesse an MCSP zunächst abschwächte. Neues Interesse keimte auf, als 300 Melanompatienten in einer klinischen Phase zur Diagnostik mittels Immunoszintigrafie einen MCSP-gerichteten Antikörper verabreicht bekamen. Der Anteil an Patienten, der mit multiplen Antikörper-Dosen behandelt wurde, wies eine signifikant gesteigerte Lebensdauer auf (Bender, H. et al., Hybridoma 16:65-68 (1997)). Obwohl die potente Lyse von Melanomzellen mit Hilfe eines MCSP-gerichteten Miniantikörpers in der Literatur einmalig beschrieben wurde (Wang, B. et al., Proc. Natl. Acad. Sci. U S A 96:1627-1632 (1999)), muss wohl eher davon ausgegangen werden, dass die hier genannten Effekte auf eine Beeinflussung der Biologie der Melanomzellen durch MCSP-Bindung zu erklären sind. Das MCSP-Antigen gilt in der Literatur fast einheitlich als überaus schlechter Mediator der CDC und ADCC (Imai, K. et al., Scand. J. Immunol. 14:369-377 (1981); Imai, K; et al., Cell. Immunol. 72:239-247 (1982); M. Schwenkert unpublizierte Daten).

Zur Therapie maligner Erkrankungen sind bislang einige gegen MCSP-gerichtete Zytostatika-Konjugate, Radioimmunkonjugate und Immuntoxine getestet worden. Bei den ersten AntikörperEffektor Konjugaten handelte es sich um ganze, MCSP-gerichtete Antikörper, chemisch fusioniert an Methotrexat (Ghose, T. et al., Cancer Immunol. Immunother. 34:90-96 (1991)), 4-Desacetylvinblastin-3-carboxyhydrazid (Schrappe, M., et al., Cancer Res. 52:3838-3844 (1992)), DT (Bumol, TF. et al., Proc. Natl. Acad. Sci. U S A 80:529-533 (1983)), Rizin A (Spitler, LE. et al., Cancer Res. 47:1717-1723 (1987)) und Purathionin (Matsui, M. et al., Jpn. J Cancer Res. 76:119-123 (1985)). Alle aufgeführten Moleküle wiesen große Erfolge im Tiermodel. Das Rizin A Konjugat zeigte sogar erste Erfolge im Menschen, wobei die Begleittoxizitäten dieses Immuntoxins formatbedingt sehr limitierend waren. Ein Radioimmunkonjugat aus dem alpha-Strahler $^{213}$Bismut und dem MCSP-gerichteten Antikörper 9.2.27 wurde zur Behandlung von Melanompatienten getestet. Die Verabreichung dieses Konjugats direkt in metastasiertes Tumorgewebe führte in 16 von 16 Melanompatienten zu massivem Zelltod der Tumorzellen, in für den Organismus verträglichen Dosen von bis zu 450 mCi (Allen, BJ. et al., Cancer Biol. Ther. 4:1318-1324 (2005)). Ein weiteres MCSP-gerichtetes Immuntoxin wurde im Jahr 2004 beschrieben. An den MCSP-gerichteten Antikörper 9.2.27 wurde chemisch die ETA Wildtyp-Variante gekoppelt. Mit diesem Immuntoxin der ersten Generation wurde eine Lebensverlängerung um 43% im xenotransplantierten Rattenmodel mit humanen Glioblastomzellen erreicht (Hjortland, GO. et al., J. Neurosurg. 100:320-327 (2004)). Diese Daten zeigen MCSP als ein validiertes Zielantigen für einen therapeutischen Einsatz mit einem Immuntoxin. Die Probleme mit diesen Immunkonjugaten sind mittlerwiele gut erklärbar und es gibt heutzutage Möglichkeiten um Immuntoxine mit aussichtsreicheren Eigenschaften herzustellen.

So wurde in der Publikation... Immuntoxin gegen MCSP konstruiert, das sich im Gegensatz zu den bis dato bekannten Formaten als wesentlich vielversprechender darstellt. Sowohl bei der Subklonierung des scFv-Fragments durch *phage display*-Technologie, als auch bei der Fusion des Antikörperfragments an das Toxin, hier z. B. an das Exotoxin A-derivat ETA', wurde eine stufenweise Verschlechterung der Bindungsstärke festgestellt, so dass das erhaltene Immunotoxin eine für klinische Anwendung unbefriedigenden Bindestärkeabilität aufweist. Der Affinitätsverlust war dabei in vergleichbarem Rahmen mit einem in der Literatur beschriebenen Fall (Huston, JS. et al., Proc. Natl. Acad. Sci. U S A 85:5879-5883 (1988)). Das Immuntoxin MCSP-ETA' hatte schließlich eine Equilibriumskonstante von 128 nM. Dieser Wert war mit anderen funktionell erprobten Immuntoxinen der Arbeitsgruppe vergleichbar. So wiesen Immuntoxine gegen CD7, CD19 und CD33 Bindungsstärken in vergleichbaren Größenordnungen auf (Peipp, M. et al., Cancer Res. 62:2848-2855 (2002); Schwemmlein, M. et al., Leukemia 21:1405-1412 (2007); Schwemmlein, M. et al., Br. J. Haematol. 133:141-151 (2006)). Trotz der erfolgreichen Charakterisierung dieser Immuntoxine liegt die Vermutung nahe, dass sich die spezifische zytotoxische Aktivität und damit die klinische Anwendbarkeit dieser Moleküle durch eine Erhöhung der Bindungsstärke verbessern würden. Die weitaus limitierendere physikalische Eigenschaft von MCSP-ETA' lag in der residuellen Bindeaktivität nach Inkubation in humanem Serum bei 37°C *in vitro.* Die ermittelte Halbwertszeit von 11 h ist für eine potentielle klinische Anwendbarkeit wahrscheinlich nicht ausreichend. Bereits das MCSP-gerichtete Antikörperfragment in seiner Ursprungsvariante wies eine vergleichsweise niedrige Halbwertszeit von 25 h auf. Somit lag die Vermutung nahe, dass durch Optimierung des scFv-Antikörperfragments auch die Stabilität des MCSP-gerichteten Immuntoxins verbessert werden könnte.

Cytotoxische T-Lymphocyten (CTL), die Tumorzellen zerstören, sind ein wichtiges Instrument des Immunsystems zur Bekämpfung von Krebs. Eine Möglichkeit, Krebspatienten mit den benötigten T-Lymphocyten zu versorgen, besteht darin, krebsspezifische T-Zellen durch die Einführung der Krebsspezifität, die durch den T-Zell-Rezeptor (TCR) bestimmt wird, in gemischte T-Lymphocyten durch genetische Methoden (d. h. "Umprogrammieren" der T-Zell-Spezifität) zu erzeugen. T-Zellen wurden durch retrovirale Übertragung von Genen, die TCR codieren, die zum Beispiel für MART-1 (Clay, T.M. et al., J. Immunol. 175:5799 (1999)), MAGE-1 (Willemsen, R.A. et al., Gene Ther. 7:1369 (2000)), MDM2 (Stanislawski, T et al., Nat. Immunol. 2:962 (2001)), gp100 (Morgan, R. A. et al., J. Immunol. 171:3287 (2003); Schaft, N. et al., J. Immunol. 170:2186 (2003)), Tyrosinase (Roszkowski, J.J. et al., Cancer Res. 65:1570 (2005)), p53 (Cohen, C.J. et al., J. Immunol. 175:5799 (2005)) und NY-ESO-1 (Zhao, Y. et al., J. Immunol. 174:4415 (2005)) spezifisch sind, und in neuerer Zeit durch Transfektion mit mRNA, die TCR codiert (Schaft, N. et al., Cancer Immunol. Immunother. 55: 1132 (2006); Morgan, R. A. et al., Science 314:126 (2006); Zhao, Y. et al., Mol. Ther. 13:151 (2006); Zhao et al., J. Immunol. 174:4415 (2005)), erfolgreich umprogrammiert. In jüngster Zeit veröffentlichte die Gruppe von Steven Rosenberg Daten über den ersten klinischen Versuch unter Verwendung von T-Zellen, die retroviral mit Genen transduziert waren, welche einen TCR gegen das Melanom-assoziierte Antigen MelanA codierten (Morgan R. A. et al., Science 314: 126 (2006)). Die Verwendung von Wildtyp-TCR hat auch Nachteile. Durch alternative Paarung mit endogenen TCR-Ketten wird der eingeführte TCR verdünnt, und neue, unerwünschte Spezifitäten, die autoreaktiv sein können, werden erzeugt (Debets, R. et al., Trends Immunol. 23:435 (2002); Xue, S. et al., Clin. Exp. Immunol. 139:167 (2005); Zhang, T. et al., Cancer Gene Ther. 11:487 (2004); Schaft, et al., J. Immunol. 170:2186 (2003); Stanislawksi, T. et al., Nat. Immunol. 2:962 (2001)). Aber das am meisten einschränkende Merkmal der Verwendung von Wildtyp-TCR ist die Notwendigkeit einer HLArestringierten Antigenpräsentation durch den Tumor. Erstens steht ein HLAangepasster TCR mit ausreichend hoher Affinität nicht immer zur Verfügung. Zweitens kann der Tumor der Therapie durch Verlust der HLA-Expression oder eine defekte Prozessierungs- und Präsentationsmaschinerie entkommen. Komplexe, die aus HLA und vom prozessierten Antigen abgeleiteten Peptiden bestehen, bilden sich also nicht mehr auf der Oberfläche, so dass der Tumor für CTL, die diese Komplexe erkennen, unsichtbar wird. Hier können gentechnisch erzeugte chimärische TCR eine Lösung darstellen, da sie native, unprozessierte Tumorantigene, die auf der Oberfläche der Tumoren exprimiert

werden, erkennen können. Diese chimärischen TCR bestehen aus den antigenbindenden Domänen eines Antikörpers, gewöhnlich in Form eines scFv, das mit signalübermittelnden Domänen, zum Beispiel des CD3-Komplexes und von costimulatorischen Rezeptoren (z. B. CD28), fusioniert ist. Ein scFv, das für irgendein Tumoroberflächenantigen spezifisch ist, wäre für diese Applikation wünschenswert.

Grundsätzlich besteht ein dringender Bedarf an wirksamen Therapieoptionen zur Behandlung von Patienten mit fortgeschrittenem Malignem Melanom. Das zentrale Ziel dieser Erfindung ist daher die Entwicklung und funktionelle Charakterisierung eines neuen, rekombinanten Immuntoxins als potentielles Therapeutikum gegen Maligne Melanome.

**Kurzbeschreibung der Erfindung**

Es wurde nunmehr gefunden, dass das bekannte gegen das MCSP-gerichtete scFv-Fragment durch molekularbiologische Methoden so verändert werden kann, dass es nicht mehr die genannten Nachteile der geringen Bindestabilität aufweist. Mit dem so optimierten scFv-Fragment konnten Immuntoxine erueugt werden, die hervoragende klinische anwendbarkeit zeigten. Schließlich konnte gezeigt werden, dass die MCSP-spezifischen scFvs als antigenbindende Domäne in chimärischen Rezeptoren verwendet werden, um T-Zellen so umzusteuern, dass sie MCSP-positive Tumorzellen erkennen und lysieren. Die Erfindung betrifft somit

(1) ein gegen das Melanom-assoziierte Chondroitinsulfat Proteoglykan (MCSP)-gerichtete scFv-Antikörperfragment umfassend die variablen Domänen des gegen MCSP gerichteten scFv-Fragments in der Orientierung $V_H$-*linker*-$V_L$, wobei $V_H$ die Aminosäresequenz von SEQ ID NOs:1 oder 2 und $V_L$ die Aminosäresequenz von SEQ ID NOs:3 oder 4 oder die entsprechenden Sequenzen, die einer oder mehreren Punktmutationen aufweisen, umfassen und *linker* eine kovalente Bindung oder ein Oligopeptid ist;
(2) eine bevorzugte Ausführungsform von (1), wobei $V_H$ und $V_L$ die in SEQ ID NO:1 1 oder 2, bzw. 3 oder 4 gezeigten Sequenzen aufweisen und *linker* die Sequenz $(G_4S)_3$ aufweist, wobei ein MCSP Antikörperfragment, das die Sequenz mit den Aminosäureresten 6 bis 254 von SEQ ID NO:5 umfasst, besonders bevorzugt ist;
(3) ein Fusionsprotein umfassend (a) eine Domaine mit einem scFv-Antikörperfragment wie vorstehend in (1) oder (2) definiert und (b) wenigstens eine Domaine mit einem funktionellen Protein oder Peptid, die mit der Domaine (a) kovalent verknüpft ist, wobei das funktionellen Protein oder Peptid vorzugsweise ein Immunotoxin oder eine Transmembran- und/oder signalübermittelnde Domäne eines Oberflächenantigens ist;
(4) eine Nukleinsäuresequenz die für ein scFv-Antikörperfragmente wie vorstehend in (1) oder (2) definiert oder für ein Fusionsprotein wie vorstehend in (3) definiert kodiert;
(5) einen Vektor umfassend eine Nukleinsäuresequenz wie vorstehend in (4) definiert;
(6) eine Zelle, die ein scFv-Antikörperfragmente wie vorstehend in (1) oder (2) definiert oder ein Fusionsprotein ein Fusionsprotein wie vorstehend in (3) definiert aufweist und/oder den Vektor wie vorstehend in (5) definiert und/oder die Nukleinsäuresequenz wie vorstehend in (4) definiert umfasst;
(7) eine bevorzugte Ausführungsform von (6) wobei die Zelle eine Expressionszelle wie transformierte oder transfizierte Pro- oder Eukaryontenzelle (einschließlich Säugerzellen aber ausgenommen humane embryonale Stammzellen), oder eine antigenpräsentierende Zelle (wie eine dendritische Zelle), oder eine antigenerkennende Zelle (wie eine T- Zelle) ist, die mit dem Antikörperfragment, dem Fusionsprotein oder den diese kodierenden Nukleinsäuren oder Vektoren beladen ist;
(8) ein Verfahren zur Herstellung

(i) eines scFv-Antikörperfragments wie vorstehend in (1) oder (2) definiert oder eines Fusionsproteins wie vorstehend in (3) definiert, umfassend das Kultivieren von Expressionszellen wie vorstehend in (7) definiert und Isolieren des scFv-Antikörperfragments oder des Fusionsproteins;
(ii) einer Expressionszellinie wie vorstehend in (7) definiert, umfassend das Transformieren oder Transfizieren einer Ausgangszelle mit dem Vektor wie vorstehend in (5) definiert und/oder der Nukleinsäuresequenz wie vorstehend in (4) definiert;
(iii) einer antigenpräsentierenden oder -erkennenden Zelle wie vorstehend in (7) definiert, umfassend das Beladen der Zelle mit einem scFv-Antikörperfragment wie vorstehend in (1) oder (2) definiert, einem Fusionsprotein wie vorstehend in (3) definiert oder mit diese kodierenden Nukleinsäuren oder Vektoren.

(9) eine Pharmazeutische Zusammensetzung, Medikament oder Diagnostische Zusammensetzung umfassend ein scFv-Antikörperfragment wie vorstehend in (1) oder (2) definiert, ein Fusionsprotein wie vorstehend in (3) definiert, eine Nukleinsäresequenz wie vorstehend in (4) definiert, einen Vektor wie vorstehend in (5) definiert oder eine antigenerkennende Zelle wie vorstehend in (7) definiert;
(10) die Verwendung eines Fusionsproteins wie vorstehend in (3) definiert oder einer antigenerkennenden Zelle wie vorstehend in (7) definiert zur Herstellung eines Medikaments zur Behandlung von Tumorerkrankungen, insbesondere zur Behandlung von Melanomen; und

(11) ein Verfahren zur Behandlung von Tumorerkrankungen, insbesondere von Melanomen in einem Patienten, umfassend das Verabreichen einer ausreichenden Menge eines Fusionsproteins wie vorstehend in (3) definiert oder einer antigenerkennenden Zelle wie vorstehend in (7) definiert an einen Patienten.

Das Immunotoxin gemäß Aspekt (3) der Erfindung kann neben der Verwndung als Melanom Therapeutikum auch bei weiteren MCSP-positiven Neoplasien wie Sarkomen, Glioblastomen und Leukämien eigesetzt werden. Die Unterdrückung der Neubildung von Blutgefäßen macht das entwickelte Immuntoxin zudem für eine Behandlung verschiedenster solider Tumore interessant, selbst wenn diese kein MCSP exprimieren.

**Kurzbeschreibung der Figuren**

Figur 1: Formate therapeutischer Antikörper und Antikörperfragmente. A-D, reduzierte Immunogenität ganzer Antikörper durch den Austausch von murinen gegen humane Regionen. A, muriner Antikörper; B, chimärer Antikörper mit humanen konstanten und murinen variablen Regionen; C, humanisierter Antikörper, nur noch die CDR-Regionen sind murinen Ursprungs; D, voll humaner Antikörper. E-I, Antikörperfragmente. E, *Fragment antigen-bindung* (F $(ab)_2$); F, *Fragment antigen-bindung* Monomer (Fab); G, *single-chain Fragment variable* (scFv); H, *tandem bispecific scFv* (tbsscFv); I, *single-chain Triple body* (scTb).

Figur 2: Direkte und indirekte Wirkmechanismen therapeutischer Antikörper. Bei der direkten Wirkung gibt es die Möglichkeit, dass durch Bindung des Antikörpers an einen Liganden oder einen Rezeptor die Signalübermittlung blockiert wird. Eine weitere Art der Blockierung ist das Unterbinden der Wechselwirkung zwischen zwei Zellen durch Besetzen der interagierenden Oberflächenstrukturen. Ebenfalls ein direkter Mechanismus ist das Auslösen apoptotischer oder anti-proliferativer Signale durch einfache Bindung oder Kreuzvernetzung von Oberflächenantigenen der Tumorzelle. Bei der indirekten Wirkung werden über die konstante Region des Antikörpers Effektoren zur Tumorzelle rekrutiert. Dies kann zum einen Zellunabhängig über die Rekrutierung und Aktivierung der Komplementkaskade erfolgen, die die Zellmembran perforiert. Die zweite Möglichkeit der indirekten Wirkung ist die Rekrutierung von Effektorzellen, die im Falle von Natürlichen Killerzellen (NK-Zellen) ebenfalls die Tumorzelle lysieren oder im Falle von Makrophagen die Tumorzelle durch Phagozytose beseitigen.

Figur 3: Formate und Wirkmechanismen von Antikörperkonjugaten. Mit Hilfe von Antikörpern und Antikörper-abgeleiteten Molekülen werden weitgehend unspezifische toxische Substanzen spezifisch an die Tumorzelle dirigiert. Zytostatika können direkt an den Antikörper gekoppelt werden, man spricht von einem Zytostatika-Immunkonjugat, oder in mit Antikörper bestückten Liposomen verpackt werden um spezifisch gerichtete Immunliposomen zu erhalten. An Antikörper gekoppelte Zytokine erhöhen die Zytokinkonzentration lokal am Tumor. Bei Radioimmunkonjugaten werden radioaktive Isotope an Antikörper gekoppelt um den Tumor durch Strahlenschäden zu eliminieren. Immuntoxine machen sich die toxischen Eigenschaften natürlich vorkommender Proteine aus Pro- oder Eukaryoten zu Nutze. Diese werden über den Antikörper zunächst zur Tumorzelle dirigiert und schließlich durch Endozytose mitsamt dem Antikörper in die Zelle aufgenommen, wo sie ihr toxisches Potential entfalten. Abgewandelt nach: Carter, 2001.

Fig. 4: Struktur des Oberflächenantigens MCSP. Das Transmembranprotein MCSP besteht aus 2322 Aminosäuren. Durch Anheftung von Glykosaminoglykanen entsteht das 450 kDa Proteoglykan. Die N-Glykosylierungsstellen befinden sich in der über Disulfidbrücken zusammengehaltenen globulären Domäne 1 des extrazellulären Teils. Auf die globuläre Domäne 1 folgt die stabförmige Domäne 2 mit Wiederholungen aus Serin und Glycin (CSPG-Domäne). Hier ist die Bindestelle für das namensgebende Chondroitinsulfat, sowie für Kollagen V und VI. Domäne D3 ist erneut globulär und schließt die extrazelluläre Domäne ab. Die kurze Transmembrandomäne beinhaltet einen Cystein-Rest. Der cytoplasmatische Teil trägt 3 Threonin-Reste als mögliche Phosphorylierungsstellen sowie ein PDZ-Motiv zur Interaktion mit intrazellulären Proteinen. Abgewandelt nach: Stallcup, 2002. N & C, N- bzw. C-terminales Ende der Polypeptidkette; D1 & D2 & D3, Domänen 1-3 der extrazellulären Domäne; S, Schwefelatom in einer Disulfidbrücke; CSPG, *chondroitin sulfate proteoglycan*; 3xT, drei Threonin-Reste; P, potentielle Phosphorylierungsstelle; PDZ; Proteininteraktionsdomäne am C-Terminus; *, Bindebereich des 9.2.27 Antikörpers.

Figur 5: Schematische Darstellung des Fusionsproteins MCSP-GFP. $V_L$ und $V_H$, variable leichte und variable schwere Kette des MCSP-gerichteten scFv-Antikörperfragments; $(G_4S)_4$, 20 Aminosäuren *linker,* aufgebaut aus vier Wiederholungen der ($Glycin_4$Serin)-Einheit; GFP, grün floureszierendes Protein; M, myc-Epitop-*tag*; H, C-terminaler Hexahistidin-*tag*.

Figur 6: Reinigung und spezifischer Nachweis des Fusionsproteins MCSP-GFP. Die Reinigung erfolgte affinitätschromatographisch mit Ni-NTA Agarose. A, Coomassie-gefärbtes SDS-PAA Gel zur Überprüfung der Reinheit des gereinigten Proteins. B, Detektion des Fusionsproteins im Western-Transfer-Experiment mit Antikörpern spezifisch für den Hexahistidin-*tag*. 1-2, Elutionsfraktionen 1-2 nach der Reinigung mit Ni-NTA Agarose.

Figur 7: Bindung des Fusionsproteins MCSP-GFP an Antigen-positiven Zellen. Die Zellen wurden vor der durchflusszytometrischen Analyse mit einer Konzentration von 2 μg/ml MCSP-GFP (schwarz) oder mit gleicher Konzentration an nicht-relevantem scFv-GFP (weiß) für 20 min inkubiert. A, MCSP-positive A2058 Zellen. B, MCSP-negative BHK-Zellen.

Figur 8: Schematische Darstellung des rekombinanten Immuntoxins MCSP-ETA' S, N-terminaler Strep-*tag*; H, Hexahistidin-*tag*; $V_L$ und $V_H$, variable leichte und variable schwere Kette des gegen MCSP-gerichteten scFv-Antikörperfragments; $(G_4S)_4$, 20 Aminosäuren *linker* zusammengesetzt aus vier Wiederholungen der ($Glycin_4$Serin)-Einheit; Exotoxin A', verkürzte Variante des *Pseudomonas* Exotoxin A Proteins, beinhaltet die Domänen Ib, II und III des Wildtyp-Proteins; K, Endoplasmatisches Reticulum-Retentionsmotiv KDEL für den retrograden Transport in die Zelle; das angegebene Molekulargewicht in kDa wurde anhand der Aminosäuresequenz ermittelt.

Figur 9: Reinigung und spezifischer Nachweis des Immuntoxins MCSP-ETA'. Die Reinigung des Immuntoxins MCSP-ETA' erfolgte affinitätschromatographisch mit Hilfe von Streptactin-Sepharose. A, Coomassie gefärbtes SDS-PAA Gel zur Überprüfung der Reinheit des gereinigten Proteins. B, C Detektion des Immuntoxins in Western-Transfer-Experimenten mit Antikörpern spezifisch für den Exotoxin A Anteil bzw. den Hexahistidin-tag des Fusionsproteins. 1-2, Elutionsfraktionen 1-2 nach der Reinigung mit Streptactin-Sepharose.

Figur 10: Analyse potentieller Aggregationstendenzen des Immuntoxins MCSP-ETA'. A, SDS-PAGE unter nicht-reduzierenden Bedingungen mit anschließendem Western-Transfer-Experiment spezifisch für den Hexahistidin-*tag*. B, Größenausschluss-Chromatographie, Elutionsprofil der gereinigten Proteinfraktion des Immuntoxins nach durchlaufen einer Sepharose 12 10/300 GL Säule; der Größenstandard wurde durch den separaten Lauf von Proteinen bekannter Größe festgelegt.

Figur 11: Antigenspezifische Bindung des Immuntoxins MCSP-ETA'. Die Zellen wurden mit 2 μg/ml MCSP-ETA' (schwarz) oder mit nicht-relevantem CD7-ETA' (weiß) inkubiert. Der Nachweis erfolgte durchflusszytometrisch mittels Maus-anti-Pentahistidin Sekundärantikörpern und RPE-gekoppelten Ziege-anti-Maus Fc Tertiärantikörpern. A, MCSP-positive M14-MCSP Zellen. B, MCSP-negative M14 Zellen. C, MCSP positive A2058 Zellen. D, MCSP positive A375M Zellen.

Figur 12: Bestimmung der Equilibriumskonstanten $K_D$ des Immuntoxins MCSP-ETA', A2058 Zellen wurden mit steigender Konzentration des Immuntoxins inkubiert. Die durchflusszytometrische Detektion erfolgte mittels Maus-anti-Pentahistidin Sekundärantikörpern und RPE-gekoppelten Ziege-anti-Maus IgG Fcγ Tertiärantikörpern. Die gemessenen Fluoreszenzintesitäten wurden mit Hilfe einer Eichgeraden in eine lineare Korrelation gebracht. Die maximale Fluoreszenzintensität wurde als 100% festgelegt und die restlichen Daten dazu in Verhältnis gesetzt. Die ermittelte halbmaximale Sättigungskonzentration ($K_D$) lag bei 128 ± 28 nM.

Figur 13: Dosisabhängiger zytotoxischer Effekt des Immuntoxins MCSP-ETA' gegen MCSP-positive Zellen. MCSP-positive A2058 Zellen (graue Balken), MCSP-positive A375M Zellen (weiße Balken) und MCSP-negative M14 Zellen wurden mit der angegebenen Konzentration MCSP-ETA' inkubiert. Nach 72 h wurde der Anteil toter Zellen durchflusszytometrisch durch hypotone PI-Färbung ermittelt. Die Daten repräsentieren Mittelwerte aus drei unabhängigen Experimenten und die Fehlerbalken zeigen die Standardabweichung (SEM). Ein Stern kennzeichnet p-Werte < 0,05. p-Werte beziehen sich auf den Vergleich zwischen behandelten und unbehandelten Zellen.

Figur 14: Antigenspezifischer zytotoxischer Effekt durch das Immuntoxin MCSP-ETA'. A, A2058 Zellen und B, A375M Zellen wurden für 48 bzw. 72 h bei 37°C inkubiert mit MCSP-ETA' (weiße Balken), CD7-ETA' (schwarze Balken), MCSP-ETA' und zehnfach molarem Überschuss des parentalen mAb 9.2.27 (graue Balken), MCSP-ETA' und 10 molarem Überschuss des Kontrollantikörpers 14G2a (quer gestreifte Balken) sowie dem Kontrollantikörper 14G2a allein (längs gestreifte Balken). Der Anteil toter Zellen wurde anschließend durchflusszytometrisch mittels hypotoner PI-Färbung ermittelt. Die Datenpunkte sind Mittelwerte aus drei unabhängig durchgeführten Experimenten und der Standardfehler (SEM) ist angegeben durch Fehlerbalken.

Figur 15: Spezifische Induktion von Apoptose durch das Immuntoxin MCSP-ETA'. A, A2058 Zellen und B, A375M Zellen wurden für 48 und 96 h bzw. für 48 und 72 h bei 37°C mit 1 μg/ml MCSP-ETA' (A, B oben) oder 1 μg/ml MCSP-ETA' und zehnfach molarem Überschuss des parentalen mAb 9.2.27 (A, B unten) inkubiert. Anschließend wurden die Zellen Annexin V / PI gefärbt und durchflusszytometrisch analysiert. Die Zahlen in den rechten Quadranten entsprechen jeweils dem prozentualen Anteil an der Gesamtpopulation. Zellen im unteren rechten Quadranten repräsentieren den Anteil früh-apoptotischer Zellen (Annexin V-positiv und PI-negativ), Zellen im oberen rechten Quadranten den Anteil toter Zellen (Annexin V-positiv und PI-positiv). Die Daten sind repräsentativ für drei unabhängig durchgeführte Experimente.

Figur 16: Apoptotische PARP-Spaltung induziert durch das Immuntoxin MCSP-ETA'. A, A2058 Zellen und B, A375M Zellen wurden für 48 h mit 1 μg/ml MCSP-ETA' bei 37°C inkubiert. Anschließend wurden Zelllysate präpariert und im Western-Transfer-Experiment mit Poly-ADP-Ribose Polymerase- (PARP) spezifischen Antikörpern analysiert. Der Pfeil zeigt das spezifische PARP-Spaltprodukt mit einem Molekulargewicht von 89 kDa. Spuren 1 und 2, Zellen behandelt mit PBS bzw. MCSP-ETA'

Figur 17: Zeitabhängige zytotoxische Wirkung von MCSP-ETA' gegen primäre Melanomzellen. Melanomzellen der

Patienten 3, 4, 7 und 8 wurden über 96 h bei 37˚C mit einer einfachen Dosis von 1 µg/ml MCSP-ETA' (grau) bzw. 1 µg/ml nicht relevantem scFv-ETA' (schwarz) inkubiert. Zu den angegebenen Zeitpunkten wurde der Anteil toter Zellen durchflusszytometrisch durch hypotone-PI Färbung ermittelt. Für jeden Zeitpunkt wurden die Daten in Dreifachansätzen bestimmt und die Fehlerbalken zeigen die Standardabweichung (SEM) um den Mittelwert.

Figur 18: Synergistischer zytotoxischer Effekt von MCSP-ETA' und CsA gegen Zellen der Melanomzelllinie A2058. A2058 Zellen wurden mit den angegebenen Konzentrationen CsA (schwarze Balken) sowie CsA in Kombination mit 100 ng/ml MCSP-ETA' (graue Balken) und CsA in Kombination mit 220 ng/ml des Parentalantikörper 9.2.27 (gestreifte Balken) inkubiert. Nach 72 h wurde der Anteil toter Zellen durchflusszytometrisch mittels hypotoner PI-Färbung ermittelt. Die gezeigten Daten sind Mittelwerte aus drei unabhängig durchgeführten Experimenten und die Standardabweichung (SEM) ist durch Fehlerbalken angegeben. Die Zahlenwerte zeigen den berechneten kooperativen Index (CI) der MCSP-ETA' und CsA Kombinationsbehandlung bei der entsprechenden CsA Konzentration.

Figur 19: Synergistischer zytotoxischer Effekt von MCSP-ETA' und CsA gegen primäre Melanomzellen. Melanomzellen von Patient 4 wurden mit 1 µg/ml MCSP-ETA' (weiße Balken), den angegebenen Konzentrationen CsA (schwarze Balken) sowie CsA in Kombination mit MCSP-ETA' (graue Balken) inkubiert. Nach 48 h wurde der Anteil toter Zellen durchflusszytometrisch mittels hypotoner PI-Färbung ermittelt. Die gezeigten Daten sind Mittelwerte aus drei unabhängig durchgeführten Experimenten und die Standardabweichung (SEM) ist durch Fehlerbalken angegeben. Die angegebenen Zahlenwerte zeigen den berechneten kooperativen Index (CI) bei der entsprechenden CsA Konzentration.

Figur 20: Stabilität von MCSP-ETA' in humanem Serum *in vitro.* MCSP-ETA' wurde über den angegeben Zeitraum bei 37˚C in humanem Serum bei einer Konzentration von 2 µg/ml gelagert. Anschließend erfolgte die durchflusszytometrische Analyse der verbleibenden Bindefähigkeit an MCSP-positiven A2058 Zellen. Eine unmittelbar vor der Messung hergestellte und nicht bei 37˚C inkubierte Probe wurde als 100%-Wert definiert und die erhaltenen mittleren Fluoreszenzaktivitäten wurden darauf normalisiert. Der Zeitpunkt halbmaximaler Bindung betrug 11 ± 2 h. Die angegebenen Werte sind Mittelwerte aus 3 unabhängigen Messungen und der Standardfehler (SEM) ist durch Fehlerbalken gekennzeichnet.

Figur 21: Stabilität des scFv-Fragments MCSP in humanem Serum *in vitro.* Das scFv-Fragment MCSP wurde über den angegebenen Zeitraum bei 37˚C in humanem Serum bei einer Konzentration von 1,5 µg/ml gelagert. Anschließend erfolgte eine durchflusszytometrische Analyse der verbleibenden Bindefähigkeit an MCSP-positiven A2058 Zellen. Eine unmittelbar vor der Messung hergestellte und nicht bei 37˚C inkubierte Probe wurde als 100% Wert definiert. Die erhaltenen mittleren Fluoreszenzaktivitäten wurden normalisiert und die Halbwertszeit wurde mit 25 ± 5 h ermittelt. Die angegeben Werte sind Mittelwerte aus 3 unabhängigen Messungen und der Standardfehler (SEM) ist durch Fehlerbalken gekennzeichnet.

Figur 22: Konstruktionsprinzip des scFv-Antikörperfragments $MCSP_{HL}$ im Vergleich zur Ausgangsvariante. F, N-terminaler FLAG-tag; $V_L$ und $V_H$, variable leichte und variable schwere Kette des gegen MCSP-gerichteten scFv-Fragments; H, C-terminaler Hexahistidin-tag; $(G_4S)_4$ bzw. $(G_4S)_3$, 20 bzw. 15 Aminosäuren *linker* bestehend aus vier bzw. drei Wiederholungen der ($Glycin_4$Serin)-Einheit; *, Aminosäure-Substitutionen in den variablen Ketten (Aminosäure-Positionen Anhand der Kabat-Datenbank).

Figur 23: Reinigung und Nachweis des scFv-Antikörperfragments $MCSP_{HL}$. Die Reinigung erfolgte in zwei Schritten: zunächst affinitätschromatographisch mit Hilfe von Ni-NTA-Agarose und später über Kationenaustausch-Chromatographie mit einer *HiTrap SP XL* Säule. A, Coomassie-gefärbtes SDS-PAA Gel zur Überprüfung der Reinheit des Proteins nach Elution von der Ni-NTA Agarose; B, Coomassie-gefärbtes SDS-PAA Gel zur Überprüfung der Reinheit des scFv-Fragments nach Elution von der *HiTrap SP XL* Säule mit 500 mM NaCl; C, Detektion des scFv-Fragments im Western-Transfer-Experiment mit Antikörpern spezifisch für den C-terminalen Hexahistidin-*tag*.

Figur 24: Antigenspezifische Bindung des scFv-Antikörperfragments $MCSP_{HL}$. A2058 Zellen wurden mit dem scFv-Fragment $MCSP_{HL}$ (dunkelgrau) oder mit gleicher Konzentration an nicht-relevantem scFv (hellgrau) inkubiert. Der Nachweis erfolgte durchflusszytometrisch mittels *AlexaFluor555*-gekoppelten Maus-anti-Pentahistidin Antikörpern. A, Vorinkubation mit dem Parentalantikörper 9.2.27 in 10 molarem Überschuss blockiert die Bindung (dicke schwarze Linie); B, Vorinkubation mit einem nicht-relevanten Antikörper (14G2a) des gleichen Isotyps in gleichem molaren Überschuss blockiert die Bindung nicht (dicke schwarze Linie).

Figur 25: Affinität des scFv-Fragments $MCSP_{HL}$ im Vergleich zum Ausgangsmolekül scFv MCSP. A2058 Melanomzellen wurden mit steigender Konzentration der scFv-Fragmente inkubiert. Die durchflusszytometrische Detektion erfolgte mittels Maus-anti-Pentahistidin Sekundärantikörpern und RPE-gekoppelten Ziege-anti-Maus Ig Fcγ Tertiärantikörpern. Die gemessenen Fluoreszenzintensitäten wurden mit Hilfe einer Eichgeraden in eine lineare Korrelation gebracht. Die maximale Fluoreszenzintensität wurde als 100% festgelegt und die restlichen Daten dazu ins Verhältnis gesetzt. A, Messung des scFv-Fragments $MCSP_{HL}$, die ermittelte Equilibriumskonstante $K_D$ war 1,65 ± 0,2 nM; B, Messung des scFv-Fragments MCSP mit einer halbmaximalen Sättigungskonzentration von 23,3 ± 2,3 nM. Die angegeben Werte sind Mittelwerte aus 3 unabhängigen Messungen und der Standardfehler (SEM) ist durch Fehlerbalken gekennzeichnet.

Figur 26: Stabilität des scFv-Fragments $MCSP_{HL}$ in humanem Serum *in vitro.* Das scFv-Fragment $MCSP_{HL}$ wurde über den angegeben Zeitraum bei 37°C in humanem Serum bei einer Konzentration von 1,0 μg/ml gelagert. Anschließend erfolgte eine durchflusszytometrische Analyse der verbleibenden Bindefähigkeit an MCSP-positiven A2058 Zellen. Eine unmittelbar vor der Messung hergestellte und nicht bei 37°C inkubierte Probe wurde als 100% Wert definiert. Nach 96 h Inkubation in humanem Serum war die Bindungsaktivität noch bei über 70%. Die angegeben Werte sind Mittelwerte aus 3 unabhängigen Messungen und der Standardfehler (SEM) ist durch Fehlerbalken gekennzeichnet.

Figur 27: Schematische Darstellung des rekombinanten Immuntoxins $MCSP_{HL}$-ETA'. tags, N-terminaler Strep- sowie Hexahistidin-*tag*; $V_H$ und $V_L$, variable schwere und variable leichte Kette des scFv-Antikörperfragments $MCSP_{HL}$; $(G_4S)_3$ bzw. $(G_4S)_4$, 15 bw. 20 Aminosäuren *linker* aufgebaut aus drei bzw. vier Wiederholungen der ($Glycin_4Serin$)-Einheit; Exotoxin A', verkürzte Variante des *Pseudomonas* Exotoxin A-Proteins, beinhaltet die Domänen Ib, II und III des Wildtyp-Proteins; K, endoplasmatisches Reticulum-Retentionsmotiv KDEL für den retrograden Transport in die Zelle. *, Aminosäure-Substitutionen in den variablen Ketten des scFv-Fragments im Vergleich zum ursprünglichen Immuntoxin.

Figur 28: Reinigung und spezifischer Nachweis des Immuntoxins $MCSP_{HL}$-ETA' sowie Analyse durch Größenausschluss-Chromatographie. Die Reinigung erfolgt affinitätschromatographisch mit Streptactin-Sepharose. A, Commassie-gefärbtes SDS-PAA Gel zur Überprüfung der Reinheit des Proteins nach der Anreicherung durch Streptactin-Sepharose. B, Detektion des Immuntoxins im Western-Transfer-Experiment mit Antikörpern spezifisch für den Hexahistidin-tag des Fusionsproteins. C, Elutionsprofil des gereinigten Immuntoxins nach Durchlaufen einer Sepharose 12 10/300 GL Säule. Der Größenstandard wurde durch den separaten Lauf von Proteinen bekannter Größe festgelegt.

Figur 29: Bindeeigenschaften des Immuntoxins $MCSP_{HL}$-ETA'. A2058 Zellen wurden mit $MCSP_{HL}$-ETA' (dunkelgrau) oder mit nicht-relevantem scFv-ETA' (hellgrau) inkubiert. Der Nachweis erfolgte durchflusszytometrisch mit AF555-gekoppelten Maus-anti-Pentahistidin Antikörpern. A, Vorinkubation mit dem Parentalantikörper 9.2.27 in 10 molarem Überschuss blockiert die Bindung (dicke schwarze Linie); B, Vorinkubation mit einem nicht-relevanten Antikörper (14G2a) blockiert die Bindung nicht (dicke schwarze Linie). C, A2058 Melanomzellen wurden mit steigender Konzentration des Immuntoxins inkubiert und die gemessenen Fluoreszenzintensitäten wurden mit Hilfe einer Eichgeraden in eine lineare Korrelation gebracht. Der ermittelte $K_D$-Wert lag bei 11,0 ± 0,8 nM. Die angegeben Werte sind Mittelwerte aus drei Messungen und der Standardfehler (SEM) ist durch Fehlerbalken gekennzeichnet.

Figur 30: Stabilität des Immuntoxins $MCSP_{HL}$-ETA' in humanem Serum *in vitro.* $MCSP_{HL}$-ETA' wurde über den angegeben Zeitraum bei 37°C in humanem Serum bei einer Konzentration von 2 μg/ml gelagert. Anschließend erfolgte eine durchflusszytometrische Analyse der verbleibenden Bindefähigkeit an MCSP-positiven A2058 Zellen. Eine unmittelbar vor der Messung hergestellte und nicht bei 37°C inkubierte Probe wurde als 100% Wert festgelegt. Die erhaltenen mittleren Fluoreszenzaktivitäten wurden darauf normalisiert. Die Bindeaktivität lag nach 96 h Inkubation in humanem Serum bei über 70%. Die angegeben Werte sind Mittelwerte aus 3 unabhängigen Messungen und der Standardfehler (SEM) ist durch Fehlerbalken gekennzeichnet.

Figur 31: Vergleichender dosisabhängiger anti-proliferativer Effekt der MCSP-gerichteten Immuntoxine. MCSP-positive A2058 Zellen wurden mit der angegebenen Konzentration MCSP-ETA' (schwarzes Quadrat) bzw. $MCSP_{HL}$-ETA' (graues Dreieck) behandelt. Nach 72 h wurde die Proliferationsaktivität mittels MTS-Test bestimmt. Der Farbumschlag unbehandelter Zellen wurde als 100% Zellviabilität definiert und die restlichen Messungen dazu ins Verhältnis gesetzt. Die Daten repräsentieren Mittelwerte aus drei unabhängigen Experimenten und die Fehlerbalken zeigen die Standardabweichung (SEM). Die halbmaximale inhibitorische Konzentration ($IC_{50}$) von MCSP-ETA' lag bei 254,6 ± 17,7 pM und von $MCSP_{HL}$-ETA' bei 52,7 ± 17,4 pM.

Figur 32: Vergleichender dosisabhängiger zytotoxischer Effekt der MCSP-gerichteten Immuntoxine. MCSP-positive A2058 Zellen wurden mit den angegebenen Konzentrationen MCSP-ETA' (schwarzes Quadrat) bzw. $MCSP_{HL}$-ETA' (graues Dreieck) behandelt. Nach 72 h wurde der Anteil toter Zellen durchflusszytometrisch mittels hypotoner PI-Färbung ermittelt. Die Daten repräsentieren Mittelwerte aus drei unabhängigen Experimenten und die Fehlerbalken zeigen die Standardabweichung (SEM). Die halbmaximale effektive Konzentration ($EC_{50}$) von MCSP-ETA' lag bei 652,0 ± 16,2 pM und von $MCSP_{HL}$-ETA' bei 150 ± 17,2 pM.

Figur 33: Vektor pet27b-STREP-His-MCSP-ETA'-KDEL

Figur 34: Vektor pAK400 $MCSP_{HL}$

Figur 35: Aufbau von chimärischen Rezeptoren. Die Konstrukte bestehen aus den schweren (VH) und leichten (VL) Ketten des ursprünglichen MCSP-lh oder des affinitätsgereiften MCSP-hl, die mit einer humanen Fc-Sequenz (hIgG-Fc) und entweder der Transmembran- und signalübermittelnden Domänen der TCRζ-Kette oder den Transmembran- und signalübermittelnden Domänen von CD28 und der signalübermittelnden Domäne der TCRζ-Kette fusioniert sind. Die Kästchen mit den Häkchen zeigen Konstrukte an, die bereits kloniert und getestet wurden.

Figur 36: Expressionskinetik der chimärischen MCSP-Rezeptoren. CD4-positive und CD8-positive T-Zellen wurden aus dem Blut von gesunden Spendern isoliert und einer Elektroporation mit mRNA, die die MCSP-lh-Konstrukte

(mit oder ohne die CD28-Domäne) codiert, unterzogen. Zu den angegebenen Zeitpunkten wurde die Oberflächenexpression durch FACS bestimmt. Einer Elektroporation ohne RNA unterzogene T-Zellen dienten als Kontrollen.

Figur 37: Spezifische Erkennung von MCSP-positiven Targetzellen. A, CD4-positive und CD8-positive T-Zellen wurden aus dem Blut von gesunden Spendern isoliert und einer Elektroporation mit mRNA, die die MCSP-Ih-Konstrukte (mit oder ohne die CD28-Domäne) codiert, unterzogen. Die MCSP-positive Zelllinie A375M, die MCSP-negative Zelllinie M14 und die MCSP-transgene Zelllinie M14-MCSP wurden als Target in einem sechzehnstündigen Cytokin-Freisetzungsassay verwendet. B, CD8-positive T-Zellen wurden einer Elektroporation mit mRNA, die die MCSP-Ih-Konstrukte mit der CD28-Domäne codiert, unterzogen. Die MCSP-positive Zelllinie A375M wurde als Target in einem vierstündigen Cytotoxizitätsassay verwendet. Einer Scheinelektroporation unterzogene CD8-positive T-Zellen dienten als negative Kontrolle.

**Detaillierte Beschreibung der Erfindung**

In Aspekt (1) und (2) der vorliegenden Erfindung wird ein gegen MCSP gerichtetes scFv-Antikörperfragment bereitgestellt, das die variablen Domänen des gegen MCSP gerichteten scFv-Fragments in der Orientierung $V_H$-*linker*-$V_L$ umfasst, wobei $V_H$ die Aminosäresequenz von SEQ ID NOs:1 oder 2 und $V_L$ die Aminosäresequenz von SEQ ID NOs: 3 oder 4 oder die entsprechenden Sequenzen, die einer oder mehreren Punktmutationen aufweisen, umfassen und *linker* eine kovalente Bindung oder ein Oligopeptid ist

Bei dem scFv-Antikörperfragment sind die Punktmutationen vorzugsweise konservative Aminosäurenaustausche. So ist dabei besonders bevorzugt, wenn (i) $V_H$ in einer oder mehreren der Positionen 3, 5, 108 und 112 bezogen auf die Sequenz von Seq ID NO:1, Punktmutationen aufweist und besonders bevorzugt eine oder mehrere der Punktmutationen Q3K, K5Q, S108T und P112S aufweist, und (ii) $V_L$ in Positionen 3, 4, 100 und 106 bezogen auf die Sequenz von Seq ID NO:3, Punktmutationen aufweist und besonders bevorzugt eine oder mehrere der Punktmutationen V3E, M4L, S100G und T106L aufweist.

Weiterhin ist bevorzugt wenn der *linker* ein hydrophiles Oligopeptid mit 1 bis 30, vorzugsweise 10 bis 20 Aminosäurereste ist und insbesondere aus Aminosäreresten ausgewählt aus Glycin , Alanin und Serin ist , wobei ein $(G_4S)_n$-Linker (worin n eine ganze von 1 bis 5 ist) besonder bevorzugt ist.

Das scFv Antkörperfragment kann darüber hinaus weitere funktionelle Sequenzen einschließlich Leader-, Sekretions- und Reinigungssequenzen wie His-Tags und STREP-Tags aufweisen.

In dem Fusionsprotein nach Aspekt (3) der Erfindung ist die Domaine (b) vorzugsweise ausgewählt aus Immunotoxinen einschließlich *Pseudomonas* Exotoxin A (ETA) und Modifikationen und Derivate derselben, Transmembran- und signalübermittelnden Domänen von Oberflächenantigenen einschließlich CD3ζ und CD28 und Modifikationen und Derivate derselben. Das Immunotoxin ist dabei vorzugsweise eine ETA-modifikation mit der Sequenz von bp 302 bis 670 von SEQ ID NO:7 und die Transmembran- und signalübermittelnde Domäne ist vorzugsweise ein Protein, das von bp 1566-2108 von SEQ ID NO:22 oder 1565-1969 von SEQ ID NO: 24 codiert wird; Ind dem Fusionsprotein können die Domainen (a) und (b) direkt oder über ein Linkermölekül miteinander verknüpt sein. Hierbei sind als Likermoleküle die vorstehen definierten Gly, Ala und Ser-haltigen Linker der scFv Domaine oder Fc C Teile von Antikörpern, insbesondere Fc C Teile von humanen IgG besonders bevorzugt.

Besonders bevorzugte Fusionsproteine sind dabei solche, die die Sequenz von AS 29-666 von SEQ ID NO: 7, AS 27-699 von SEQ ID NO:23 oder AS 27-651 von SEQ ID NO:25 umfasst.

Zur Optimierung des MCSP-gerichteten scFv-Antikörperfragments wurde das Antikörperfragment zur Stabilitätsverbesserung weitreichender umgestaltet. Die bevorzugte Reihenfolge der variablen Regionen innerhalb der Polypeptidkette eines scFv-Fragments ist in der Literatur nicht festgelegt. Das MCSP-gerichtete scFv-Fragment wurde nach publizierten Protokollen in der Orientierung $V_L$-*linker*-$V_H$ hergestellt (Krebber, A. et al., J. Immunol. Methods 201: 35-55 (1997); Peipp, M. et al., Cancer Res. 62:2848-2855 (2002)). Um die Tendenz zur Aggregation des scFv-Fragments zu reduzieren (Desplancq, D. et al., Protein Eng. 7:1027-1033 (1994)), wurde der *linker* in diesen Protokollen von 15 auf 20 Aminosäuren verlängert. Ursprünglich wurden die ersten scFv-Fragmente jedoch in der Orientierung $V_H$-*linker*-$V_L$ konstruiert und hier zeigte sich ein 15 Aminosäuren *linker* als optimal (Huston, JS. et al., Proc. Natl. Acad. Sci. U S A 85:5879-5883 (1988); Bird, RE. et al., Science, 242:423-426 (1988)). In einer Arbeitsgruppe der Universität Tübingen, die ebenfalls mit einem MCSP-gerichteten Antikörperfragment arbeitet, war die Konstruktion dieses scFv-Fragments in der Orientierung $V_H$-*liker*-$V_L$ erfolgreich (Grosse-Hovest, L. et al., Eur. J. Immunol. 33:1334-1340 (2003)). Im Vergleich mit der publizierten Aminosäuresequenz des Parentalantikörpers 9.2.27 wies das verwendete scFv-Fragment zusätzlich acht veränderte Aminosäurereste in den distalen Bereichen der variablen Regionen auf. Diese Punktmutationen sind wahrscheinlich bei der Subklonierung des Hybridoms zum scFv-Fragment entstanden. Die variablen Regionen wurden mittels PCR-Technik mit degenerierten Primern amplifiziert (Krebber, A. et al., J. Immunol. Methods 201:35-55 (1997)), wobei es in den Bereichen der Primeranlagerung möglicherweise zu Fehlpaarungen kam.

Aufgrund dieser Gegebenheiten wurde das scFv-Fragment $MCSP_{HL}$ konstruiert. Es zeichnete sich neben einer umgekehrten Orientierung der variablen Regionen, inklusive der Verkürzung des *linkers* durch die Rückmutation von

acht Aminosäureresten entsprechend der Sequenz des Parentalantikörpers aus. Bei der Charakterisierung dieses scFv-Fragments zeigte sich, neben der erhofften Steigerung der Stabilität, eine drastische Verbesserung der Affinität. Die tatsächliche Ursache dieser Verbesserung wurde im Rahmen dieser Arbeit nicht analysiert. Die Zielsetzung war die Konstruktion eines optimierten stabileren Immuntoxins. Allerdings wäre es interessant, wietere Varianten des MCSP-gerichteten scFv-Fragments, bei denen die Änderungen einzeln vorgenommen werden würden, vergleichend zu analysieren, um die Ursache dieser Verbesserung genauer zu untersuchen. Wahrscheinlich ist vor allem die Steigerung der Stabilität für den gemessenen Affinitätsgewinn verantwortlich. Möglicherweise war die Ursprungsvariante des MCSP-gerichteten scFv-Fragments bereits zu Beginn der Affinitätsmessungen zu einem großen Teil stabilitätsbedingt nicht bindefähig, sodass diese vergleichsweise niedrige Affinität ermittelt wurde.

Nachdem weder die Disulfidstabilisierung noch die CDR-graft-Methode zu einer Verbesserung der Stabilität des MCSP-gerichteten scFv-Fragments geführt hatten, wurde nur das Immuntoxin mit dem scFv-Fragment $MCSP_{HL}$ charakterisiert. Die Herstellung und Reinigung erfolgte nach den gleichen Protokollen wie bei der ursprünglichen Variante des Immuntoxins. Die Antigenspezifität wurde durch die durchgeführten Änderungen nicht beeinträchtigt. Wie bereits beim scFv-Fragment $MCSP_{HL}$ beobachtet, zeigte das überarbeitete Immuntoxin ein Verbesserung der Stabilität sowie einen starken Affinitätsgewinn. Ein wichtiger Parameter bei der funktionellen Bewertung eines Immuntoxins ist dessen Bindungsstärke. Je höher die Affinität, umso vielversprechender wird der therapeutische Nuzen eines Immuntoxin eingeschätzt (Pastan, I. et al., Nat. Rev. Cancer 6:559-565 (2006); Kreitman, RJ., Aaps. J. 8:E532-551 (2006)). Das optimierte Immuntoxin bewegt sich mit einer Equilibriumskonstante von 11 nM in einem vergleichbaren Rahmen mit dem klinisch evaluierten Immuntoxin BL22 das eine $K_D$ von 5,8 nM aufweist (Ho, M. et al., J. Biol. Chem. 280:607-617 (2005)). Die erhaltenen Daten bei der vergleichenden Bestimmung des zytotoxischen Potentials beider MCSP-gerichteter Immuntoxine bestätigen den wichtigen Einfluss der Affinität. Der wahrscheinlich größtenteils durch die Verbesserung der Stabilität erzielte Affinitätsgewinn bewirkte eine drastische Verbesserung des $IC_{50}$-Wertes in einen Bereich von 50 pM. Auch hier zeigt der Vergleich mit dem klinisch erfolgreichen Immuntoxin BL22, welches *in vitro* einen $IC_{50}$-Wert von etwa 20 pM aufweist (Bang, S. et al., Clin. Cancer Res. 11:1545-1550 (2005)), das Potential des hier entwickelten Immuntoxins bei der Bekämpfung maligner Erkrankungen.

Gemäß Aspekt (3) der Erfindung wird weiterhin ein Fusionsprotein des erfindungsgemäßen scFv gegen das hochgradig auf Malignen Melanomen exprimierte Oberflächenantigen *melanoma-associated chondroitin sulfate proteoglycan* (MCSP) mit einem Immuntoxin zur Verfügung gestellt. Das rekombinante Fusionsprotein besteht aus einem *single-chain Fragment variable-* (scFv) Antikörperfragment und einer verkürzten Variante des Exotoxin A (ETA') aus *Pseudomonas aeruginosa.* Das Immuntoxin wurde konstruiert, bakteriell exprimiert und unter nativen Bedingungen affinitätschromatographisch gereinigt. Es band antigen-spezifisch und induzierte innerhalb von 72 h bis zu 80% Zelltod in Melanom-abgeleiteten Zellen. Die Untersuchungen der Zytotoxizität gegen Zellen biopsierter Melanommetastasen von 10 Patienten mit fortgeschrittener Erkrankung zeigten in vier Fällen einen spezifischen Zelltod von 44 - 69% und in weiteren vier Fällen 17 - 25% Zelltod nach einmaliger Gabe von 14 nM MCSP-ETA'. Das Immuntoxin wirkte sowohl gegen Melanomzelllinien als auch gegen primäre Melanomzellen synergistisch mit dem zytotoxischen Potential des immunsupprimierenden Medikaments Cyclosporin A.

Im Hinblick auf einen potentiellen klinischen Einsatz waren die physikalischen Eigenschaften des Immuntoxins MCSP-ETA' nicht optimal. Vor allem die Halbwertszeit der Bindeaktivität nach Inkubation in humanem Serum *in vitro* lag bei nur 11 h. Daher wurde der scFv-Bindekopf des Immuntoxins optimiert. Sowohl die Disulfidstabilisierung als auch die *loop-graft* Methode führten nicht zu der gewünschten Verbesserung der Stabilität des MCSP-gerichteten Antikörperfragments. Das optimierte scFv-Fragment $MCSP_{HL}$ unterschied sich von der Ursprungsvariante durch umgekehrte Anordnung der variablen Domänen, einen verkürzten Peptid-linker, sowie acht veränderte Aminosäurereste entsprechend der Sequenz des Parentalantikörpers. Das mit diesem scFv-Fragment konstruierte Immuntoxin $MCSP_{HL}$-ETA' wurde analog hergestellt und zeigte eine Serumhalbwertszeit der Bindestabilität von über 96 h. Außerdem zeichnete sich $MCSP_{HL}$-ETA' durch eine 11-fach bessere Affinität ($K_D$ = 11 nM) gegenüber der Ursprungsvariante ($K_D$ = 128 nM) aus. Zusätzlich zeigte sich eine gesteigerte Zytotoxizität mit einem vierfach verbesserten $EC_{50}$-Wert von 150 pM und einem fünffach verbesserten $IC_{50}$-Wert von 53 pM.

Die hoch antigenspezifische zytotoxische Wirkung des hier entwickelten Immuntoxins im pikomolaren Konzentrationsbereich sowie die Synergie mit Cyclosporin A stellen möglicherweise ein neue, vielversprechende Therapieoption bei der Behandlung Maligner Melanome sowie weiterer MCSP-positiver Neoplasien dar. Deshalb sollte dieses Immuntoxin im Hinblick auf eine klinische Anwendung weiter charakterisiert werden.

Zur Herstellung des MCSP-gerichteten Immuntoxins sollte die kodierende Sequenz des bereits vorliegenden, MCSP-gerichteten scFv-Antikörperfragments an die codierende Sequenz einer verkürzten Variante von *Pseudomonas* Exotoxin A mit optimiertem ER-Retentionsmotiv fusioniert werden. Anschließend sollte dieses Fusionsprotein bakteriell exprimiert, aus dem Periplasma extrahiert, und affinitätschromatographisch gereinigt werden. Nach der Analyse der Bindeeigenschaften sollte dieses Immuntoxin auf seine Fähigkeit untersucht werden, antigenspezifisch Apoptose zu induzieren. Dies sollte an Langzeit-kultivierten humanen Melanomzellen sowie an biopsierten primären Zellen aus Melanompatienten untersucht werden. Außerdem sollte eine mögliche Synergie des Immuntoxins mit dem immunsupprimierenden Medikament Cyclosporin A bei der Eliminierung von Melanomzellen untersucht werden. Für den Fall, dass sich dieses Immuntoxin als potenter spezifischer Induktor der Apoptose in Melanomzellen herausstellte, sollten die biophysikalischen Eigenschaften dieses Moleküls im Hinblick auf eine potentielle klinische Anwendung genauer analysiert werden.

Die Prognose eines MM hängt stark davon ab, ob zum Zeitpunkt der Diagnose bereits eine Metastasierung stattgefunden hat. Bei ausbleibender Metastasierung beträgt die 10-Jahres Überlebensrate 95%, wohingegen nach Fernmetastasierung die mittlere Lebenserwartung auf sechs bis neun Monate abfällt. Chemotherapie und Strahlenbehandlung wirken meist nicht lebensverlängernd und werden daher häufig nur unter palliativem Gesichtspunkt angewandt. Trotzdem verursachen diese Therapieformen durch ihre unspezifische Wirkung gegen schnell proliferierende Zellen starke Nebenwirkungen. Diese unbefriedigenden Behandlungsschemata fordern neuartige und wirksame Therapieoptionen. Aufgrund ihrer spezifischen Bindefähigkeit zeichnen sich Antikörper und Antikörper-abgeleitete Immuntherapeutika als aussichtsreiche Alternativen bzw. Ergänzungen zu herkömmlichen Therapieoptionen aus.

In der vorliegenden Erfindung wurde nunmehr ein vielversprechendes Immuntoxin gegen das Oberflächenantigen MCSP zur selektiven Eliminierung von Melanomzellen entwickelt. Das rekombinante Fusionsprotein besteht aus einem scFv-Antikörperfagment und einer verkürzten Variante des Exotoxin A aus *Pseudomonas aeruginosa.* Vergleichbare Exotoxin A-abgeleitete Immuntoxine waren bereits in klinischen Studien erfolgreich (Kreitman, RJ. et al., Blood 94:3340-3348 (1999); Kreitman, RJ. et al., J. Clin. Oncol. 18:1622-1636 (2000); Kreitman, RJ. et al., N. Engl. J. Med. 345:241-247 (2001); Kreitman, RJ. et al., J. Clin. Oncol. 23:6719-6729 (2005)). Das entwickelte Immuntoxin zeigte antigenspezifische Induktion von Apoptose in Langzeit-kultivierten Melanomzellen, sowie biopsierten Metastasen von Melanompatienten *in vitro.* Zusätzlich wurde ein stark synergistisches zytotoxisches Potential mit dem immunsupprimierenden Medikament Cyclosporin A gefunden. Aufgrund der geringen Bindestabilität dieses Fusionsproteins nach Inkubation in humanem Serum *in vitro* wurde eine optimierte Variante des MCSP-gerichteten Immuntoxins hergestellt und charakterisiert. Neben der Verbesserung der Stabilität zeigte dieses Toxin eine signifikant erhöhte spezifische Toxizität gegen Melanomzellen.

Das Oberflächenantigen MCSP zeichnet sich durch sein Expressionsprofil sehr gut als Zielantigen für eine Antikörper-basierte Therapie Maligner Melanome und anderer MCSP-positiver Neoplasien aus (Campoli, MR. et al., Crit. Rev. Immunol. 24:267-296 (2004)). MCSP wird nur vereinzelt auf gesunden Geweben exprimiert, während über 85% der Melanomgewebe für dieses Antigen hochgradig positiv sind. MCSP wird auf Melanomzellen in sehr hoher Oberflächendichte von bis zu 500.000 Molekülen pro Zelle exprimiert. Die MCSP-Oberflächendichte auf den Zellen der beiden Melanom-abgeleiteten Linien A2058 und A375M war 168 bzw. 195 x $10^3$ Moleküle pro Zellen (Schwenkert, M. et al., Melanoma Res. 18:73-84 (2008)). Diese hohe Antigendichte ist für einen Antikörper-basierten Einsatz gegen MCSP ein großer Vorteil, da sich so das therapeutisch wirksame Molekül verstärkt im Tumorgewebe anreichert. Zusätzlich wird die MCSP-Expression durch den Einsatz von Chemotherapeutika nicht beeinflusst (Ferrone *et al.,* 2003), wodurch eine Kombination MCSP-gerichteter Therapeutika mit Zytostatika prinzipiell möglich ist. Die Expression von MCSP korreliert häufig mit einem unreifen Charakter der betroffenen Zellen. Stammzellartige Eigenschaften wurden für MCSP-positive Melanomzellen und für leukämische Blasten mit einer Translokation in das MLL-Gen beobachtet (Fang, D. et al., Cancer Res. 65:9328-9337 (2005); Hrusak, O. & Porwit-MacDonald, A., Leukemia 16:1233-1258 (2002); Somervaille, TC. & Cleary, ML., Cancer Cell. 10:257-268 (2006)). Zusätzlich wird MCSP auf Vorläufern von Epithel- und Haarfollikelzellen exprimiert (Ghali, L. et al., J. Invest. Dermatol. 122:433-442 (2004)). Dies deutet darauf hin, dass MCSP auf stammzellartigen Zellen und wahrscheinlich auch auf Melanom-Tumorstammzellen exprimiert wird (Fang, D. et al., Cancer Res. 65:9328-9337 (2005)). Diese Eigenschaften machen MCSP als Zielantigen noch attraktiver, da die Eliminierung dieser Tumorstammzellen in der Zukunft ein wichtiger Aspekt bei der Therapie maligner Erkrankungen werden muss, um die Repopulation der Tumormasse zu unterbinden.

Die Eigenschaften von Antikörpern, spezifisch an Zellen zu binden und damit direkte oder indirekte Effekte zu induzieren, oder als zielgerichtete Vehikel Effektormoleküle zu transportieren, wurden in den letzten Jahren verstärkt ausgenutzt, um therapeutisch wirksame Moleküle zu entwickeln. Ganze Antikörper wirken dabei meist über körpereigene Effektoren. Am Beispiel von Rituximab wird deutlich, dass die Abhängigkeit von Effektorzellen auch Nachteile mit sich bringt. So zeigen Patienten mit einer allelischen Variante von FcγRIIIa ein schlechteres Ansprechen auf Rituximab-Behandlung (Cartron, G. et al., Blood 99:754-758 (2002); Weng, WK. & Levy, R., J. Clin. Oncol. 21:3940-3947 (2003)). Als Ursache hierfür wurde eine direkte Korrelation zwischen dem Polymorphismus dieses Allels und der Affinität des IgG Antikörpers zum Rezeptor gefunden (Koene, HR. et al., Blood 90:1109-1114 (1997)). Die Bindung an inhibitorische FcRs kann außerdem dazu führen, dass das zytotoxische Potential abgeschwächt, oder sogar aufgehoben wird. Trastuzumab-Behandlung führte im Mausmodell humaner Brustkarzinome nur zum Erfolg, wenn in den Mäusen gleichzeitig der inhibitorische FcγRIIb eliminiert wurde (Clynes, RA. et al., Nat. Med. 6:443-446 (2000)). Die Abhängigkeit von Effektorzellen kann auch problematisch werden, wenn aufgrund einer vorrangegangenen Chemotherapie das hämatopoietische System des Patienten stark dezimiert wurde und die nötigen Mengen an Effektorzellen fehlen. Im Gegensatz dazu wirken Immunkonjugate bzw. Immuntoxine direkt und sind von zusätzlichen Effektoren und deren Eigenschaften unabhängig.

Neben den erwähnten Vorteilen von Immuntoxinen ist deren klinischer Einsatz auch mit Nachteilen verbunden. Ein großes Problem ist das Auftreten des VLS, was durch die Bindung der Toxinkomponenten wie Rizin oder ETA an die Endothelzellen des Blutgefäßsystems zustande kommt. Die Aminosäuremotive des Rizin, die für diese Bindung verantwortlich sind, wurden identifiziert und Punktmutationen führten in Mäusen zu weniger VLS ohne die spezifische Wirksamkeit zu verlieren (Baluna, R. & Vitetta, ES., J. Immunother. 22:41-47 (1999); Smallshaw, JE. et al., Nat. Biotechnol. 21:387-391 (2003)). Bei ETA-abgeleiteten Immuntoxinen zeigte sich VLS nicht als dosislimitierender Faktor, was sich im Vergleich zu Rizin durch die $10^3$-fach geringere Affinität von ETA zu den Endothelzellen erklären lässt (Baluna, R. & Vitetta, ES., J. Immunother. 22:41-47 (1999); Kreitman, RJ. et al., Blood 94:3340-3348 (1999); Kreitman, RJ. et al., J. Clin. Oncol. 18:1622-1636 (2000); Kreitman, RJ. et al., N. Engl. J. Med. 345:241-247 (2001); Kreitman, RJ. et al., J. Clin. Oncol. 23:6719-6729 (2005)).

Eine weitere Einschränkung von Immuntoxinen ist die Immunogenität des bakteriellen bzw. pflanzlichen Toxinanteils im Menschen. Die Entwicklung neutralisierender Antikörper verhindert die kontinuierliche Applikation des Immuntoxins und führt zu Nebenwirkungen wie beispielsweise Allergien. In Patienten, die mit Rizin-basierten Immuntoxinen behandelt wurden, zeigten sich neben der Entwicklung von HAMA-Antikörpern gegen den murinen Antikörper zusätzlich humane anti-Rizin Antikörper (*human-anti-ricin antibodies,* HARA; Amlot, PL. et al., Blood 82:2624-2633 (1993); Longo, DL. et al., Cancer J. 6:146-150 (2000)). Vergleichbare Antikörperantworten wurden auch bei DT- und ETA-abgeleiteten Molekülen beobachtet (Hall, PD. et al., Leukemia 13:629-633 (1999); von Minckwitz, G. et al., Breast Cancer Res. 7: R617-626 (2005); Kreitman, RJ. et al., J. Clin. Oncol. 23:6719-6729 (2005)). Im Fall ETA-abgeleiteter Immuntoxine gibt es einige Ansätze, die Immunogenität zu reduzieren. Die chemische Modifikation von LMB-2 mit Polyethylenglykol führte neben einer Stabilisierung des Moleküls und einer Reduktion der Nebenwirkungen, zu einer verringerten Immunogenität in Mäusen (Tsutsumi, Y. et al., Proc. Natl. Acad. Sci. U S A 97:8548-8553 (2000)). Außerdem führte die Punktmutation der kürzlich charakterisierten B-Zell Epitope in der Polypeptidkette von ETA', zu einer reduzierten Bildung neutralisierender Antikörper in der Maus, ohne dabei das zytotoxische Potential zu beeinflussen (Onda, M. et al., J. Immunol. 177:8822-8834 (2006)). Neben einer direkten Reduktion der Immunogenität der Moleküle wurde auch versucht das Immunsystem des Patienten zu unterdrücken, um die Bildung der neutralisierenden Antikörper zu verhindern. In einer Studie mit dem Lewis Y-gerichteten Immuntoxin LMB-1 wurde den Patienten zusätzlich Rituximab verabreicht. Obwohl dadurch die CD20-positiven B-Zellen weitestgehend depletiert wurden, entstanden neutralisierende anti-ETA' Antikörper (Hassan *et al.,* 2004). Vor allem bei der Behandlung von soliden Tumoren besteht auf diesem Gebiet großer Handlungsbedarf, um eine kontinuierliche Verabreichung eines Immuntoxins zu ermöglichen. Die Ergebnisse dieser Arbeit bieten eine weitere interessante Option, nämlich mit CsA die Immunantwort des Patienten zu schwächen und gleichzeitig die Tumorzellen für das spezifische toxische Potential von Exotoxin A zu sensibilisieren.

MCSP war bereits im Vorfeld dieser Arbeit ein validiertes Oberflächenantigen für Immunkonjugate. Sowohl Zytostatika-Immunkonjugate als auch Konjugate mit Wildtyp-ETA zeigten, dass MCSP nach Bindung eines monoklonalen Antikörpers internalisiert (Spitler, LE. et al., Cancer Res. 47:1717-1723 (1987); Ghose, T. et al., Cancer Immunol. Immunother. 34:90-96 (1991); Hjortland, GO. et al., J. Neurosurg. 100:320-327 (2004)). Allerdings wurde bislang kein rekombinantes scFv-ETA' Immuntoxin beschrieben. Somit war im Vorfeld dieser Arbeit nicht klar, ob eine monovalente Bindung an MCSP ausreichen würde, um effiziente Internalisierung auszulösen. Im Vergleich zu dem bereits in der Literatur beschriebenen Konjugat aus dem 9.2.27 Antikörper, chemisch gekoppelt an Wildtyp-ETA, würde das hier entwickelte Immuntoxin wahrscheinlich weniger Nebenwirkungen in einem klinischen Einsatz aufweisen. Sowohl die Verwendung des ganzen murinen Antikörpers als auch des Wildtyp-ETA Proteins sind für einen klinischen Einsatz nicht notwendig. Vielmehr zeigt ein solches Immuntoxin einige Nachteile. Aufgrund des murinen Fc-Teils würde das publizierte Konjugat wahrscheinlich von humanen, Fc-Rezeptor-positiven Zellen gebunden und evtl. sogar aufgenommen, was die betroffenen Zellen schädigen würde. Zusätzlich würde ein großer Teil des Therapeutikums bei systemischer Applikation nicht das Tumorgewebe erreichen, sondern frühzeitig abgefangen werden. Der murine Fc-Anteil erhöht außerdem die Immunogenität im Menschen, was zu einer verstärkten Produktion neutralisierender Antikörper führt. Die chemische Kopplung des Wildtyp-ETA ist ebenfalls nicht optimal. Derartige Verknüpfungen müssen zur Gewährleistung der Zytotoxizität säurelabil sein, was dazu führt, dass ein Teil des ETA bereits außerhalb der Zelle abgespalten wird. Das abgespaltene ETA wäre dann über die natürliche Bindedomäne in der Lage, in gesunde Zellen einzudringen, und diese zu töten. Die genetische scFv-ETA' Fusion löst nicht nur diese Problematik, sondern lässt sich zusätzlich in homogenerer Form herstellen und zeichnet sich mit einer Größe von nur 67 kDa als besser gewebegängig aus, als das Immunkonjugat mit ca. 215 kDa, was im Fall solider Tumore ein wichtige Rolle spielt.

Das in dieser Arbeit konstruierte Immuntoxin MCSP-ETA' wurde periplasmatisch in Bakterien exprimiert und anschließend einmalig affinitätschromatographisch gereinigt. Der erzielte Reinheitsgrad, abgeschätzt im Coomassie-gefärbten SDS-PAA Gel, war ausreichend für die Charakterisierung dieses Moleküls *in vitro.* Gleiches gilt für die erhaltene Ausbeute von 40 - 60 μg Immuntoxin pro Liter Bakterienkultur. Für eine Herstellung größerer Mengen müssten die Expressionsprotokolle abgeändert werden. Eine Orientierung wäre hier möglicherweise das ähnlich aufgebaute Immuntoxin LMB-2, welches in entsprechend großem Maßstab für eine klinische Applikation hergestellt wird.

Wie bereits erwähnt, war vor der Charakterisierung des zytotoxischen Potentials von MCSP-ETA' nicht klar,

ob die monovalente Bindung an MCSP ausreichen würde, um die Internalisierung auszulösen. Obwohl gezeigt worden war, dass humane Glioblastomzellen durch ein MCSP-gerichtetes, ETA'-basiertes Immunkonjugat eliminiert werden konnten, war nicht sicher, ob ETA in der Lage sein würde Melanomzellen zu töten. Das Immuntoxin MCSP-ETA' eliminierte Zellen zweier humaner Melanom-abgeleiteter Zelllinien mit vergleichbarer Effektivität. Nach 72 h Inkubation mit einer einmaligen Dosis wurden bis zu 80% der Zellen abgetötet, wobei signifikannte Sterberaten bereits mit 0,14 nM des Immuntoxins gemessen wurden. Der ermittelte $EC_{50}$ Wert lag im Bereich von 0,6 - 1 nM und war somit in einem vergleichbar niedrigen Rahmen mit ähnlich konstruierten Immuntoxinen anderer Spezifität (Kreitman, R.J. et al., N. Engl. J. Med. 345:241-247 (2001); Tur, MK. et al., Cancer Res. 63:8414-8419 (2003)). Die Zytotoxizität wurde dabei antigensspezifisch vermittelt und der Zelltod erfolgte über die Induktion von Apoptose. Dieses Kriterium war sehr wichtig, da im Fall einer Tumorbehandlung das nekrotische Sterben einer großen Tumormasse eine hohe Belastung für den geschwächten Organismus des Patienten darstellen würde.

Langzeit-kultivierte Melanomzellen spiegeln nur sehr ungenau die Eigenschaften akuter Melanomzellen wieder. Aus diesem Grund wurde das zytotoxische Potential von MCSP-ETA'an primären Melanomzellen biopsierter Metastasen *in vitro* untersucht. Die Untersuchungen an Melanomzellen von 10 Patienten mit fortgeschrittener Erkrankung zeigten, dass die Zellen einiger Patienten besser auf das Immuntoxin ansprachen als andere (Tabelle 1). Das schlechte Ansprechen der Patienten 5 und 6 konnte nicht auf eine geringe MCSP-Expression der Tumorzellen zurückgeführt werden, da in beiden Fällen über 90% der Zellen MCSP-positiv waren. Eine mögliche Erklärung für die unterschiedliche Suszeptibilität der Melanomzellen könnte in der Heterogenität der genetischen Veränderungen innerhalb eines jeden Tumors liegen. Tumorgenese geht immer mit einer steigenden genomischen Instabilität einher, so dass sich im Verlauf der Tumorentwicklung unterschiedliche sub-klonale Linien bilden, die möglicherweise unterschiedlich gut durch MCSP-ETA' eliminierbar sind. Diese Theorie wird durch eine aktuelle Studie an primären Brust- und Kolonkarzinomen unterstützt, bei der eine überraschend hohe Anzahl von bis zu 100 Mutationen pro Zelle in bekannten Tumorsuppressor Genen und Protoonkogenen gefunden wurde (Sjoblom *et al.,* 2006). Ausgehend von der Annahme, dass im Fall des MMs eine ähnlich große Zahl an Mutationen vorliegt, ließen sich sowohl das heterogene Ansprechen der unterschiedlichen Zellen der zehn Melanompatienten als auch die verschiedenen Ansprechraten innerhalb der Tumorzellpopulation eines Patienten erklären. Eine weitere Erklärungsmöglichkeit für das unterschiedliche Ansprechen der Zellen auf das Immuntoxin könnte in einer unterschiedlich starken Internalisierungsrate von MCSP nach Antikörperbindung liegen. Obwohl dieses Phänomen für MCSP bislang nicht beschrieben wurde, sind derartige Einflüsse für die Wirksamkeit von Gemtuzumab Ozogamicin gezeigt worden (van Der Velden, VH. et al., Blood 97:3197-3204 (2001)). Neben der Internalisierungsrate könnten auch Mutationen, die den Transport des Toxins vom Endosom ins Zytoplasma beeinflussen, Ursache für das unterschiedliche Ansprechen darstellen. Die zellulären KDEL-Rezeptoren sind für diesen Transport unerlässlich. Sind diese Rezeptoren defekt, oder wird deren Transport ins ER inhibiert, dann wird die zytotoxische Wirkung von ETA aufgehoben (Jackson, ME. et al., J. Cell. Sci. 112 (Pt 4):467-475 (1999)). Möglicherweise zeigten die Tumorzellen mancher Patienten eine niedrigere Expression dieses Rezeptors, wodurch die Wirkung des Immuntoxins abgeschwächt oder aufgehoben gewesen wäre.

Unter Anbetracht der Umstände, dass alle 10 Melanompatienten in fortgeschrittenen Stadien der Erkrankung waren und vor der Biopsie des Tumorgewebes Standardbehandlung erhielten, sind die erhaltenen Resultate vielversprechend. Der Kontakt mit DTIC hat möglicherweise weitere Resistenzen in den Tumorzellen selektiert. Insgesamt wurde bei den Zellen von sechs der zehn Patienten ein klares Ansprechen ermittelt und bei zwei weiteren zeigte sich ein schwaches Ansprechen. Hinsichtlich der schlechten Erfolge bei der Eliminierung von Melanomzellen sollten diese Ergebnisse als positiv gewertet werden.

Wie bereits beschrieben, ist die Immunogenität der bakteriellen Toxinkomponente ein limitierender Faktor für den klinischen Einsatz von Immuntoxinen. Trotzdem werden derzeit ETA-abgeleitete Immuntoxine zur Behandlung von Brustkarzinomen sowie Kopf-Hals-Karzinomen entwickelt und klinisch erprobt (Azemar, M. et al., Breast Cancer Res. Treat. 82:155-164 (2003); Di Paolo, C. et al., Clin. Cancer Res. 9:2837-2848 (2003)). Um die Immunogenität des bakteriellen Toxins zu reduzieren, wird aktuell eine Kombinationstherapie mit immunsupprimierenden Medikamenten wie CsA in Erwägung gezogen. Interessanterweise unterdrückt CsA nicht nur die Immunrektion sondern zeigte auch Apoptose-induzierendes Potential in Melanom-, Gliom- und Leukämiezellen (Ciechomska, I. et al., Int. J. Cancer 117: 59-67 (2005); Mosieniak, G. et al., J. Neurochem. 68:1142-1149 (1997); Ito, C. et al., Blood 91:1001-1007 (1998)).

Es ist bekannt, dass CsA, als ein Modulator eines mitochondriellen Kalzium-Kanals, Apoptose induzieren kann (Crompton, M. et al., Biochem. J. 255:357-360 (1988)) und eine ähnliche Wirkung wurde auch für ETA publiziert (Andersson, Y. et al., Int. J. Cancer 112:475-483 (2004)). Bei Leukämiezellen wurde gezeigt, dass CsA mit dem Tumorschädigenden Antioxidans Resveratrol, welches ebenfalls über dir Mitochondrien wirkt, synergistisch die Tumorzellen eliminiert (Zunino, SJ. & Storms, DH., Cancer Lett. 240:123-134 (2006)). Zusätzlich bindet CsA an Cyclophiline und inhibiert auf diesen Weg deren Petidyl-propyl-cis-trans-Isomerase (PPIase)-ähnliche Aktivität (Gothel, SF. & Marahiel, MA., Cell Mol. Life Sci. 55:423-436 (1999)). PPIase-ähnliche Enzyme fungieren als molekulare Chaperone und unterstützen die Proteinfaltung, den intrazellulären Transport von Proteinen und die Stabilität von Multiproteinkomplexen (Hamilton, GS. & Steiner, JP., J Med. Chem. 41:5119-5143 (1998)). Somit besteht die Möglichkeit, dass beide Agenzien

gleichzeitig auf die Mitochondrien wirken und zusätzlich die Proteinsynthese beeinflussen, was zu dem synergistischen zytotoxischen Potential führen könnte.

Von einer Steigerung des zytotoxischen Potentials durch CsA wurde bereits bei einem anti-TAC-ETA' Immuntoxin gegen Leukämiezellen berichtet (Ohno, N. et al., Leuk. Lymphoma. 43:885-888 (2002)). Die synergistische Wirkung beider Agenzien bei der Behandlung von Melanomzellen ist ein, für zukünftige Therapieoptionen, interessanter neuer Befund. In einem kombinationstherapeutischen Ansatz könnte durch CsA die Produktion neutralisierender Antikörper unterdrückt werden und gleichzeitig würden die Tumorzellen für die ETA-basierte Behandlung sensibilisiert. Diese Sensibilisierung würde eine Absenkung der erforderlichen Dosen des Immuntoxins ermöglichen, wodurch die Therapie für den Patienten verträglicher werden würde.

Im Anbetracht der aktuellen Therapiesituation bei der Behandlung von MM Patienten sowie erster Erfolge von scFv-ETA' Immuntoxinen in der Klink, könnte das hier entwickelte Immuntoxin eine Verbesserung der Behandlung des MM sowohl als Monotherapie als auch in kombinationstherapeutischen Ansätzen darstellen und sollte daher im Hinblick auf eine klinische Evaluierung weiter charakterisiert werden.

Die vorliegende Erfindung wird anhand der nachfolgenden Beispiele näher erläutert. Diese schränken jedoch den Schutzbereich der Erfindung in keinster Weise ein.

**Beispiele**

Material und Methoden

Chemikalien und Enzyme: Die verwendeten Chemikalien wie Antibiotika, Lösungsmittel, Puffersubstanzen und Salze etc. wurden von den Firmen *Bio Whittaker* (Walkersville, Maryland, USA), *Costar* (Bodenheim), *Dianova* (Hamburg), *Dako* (Glostrup, Hovedstaden, Dänemark), *Invitrogen* (Karlsruhe), *Merck* (Darmstadt), *Qiagen* (Hilden), *Roche* (Mannheim), *Roth* (Karlsruhe) und *Sigma-Aldrich* (Diesenhofen) bezogen. Die Nährsubstanzen für Bakterien stammen von den Firmen *Difco* (Michigan, USA) und *Roth,* Filter von den Firmen *Millipore* (Eschborn) und *Schleicher & Schuell* (Dassel). Enzyme wurden von *MBI Fermentas* (St. Leon-Rot), *New England Biolabs* (Frankfurt am Main), *Peqlab* (Erlangen), *Pharmacia* (Freiburg), *Promega* (Heidelberg), *Qiagen, Roche* und *Stratagene* (Heidelberg) bezogen.
Allgemeine Puffer und Lösungen: Für die Herstellung von Verdünnungen und Lösungen wurde Wasser aus einer Deionisationsanlage MilliQ der Firma *Millipore* mit einem spezifischen Widerstand von 18,2 MΩ x cm verwendet. Falls nicht anders beschrieben wurden Puffer und Lösungen bekannten Protokollsammlungen entnommen (Sambrook, J. & Russel, DW., Molecular Cloning: A Laboratory Manual, Ed.3. Cold Spring Harbour Laboratory Press, Cold Spring Harbor, NY (2001)). Vektoren: pAK 100 und pAK 400 (Krebber, A. et al., J. Immunol. Methods 201:35-55 (1997)); pASK 4 und pASK 6 *IBA* (Göttingen); pcDNA *Invitrogen;* pET27b(+) *Novagen* (Darmstadt); pSecTag2HygroC *Invitrogen.*

Oligonukleotide: Die in PCR Experimenten und zur DNA-Sequenzierung verwendeten Oligonukleotide wurden von den Firmen *MWG-Biotechnology* (Ebersberg) und *Sigma-Aldrich* synthetisiert.

Tabelle 3: Übersicht über die verwendeten Oligonukleotide.

| Oligonukleotid (SEQ ID NO:) | Sequenz (5' nach 3') |
|---|---|
| *MCSP vL for BamHI* (9) | AAA GGA TCC GGT GGT GGC GGC AGC GGT GGT GGC GGC AGC GAT ATT GAA CTG ACG CAA TCT CCA GCT TCT TTG GCT GTG |
| *MCSP vL back AvaI* (10) | TTT GGC CCC CGA GGC CGA ACG TTT CAG TTC CAG CTT GGT CCC GCC GCC AAA CGT CAG AGG GTC TTC ATT ATT TTG TTG ACA GTA ATA GGT TGC AG |
| *MCSP vH for NcoI* (11) | AAA CCA TGG CCG ATT ATA AAC AGG TGA AAC TGC AGC AGT CTG GAC CTG AGC TGG TGA AGC C |
| *MCSP vH back BamHI* (12) | TTT GGA TCC ACC GCC ACC CGA ACT CAC GGT CAC GGT GGT TCC TTG ACC CCA GTA GTC CAT AG |
| *MCSP mut for* (13) | AGT GGC AGT GGA TCT AGG |
| *MCSP mut back* (14) | GTA GGC TGT GCT GGA GG |
| *pASK4 SH Seq for* (15) | GTC GCA CTG GCT GGT TTC G |
| *pASK4 SH Seq back* (16) | GTA GCG GTA AAC GGC AGA C |

(fortgesetzt)

| Oligonukleotid (SEQ ID NO:) | Sequenz (5' nach 3') |
|---|---|
| *pAK400 lac for* (17) | CAC AGG AAA CAG CTA TGA C |
| *pAK400 lac back* (18) | GAC GCA GTA GCG GTA AAC |
| *PelB* (19) | GCC TAC GGC AGC CGC TGG |
| *Seq2* (20) | TTC ACT TCA CAG GTC AAG C |
| *KDEL back Seq* (21) | TAT AGT TCC TCC TTT CAG C |

Bakterienstämme: *E. coli* XL1-blue endA1, supE44, thi-1, recA1, gyrA96, relA1, lac [F'proAB, laclqZ$\Delta$M15, tn10, ($tet^R$)]; *E. coli* HB2151 ara, delta (lac-pro), thi/F'-proAB, laclqZ$\Delta$M15; *E. coli* BL21(DE3) F' ompT, $hsdS_B$ ($r'_B m'_B$), gal, dcm (DE3). Zellkulturmedien: Alle Zellkulturmedien und Zusätze wurden von der Firma *Invitrogen* bezogen.

Tabelle 4: Übersicht über die verwendeten Zellkulturmedien

| *Zellen* | Kulturmedium |
|---|---|
| *NG2-Hybridom* | Serum-free Hybridoma Medium (SFM), 0,5 % Penicillin / Streptomycin |
| *GD2-Hybridom* | *Roswell Park Memorial Institute* (RPMI) Medium 1640 mit Glutamax, 5 % fötales Kälberserum, 1 % Penicillin / Streptomycin |
| *293T, A2058* | *Dubelcco's Modified Eagle Medium* (DMEM) mit L-Gutamin, 10 % fötales Kälberserum, 1 % Penicillin / Streptomycin |
| A375M, BHK, CEM, M14 | *Roswell Park Memorial Institute* (RPMI) Medium 1640 mit Glutamax, 10 % fötales Kälberserum, 1 % Penicillin / Streptomycin |
| *M14 MCSP* | *Roswell Park Memorial Institute* (RPMI) Medium 1640 mit Glutamax, 10 % fötales Kälberserum, 1 % Penicillin / Streptomycin, 400 μg/ml Geneticin |
| *primäre Melanomzellen* | *Dubelcco's Modified Eagle Medium* (DMEM) mit 4mM L- Gutamin, 10 % fötales Kälberserum, 1 % Penicillin / Streptomycin, 40 μg/ml Gentamycin |

Zelllinien

Tabelle 5: Übersicht über die verwendeten Zelllinien

| *Zelllinie* | Zelltyp | Referenz |
|---|---|---|
| *293T* | humane Nierenfibroblasten stabil transifiziert mit dem *large* T-Antigen des SV40 Virus | (Pear *et al.,* 1993) |
| *A2058* | humane Melanomzellen (gewonnen aus Metastasen im Lymphknoten) | (Todaro *et al.,* 1980) |
| *A375M* | humane Melanomzellen(gewonnen aus Metastasen in der Haut) | (Kozlowski *et al.,* 1984) |
| *BHK* | *in vitro* transformierte Nieren Zellen des sy-rischen Goldhamsters (*Mesocricetus auratus*) | (MacPherson *et al.,* 1962) |
| *CEM* | humane akute T-Zell Leukämie | (Foley *et al.,* 1965) |
| *GD2* | murines Hybridom; mAb 14G2a (anti GD2) | (Mujoo *et al.,* 1989) |
| *M14* | humane Melanomzellen, MCSP negativ | (Chee *et al.,* 1976) |
| *M14 MCSP* | humane Melanomzellen, stabil transfiziert mit MCSP-cDNA | (Campoli *et al.,* 2004) |
| *NG2* | murines Hybridom; mAb 9.2.27 (anti MCSP) | (Morgan *et al.,* 1981) |

Antikörper

Tabelle 6: Übersicht über die verwendeten Antikörper.

| *Antikörper* | Klonalität | Referenz |
|---|---|---|
| *9.2.27 (Maus-anti-human MCSP), IgG2a* | monoklonal | (Morgan *et al.,* 1981) |
| *14G2a (Maus-anti-human GD2), IgG2a* | monoklonal | (Mujoo *et al.,* 1989) |
| *Maus IgG2a Negativkontrolle* | monoklonal | *Dako* |
| *Maus-anti-Pentahistidin* | monoklonal | *Qiagen* |
| *Maus-anti-Pentahistidin, AlexaFlour555-gekoppelt* | monoklonal | *Qiagen* |
| *Maus-anti-Poly-ADP-Ribose-Polymerase (PARP)* | monokonal | *BD Biosciences* |
| *Maus-anti-Strep, HRP-gekoppelt* | monoklonal | *IBA* |
| *Kaninchen-anti-Pseudomonas Exotoxin A* | polyklonal | *Sigma* |
| *Ziege-anti-Maus $F(ab)_2$, RPE-gekoppelt* | polyklonal | *DAKO* |
| *Ziege-anti-Maus $F(ab)_2$, FITC-gekoppelt* | polyklonal | *DAKO* |
| *Ziege-anti-Maus IgG Fcγ, RPE-gekoppelt* | polyklonal | *DAKO* |
| *Ziege-anti-Maus IgG Fcγ, HRP-gekoppelt* | polyklonal | *Dianova* |
| *Ziege-anti-Kaninchen IgG, HRP-gekoppelt* | polyklonal | Sigma |
| *Schwein-anti-Kaninchen, FITC-gekoppelt* | Polyklonal | *Sigma* |
| *Schwein-anti-Kaninchen, RPE-gekoppelt* | Polyklonal | *Dako* |

Methoden

Standardmethoden: Die folgenden Methoden sind, wenn nicht anderweitig gekennzeichnet, aus bekannten Protokollsammlungen entnommen (Sambrook, J. & Russel, DW., Molecular Cloning: A Laboratory Manual, Ed.3. Cold Spring Harbour Laboratory Press, Cold Spring Harbor, NY (2001)): Elektroporation von *E. coli;* Fällungsmethoden für Nukleinsäuren; Gelelektrophoresen; Herstellung elektrokompetenter *E. coli* Bakterien; Ligationsreaktionen; Minilysat-Präparation von Plasmid DNA aus *E. coli* nach der Methode der alkalischen Lyse; Photometrische Dichtebestimmung von Bakterienkulturen; Photometrische Konzentrationsbestimmung von Nukleinsäuren; Photometrische Konzentrationsbestimmung von Proteinen; Spaltung von DNA mit Restriktionsnukleasen.

Klonierungen: Alle Zwischenschritte der Klonierungen wurden mittels Restriktionsverdau analysiert und die DNA-Sequenzen der Endprodukte durch mehrfache Sequenzierung überprüft. Die Sequenzanalyse erfolgte mit der Didesoxynukleotid-Methode (Sambrook, J. & Russel, DW., Molecular Cloning: A Laboratory Manual, Ed.3. Cold Spring Harbour Laboratory Press, Cold Spring Harbor, NY (2001)) auf einem automatisierten *Applied Biosystems* DNA Sequenziergerät *(ABI Prism 310 Genetic Analyzer, Perkin Eimer,* Überlingen). Eukaryotische Expression und Reinigung der scFv-GFP Fusionsproteine: Zur Herstellung rekombinanter Proteine in Säugerzellen wurden 293T Zellen in Zellkulturschalen (∅ 10 cm) ausgesät und bei einer Konfluenz von ca. 70% mit 20 μg der entsprechenden Expressionsvektor-DNA mittels der Calcium-Phosphat-Methode und 5 mM Chloroquin transfiziert. Nach 10 h wurde das Transfektions-Medium durch frisches Kulturmedium ersetzt. Die Überstände wurden über einen Zeitraum von 7 Tagen täglich gesammelt und anschließend wurden die Pufferbedingungen für die Reinigung über Ni-NTA-Agarose durch Dialyse angepasst. Nach dem Pelletieren restlicher Zellbruchstücke durch Zentrifugation wurde der Überstand mit Ni-NTA-Agarose Perlen bei 4°C über mehrere Stunden inkubiert. Anschließend wurden die Sepharose-Perlen durch Zentrifugation eingeengt, auf ein Chromatographie-Säulchen aufgetragen und es erfolgte die Reinigung gemäß Angaben des Herstellers. Abschließend wurde das eluierte Protein gegen PBS dialysiert und bei 4°C unter Lichtausschluss gelagert.

Prokaryotische Expression und Reinigung der scFv-Fragmente: Zur Expression der scFv-Antikörperfragmente wurde zunächst das Expressionsplasmid durch Elektroporation in *E. coli* HB2151 eingebracht. Die Expression erfolgte nach publizierten Protokollen (Peipp *et al.,* 2001). Anschließend wurden die Bakterien geerntet und das Periplasma wurde osmotisch aufgeschlossen. Nach der Sedimentation der Zellbruchstücke durch Zentrifugation wurde der Überstand durch Dialyse den Pufferbedingungen für die darauf folgende Reinigung mittels Ni-NTA-Agarose angepasst. In den dialysierten Periplasmaextrakt wurden Ni-NTA-Agarose Perlen (*Qiagen*) über einen Zeitraum von mehreren Stunden bei 4°C eingerührt. Anschließend wurden die Perlen sedimentiert, in ein Chromatographie-Säulchen (*Bio-Rad,* München) überführt, gemäß der Herstellerangaben gewaschen und die scFv-Fragmente eluiert. Aufgrund der unbefriedigenden Reinheit der scFv-Fragmente nach der Ni-Chelat Anreicherung wurde ein zweiter Reinigungsschritt mittels Ionen-Austausch Chromatographie durchgeführt. Dementsprechend wurde der pH-Wert des Ni-NTA-Agarose Elutionspuffers auf 6,5 eingestellt. Bei diesem pH-Wert waren die scFv-Fragmente positiv geladenen (isoelektrischer Punkt im Bereich 7,2) und banden an die Kationen-Austauscher Säule (*HiTrap SP XL* Säule, *GE Healthcare*). Anschließend wurde das Protein mit steigenden NaCl-Konzentrationen zischen 30 und 750 mM eluiert. Abschließend erfolgte ein erneuter Pufferaustausch mittels Dialyse gegen PBS und das gereinigte Protein wurde bei 4°C für weitere Experimente gelagert. Prokaryotische Expression und Reinigung der scFv-ETA' Immuntoxine: Die Expression der Immuntoxine erfolgte unter osmotischen Stress Bedingungen im Bakterienstamm BL21(DE3) nach publizierten Protokollen (Barth *et al.,* 2000a). Zunächst wurde das entsprechende Expressionsplasmid in die Bakterien elektroporiert und die transformierten Bakterien wurden nach 45 min Schüttelinkubation in Antibiotika-freiem SOC-Medium auf *2xYT* Agarplatten mit 50 μg/ml Kanamycin und 1% Glucose ausgestrichen und über Nacht bei 37°C inkubiert. Von den bewachsenen Platten wurde zunächst eine Vorkultur in 100 ml *teriffic broth* (TB)-Medium mit 50 μg/ml Kanamycin, 0,5 mM $ZnCl_2$ und 1% Glucose polyklonal beimpft und über Nacht bei 28°C im Schüttler inkubiert. Anschließend wurden zehn 2 l Schikanekolben mit je 500 ml TB-Medium inklusive 50 μg/ml Kanamycin und 0,5 mM $ZnCl_2$ mit der Übernachtkultur beimpft, sodass die Bakteriendichte der Hauptkultur ein $OD_{600}$ von 0,1 aufwies. Bei Erreichen einer $OD_{600}$ von 1,5, was ca. einer vierstündigen Inkubation bei 28°C entsprach, wurde in den Kulturen osmotischer Stress induziert. Hierzu wurden 0,5 M Sorbitol, 4% NaCl und 10 mM Betain zugegeben und die Kultur wurde für 60 min bei 26°C inkubiert. Nach der Induktion der Expression mit 2 mM IPTG wurden die Bakterien für weitere 16-18 h bei 26°C bebrütet. Die Ernte der Kulturen erfolgte durch Zentrifugation und die pelletierten Bakterien wurden in 20% des ursprünglichen Volumens Periplasma-Extraktionspuffers (100 mM Tris/HCl pH 8,0; 500 mM Saccharose; 1 mM EDTA) resuspendiert und bei 4°C für 4 h gerührt. Nach dem Pelletieren der Zellreste wurde der Überstand mit Streptactin-Sepharose Perlen (*IBA*) versetzt und für 2 h bei 4°C unter Rühren inkubiert. Anschließend wurden die Sepharose-Perlen durch Zentrifugation eingeengt, auf eine Chromatographie-Säule (*Bio-Rad*) aufgetragen und die Reinigung erfolgte nach Angaben des Herstellers. Die Elutionsfraktionen wurden abschließend gegen PBS dialysiert, und das gereinigte Protein wurde für weitere Experimente bei 4°C gelagert.

Analyse der Immuntoxine durch Größenausschluss-Chromatographie: Zur Analyse potentieller Aggregationstendenzen der Immuntoxine wurden die gereinigten Proteinfraktionen durch Größenausschluss-Chromatographie untersucht. Hierfür wurden jeweils 100 μg des gereinigten Proteins in 500 μl PBS-Puffer mit 10% Glycerin und 0,02% $NaN_3$ auf eine Sepharose 12 10/300 GL Säule (*GE Healthcare,* München) aufgetragen und bei 4°C unter gleichen Pufferbedingungen eluiert. Die Elutionsfraktionen wurden photometrisch auf Proteinkonzentration untersucht. Als Eichstandard dienten die jeweils einzeln analysierten Substanzen bzw. Proteine Dextran (2000 kDa), Aldolase (158 kDa), bovines Serum Albumin (67 kDa), Ovalbumin (43 kDa) und RNAse A (15 kDa).

SDS-PAGE und Western-Transfer Experimente: Sodiumdodecylsulfat-Polyacrylamid Gelelektrophorese (SDS-PAGE) unter reduzierenden und nicht reduzierenden Bedingungen wurde nach Standard Methoden durchgeführt (Laemmli, 1970). Die Gele wurden anschließend mit Coomassie-Brillant-Blau R250 (*Sigma*) gefärbt. Für Western-Transfer-Experimente wurden die aufgetrennten Proteine auf eine Nitrozellulose-Membran (*Schleicher & Schuell*) transferiert. Der Transfer erfolgte in einem Halbtrockenen-Transferapparat (Semi-Phor™, *Hoefer Scientific Instruments,* San Francisco, USA) mit 1 $mA/cm^2$ für 90 min. Der Nachweis der Immuntoxine erfolgte mit einem polyklonalen Kaninchen-anti-*Pseudomonas* Exotoxin A Serum (*Sigma*). Proteine mit einem Hexahistidin-*tag* wurden mit einem Maus-anti-Pentahistidin Antikörper (*Qiagen*) detektiert. Der Nachweis des PARP-Proteins und dessen Spaltprodukt erfolgte über einen Maus-anti-Poly-ADP-Ribose-Polymerase Antikörper (*BD Biosciences*). Als Sekundäres Nachweisreagenz diente ein Ziege-anti-Maus IgG Fcγ HRP-gekoppelter Antikörper (*Dianova*) bzw. ein Ziege-anti-Kaninchen IgG Fcγ HRP-gekoppelter Antikörper (*Sigma*). Zum Nachweis von Proteinen mit einem Strep-*tag* diente ein Maus-anti-Strep HRP-gekoppelter Antikörper (*IBA*). Für die Entwicklung auf einem Röntgenfilm wurde *Enhanced Chemoluminescence* Lösung (ECL) (*Amersham*) verwendet.

Durchflusszytometrische Analysen : Alle durchflusszytometrischen Analysen wurden an einem FACS*Calibur* Apparat (*Becton Dickinson,* Mountain View, CA, USA) nach Anleitung des Herstellers durchgeführt. Sowohl für die Aufnahme der Daten, als auch für deren Auswertung wurde die zugehörige Software *CellQuest* benutzt. Für eine Messung wurden jeweils 10.000 Zellen gezählt und ausgewertet.

Nachweis der spezifischen Bindung an Antigen-positiven Zellen: Zum Bindungsnachweis wurden pro Ansatz $3 \times 10^5$ Zellen in 12 x 75 mm Falcon-Gefäße portioniert und anschließend mit PBA (PBS; 0,1 % BSA; 7 mM Na-Azid) gewaschen. Im Fall der Immuntoxine wurden die Zellen mit 25 μl des gereinigten Fusionsproteins bei einer Konzentration von 2 μg/ml für 30 min inkubiert. Als Kontrolle diente ein vergleichbares Immuntoxin anderer Spezifität. Anschließend wurden die Zellen erneut mit PBA gewaschen und für weitere 30 min mit 20 μl eines Maus-anti-Pentahistidin Antikörpers *(Sigma)* bei einer Konzentration von 10 μg/ml inkubiert. Nach abermaligem Waschen mit PBA wurden die Zellen für 30 min mit dem RPE-gekoppelten Ziege-anti-Maus $F(ab)_2$ Antikörper (*Dako*) in einer 1:100 Verdünnung unter Lichtausschluss inkubiert. Für Blockier-Experimente wurden die Zellen mit den monoklonalen Antikörpern in 10 molarem Überschuss für 30 min auf Eis vorinkubiert. Anschließend erfolgte die Inkubation mit dem Immuntoxin wie oben beschrieben. Die Detektion erfolgte in diesem Fall mit einem *AlexaFluor555*-gekoppelten Maus-anti-Pentahistidin Antikörper (*Qiagen*) bei einer Konzentration von 10 μg/ml. Zum Nachweis der spezifischen Bindung des MCSP-GFP Fusionsproteins, wurden die Zellen mit dem gereinigten Protein für 30 min in Dunkelheit bei einer Konzentration von 2 μg/ml inkubiert. Alle Inkubationsschritte erfolgten auf Eis. Nach dem letzten Waschschritt wurden die Zellen in 400 μl PBA aufgenommen und vermessen.

Bestimmung der Equilibriumskonstanten ($K_D$): Die Equilibriumskonstante $K_D$ wurde anhand publizierter Protokolle durchflusszytometrisch ermittelt (Benedict *et al*., 1997). Hierfür wurden Antigen-positive Zellen mit unterschiedlicher Konzentration des zu messenden Antikörperderivats im Bereich von 1 ng/ml bis 10 μg/ml inkubiert und mit Hilfe der ermittelten durchschnittlichen Floureszenzintensität wurden Bindungskurven als eine Funktion steigender Konzentration erstellt. Für die Kalibrierung vorgesehene Latex Fluoreszenzpartikel (*Spherotech,* Libertyville, IL, USA) wurden in jeder Versuchsreihe mitgeführt. Mit Hilfe dieser Partikel wurden die Messdaten in einen linearen Zusammenhang zwischen der gemessenen Fluoreszenzintensität und der Anzahl gebundener Fluorochrome gebracht und in Form einer Eichgeraden dargestellt. Sowohl die graphisch Auswertung als auch die Bestimmung des $K_D$-Wertes erfolgte aus den geeichten Werten mit Hilfe der Software *GraphPad Prism* V.3.0 (*GraphPad Software Inc*., San Diego, CA, USA). Nachweis des Zelltods durch hypotone PI Färbung der Zellkerne: Der prozentuale Anteil toter Zellen wurde durch Färbung der Zellkerne mit hypotoner Propidiumiodid (PI)-Lösung (*Sigma*) und durchflusszytometrischer Analyse ermittelt. Der Prozentsatz der gemessenen Zellkerne mit einem subdiploiden DNA-Gehalt wurde als Anteil toter Zellen festgelegt. Für die Färbung eines Versuchsansatzes mussten sowohl die Zellen im Überstand, als auch die mit Hilfe von Trypsin abgelösten noch adhärierenden Zellen, vereint werden. Die anschließende Färbung erfolgte nach publizierten Protokollen (Nicoletti, I. et al., J. Immunol. Methods 139:271-279 (1991)). Nachweis der Induktion von Apoptose durch Annexin V/PI-Färbung: Für die Messung eines Versuchsansatzes mussten die Zellen im Überstand und die noch adhärierenden Zellen vereinigt werden (s.o.). Nach dem Waschen wurden die Zellen in 200 μl Annexin-Bindepuffer (*Caltag Laboratories,* Hamburg) aufgenommen und mit 2,5 μl FITC-gekoppeltem Annexin V (*Caltag Laboratories*) sowie 5 μl einer 50 mg/ml konzentrierten PI-Lösung (*Sigma*) für 15 min unter Lichtausschluss bei Raumtemperatur inkubiert. Anschließend wurde der Prozentsatz toter Zellen durchflusszytometrisch anhand der Summe aus Annexin V/PI doppelt positiven sowie Annexin V einfach positiven Zellpopulationen ermittelt. Die Annexin V-positiven PInegativen Zellen, die sogenannten früh-apoptotischen Zellen, waren ein Nachweis für den Zelltod durch Apoptose (Vermes, I. et al., J. Immunol. Methods 184:39-51 (1995)).

Zytotoxizitätsexperimente

Ermittlung des zytotoxischen Potentials der Immuntoxine gegen Zelllinien: Zur Untersuchung der zytotoxischen Wirkung der Immuntoxine wurden je 1,5 x $10^4$ Zellen in 500 ml Kulturmedium in 24 Well Patten mit steigenden Konzentrationen der Proteine inkubiert. Nach 24, 48, 72 und 96 h wurden die Zellen mittels Annexin V/PI Doppelfärbung bzw. hypotoner PI-Färbung auf den Prozentsatz toter Zellen untersucht. Mit Hilfe der Software *GraphPad Prism* wurden dosisabhängige Kurven, sowie halbmaximale effektive Konzentrationen ($EC_{50}$) ermittelt. Um den Einfluss der Immuntoxine auf die Zellviabilität zu messen wurde der *CellTiter 96® $AQ_{ueous}$ Non-Radioactive* Zell Proliferations Test *(MTS; Promega*) durchgeführt. In 96-Well Platten (*Costar*) wurden 2 x $10^3$ Zellen in 100 μl Medium mit unterschiedlichen Konzentrationen an Immuntoxin inkubiert. Nach 72 h wurden 20 μl MTS/PMS Lösung nach Angaben des Herstellers zugegeben. Nach vierstündiger Inkubation bei 37˚C wurde die Absorption mit einem Mikroplatten Lesegerät (*Spectra Rainbow Thermo, Tecan,* Salzburg, Österreich) bei λ=490 nm photometrisch vermessen (Referenzwellenlänge λ=620 nm). Mit Hilfe der Software *GraphPad Prism* wurden dosisabhängige Kurven und halbmaximale inhibitorische Konzentrationen ($IC_{50}$) ermittelt.

Ermittlung des zytotoxischen Potentials gegen primäre Melanomzellen: Operativ entfernte Metastasen-Biopsien von Melanompatienten der klinischen Phasen IIIC und IV wurden mechanisch zerkleinert, durch einen Zellsieb (*Cell strainer* 40 mm Nylonmembran, *Becton Dickinson*) gepresst und anschließend in Kultur genommen. Für Zytotoxizitätsexperimente wurden 3 x $10^4$ Zellen in 500 μl Kulturmedium mit 1 μg/ml Immuntoxin behandelt. Nach 24, 48, 72 und 96 h wurde der Anteil toter Zellen durch hypotone PI-Färbung analysiert.

Kooperativität von Immuntoxinen und Cyclosporin: Zur Untersuchung synergistischer anti-Tumor Effekte, ausgelöst durch eine Behandlung mit dem MCSP-gerichteten Immuntoxin in Verbindung mit Cyclosporin A (CsA, *Sigma*) wurden Melanomzellen mit den jeweiligen Reagenzien alleine sowie in Kombination behandelt. Mit Hilfe des Kooperativitäts-Index (CI) wurde anschließend ermittelt, ob es sich um einen antagonistischen, einen additiven oder einen synergistischen Effekt beider Agenzien handelte.

$$CI = Zelltod\ (MCSP\text{-}ETA') + Zelltod\ (CsA) / Zelltod\ (MCSP\text{-}ETA' + CsA)$$

Ein CI kleiner als eins zeigt ein synergistischer Effekt zwischen beiden Agenzien, ein CI gleich eins eine additive Wirkung und ein CI größer eins einen antagonistischen Effekt.

Es wurden 1,5 x $10^4$ A2058 Zellen mit 100 μg/ml MCSP-ETA' bzw. 220 μg/ml mAb 9.2.27 sowie mit variierenden Konzentrationen an CsA von 0 - 100 μg/ml inkubiert und nach 72 h wurde der Zelltod durchflusszytometrisch mittels hypotoner PI-Färbung ermittelt. Bei den Experimenten mit den primären Melanomzellen von Patient 4 wurden 3 x $10^4$ Zellen mit 1 μg/ml MCSP-ETA' sowie 5 bzw. 10 μg/ml CsA behandelt und nach 48 h wurde der Anteil toter Zellen durch hypotoner PI-Färbung analysiert.

Nachweis der Induktion von Apoptose durch PARP-Spaltung: Zum Nachweis der Apoptose als Ursache des Zelltods wurden 4,5 x $10^5$ Zellen in 5 ml Zellkulturmedium mit 1 $\mu$g/ml des Immuntoxins über 24 bzw. 48 h bei 37˚C inkubiert. Für Blockier-Experimente wurden die Zellen mit dem mAb 9.2.27 in 10-molarem Überschuss 1 h vor Immuntoxin Zugabe vorinkubiert. Nach Ernte der Zellen wurden diese gezählt, und abhängig von ichrer Anzahl in Zelllyse-Puffer resuspendiert. Nach Zentrifugation der Zellbruchstücke und der photometrischen Ermittlung der Proteinkonzentration im Überstand mit der Bradford-Methode wurde die PARP-Spaltung im Western-Transfer Experiment analysiert.

Bestimmung der Serumstabilität rekombinanten Proteine *in vitro*: Die Antikörper-abgeleiteten Proteine wurden auf ihr residuelle Bindefähigkeit nach *in vitro* Inkubation in humanem Serum bei 37˚C untersucht. Hierfür wurden die Proteine bei einer, hinsichtlich der durchflusszytometrischen Analyse, sub-saturativen Konzentration über einen unterschiedlich langen Zeitraum in humanem Serum bei 37˚C inkubiert. Die verbleibende Bindefähigkeit wurde durchflusszytometrisch an Antigen-positiven Zellen ermittelt (Bruenke, J. et al., Br. J. Haematol. 130:218-228 (2005)). Die Bindefähigkeit einer frisch präparierten Antikörperverdünnung in humanem Serum wurde hierbei als 100% Bindung zum Zeitpunkt 0 h definiert und die restlichen Messwerte dazu normalisiert. Mit den so erhaltenen Daten wurde mit Hilfe der Software *GraphPad Prism* die Halbwertszeit der Bindeaktivität der Antikörperderivate ermittelt.

Statistische Analysen: Für alle Experimente wurden mindestens drei unabhängige Versuche durchgeführt. Soweit nicht anders angegeben, sind die gezeigten Daten Mittelwerte $\pm$ Standardabweichung (SEM). Unterschiede zwischen zwei Datensätzen wurden mit dem Student t-Test abhängig von den Versuchsparametern einseitig bzw. zweiseitig und gepaart bzw. ungepaart berechnet. Die graphische Darstellung der Daten wurde mit *Microsoft Excel* sowie *GraphPad Prism* durchgeführt. Zur Berechnung von halbmaximalen effektiven Konzentrationen ($EC_{50}$), halbmaximalen inhibitorischen Konzentrationen ($IC_{50}$), Halbwertszeiten oder halbmaximalen Sättigungskonzentrationen ($K_D$) sowie potentieller signifikannter Unterschiede dieser Werte diente erneut die Software *GraphPad Prism.* Für alle statistischen Aussagen wurde eine Irrtumswahrscheinlichkeit von weniger als 5 % als signifikant und von weniger 1 % als hoch signifikant festgelegt.

Abkürzungsverzeichnis: $\mu$ - Micro; A - Adenin oder Ampere; AA - Acrylamid; ABCD - Asymmetrie, Begrenzung, Farbe, Durchmesser (*asymmetry, border, color, diameter)*; ADCC - Antikörpervermittelte zelluläre Zytotoxizität (*antibody dependent cellular cytotoxicity*); AF - AlexaFluor; ALL - akute lymphoblastische Leukämie, ALM - akral-lentiginöses Melanom; AML - akute myeloische Leukämie; Amp - Ampicillin; AS - Aminosäure; bp - Basenpaar; BSA - Bovines Serum Albumin; bsscFv - *bispecific single-chain Fragment variable*; ˚C - Grad Celius; C - Cytosin; c - Konzentration; CAM - Chloramphenicol; Carb - Carbenicillin; CD - Differenzierungsantigen (*cluster of differentiation*); CDC - Komplement-abhängige Zytotoxizität (*complement dependent cytotoxicity*); cDNA - copy-DNA; CDR - *complementarity determining region*; CH - konstante Region der Immunglobulin schweren Kette; CHO - *chinese hamster ovary;* Ci - Curie; CI - kooperativer Index (*cooperative index*); CLL - chronische lymphoblastische Leukämie; CML - chromische myeloische Leukämie; CMV - *Cytomegalovirus;* CTL - zytotoxischer T-Lymphozyt (*cytotoxic T-lymphocyte*); Cys - Cystein; Da - Dalton; DMEM - *Dulbecco's modified eagle medium;* DNA - Desoxyribonukleinsäure (*desoxyribonucleic acid*); dNTP - Desoxynukleotid Triphosphat; dsDNA - Doppelstrang DNA; dsMCSP - disulfid-stabilisierte Variante des MCSP-gerichteten scFv-Fragments; DSMZ - Deutsche Sammlung von Mikroorganismen und Zellkultur; DT - Diphterietoxin; DTIC - Dacarbacin (Dimethyl-trizeno-imidazol-Carboxamid); E. coli - *Escherichia coli;* ECL - *enhanced chemoluminescence;* EDTA - Ethylendiamintetraaetat; EGF - epidermaler Wachstumsfaktor (*epidermal growth factor*); EGFR - EGF-Rezeptor; ER - endoplasmatisches Reticulum; ERK - *extracellular regulated kinase;* et al. - und andere (*et alii*); ETA - Exotoxin A; ETA' - verkürzte Variante von ETA, Domänen Ib, II und III des Wildtyps; EtBr - Ethidium Bromid; EZM - extrazelluläre Matrix; Fab - *Fragment antigen binding;* FACS - Durchflußzytometer (*flourescence activated cell sorter*); FAK - *focal adhesion kinase;* Fc - F*ragment crystalline*; FcR - Fc-Rezeptor; FDA - *food and drug administration*; FITC - Fluorescein-Iso-Thio-Cyanat; FKS - fötales Kälber Serum; FR - Gerüstregion *(framework region);* Fv - *fragment variable;* g - Gramm; G - Guanin; GFP - grün fluoreszierendes Protein (*green fluorescent protein*); GO - Gemtuzumab Ozogamicin (*Mylotarg*®); Golgi - Golgi Apparat; graft - *loop-graft* Variante eines scfv-Fragments; h - Stunde; HAMA - Mensch-anti-Maus Antikörper (*human-anti-mouse antibody*); HLA - *human leukocyte antigen;* HCl - Salzsäure; His - Histidin; HMW-MAA - *high-molecular-weight melanoma-associated antigen;* HRP - Meerrettich-Peroxidase (*horse radish peroxidase*); Ig - Immunglobulin; IL - Interleukin; INF$\alpha$ - Interferon $\alpha$; IPTG - Isopropyl-Thio-Galaktosid; k - Kilo; $K_D$.- Equilibriumskonstante; l - Liter; LB - *Luria broth;* LMM - Lentigo-maligna-Melanom; m - Milli; M - Molar oder Mega; mAb - monoklonaler Antikörper (*monoclonal antibody*); MCSP - Melanom-assoziiertes Chondroitinsulfat Proteoglykan; ME - Mercaptoethanol; min - Minute; MLL - Mischlinien Leukämie (*mixed lineage leukemia*); MM - Malignes Melanom; MQ - MilliQ; MRD - minimale Resterkrankung (*minimal residual disease*); MRT - Magnet-Resonanz-Tomographie; mRNA - boten-RNA (*messenger-RNA*); MTS - 3-(4,5-dimethylthiazol-2-yl)-5-(3-caboxymethoxyphenyl)-2-(4-sulfophenyl)-2H-tetrazolium, inner salt; n - nano; NAD - Nicotinamidadenindinukleotid; NK-Zelle - Natürliche Killer-Zelle; NHL - *non-Hodgkin* Lymphom; NM - noduläres Melanom; NTA - *Nitrilotriacetic acid;* OmpA - *outer membrane protein A;* OD - optische Dichte; p - pico; pH - *pondus Hydrogenii;* PMS - *phenazine methosulfate;* PAA - Polyacrylamid; PAGE - Polyacrylamid-Gel-Elektrophorese; PBS - *phosphate buffered saline;* PCR - Polymerase-Ketten-Reaktion (*polymerase chain reaction*); PET - Positronen-Emissions-Tomographie; PI - Propidium Iodid; RIP - Ribosomeninaktivierendes Protein (*ribosome-inactivating protein*);

RKI - Robert Koch Institut; RNA - Ribonukleinsäure (*ribonucleic acid*); RPE - *red phycoerythrin;* RPMI - *Roswell Park Memorial Institute;* S-Phase - Synthese Phase; SB - *super broth;* SCID - *severe combined immunodeficiency;* scFv - *single-chain Fragment variable;* SDS - Natrium Dodecylsulfat (*sodium dodecyl sulfate*); SEM - Standardabweichung (*standard error of the mean*); SSM - superfiziell spreitendes Melanom; SV40 - *simian virus 40;* T - Thymin; TB - *terrific broth;* TBS - Tris *buffered saline;* TGN - trans-Golgi Netzwerk; TNFa - Tumor Nekrose Faktor $\alpha$, Tris - Trihydoxymethylaminoethan; UV - ultraviolett; V - Volt; VEGF - vaskulärer endothelialer Wachstumsfaktor (*vascular endothelial growth factor*); $V_H$ - variable Region der Immunglobulin schweren Kette; $V_L$ - variable Region der Immunglobulin leichten Kette; VLS - *vaskular leak syndrome;* wt - Wildtyp (*wildtype*); YT - *yeast trypton;* ZNS - Zentrales Nervensystem.

Beispiel 1: Herstellung von MCSP scFv und von Fusionsproteinen davon (Vergleichsbeispiel)

Klonierung und Charakterisierung des Fusionsproteins MCSP-GFP: Zur schnellen durchflusszytometrischen Analyse MCSP-positiver Zellen wurde zunächst ein Fusionsprotein aus einem gegen MCSP gerichteten scFv-Antikörperfragment und dem grün fluoreszierenden Protein (*green-fluorescent-protein,* GFP) hergestellt.

Zu Beginn der Klonierung lag der eukaryotische Expressionsvektor pSecTag2-HygroC-His-CD7-GFP bereits vor (Peipp, M. et al., J. Immunol. Methods 285:265-280 (2004)). Die Ursprungsvariante des scFv-Antikörperfragments gegen MCSP, subkloniert aus dem Hybridom 9.2.27 (Morgan, AC. Jr. et al., Hybridoma 1:27-36 (1981)), lag ebenfalls vor (Peipp, M. et al., Cancer Res. 62:2848-2855 (2002)) und dessen cDNA wurde aus dem Vektor pAK100 MCSP als SfiI-Kassette in den Vektor pSecTag2HygroC-His-CD7-GFP ligiert. Das Plasmid pSecTag2HygroC-His-MCSP-GFP war derart konstruiert, dass die cDNA des scFv-Fragments MCSP 3' der codierenden Sequenz des Sektretions-*leader*-Peptids der murinen $\kappa$-leichten Kette und 5' der codierenden Sequenz des GFP-Fusionspartners lag. Die Fusion erfolgte durch den gleichen flexiblen $(G_4S)_4$-*linker,* durch den auch die variablen Ketten des scFv-Fragments miteinander verbunden waren. Zur Expression in Säugerzellen stand das Konstrukt unter der Kontrolle des *Cytomegalovirus* (CMV) *immediate-early* Promotors und der murine Ig$\kappa$-*leader* sorgte für die Sekretion des rekombinanten Proteins in den Zellkulturüberstand. Figur 5 zeigt schematisch das Fusionsprotein MCSP-GFP. Der fertige Expressionsvektor wurde durch Sequenzierung überprüft und bestätigt.

Herstellung und Reinigung des Fusionsproteins MCSP-GFP: Zur Expression des Fusionsproteins wurden 293T-Zellen transient mit dem Vektor pSecTag2HygroC-His-MCSP-GFP transfiziert und der Zellkulturüberstand wurde täglich über einen Zeitraum von sieben Tagen gesammelt. Anschließend wurden die Pufferbedingungen des Zellkulturüberstands durch Dialyse auf die folgende Reinigung über den c-terminalen Hexahistidin-tag angepasst. Die Reinigung erfolgte affinitätschromatographisch mit Ni-NTA Agarose. Das eluierte Protein wurde anschließend durch SDS-PAGE und Coomassie-Färbung sowie im Western-Transfer-Experiment untersucht (siehe Figur 6).

Sowohl im Coomassie-gefärbten Polyacrylamid- (PAA) Gel als auch im Western-Transfer-Experiment ist eine Bande entsprechend der zu erwartenden Größe von ca. 60 kDa zu sehen. Die erzielten Ausbeuten lagen im Bereich von 100 - 200 $\mu$g nach Reinigung des rekombinanten Proteins aus dem Überstand von zehn transfizierten Zellkulturschalen (∅ 10 cm). Für durchflusszytometrische Analysen der MCSP-Expression wurde der Grad der Reinheit des Fusionsproteins, wie im Coomassie-gefärbten SDS-PAA Gel ersichtlich, als ausreichend hingenommen. Im folgenden Experiment sollte die Bindefähigkeit des Fusionsproteins MCSP-GFP analysiert werden.

Analyse der Bindefähigkeit des Fusionsproteins MCSP-GFP: Nachdem das rekombinante Fusionsprotein, bestehend aus dem MCSP-gerichteten scFv-Fragment und GFP, über Ni-NTA-Agarose erfolgreich gereinigt worden war, wurde die Bindefähigkeit an MCSP-positiven Zellen untersucht. Hierfür wurden A2058-Zellen mit dem Fusionsprotein für 20 min inkubiert und anschließend durchflusszytometrisch analysiert (siehe Figur 7).

Das Fusionsprotein MCSP-GFP band an MCSP-positiven A2058 Zellen, jedoch nicht an MCSP-negativen BHK-Zellen. Somit stand ein sehr handliches Werkzeug zur schnellen Überprüfung der MCSP-Expression auf Zellen zur Verfügung. Konstruktion des Immuntoxins MCSP-ETA': Die cDNA des scFv-Fragments MCSP wurde aus dem Vektor pAK100 MCSP über das Restriktionsenzym SfiI ausgeschnitten und in den Vektor pASK6-linker (Dissertation M. Peipp) ligiert. Anschließend wurde aus dem Vektor pASK6-MCSP-linker das scFv-Fragment inklusive der codierenden Sequenzen für den N-terminalen Strep- und Hexahistidin-tag sowie der Sequenz für den C-terminalen 20 Aminosäuren $(G_4S)_4$-*linker* über NotI und XbaI in den Vektor pET27b-His-CD7-ETA'-REDLK 5' der codierenden Sequenz einer verkürzten Variante von *Pseudomonas* Exotoxin A eingesetzt. In dem so entstandenen Vektor pET27b-Strep-His-MCSP-ETA'-REDLK wurde anschließend die codierende Sequenz für das C-terminale ER-Retentionsmotiv REDLK gegen die Sequenz des eukaryotischen ER-Retentionsmotivs KDEL ersetzt. Dieser Austausch führte zu einer gesteigerten Zytotoxizität ähnlich konstruierter ETA-abgeleiteter Immuntoxine (Brinkmann *et al.,* 1991; Seetharam, S. et al., J. Biol. Chem. 266:17376-17381 (1991)). Zu diesem Zweck wurde aus dem Vektor pET27b-Strep-His-CD33-ETA'-KDEL (Schwemmlein, M. et al., Br. J. Haematol. 133:141-151 (2006)) ein Fragment mit der codierenden Sequenz des KDEL-Motivs über XhoI und XmaI ausgeschnitten und in den ebenso aufgeschnittenen Vektor pET27b-Strep-His-MCSP-ETA'-REDLK ligiert. Die DNA-Sequenz des Immuntoxins im so entstandenen Vektor pET27b-Strep-His-MCSP-ETA'-KDEL wurde anschließend durch Sequenzierung auf Richtigkeit überprüft und bestätigt. Figur 8 zeigt eine schematische

Darstellung des konstruierten Immuntoxins, die Proteinsequenz des exprimierten Fusionsproteins ist in SEQ ID NO:8 gezeigt.

Expression und Reinigung des Immuntoxins MCSP-ETA': Die Expression des Immuntoxins MCSP-ETA' erfolgte bakteriell. Hierzu wurde zunächst der Vektor pET27b-Strep-His-MCSP-ETA'-KDEL in den zur Expression verwendeten Bakterienstamm BL21(DE3) elektroporiert. Die Expression des Immuntoxins stand in diesem Vektor sowohl unter der Kontrolle des lac-Operons, als auch unter der Kontrolle des T7 Promotors. Im Gegensatz zum Bakterienstamm XL-1 *blue*, der für die Klonierung verwendet wurde, besitzt der Expressionsstamm BL21(DE3) das Gen für die T7-Polymerase. Durch Zugabe von IPTG zur Bakterienkultur wurde zunächst die T7-Polymerase hergestellt, welche ebenfalls unter der Kontrolle eines lac-Operons steht, und anschließend wurde die Expression des rekombinanten Fusionsproteins induziert. Das pET-Expressionssystem erlaubt ein hohes Maß an Kontrolle über den Zeitpunkt der Induktion der Proteinexpression und minimiert die basale Expressionsaktivität in Abwesenheit von IPTG oder der T7-Polymerase. Mittels eines N-terminalen OmpA-leaders wurde das Protein in das Periplasma der Bakterien sekretiert. Die Erzeugung von osmotischem Stress durch NaCl sowie die Zuführung der kompatiblen Solute Sorbitol und Betain sorgte für eine erhöhte Menge an funktionell richtig gefaltetem Protein (Arakawa, T. & Timasheff, SN. Biophys. J., 47:411-414 (1985); Barth, S. et al., Appl. Environ. Microbiol. 66:1572-1579 (2000a)). Nach der Extraktion des Periplasmas erfolgte die Reinigung der Proteine mit Hilfe von Streptactin-Sepharose. Die Reinheit sowie die Spezifität des gereinigten Fusionsproteins wurden mit Hilfe von Coomassie-gefärbten SDS-PAA Gelen bzw. Western-Transfer-Experimenten überprüft (siehe Figur 9).

Das Coomassie-gefärbte PAA Gel zeigte wenig Kontaminationen nach einmaliger Reinigung mit Streptactin-Sepharose, so dass dieser Grad an Reinheit für die folgenden Experimente als ausreichend hingenommen wurde. Sowohl durch Coomassie-Färbung im PAA Gel, als auch im spezifischen Nachweis gegen den ETA'-Anteil und den Hexahistidin-tag zeigte sich eine Bande entsprechend der erwarteten Größe von etwa 70 kDa, die dem Molekulargewicht des Immuntoxins entspricht (errechnete Größe: 66,7 kDa). Die Ausbeute lag bei ca. 30 μg Fusionsprotein pro Liter Bakterienkultur.

Die erhaltenen Banden bei einer Größe von etwa 70 kDa erlauben im reduzierenden SDS-PAA Gel keine Aussage über potentielle Aggregationstendenzen des nativen Proteins. Hierzu wurde das Protein einer SDS-PAGE unter nicht-reduzierenden Bedingungen unterzogen (siehe Figur 10A). Um nicht-kovalente Aggregationsformen ebenfalls auszuschließen wurde die gereinigte Proteinfraktion durch Größenausschluss-Chromatographie aufgetrennt und die Elutionsfraktionen photometrisch auf Proteingehalt untersucht. Figur 10B zeigt das Elutionsprofil der Größenausschluss-Chromatographie.

Im nicht-reduzierenden SDS-PAA Gel zeigte sich ein Protein in der zu erwartenden Größe von ca. 70 kDa, wodurch die Ausbildung disulfidstabilisierter Aggregate ausgeschlossen wurde. Ebenso zeigte sich im Elutionsprofil nach Größenausschluss-Chromatographie ein einzelnes Maximum bei der erwarteten Größe. Somit konnte davon ausgegangen werden, dass das gereinigte Protein in monomerer Form vorlag.

Analyse der Bindeeigenschaften von MCSP-ETA': Im Anschluss an die Reinigung des Immuntoxins sollte dessen spezifische Bindefähigkeit an Antigen-positiven Melanomzellen überprüft werden. Zu diesem Zweck wurde die Bindung an M14-MCSP Zellen, einer stabil mit MCSP-cDNA transfizierten, MCSP-positiven Zelllinie, analysiert. Zur Kontrolle wurden nicht transfizierte MCSP-negative M14 Zellen mitgeführt. Zusätzlich wurde die Bindung an Zellen der beiden MCSP-positiven Melanomzelllinien A2058 und A375M untersucht. Figur 14 zeigt die durchflusszytometrische Analyse der Bindeeigenschaften von MCSP.

Das Immuntoxin MCSP-ETA' band an MCSP-positiven Zellen. Die Bindung an M14-MCSP Zellen bei ausbleibender Bindung an M14 Zellen zeigte die antigenspezifische Bindung des rekombinanten Fusionsproteins an MCSP.

Die Affinität des Immuntoxins wurde durchflusszytometrisch bestimmt (Benedict et al., 1997). Aus einer konzentrationsabhängigen Sättigungskurve der mittleren Fluoreszenzaktivität wurde die Antikörper-Konzentration berechnet, bei der halbmaximale Sättigung eintrat. Die so erhaltene Equilibriumskonstante $K_D$ ist ein Maß für die Bindungsstärke des Immuntoxins an das Antigen MCSP (siehe Figur 12).

Die für das Immuntoxin MCSP-ETA' ermittelte halbmaximale Sättigungskonzentration lag bei 128 ± 28 nM. Dieser Wert ist mit anderen, in der Arbeitsgruppe ermittelten Equilibriumskonstanten von Immuntoxinen vergleichbar. So zeigte ein CD33-ETA' Fusionsprotein eine $K_D$ von 98 ± 14 nM (Schwemmlein et al., 2006) und ein gegen CD19 gerichtetes Immuntoxin wies eine $K_D$ von 153 ± 23 nM auf (Dissertation M. Schwemmlein).

Nachweis der spezifischen Zytotoxizität durch MCSP-ETA': Nach der Charakterisierung der Bindeeigenschaften des Immuntoxins wurde dessen zytotoxisches Potential gegen Melanom-abgeleitete Zellen untersucht. Hierfür wurden Zellen der beiden MCSP-positiven Zelllinien A2058 und A375M sowie der MCSP-negative Zelllinie M14 mit steigenden Konzentrationen des Immuntoxins inkubiert. Nach 72 h wurde der Anteil toter Zellen mittels hypotoner PI-Färbung durchflusszytometrisch bestimmt. Diese Methode basiert darauf, dass PI in den DNA-Doppelstrang interkaliert und somit die Zellkerne proportional zu ihrem DNA-Gehalt anfärbt. Mittels durchflusszytometrischer Analyse PI-gefärbter Zellkerne lässt sich der Anteil der Zellpopulation bestimmen, der infolge von Kernfragmentierung einen weniger als diploiden DNA-Gehalt aufweist. Figur 13 zeigt die Dosis-abhängige Wirkung des Immuntoxins auf Langzeit-kultivierte

Melanomzellen.

Das Immuntoxin MCSP-ETA' zeigte einen dosisabhängigen zytotoxischen Effekt gegen Melanom-abgeleitete, MCSP-positive A2058 und A375M Zellen mit maximalen Zelllysen von 80%. Bei den ebenfalls Melanom-abgeleiteten MCSP-negativen M14 Zellen war kein zytotoxischer Effekt messbar. Bereits ab einer Konzentration von 10 ng/ml zeigte sich bei A2058 und A375M eine signifikante Zytotoxizität von MCSP-ETA'.

Nachdem die Dosis-abhängige Zytotoxizität an Antigen-positiven Melanomzellen gezeigt worden war, sollte die Antigen-spezifische Induktion des Zelltods untersucht werden. Hierfür wurden A2058 und A375M Zellen vor der Zugabe des Immuntoxins mit dem Parentalantikörper 9.2.27, sowie einem Kontrollantikörper vom gleichen Isotyp aber anderer Spezifität (14G2a) in 10-fach molarem Überschuss vorbehandelt. Bei Antigen-spezifisch vermitteltem Zelltod durch das Immuntoxin sollten die Antigenbindestellen durch den Parentalantikörper blockiert und der zytotoxische Effekt aufgehoben werden (siehe Figur 14).

Der parentale gegen MCSP gerichtete Antikörper 9.2.27, nicht jedoch der Isotyp-Antikörper 14G2a, blockierte die Zytotoxizität des Immuntoxins MCSP-ETA' auf ein nicht mehr messbares Niveau. Somit wurde neben der Dosisabhängigkeit auch die Antigenspezifität des induzierten Zelltods nachgewiesen.

<u>Nachweis der Induktion von Apoptose durch MCSP-ETA':</u> Ein wichtiges Kriterium für ein Immunkonjugat ist die Induktion des Zelltods durch Apoptose. Während die Nekrose eine inflammatorische Reaktion hervorruft, werden apoptotische Zellen ohne Entzündungsreaktion aus dem Körper eliminiert. Daher war es wichtig, die Art des ausgelösten Zelltods zu untersuchen. Eine Möglichkeit zum Nachweis der Apoptose ist eine Doppelfärbung sterbender Zellen mit Annexin V und PI. Ein frühes Zeichen der Apoptose ist der Verlust der Membranintegrität, wodurch die normalerweise zum Zytoplasma gerichteten Phosphatidylserine auf die Außenseite der Membran gelangen. Bei diesem Prozess bleibt die Barrieren-Funktion der Membran intakt, wodurch PI nicht in den Zellkern gelangt. Das Protein Annexin V bindet spezifisch an Phosphatidylserine, kann aber ebenfalls eine intakte Zellmembran nicht durchdringen. Ein Auftreten von Annexin V-positiven und gleichzeitig PInegativen Zellen ist somit ein sicherer Nachweis für die Induktion der Apoptose. MCSP-positive Zellen wurden mit MCSP-ETA' behandelt und zu unterschiedlichen Zeiten durchflusszytometrisch mittels Annexin V/PI Doppelfärbung analysiert. Als Kontrolle wurden die Zellen mit dem Parentalantikörper 9.2.27 vorinkubiert um den apoptotischen Effekt zu blockieren (siehe Figur 15). Sowohl bei A2058 als auch bei A375M Zellen zeigte sich nach Behandlung mit dem Immuntoxin durch AnnexinV/PI Doppelfärbung und Durchflusszytometrie eine früh-apoptotische Zellpopulation (Annexin V-positiv und PI-negativ). Früh-apoptotische Merkmale wurden in 40% der mit dem Immuntoxin behandelten A2058 Zellen sowie in 32% der behandelten A375M Zellen nach 96 bzw. 72 h gemessen. Der apoptotische Effekt war antigen-spezifisch, da er Effekt durch prä-Inkubation mit dem Parentalantikörper blockiert wurde.

Ein weiterer Nachweis der Induktion von Apoptose ist die Caspase 3-vermittelte Spaltung der Poly-ADP-Ribose Polymerase (PARP; 116 kDa), einem Reparaturenzym für DNA-Strangbrüche, in einem frühen Stadium des programmierten Zelltods. Bei dieser Spaltung entstehen zwei Fragmente mit 89 kDa und 27 kDa. Dieser Vorgang findet nicht in nekrotischen Zellen statt und ermöglicht daher die Diskriminierung beider Arten des Zelltods. A2058 und A375M Zellen wurden mit MCSP-ETA' und zur Kontrolle mit PBS behandelt. Nach 48 h wurden Zelllysate von den behandelten Zellen hergestellt und das PARP-Protein in Western-Transfer-Experimenten nachgewiesen (siehe Figur 16).

Sowohl die Existenz früh-apoptotischer Zellen in der Annexin-PI Doppelfärbung als auch das Auftreten des spezifischen Spaltproduktes des PARP-Proteins zeigten, dass der Zelltod der Melanom-abgeleiteten A2058 und A375M Zellen durch MCSP-ETA' über die Induktion von Apoptose stattfand.

<u>Analyse der Zytotoxizität gegen primäre Melanomzellen:</u> Über lange Zeit *in vitro* kultivierte Zelllinien humaner Tumorzellen repräsentieren nur sehr ungenau die Eigenschaften von Tumorzellen in Patienten. Aus diesem Grund wurde das Immuntoxin zusätzlich an primären Tumorzellen von Melanom Patienten getestet. Die Melanomzellen wurden aus operativ entfernten Metastasen von zehn Melanom Patienten der klinischen Stadien IIIC und IV (Balch, CM. et al., J. Clin. Oncol. 19:3635-3648 (2001)) gewonnen. Die Gewebestücke wurde mechanisch zerkleinert und durch einen Zellsieb gepresst. Anschließend wurden die Zellen in Kulturmedium aufgenommen und über mehrere Wochen passagiert. Unmittelbar vor der Ermittlung des zytotoxischen Potentials durch das Immuntoxin wurden die Zellen mit dem Fusionsprotein MCSP-GFP durchflusszytometrisch auf MCSP-Expression untersucht. Anschließend wurden die Zellen über einen Zeitraum von 96 h mit einer einfachen Dosis MCSP-ETA' bzw. CD33-ETA' bei einer Konzentration von 1 μg/ml inkubiert. Alle 24 h wurde mittels hypotoner PI-Färbung der Anteil toter Zellen bestimmt. Figur 17 zeigt die zeitabhängige Induktion des Zelltods in den Melanomzellen der Patienten 3, 4, 7 und 8.

Das Immuntoxin induzierte in den Melanomzellen der Patienten 3, 4, 7 und 8 nach 96 h 44-69% Zelltod bei einem Hintergrund von 17-23%. Mit dem nicht relevanten CD33-ETA' Konstrukt war keine vergleichbare Erhöhung des Anteils toter Zellen messbar. Unabhängig von dem Grad der MCSP-Expression sprachen jedoch nicht alle Zellpopulationen gleichermaßen auf das Immuntoxin an. Die Ergebnisse der zehn untersuchten Patientenzellen sind in Tabelle 7 zusammengestellt. Zusammenfassend wurde in vier der zehn Patienten ein spezifischer Zelltod zwischen 44 - 69 % erzielt, in weiteren vier Patienten ein spezifischer Zelltod zwischen 17 und 25 % und bei zwei Patienten lag der Prozentsatz ansprechender Zellen bei unter 10 %. Somit zeigte das Immuntoxin MCSP-ETA' auch einen zytotoxischen Effekt gegen primäre Melanomzellen von Patienten mit fortgeschrittener Erkrankung.

Tabelle 7: Zytotoxische Aktivität von MCSP-ETA' gegen primäre Melanomzellen

| *Patient* | Geschlecht | Alter | Stadium* | entfernte Metastasen* | %MCSP§ | ΔMFI‡ | % spezifischer Zelltod† | | | |
|---|---|---|---|---|---|---|---|---|---|---|
| | | | | | | | 24h | 48h | 72h | 96h |
| *1* | weiblich | 32 | VI | M1c | 94 | 84 | 2 | 16 | 14 | 21 |
| *2* | weiblich | 77 | VI | M1b | 98 | 320 | 6 | 8 | 14 | 25 |
| *3* | weiblich | 40 | VI | M1c | 64 | 182 | 15 | 26 | 26 | 45 |
| *4* | weiblich | 71 | IIIC | - | 95 | 329 | 12 | 27 | 39 | 44 |
| *5* | männlich | 30 | VI | M1c | 98 | 475 | 3 | 4 | 3 | 8 |
| *6* | männlich | 30 | VI | M1a | 94 | 425 | 5 | 4 | 7 | - |
| *7* | weiblich | 67 | IIIC | - | 99 | 504 | 12 | 19 | 35 | 55 |
| *8* | männlich | 40 | VI | M1c | 99 | 627 | 6 | 39 | 57 | 69 |
| *9* | weiblich | 61 | VI | M1c | 14 | 11 | 2 | 11 | 14 | 17 |
| *10* | weiblich | 59 | VI | M1c | 97 | 507 | 3 | 4 | 20 | - |

* nach: Balch *et al.,* 2001
§ Prozent MCSP-positive Zellen zu Beginn der Behandlung
‡ Mittlere Fluoreszenzaktivität über dem Hintergrund
† Prozent toter Zellen über dem Hintergrund

Kooperativität zwischen MCSP-ETA' und Cyclosporin A: Ein viel diskutierter Kritikpunkt bei der Verwendung des *Pseudomonas* Exotoxin A als Effektordomäne eines Immuntoxins ist die hohe Immunogenität nach Applikation im Patienten. Nach mehrmaliger Gabe eines Exotoxin A-abgeleiteten Immuntoxins entwickelt der Patient neutralisierende Antikörper gegen das bakterielle Toxin, was eine Weiterführung der Therapie unmöglich macht (Roscoe, DM. et al., Eur. J. Immunol. 27:1459-1468 (1997); Kreitman, RJ. et al., J. Clin. Oncol. 18:1622-1636 (2000)). Cyclosporin A (CsA) ist ein aus Schlauchpilzen isoliertes, zyklisches Peptid aus 11 Aminosäuren. Es wird in der Klinik als Immunsuppressivum eingesetzt. Neben den immunsupprimierenden Eigenschaften wurde jüngst gezeigt, dass CsA apoptotische Wirkung gegen Melanomzellen induziert (Ciechomska, I. et al., Int. J. Cancer 117:59-67 (2005)).

Zur Untersuchung eines potentiellen synergistischen Effekts zwischen CsA und MCSP-ETA', wurden A2058-Zellen mit steigender Konzentration an CsA, sowie mit CsA in Kombination mit einer konstanten Konzentration MCSP-ETA' inkubiert. Nach 72 h wurde der Anteil toter Zellen durchflusszytometrisch mittels hypotoner PI-Färbung analysiert (siehe Figur 18).

Cyclosporin A löste in den Melanomzellen ab einem Konzentrationsbereich von 10 - 25 μg/ml den Zelltod aus. In Kombination mit 100 ng/ml MCSP-ETA' war der zytotoxische Effekt verstärkt. Der kooperative Index (CI), berechnet aus dem Quotienten der Summe der jeweiligen Einzelbehandlung und der Kombinationsbehandlung, bei den CsA Konzentrationen 1, 5 und 10 μg/ml lag zwischen 0,56 und 0,66, was für einen stark synergistischen zytotoxischen Effekt von CsA und MCSP-ETA' spricht. Um auszuschließen, dass dieser Effekt mit der MCSP Bindung und den damit ausgelösten intrazellulären Signalen zusammenhängt, wurden die Zellen mit CsA und dem parentalen Antikörper anstelle des Immuntoxins inkubiert. Hier war kein veränderter zytotoxischer Effekt im Vergleich zur alleinigen Gabe von CsA messbar.

Nachdem der Synergismus zwischen MCSP-ETA' und CsA an Zellen der über lange Zeit kultivierten, Melanom-abgeleiteten Zelllinie A2058 gezeigt worden war, sollte überprüft werden ob auch bei primären Melanomzellen ein ähnlicher Effekt messbar war. Hierfür wurden die Tumorzellen des Patienten 4 als Zielzellen untersucht. Die Zellen wurden bei 37°C mit 5 μg/ml bzw. 10 μg/ml CsA allein, mit 1 μg/ml MCSP-ETA' allein sowie mit CsA und MCSP-ETA' in Kombination inkubiert. Nach 48 h erfolgte die durchflusszytometrische Auswertung mittels hypotoner PI-Färbung (siehe Figur 19).

Wie bei Zellen der Melanomzelllinie A2058 wurde auch bei primären Melanomzellen von Patient 4 der zytotoxische Effekt von MCSP-ETA' durch CsA angehoben. Während die eingesetzten CsA Konzentrationen bei alleiniger Gabe keinen zytotoxischen Effekt ausübten, wurde der Zelltod in Verbindung mit MCSP-ETA' synergistisch verstärkt, wie die CI Werte von 0,65 zeigten.

Bestimmung der Serumstabilität von MCSP-ETA' *in vitro*: Ein wichtiger Parameter eines zu Therapiezwecken entwickelten Immuntoxins ist dessen Stabilität in humanem Serum. Zur Untersuchung dieser Eigenschaft wurde das Immuntoxin über 24, 48, 72 und 96 h bei 37°C in humanem Serum *in vitro* inkubiert. Anschließend wurde die verbliebene Bindefähigkeit an Antigen-positiven Zellen durchflusszytometrisch ermittelt. Ein Ansatz wurde unmittelbar vor der Vermessung mit Serum versetzt und dessen gemessene mittlere Fluoreszenzintensität wurde als 100% Bindeaktivität

definiert. Figur 20 zeigt den zeitlichen Abfall der Bindefähigkeit von MCSP-ETA' nach Inkubation in humanem Serum *in vitro*.

Die ermittelte Halbwertszeit der Bindefähigkeit des Immuntoxins MCSP-ETA' nach Inkubation in humanem Serum bei 37°C war 11 ± 2 h. Dieser Wert ist im Vergleich mit anderen in der Arbeitsgruppe entwickelten scFv-ETA' Immuntoxinen relativ niedrig. So zeigte CD7-ETA' eine Halbwertszeit von 70 h und CD33-ETA' wies nach 96 h noch über 80% Bindeaktivität auf (Arbeitsgruppe Fey; N. Rohner, M. Schwemmlein, unpublizierte Daten). Diese Daten zeigten, dass eine derart niedrige Halbwertszeit nicht notwendigerweise bei scFv-ETA' Fusionsproteinen auftritt. Es lag nahe, dass diese geringe Stabilität wahrscheinlich auf das MCSP-gerichtete scFv-Antikörperfragment zurückzuführen ist. Daher wird im folgenden Teil dieser Arbeit auf die verschiedenen Ansätze zur Verbesserung des MCSP-gerichteten scFv-Fragments eingegangen.

Bestimmung der Serumstabilität von MCSP *in vitro*: Die unbefriedigende Halbwertszeit von MCSP-ETA' nach Inkubation in humanem Serum ließ vermuten, dass bereits das MCSP-gerichtete Antikörperfragment eine eingeschränkte Stabilität in humanem Serum aufweisen würde. Um diese Hypothese zu prüfen wurde die Serum-Halbwertszeit des gereinigten scFv-Fragments MCSP (SEQ ID NO:6) analog der Vorgehensweise bei der Stabilitätsmessung des Immuntoxins analysiert. Figur 21 zeigt die Auswertung der Stabilitätsmessungen des scFv-Fragments MCSP.

Die Halbwertszeit der Bindungsaktivität des scFv-Fragments MCSP nach Inkubation in humanem Serum mit 25 ± 5 h deutlich unter den Werten anderer, in der Arbeitsgruppe charakterisierter scFv-Antikörper mit der hier verwendeten Orientierung der variable Ketten ($V_L$-*linker*-$V_H$). Sowohl das scFv-Fragment gegen CD7, als auch das scFv-Fragment K132 gegen CD33 wiesen nach 96 h Inkubation in humanem Serum eine Bindeaktivität von über 80% auf (Fey; N. Rohner, M. Schwemmlein, unpublizierte Daten).

Beispiel 2 Konstruktion und Charakterisierung des scFv-Fragments $MCSP_{HL}$: In Grosse-Hovest, L. et al., Eur. J. Immunol. 33:1334-1340 (2003) ist ein aus dem 9.2.27 Hybridom subkloniertes scFv-Fragment im Kontext des bispezifischen scFv (bsscFv) Antikörpers [MCSPxCD28] beschrieben, das im Blut transgener Rinder hergestellt wird, was auf eine relativ hohe Stabilität hindeutet. In diesem Konstrukt sind die variablen Domänen des gegen MCSP gerichteten scFv-Fragments in umgekehrter Orientierung ($V_H$-*linker*-$V_L$) angeordnet und durch einen 15 Aminosäuren *linker* miteinander verknüpft.

Ausgehend vom scFv-Fragment MCSP (SEQ ID NO:6) wurde ein Antikörperfragment mit umgekehrter Orientierung der variablen Ketten und einem nur 15 Aminosäuren $(G_4S)_3$-*linker* konstruiert. Zusätzlich wurden ausgehend von der Aminosäuresequenz des Parentalantikörpers 9.2.27 acht Punktmutationen in den distalen Bereichen der variablen Ketten rückgängig gemacht. Diese Mutationen entstanden wahrscheinlich methodenbedingt während der Generierung des scFv-Fragments aus dem Hybridom (Krebber, A. et al., J. Immunol. Methods 201:35-55 (1997)). Bei den durchgeführten Rückmutationen handelte es sich um Q3K, K5Q, S108T und P112S in der variablen schweren Kette sowie V3E, M4L, S100G und T106L in der variablen leichten Kette (Position der Aminosäuren anhand der Kabat-Datenbank, die ein "Raster" für alle scFvs mit allen möglichen Aminosäurepositionen darstellt), was den Punktmutationen Q3K, K5Q, S116T und P120S in SEQ ID NO:1 und V3E, M4L, S104G und T110L in SEQ ID NO:3 entspricht. Figur 22 zeigt einen schematischen Überblick über die durchgeführten Änderungen bei der Konstruktion des scFv-Fragments $MCSP_{HL}$, die Sequenz des exprimierten Proteins ist in SEQ ID NO:5 gezeigt.

Zur Klonierung des scFv-Fragments $MCSP_{HL}$ wurden zunächst die variablen Ketten mittels PCR ausgehend vom scFv MCSP aus dem Vektor pAK400 MCSP getrennt amplifiziert. Die verwendeten Primer wiesen hierfür die nötigen Fehlpaarungen auf, um die kodierenden Bereiche der auszutauschenden Aminosäuren in den distalen Bereichen der variablen Ketten zu verändern. Zusätzlich beinhalteten die entsprechenden Primer jeweils einen Teil des 15 Aminosäuren langen $(G_4S)_3$-*linkers*. Die amplifizierten variablen Ketten wurden getrennt über NcoI und BamHI im Fall der schweren Kette, sowie über BamHI und AvaI im Fall der leichten Kette, in den Vektor pASK4-Strep-His-4G7-G4C kloniert. Anschließend wurde aus dem Vektor pASK4-Strep-His-MCSP$v_H$-G4C die variable schwere Kette als NcoI/BamHI-Kassette in den Vektor pASK4-Strep-His-MCSP$v_L$-G4C ligiert. Die cDNA des scFv-Fragments $MCSP_{HL}$ wurde abschließend als SfiI-Kassette in den Expressionsvektor pAK400 eingesetzt.

Expression und Reinigung des scFv-Fragments $MCSP_{HL}$: Die Expression des scFv-Fragments $MCSP_{HL}$ erfolgte im *E. coli* Stamm HB21/51. Anschließend wurden die Bakterien geerntet und es erfolgte ein osmotischer Aufschluss des Periplasmas. Nach dem Pelletieren der Zelltrümmer durch Zentrifugation wurden die Pufferbedingungen des Überstands durch Dialyse an die Reinigung über den Hexahistidin-tag angepasst. Die Reinigung erfolgte affinitätschromatographisch mit der Ni-Chelat Methode. Aufgrund der unbefriedigenden Reinheit nach dem ersten Reinigungsschritt wurde ein zweiter Reinigungsschritt mittels Ionen-Austausch Chromatographie durchgeführt. Bei einem pH-Wert von 6,5 band das positiv geladene scFv-Fragment (isoelektrischer Punkt 7,2) an eine Kationen-Austauscher Säule und das Protein wurde anschließend mit steigender Konzentration an NaCl von 30 - 750 mM eluiert. Figur 23 zeigt die Reinigung des scFv-Fragments $MCSP_{HL}$.

Nach der Anreicherung des Proteins mit Ni-NTA-Agarose war eine Bande in der zu erwartenden Größe von ca. 29 kDa im Coomassie-gefärbten SDS-PAA Gel zu sehen. Allerdings betrug der Anteil dieses Proteins nur schätzungsweise 50% am Gesamtprotein. Nach der anschließenden Kationenaustausch-Chromatographie sieht man im

Coomassie-gefärbten SDS-PAA Gel eine relativ reine Proteinfraktion mit einem dominanten Protein bei ca. 30 kDa. Dieses Protein reagierte im Western-Transfer-Experiment mit Antikörpern spezifisch gegen den Hexahistidin-tag. Somit wurde das Protein erfolgreich exprimiert und gereinigt. Die erhaltene Ausbeute lag bei ca. 300-400 µg pro Liter Bakterienkultur.

Analyse der Bindeeigenschaften des scFv-Fragments $MCSP_{HL}$: Nach der Reinigung des scFv-Fragments $MCSP_{HL}$ wurde untersucht, ob die Antigen-spezifische Bindefähigkeit durch die Änderungen im Molekül erhalten geblieben ist. Hierzu wurden MCSP-positive A2058 Zellen mit dem scFv-Fragment $MCSP_{HL}$ inkubiert. Anschließend erfolgte die Detektion über einen murinen *AlexaFluor555*-gekoppelten anti-Pentahistidin Antikörper. Der Nachweis der spezifischen Bindung erfolgte über Blockier-Experimente mit dem parentalen Antikörper 9.2.27 sowie einem nicht relevanten Antikörper vom gleichen Isotyp (14G2a) in jeweils 10 molarem Überschuss, die 30 min vor Zugabe des scFv-Fragments mit den Zellen inkubiert wurden. Figur 24 zeigt die durchflusszytometrische Auswertung dieser Experimente. Das Antikörperfragment $MCSP_{HL}$ band an MCSP-positiven A2058 Zellen. Diese Bindung war Antigen-spezifisch, da der Parentalantikörper 9.2.27, nicht jedoch der nicht-relevanter Antikörper 14G2a, die Bindung blockierte. Die Umstrukturierung des gegen MCSP gerichteten Antikörperfragments hat somit die Bindungsspezifität nicht beeinträchtigt.

Als nächstes wurde untersucht, ob die Veränderungen des scFv-Fragments die Bindungsstärke an MCSP beeinflusst hatten. Hierzu wurde die Equilibriumskonstante $K_D$ durchflusszytometrisch ermittelt. Figur 25 zeigt die Sättigungskurven zur Bestimmung der halbmaximalen Sättigungskonzentration des scFv-Fragments $MCSP_{HL}$ im Vergleich zum ursprünglichen scFv-Fragment MCSP.

Die ermittelte Equilibriumskonstante des scFv-Fragments $MCSP_{HL}$ lag bei 1,65 ± 0,2 nM. Im Vergleich zum ursprünglichen MCSP-gerichteten scFv-Fragment mit einer $K_D$ von 23 ± 2,3 nM hat sich die Affinität um etwa den Faktor 14 verbessert. Bestimmung der Serumstabilität des scFv-Fragments $MCSP_{HL}$ *in vitro*: Das Ziel bei der Umstrukturierung des MCSP-gerichteten Antikörperfragments war eine deutliche Verbesserung der Bindeaktivität nach Inkubation in humanem Serum. Nachdem die Spezifität der Bindung sowie eine sehr guten Affinität nachgewiesen worden waren, wurde die Stabilität in humanem Serum, wie bereits beschrieben, ermittelt (siehe Figur 26). Die Bestimmung der Serumstabilität ergab für den $MCSP_{HL}$ scFv nach 96 h Inkubation eine Bindeaktivität von ca. 70%. Der ursprüngliche scFv hatte eine Halbwertszeit von ca. 25 h (siehe Figur 21). Aufgrund der verbesserten Eigenschaften des neuen scFv-Fragments wurde das Immuntoxin $MCSP_{HL}$-ETA' kloniert und funktionell getestet.

Herstellung des Immuntoxins $MCSP_{HL}$-ETA': Eine sehr unvorteilhafte Eigenschaft des Immuntoxins MCSP-ETA' im Hinblick auf eine potentielle klinische Anwendung war die eingeschränkte Halbwertszeit der Bindestabilität von 11 h nach Inkubation in humanem Serum *in vitro* (siehe Figur 20). Als Ursache für diesen niedrigen Wert wurde die vergleichsweise geringe Stabilität des MCSP-gerichteten scFv-Fragments angenommen. Mit der beschriebenen Variante scFv $MCSP_{HL}$ stand nun ein Antikörperfragment mit sehr aussichtsreichen physikalischen Eigenschaften zur Verfügung.

Um das Immuntoxin $MCSP_{HL}$-ETA' mit Hilfe des scFv-Fragments $MCSP_{HL}$ herzustellen, wurde aus dem Vektor, pASK4-Strep-His-$MCSP_{HL}$-G4C der scFv $MCSP_{HL}$ als SfiI-Kassette ausgeschnitten und in den ebenso verdauten Vektor pASK6-linker (s.o.) eingesetzt. Abschließend wurde aus dem Vektor pASK6-$MCSP_{HL}$-linker das Fragmente welches die beiden *tags,* den $MCSP_{HL}$-scFv sowie den 20AS $(G_4S)_4$-*linker* enthält über NotI und XbaI in den Vektor pET27b-Strep-His-MCSP-ETA'-KDEL vor die codierende Sequenz des verkürzten *Pseudomonas* Exotoxin A ligiert. Im fertigen Vektor pET27b-Strep-His-$MCSP_{HL}$-ETA'-KDEL wurde die codierende Sequenz des Immuntoxins durch Sequenzierung validiert. Figur 27 zeigt das Immuntoxin $MCSP_{HL}$-ETA' schematisch, die Sequenz des exprimierten Fusionsproteins ist in SEQ ID NO:7 gezeigt.

Expression und Reinigung des Immuntoxins $MCSP_{HL}$-ETA': Die bakterielle Expression des Immuntoxin $MCSP_{HL}$-ETA' erfolgte nach den gleichen Protokollen wie bei dem Immuntoxin MCSP-ETA' (siehe Kapitel 4.2.2). Die Reinheit sowie die Spezifität des gereinigten Proteins wurde im Coomassie-gefärbten SDS-PAA Gel bzw. im Western-Transfer-Experiment überprüft. Zusätzlich wurden potentielle Aggregationstendenzen mittels Größenausschluss-Chromatographie analysiert (siehe Figur 28).

Das Immuntoxin $MCSP_{HL}$-ETA' zeigte im Coomassie-gefärbt SDS-PAA Gel wenig Kontaminationen nach einmaliger Anreicherung mit Streptactin-Sepharose. Sowohl im gefärbten SDS-PAA Gel, als auch im spezifischen Western-Transfer-Experiment zeigte sich eine Bande mit der zu erwartenden Größe von ca. 70 kDa. Das Elutionsprofil nach der Größenausschluss-Chromatographie zeigte ein einzelnes Maximum in der zu erwartenden Größe. Somit konnte davon ausgegangen werden, dass das gereinigte Immuntoxin $MCSP_{HL}$-ETA in monomerer Form vorliegt. Die erhaltene Ausbeute lag bei ca. 60-80 µg Fusionsprotein pro Liter Bakterienkultur. Analyse der Bindungseigenschaften von $MCSP_{HL}$-ETA': Im Anschluss an die Reinigung des Immuntoxins wurde die spezifische Bindung an MCSP-positiven Zellen überprüft. Hierzu wurden A2058 Zellen mit $MCSP_{HL}$-ETA' inkubiert. Der Nachweis der spezifischen Bindung erfolgte über Blockier-Experimente mit dem Antikörper 9.2.27 in 10-molarem Überschuss. Zusätzlich wurde die Equilibriumskonstante $K_D$ des neu konstruierten Immuntoxins ermittelt. Figur 29 zeigt die durchflusszytometrische Auswertung dieser Experimente.

Das Immuntoxin band, wie bereits das Antikörperfragment $MCSP_{HL}$, spezifisch an MCSP. Der ermittelte

$K_D$-Wert des Immuntoxins $MCSP_{HL}$-ETA' war 11,0 ± 0,2 nM. Im Vergleich zum ursprünglichen MCSP-gerichteten Immuntoxin mit einem $K_D$-Wert von 128 ± 28 nM (Figur 12) hatte sich die Affinität durch den Austausch des scFv-Fragments um etwa den Faktor 12 verbessert.

Bestimmung der Serumstabilität von $MCSP_{HL}$-ETA' in vitro: Der Grund für die Konstruktion eines abgewandelten MCSP-gerichteten Immuntoxins lag in der unbefriedigenden Serumstabilität des Prototyps MCSP-ETA'. Das mit dem vielversprechenden scFv-Fragment $MCSP_{HL}$ konstruierte Immuntoxin wurde daher auf seine Bindeaktivität nach Inkubation in humanem Serum untersucht (siehe Figur 30).

Die Bestimmung der Serumstabilität ergab für das Immuntoxin $MCSP_{HL}$-ETA' nach 96 h Inkubation eine Bindeaktivität von über 70%. Das ursprüngliche Immuntoxin MCSP-ETA' hatte eine Halbwertszeit von ca. 11 h (siehe Figur 20). Der Austausch des scFv-Antikörperfragments hatte die gewünschte Verbesserung der Serumstabilität mit sich gebracht. Neben der Stabilität ist v.a. die Affinität eines derart konstruierten Immuntoxins von großer Bedeutung für dessen zytotoxische Aktivität. Aufgrund der sehr drastischen Verbesserung der Affinität des Immuntoxins $MCSP_{HL}$-ETA' um mehr als den Faktor zehn wurde das zytotoxische Potential mit dem Ausgangskonstrukt vergleichend charakterisiert.

Anti-Tumor Aktivität von MCSP-ETA' und $MCSP_{HL}$-ETA' in vitro: Zunächst wurde der Einfluss beider Immuntoxine auf die Zellproliferation untersucht. Mit Hilfe eines Zellproliferations Tests wurde die Teilungsaktivität behandelter Zellpopulationen gemessen. A2058 Zellen wurden mit unterschiedlichen Konzentrationen an Immuntoxin inkubiert. Nach 72 h wurden 20 µl MTS/PMS Lösung zugegeben und nach vierstündiger Inkubation bei 37°C wurde die Absorption bei λ=490 nm photometrisch vermessen. Figur 31 zeigt die gemessenen Dosis-abhängigen Kurven, aus denen die halbmaximalen inhibitorischen Konzentrationen ($IC_{50}$) ermittelt wurden.

Beide Immuntoxine bewirkten eine konzentrationsabhängige Reduktion der Zellviabilität gemessen an der Zelldichte nach 72-stündiger Inkubation mit den Fusionsproteinen. Die ermittelten $IC_{50}$ Konzentrationen der Immuntoxine waren 254,6 ± 17,7 pM für MCSP-ETA' und 52,7 ± 17,4 pM für $MCSP_{HL}$-ETA'. Die beiden gemessenen $IC_{50}$-Werte unterschieden sich dabei hoch signifikant ($p<0,01$). Das Immuntoxin mit dem verbesserten Lesekopf wies somit eine um etwa den Faktor 5 geringere halbmaximale inhibitorische Konzentration auf.

Nach der Analyse des anti-proliferativen Potentials der MCSP-gerichteten Immuntoxine wurde deren zytotoxisches Potential vergleichend gemessen. Hierfür wurden MCSP-positive A2058 Zellen mit steigenden Konzentrationen beider Immuntoxine inkubiert und nach 72 h wurde der Anteil toter Zellen mittels hypotoner PI-Färbung durchflusszytometrisch bestimmt. Figur 32 zeigt die dosisabhängige Wirkung beider Immuntoxine gegen MCSP-positive A2058 Melanomzellen.

$MCSP_{HL}$-ETA' induzierte konzentrationsabhängig den Zelltod in A2058 Zellen. Die ermittelten halbmaximalen effektiven Konzentrationen ($EC_{50}$) der Immuntoxine waren 652,0 ± 16,2 pM für MCSP-ETA' und 150,0 ± 17,2 pM für $MCSP_{HL}$-ETA'. Wie die $IC_{50}$-Werte unterschieden sich auch die $EC_{50}$-Werte hoch signifikant ($p<0,01$). Erneut wies das Immuntoxin mit dem verbesserten Lesekopf eine höhere anti-Tumoraktivität im Vergleich zu MCSP-ETA' auf. Der ermittelte $EC_{50}$-Wert von $MCSP_{HL}$-ETA' war ca. 4,5-fach niedriger als bei MCSP-ETA'.

In der nachfolgenden Tabelle 8 ist ein Überblick über die in dieser Arbeit charakterisierten MCSP-gerichteten Moleküle hinsichtlich der Ausbeute nach bakterieller Expression, der Affinität für das Zielantigen MCSP, der Bindeaktivität nach Inkubation in humanem Serum *in vitro* sowie des zytotoxischen Potentials der Immuntoxine gegeben. Die Optimierung des gegen MCSP gerichteten Immuntoxins bewirkte neben einer deutlichen Verlängerung der Halbwertszeit der Bindeaktivität nach Inkubation in humanem Serum vor allem eine gesteigerte zytotoxische Aktivität. Nicht zuletzt durch die Verbesserung dieser Parameter ist das Immuntoxin ein interessanter Kandidat für weitere vorklinische Untersuchungen im Tiermodell in Richtung eines potentiellen Einsatzes bei der Behandlung von Patienten mit MM sowie möglicherweise weiterer MCSP-positiver maligner Erkrankungen.

Tabelle 8: Überblick über die Eigenschaften der charakterisierten MCSP-gerichteten Moleküle

| *rekombinantes Protein* | Ausbeute [µg/ml] | $K_D$-Wert [nM] | Serumhalbwertszeit *in vitro* [h] | $IC_{50}$* [pM] | $EC_{50}$* [pM] |
|---|---|---|---|---|---|
| *MCSP-scFv* | 100 - 150 | 23,3 ± 2,3 | 25 ± 5 | - | - |
| *$MCSP_{HL}$-scFv* | 300 - 400 | 1,65 ± 0,2 | > 70% nach 96 | - | - |
| *MCSP-ETA'* | 25 - 35 | 128 ± 28 | 11 ± 2 | 254,6 ± 17,7 | 652,0 ± 16,2 |
| *$MCSP_{HL}$-ETA'* | 60 - 80 | 11,0 ± 0,8 | > 70% nach 96 | 52,7 ± 17,4 | 150 ± 17,2 |

* Anti-Tumor Aktivität gegenüber A2058-Zellen

Beispiel 3: MCSP-spezifische chimärische TCRs

MCSP als Tumoroberflächenantigen kann als ebenfalls als Angriffspunkt für chimärische Rezeptoren verwendet werden. Mehrere MCSP-spezifische chimärische TCR-Konstrukte wurden entworfen, die entweder aus dem MCSP-spezifischen MCSP-Ih-scFv von Beispiel 1 oder dem stabilitäts- und affinitätsverbesserten MCSP-hl-scFv von Beispiel 2 bestehen, das mit den Transmembran- und signalübermittelnden Domänen von CD3ζ oder den Transmembran- und signalübermittelnden Domänen von CD28 und der signalübermittelnden Domäne von CD3ζ fusioniert ist (Figur 35; SEQ ID Nos:22, 24, 26 und 28).

Die Konstrukte, die das ursprüngliche MCSP-Ih-scFv enthalten, wurden analog der von Hombach, A. et al. in J. Immonol 167:6123-31 (2001) und in Gut 55:1156-64 (2006) publizierten Methode einem mRNA-Format erzeugt und wurden wie in Birkholz, k. et al. Gene Therapy, 1-9 (2009) beschrieben durch Elektroporation in ex vivo erzeugte T-Zellen eingeschleust (Figur 36). Die Expression beider MCSP-LHscFv-Rezeptoren (einer mit CD3ζ- und einer mit CD28-CD3ζ-Domänen) wurde sowohl auf der Oberfläche von transfizierten CD4-positiven als auch CD8-positiven T-Zellen nachgewiesen, während das Konstrukt, das eine von CD28 abgeleitete Domäne enthielt, besser und länger exprimiert wurde (Figur 36), was auf eine stabilisierende Funktion dieser Domäne hinweist.

Um die transfizierten T-Zellen funktionell zu testen, wurden diese mit MCSP-positiven Tumorzellen inkubiert, und die Produktion von Interferon-γ (Figur 37A) und anderer Cytokine (nicht gezeigt) wurde bestimmt. Um die Spezifität der Erkennung nachzuweisen, wurden eine MCSP-transgene Zelllinie und ihre MCSP-negative Stammlinie verwendet (Figur 37A). Die MCSP-positiven Zellen wurden erkannt, die MCSP-negativen aber nicht (Figur 37A). T-Zellen, die das Konstrukt trugen, das die von CD28 abgeleitete Domäne enthielt, erzeugten eine wesentlich größere Menge der Cytokine, zeigten aber einen unspezifischen Hintergrund (Figur 37A). IL-2 und TNF wurden in einem ähnlichen Muster sezerniert (Daten nicht gezeigt).

Weiterhin wurde getestet, ob die umprogrammierten T-Zellen auch eine MCSP-positive Tumorzelllinie lysieren können. Wie in Figur 37B gezeigt ist, waren CD8-positive T-Zellen, die einer Elektroporation mit RNA, die den chimärischen TCR (der die CD28-Domäne enthielt) codiert, unterzogen worden waren, in der Lage, die Tumorzelllinie A375M abzutöten. Einer Scheinelektroporation unterzogene T-Zellen lysierten diese Targetzelllinie nicht (Figur 37B).

**Sequenzprotokoll - Freier Text**

| Seq ID NO: | Bezeichnung |
|---|---|
| 1 | MSCP $V_H$ |
| 2 | modifiziertes MSCP $V_H$ |
| 3 | MSCP $V_L$ |
| 4 | modifiziertes MSCP $V_L$ |
| 5 | scFv-MSCPHL (AS 1-5 Leader, AS 6-253 scFv, AS 254-265 His-Tag) |
| 6 | scFv-MSCP (AS 1-5 Leader, AS 6-258 scFv, AS 259-269 His-Tag) |
| 7 | Fusionsprotein scFv-MSCPHL-ETA' (AS 29-276 scFv, 302-666 ETA') |
| 8 | Fusionsprotein scFv-MSCP-ETA' (AS 29-281 scFv, 306-670 ETA') |
| 9-21 | Primer |
| 22/23 | MCSPhl CD28/TCRζ (in SEQ ID NO:22: bp 1-74 Leader, bp 75-848 scFv, bp 849-1565 humaner Fc Teil, bp 1566-1790 CD28 TM Bereich, bp 1791-2108 Zeta-Kette) |
| 24/25 | MCSPhl TCRζ (In SEQ ID NO:24: bp 1-74 Leader, bp 75-848 scFv, bp 849-1564 humaner Fc Teil, bp 1565-1646 TM Bereich Zeta-Kette; bp 1647-1964 Zeta-Kette) |
| 26/27 | MCSP CD28/TCRζ (in SEQ ID NO:26: bp 1-74 Leader, bp 75-860 scFv, bp 861-1586 humaner Fc Teil, bp 1587-1811 CD28 TM Bereich, bp 1812-2129 Zeta-Kette) |
| 28/29 | MCSP TCRζ (in SEQ ID NO:28: bp 1-74 Leader, bp 75-860 scFv, bp 861-1574 humaner Fc Teil, bp 1575-1655 TM Bereich Zeta-Kette, bp1656-1973 Zeta-Kette |

## Patentansprüche

1. Gegen das Melanom-assoziierte Chondroitinsulfat Proteoglykan (MCSP)-gerichtete scFv-Antikörperfragment umfassend die variablen Domänen des gegen MCSP gerichteten scFv-Fragments in der Orientierung $V_H$-*linker*-$V_L$, wobei $V_H$ die Aminosäresequenz von SEQ ID NOs:1 oder 2 und $V_L$ die Aminosäresequenz von SEQ ID NOs:3 oder 4 oder die entsprechenden Sequenzen, die einer oder mehreren Punktmutationen aufweisen, umfassen und *linker* eine kovalente Bindung oder ein Oligopeptid ist.

2. MCSP scFv-Antikörperfragment nach Anspruch 1, wobei die Punktmutationen konservative Aminosäurenaustausche sind und/oder die Zahl der Punktmutationen pro variabler Domäne 1 bis 5 beträgt, und wobei vorzugsweise (i) $V_H$ in einer oder mehreren der Positionen 3, 5, 116 und 120, bezogen auf die Sequenz von SEQ ID NO:1, Punktmutationen aufweist und besonders bevorzugt eine Sequenz von SEQ ID NO:1 ist, die eine oder mehrere der Punktmutationen Q3K, K5Q, S116T und P120S aufweist, und

   (ii) $V_L$ in Positionen 3, 4, 104 und 110, bezogen auf die Sequenz von Seq ID NO:3, Punktmutationen aufweist und besonders bevorzugt eine Sequenz von SEQ ID NO:3 ist, die eine oder mehrere der Punktmutationen V3E, M4L, S104G und T110L aufweist.

3. MCSP scFv-Antikörperfragment nach Anspruch 1 oder 2, wobei

   (i) der *linker* ein hydrophiles Oligopeptid mit 1 bis 30, vorzugsweise 10 bis 20 Aminosäureresten ist und das insbesondere im Wesentlichen aus Aminosäreresten ausgewählt aus Glycin, Alanin und Serin besteht, wobei ein $(G_4S)_n$-Linker (worin n eine ganze Zahl von 1 bis 5 ist) besonder bevorzugt ist; und/oder
   (ii) das Antkörperfragment weitere funktionelle Sequenzen einscließlich Leader-, Sekretions- und Reinigungssequenzen wie His-Tags und STREP-Tags aufweist.

4. MCSP scFv-Antikörperfragment nach einem oder mehreren der Ansprüche 1 bis 3, wobei $V_H$ und $V_L$ die in SEQ ID NO:1 oder 2, bzw. 3 oder 4 gezeigten Sequenzen aufweisen und *linker* die Sequenz $(G_4S)_3$ aufweist, wobei ein MCSP Antikörperfragment, das die Sequenz mit den Aminosäureresten 6 bis 254 von SEQ ID NO:5 umfasst, besonders bevorzugt ist.

5. Fusionsprotein umfassend (a) eine Domaine mit einem MCSP scFv-Antikörperfragment nach Anspruch 1 bis 4 und (b) wenigstens eine Domaine mit einem funktionellen Protein- oder Peptidsequenz, die mit der Domaine (a) kovalent verknüpft ist.

6. Fusionsprotein nach Anspruch 5, wobei

   (i) die Domaine (b) ausgewählt ist aus Immunotoxinen einschließlich *Pseudomonas* Exotoxin A (ETA) und Modifikationen und Derivate derselben, Transmembran- und signalübermittelnden Domänen von Oberflächenantigenen einschließlich CD3ζ und CD28 und Modifikationen und Derivate derselben, wobei das Immunotoxin vorzugsweise eine ETA-modifikation mit der Sequenz von 302 bis 670 von SEQ ID NO:7 ist und die Transmembran- und signalübermittelnde Domäne von bp 1566-2108 von SEQ ID NO:22 oder 1565-1969 von SEQ ID NO: 24 codiert wird;
   (ii) die Domainen (a) und (b) direkt oder über ein Linkermölekül miteinander Verknüpt sind, wobei das Likermolekül vorzugsweise ein wie in Anspruch 3 definierter Linker oder ein Fc C Teil eines Antikörper, insbesondere ein Fc C Teil eines humanen IgG, ist.

7. Fusionsprotein nach Anspruch 5 oder 6, wobei das Fusionsprotein die Sequenz von AS 29-666 von SEQ ID NO: 7, AS 27-699 von SEQ ID NO:23 oder AS 27-651 von SEQ ID NO:25 umfasst.

8. Nukleinsäuresequenz die für ein scFv-Antikörperfragment nach einem oder mehreren der Ansprüche 1 bis 4 oder für ein Fusionsprotein nach einem oder mehreren der Ansprüche 5 bis 7 kodiert.

9. Vektor umfassend eine Nukleinsäuresequenz nach Anspruch 8.

10. Zelle, die ein scFv-Antikörperfragment nach einem oder mehreren der Ansprüche 1 bis 4 oder ein Fusionsprotein nach einem oder mehreren der Ansprüche 5 bis 7 aufweist und/oder den Vektor nach Anspruch 9 und/oder die Nukleinsäuresequenz nach Anspruch 8 umfasst.

**11.** Zelle nach Anspruch 10, die eine Expressionszelle wie transformierte oder transfizierte Pro- oder Eukaryontenzelle einschließlich Säugerzellen ist, wobei humane embryonale Stammzellen ausgenommen sind, oder die eine antigenpräsentierende Zelle, wie eine dendritische Zelle, oder eine antigenerkennende Zelle, wie eine T- Zelle, ist, die mit dem Antikörperfragment, dem Fusionsprotein oder mit diese kodierenden Nukleinsäuren oder Vektoren beladen sind.

**12.** Verfahren zur Herstellung

(i) eines scFv-Antikörperfragmente nach einem oder mehreren der Ansprüche 1 bis 4 oder eines Fusionsprotein nach einem oder mehreren der Ansprüche 5 bis 7 umfassend das Kultivieren von Expressionszellen nach Anspruch 11 und Isolieren des scFv-Antikörperfragments oder des Fusionsproteins;
(ii) einer Expressionszellinie nach Anspruch 11 umfassend das Transformieren oder Transfizieren einer Ausgangszelle mit dem Vektor nach Anspruch 9 und/oder der Nukleinsäuresequenz nach Anspruch 8;
(iii) einer antigenpräsentierenden oder -erkennenden Zelle nach Anspruch 11 umfassend das Beladen der Zelle mit einem scFv-Antikörperfragmente nach einem oder mehreren der Ansprüche 1 bis 4 oder ein Fusionsprotein nach einem oder mehreren der Ansprüche 5 bis 7 oder mit diese kodierenden Nukleinsäuren oder Vektoren.

**13.** Pharmazeutische Zusammensetzung, Medikament oder Diagnostische Zusammensetzung umfassend ein scFv-Antikörperfragment nach einem oder mehreren der Ansprüche 1 bis 4, eines Fusionsprotein nach einem oder mehreren der Ansprüche 5 bis 7, eine Nukleinsäresequenz nach Anspruch 8, einen Vektor nach Anspruch 9 oder eine antigenpräsentierende oder -erkennende Zelle nach Anspruch 11.

**14.** Verwendung eines Fusionsproteins nach einem oder mehreren der Ansprüche 5 bis 7 oder einer antigenpräsentierenden oder -erkennenden Zelle nach Anspruch 11 zur Herstellung eines Medikaments zur Behandlung von Tumorerkrankungen.

**15.** Verfahren zur Behandlung von Tumorerkrankungen in einem Patienten, umfassend das Verabreichen einer ausreichenden Menge des Fusionsproteins nach einem oder mehreren der Ansprüche 5 bis 7 oder einer antigenpräsentierenden oder -erkennenden Zelle nach Anspruch 11 an einen Patienten.

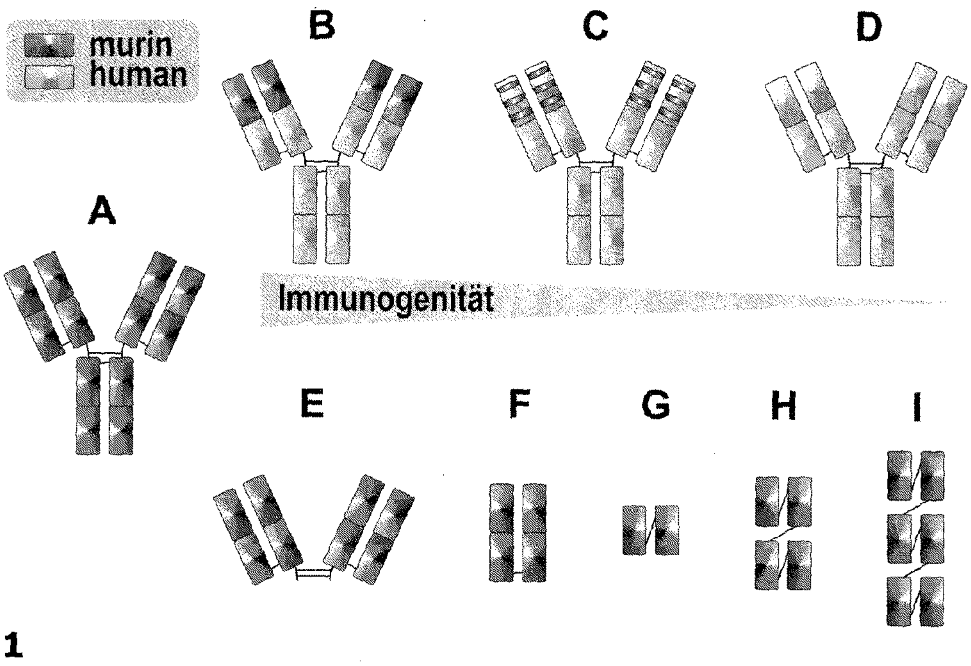
murin
human
A
B
C
D
Immunogenität
E
F
G
H
I

Fig.1

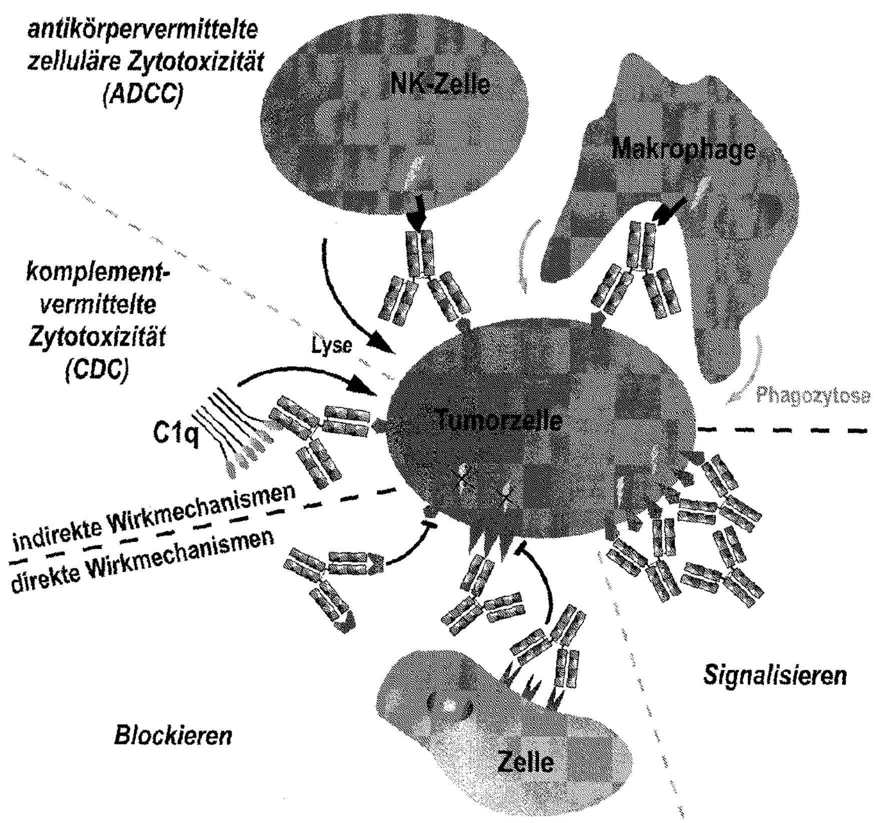
antikörpervermittelte zelluläre Zytotoxizität (ADCC)
NK-Zelle
Makrophage
komplement-vermittelte Zytotoxizität (CDC)
Lyse
C1q
Tumorzelle
Phagozytose
indirekte Wirkmechanismen
direkte Wirkmechanismen
Signalisieren
Blockieren
Zelle

Fig.2

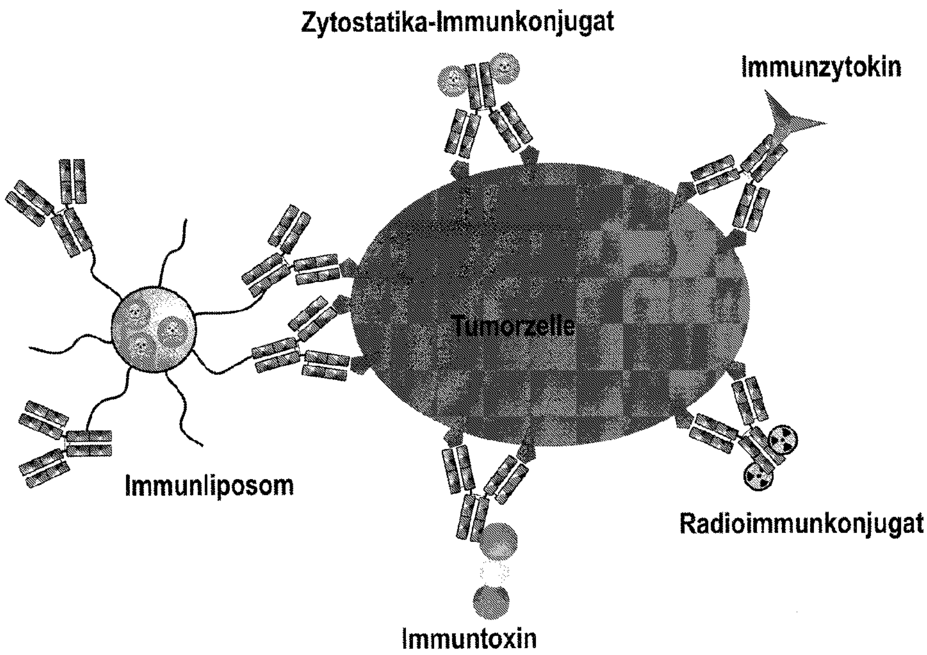
Zytostatika-Immunkonjugat
Immunzytokin
Tumorzelle
Immunliposom
Radioimmunkonjugat
Immuntoxin

**Fig.3**

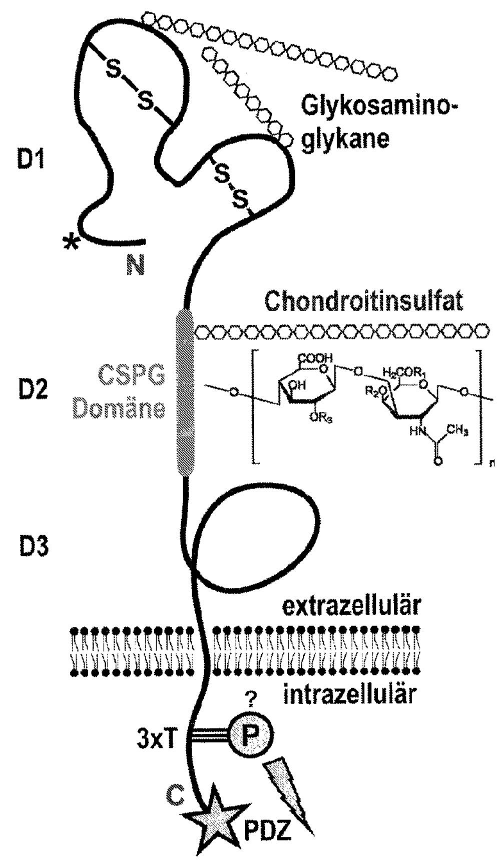
Glykosamino-
glykane
D1
N
Chondroitinsulfat
D2
CSPG
Domäne
D3
extrazellulär
intrazellulär
?
3xT
P
C
PDZ

**Fig.4**

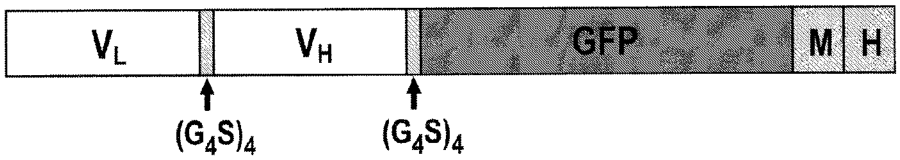


Fig.5

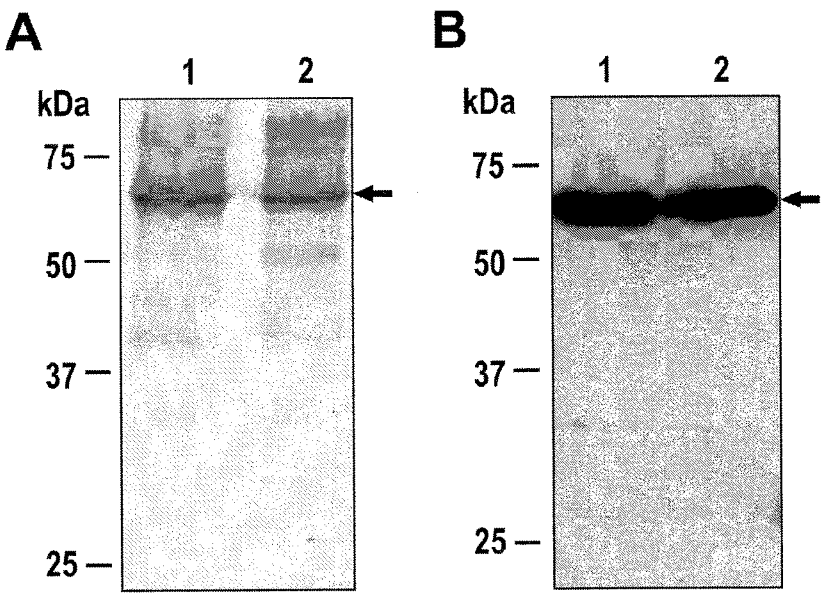


Fig.6

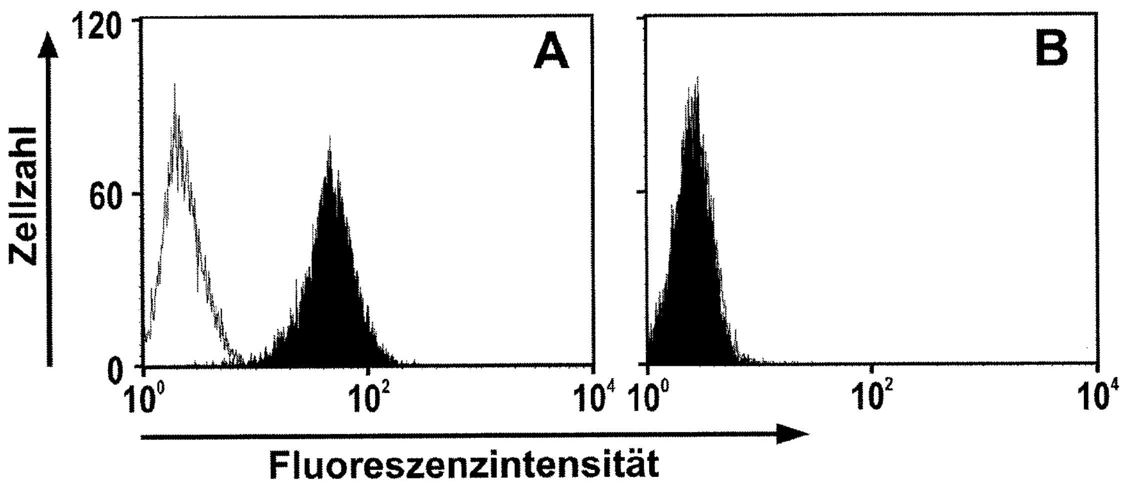


Fig.7

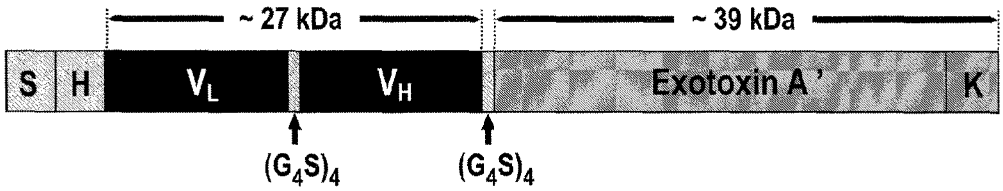


Fig.8

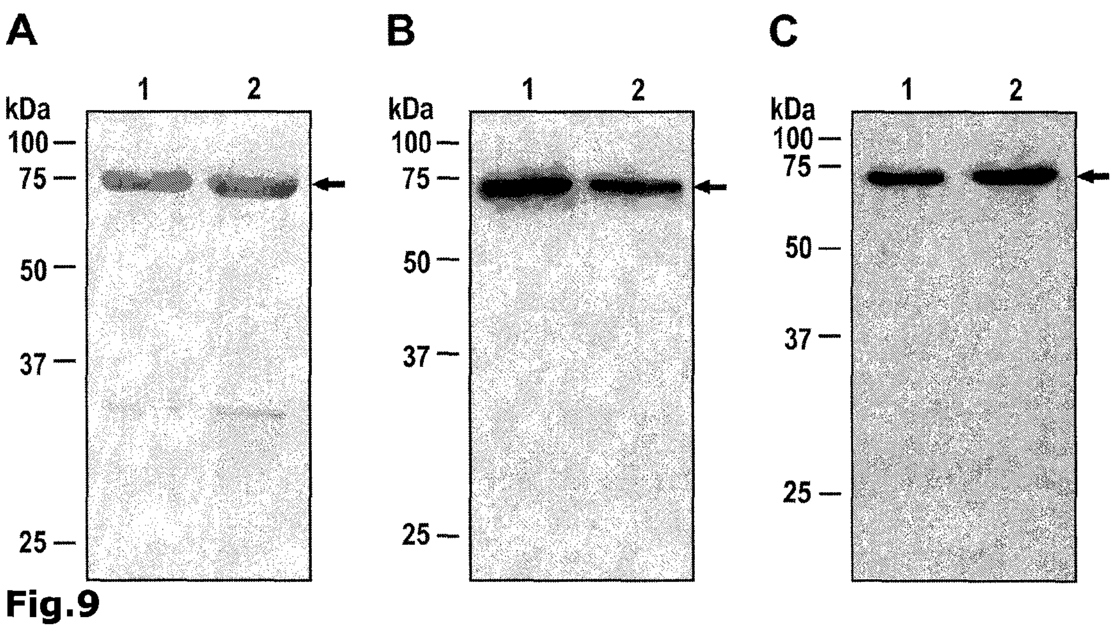


Fig.9

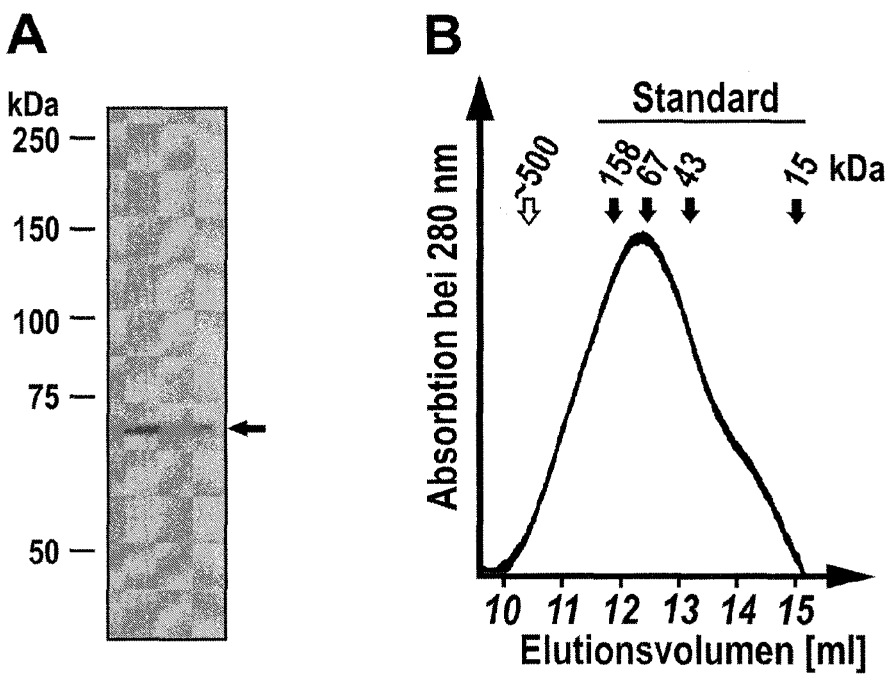


Fig.10

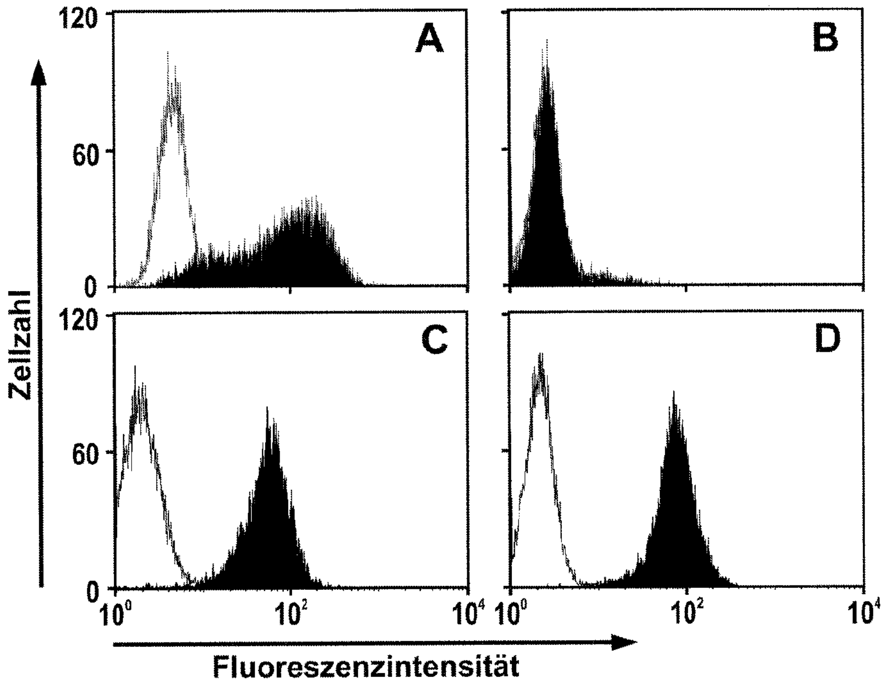
A
B
C
D
120
60
0
Zellzahl
$10^0$
$10^2$
$10^4$
Fluoreszenzintensität

Fig.11

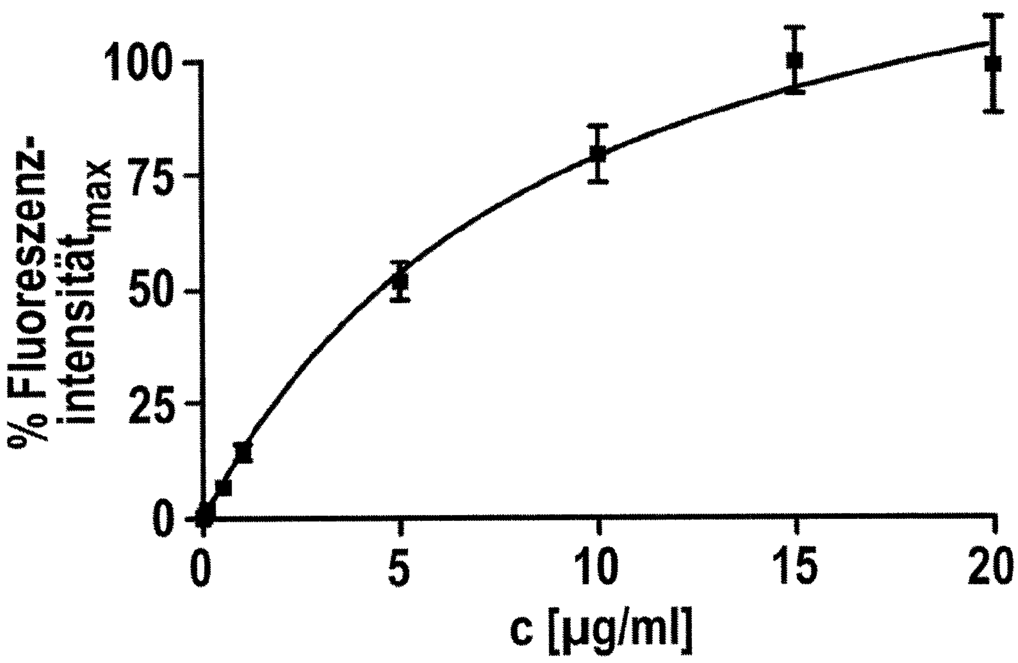
100
75
50
25
0
% Fluoreszenz-intensität$_{max}$
0
5
10
15
20
c [µg/ml]

Fig.12

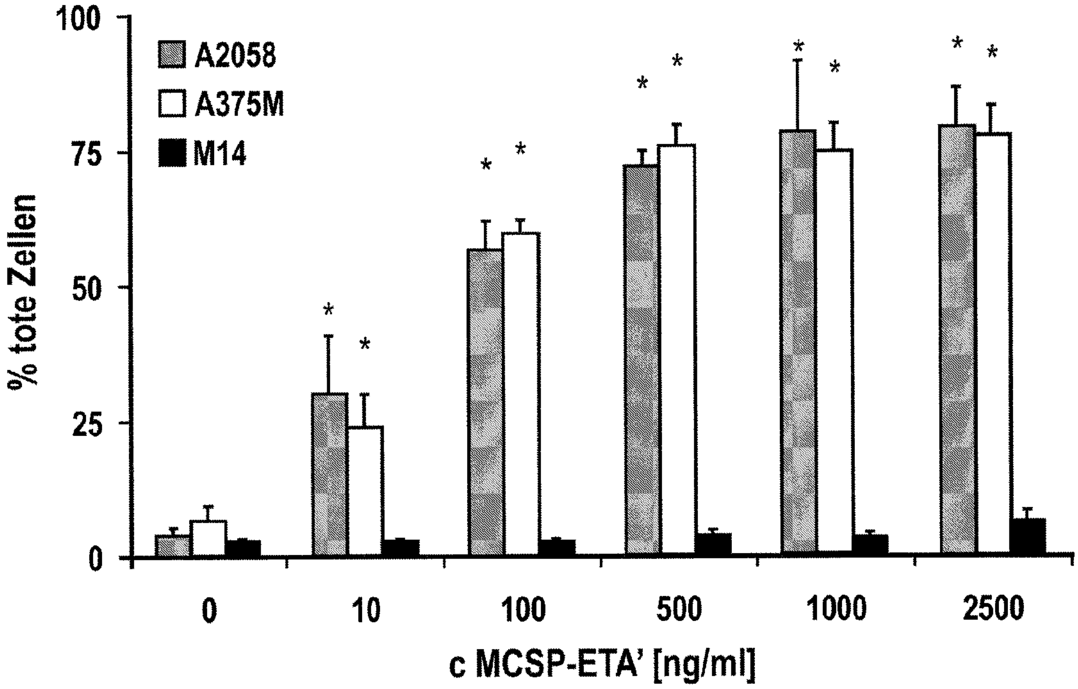
100
75
50
25
0
% tote Zellen
A2058
A375M
M14
0
10
100
500
1000
2500
c MCSP-ETA' [ng/ml]

Fig.13

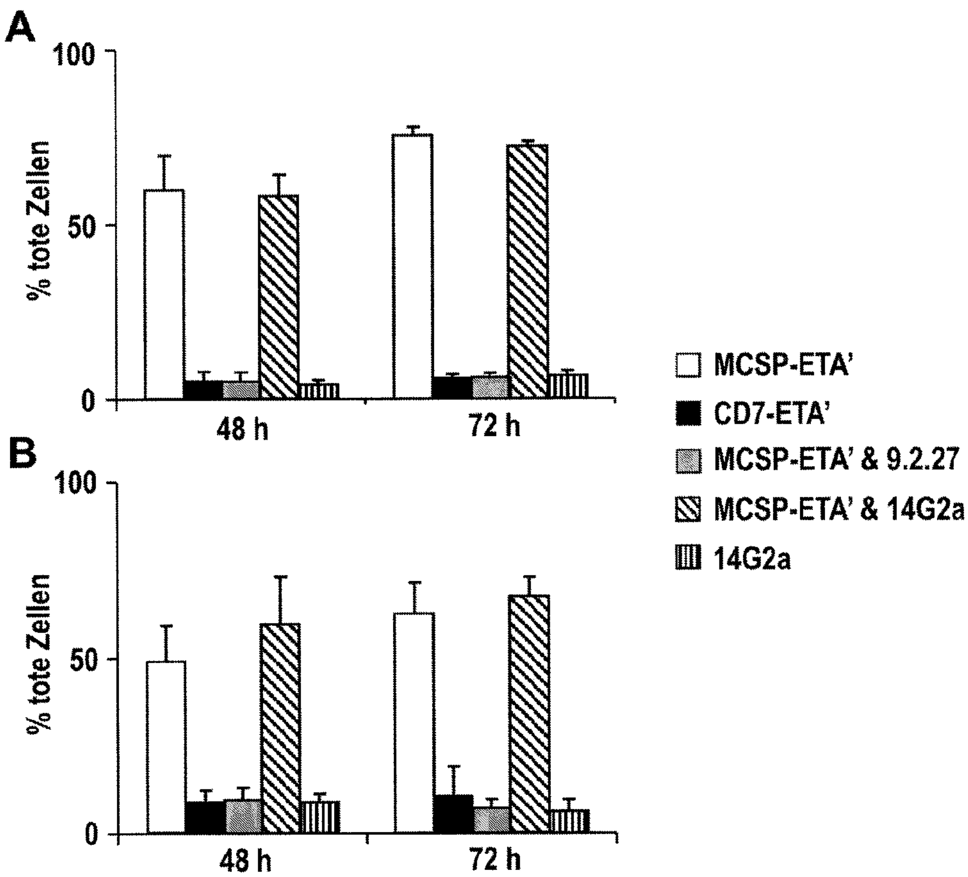
A
B
100
50
0
% tote Zellen
48 h
72 h
MCSP-ETA'
CD7-ETA'
MCSP-ETA' & 9.2.27
MCSP-ETA' & 14G2a
14G2a

Fig.14

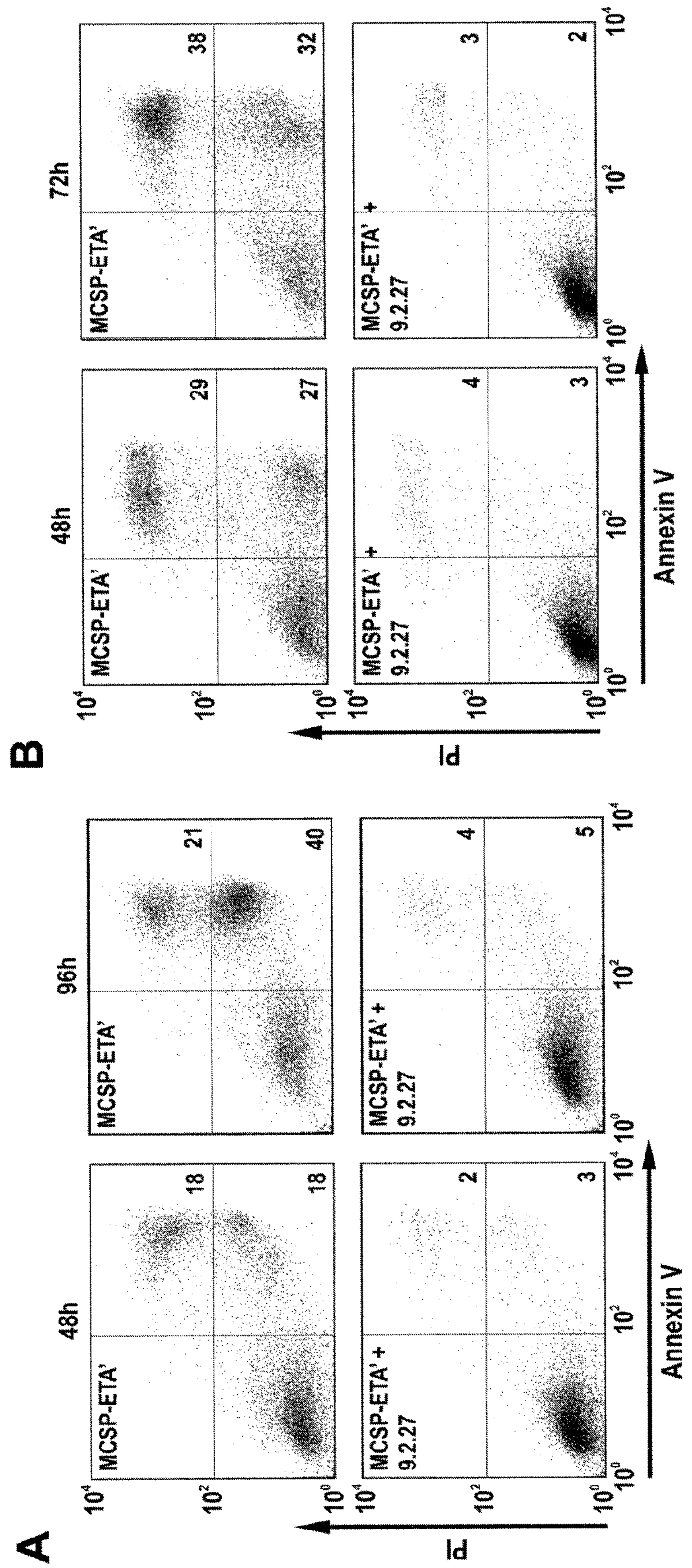
A
48h
96h
MCSP-ETA'
MCSP-ETA' +
9.2.27
18
18
2
3
21
40
4
5
PI
Annexin V
B
48h
72h
29
27
4
3
38
32
3
2


Fig.15

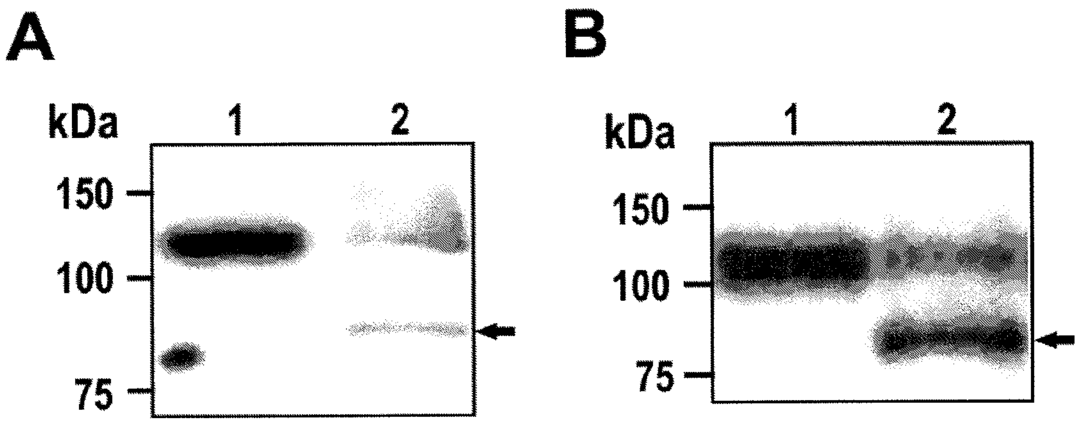
A
kDa
1
2
150
100
75
B
kDa
1
2
150
100
75

Fig.16

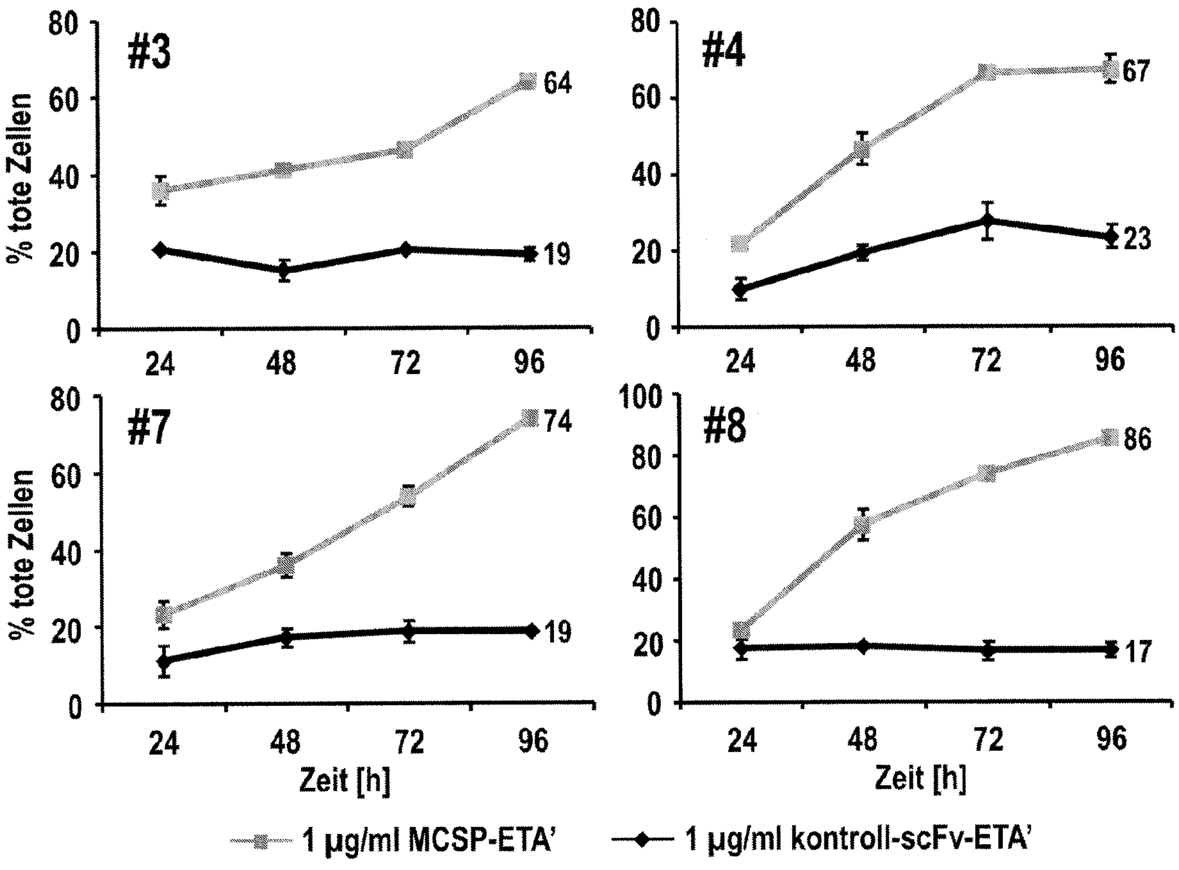
#3
#4
#7
#8
% tote Zellen
Zeit [h]
24
48
72
96
64
19
67
23
74
19
86
17
1 µg/ml MCSP-ETA'
1 µg/ml kontroll-scFv-ETA'

Fig.17

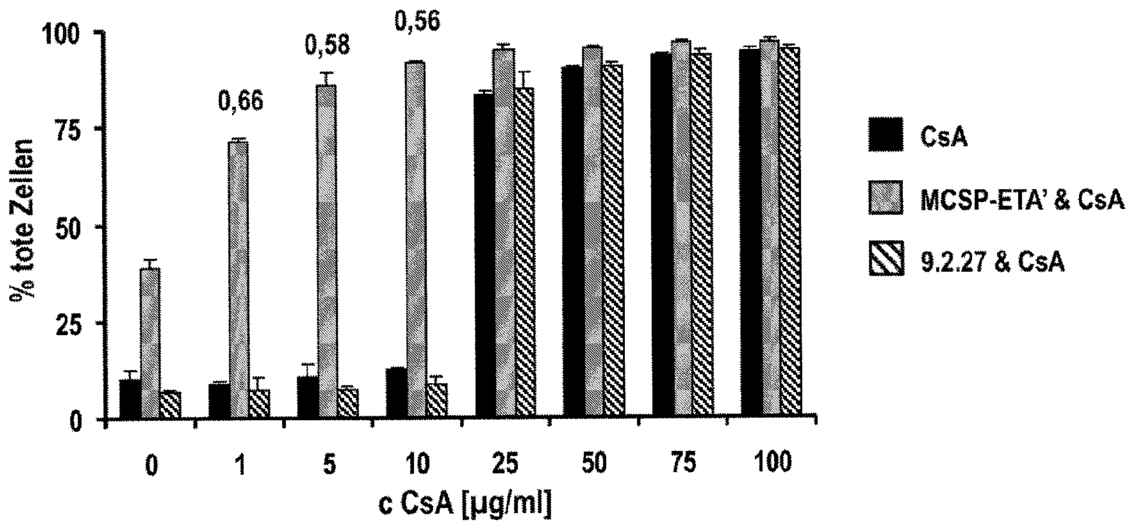
100
75
50
25
0
% tote Zellen
0,66
0,58
0,56
0
1
5
10
25
50
75
100
c CsA [µg/ml]
CsA
MCSP-ETA' & CsA
9.2.27 & CsA

Fig.18

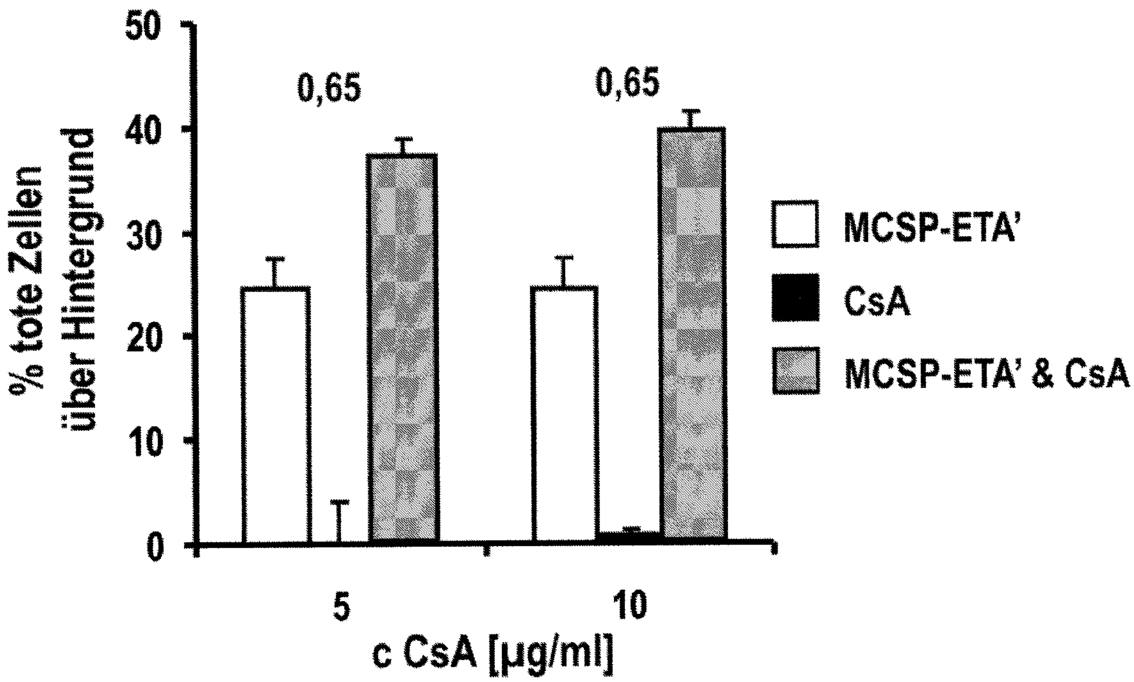
50
40
30
20
10
0
% tote Zellen
über Hintergrund
0,65
0,65
5
10
c CsA [µg/ml]
MCSP-ETA'
CsA
MCSP-ETA' & CsA

Fig.19

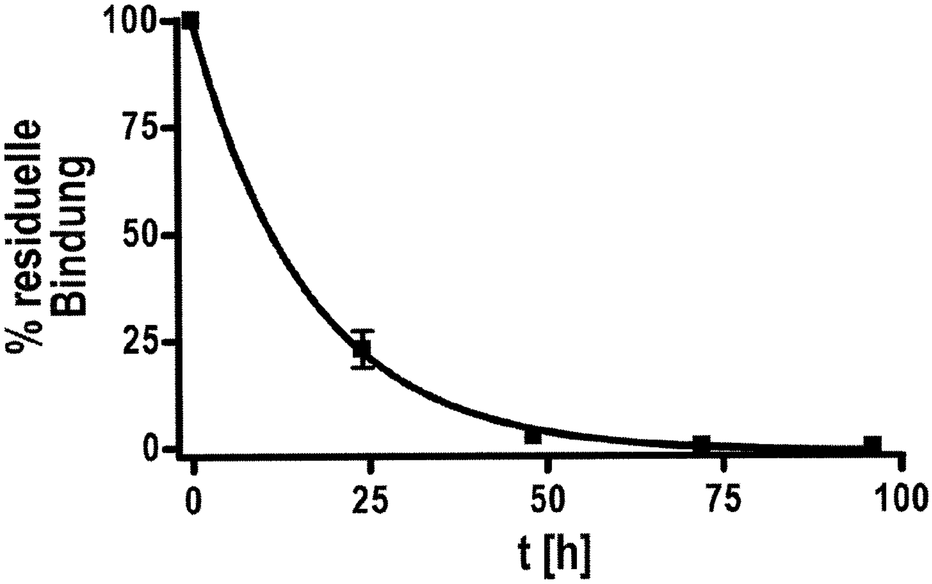
100
75
50
25
0
% residuelle Bindung
0
25
50
75
100
t [h]

Fig.20

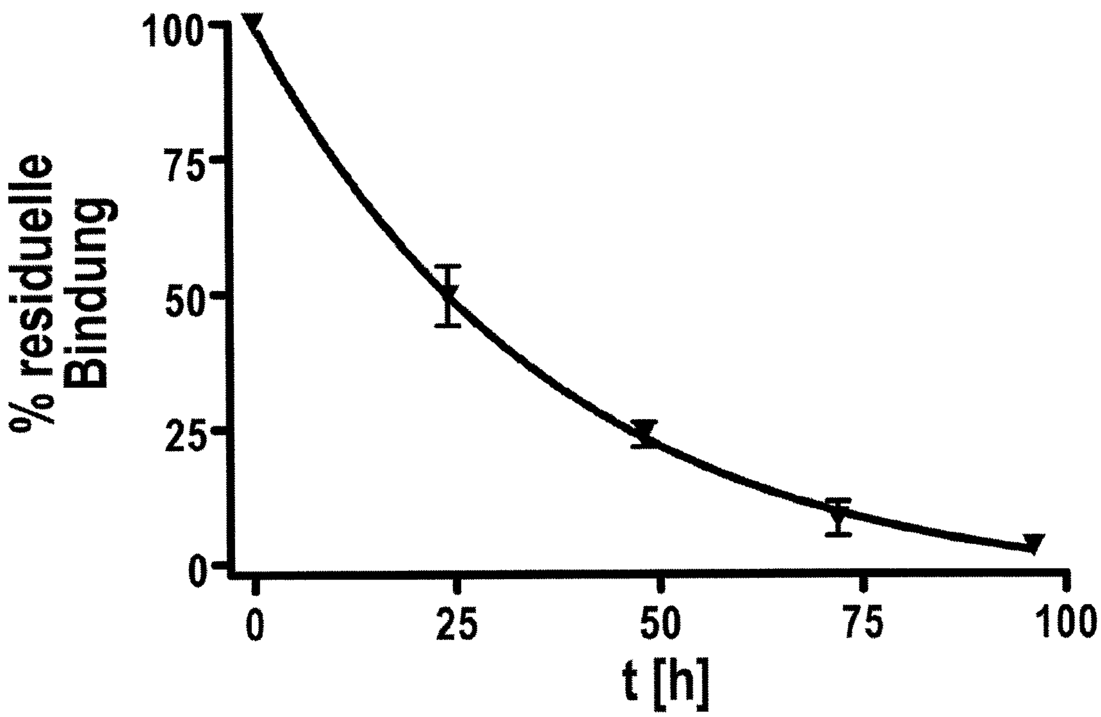
100
75
50
25
0
% residuelle Bindung
0
25
50
75
100
t [h]

Fig.21

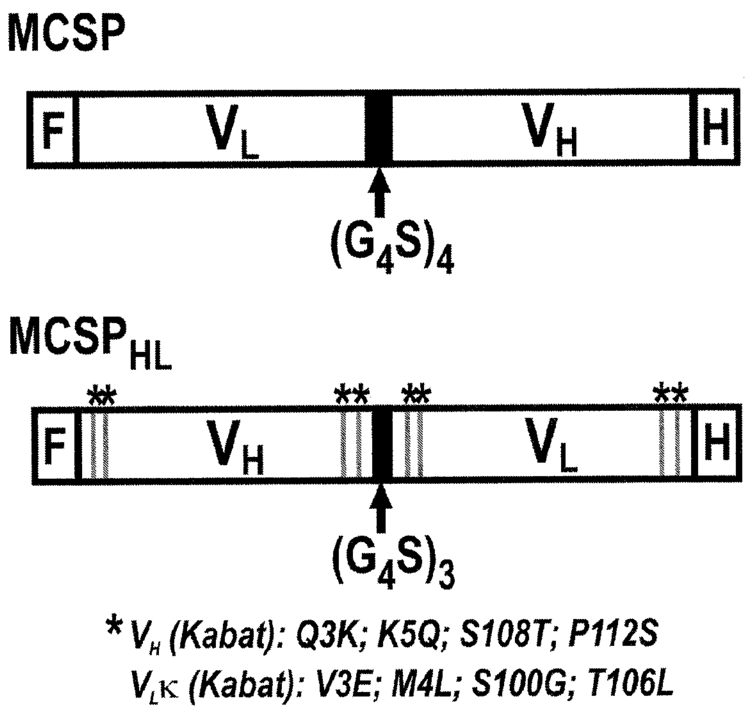
MCSP
F
V L
V H
H
(G4S)4
MCSPHL
F
V H
V L
H
(G4S)3
* VH (Kabat): Q3K; K5Q; S108T; P112S
VLκ (Kabat): V3E; M4L; S100G; T106L

Fig.22

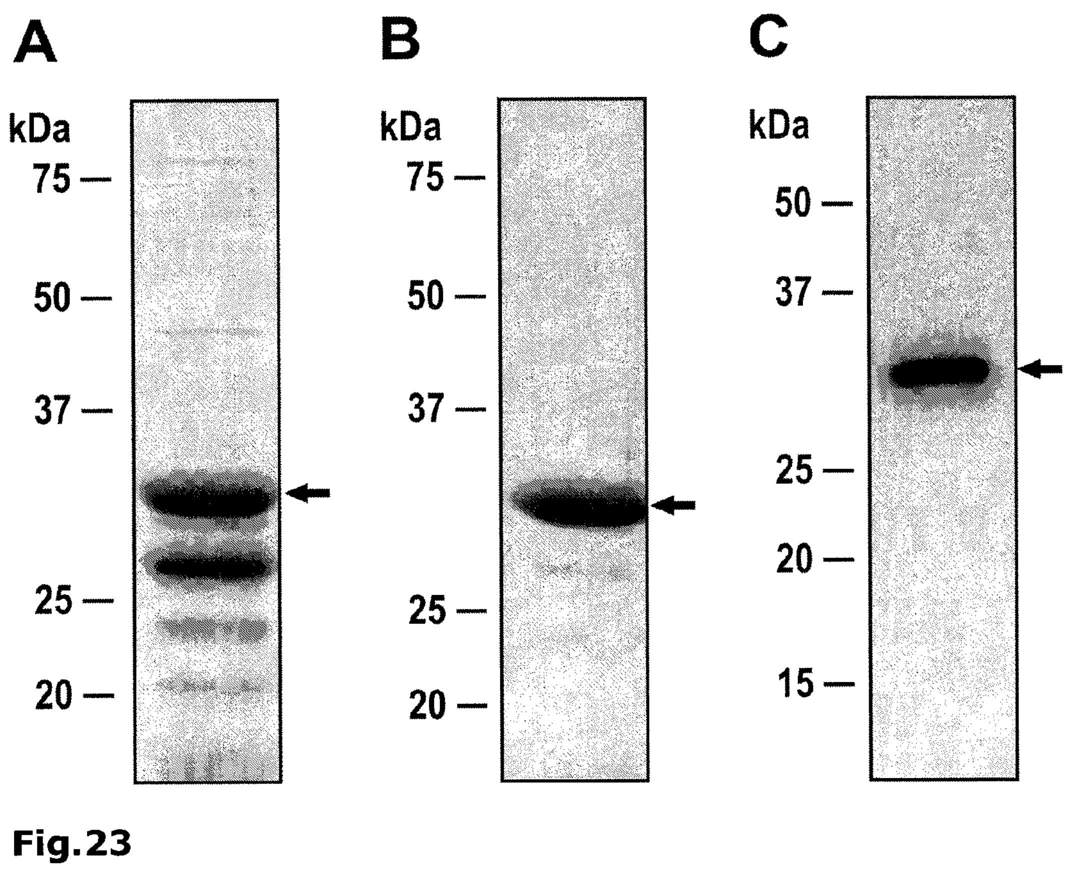
A
kDa
75
50
37
25
20
B
kDa
75
50
37
25
20
C
kDa
50
37
25
20
15

Fig.23

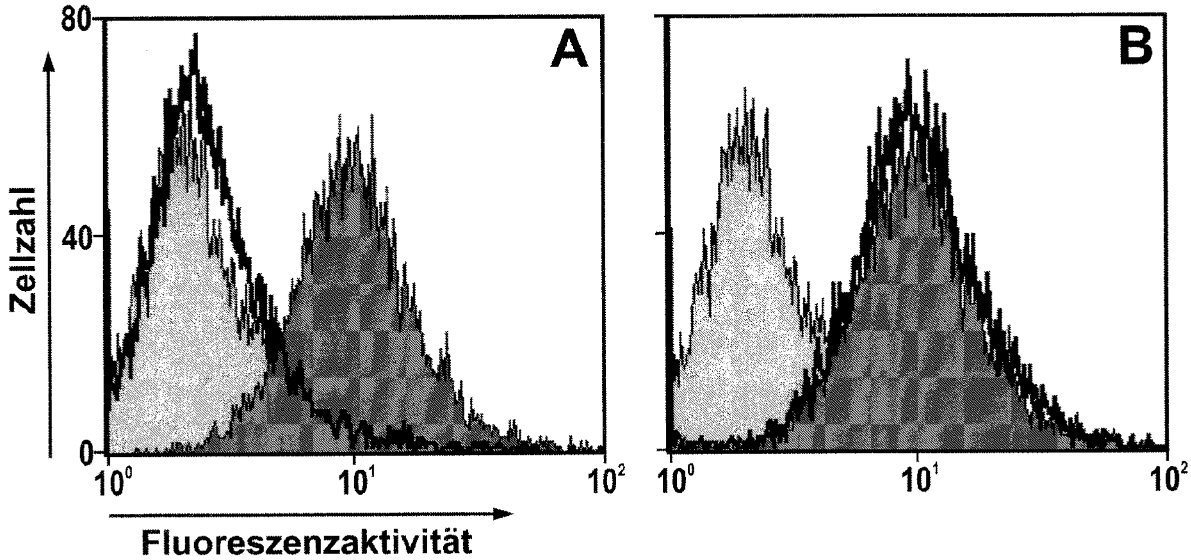


Fig.24

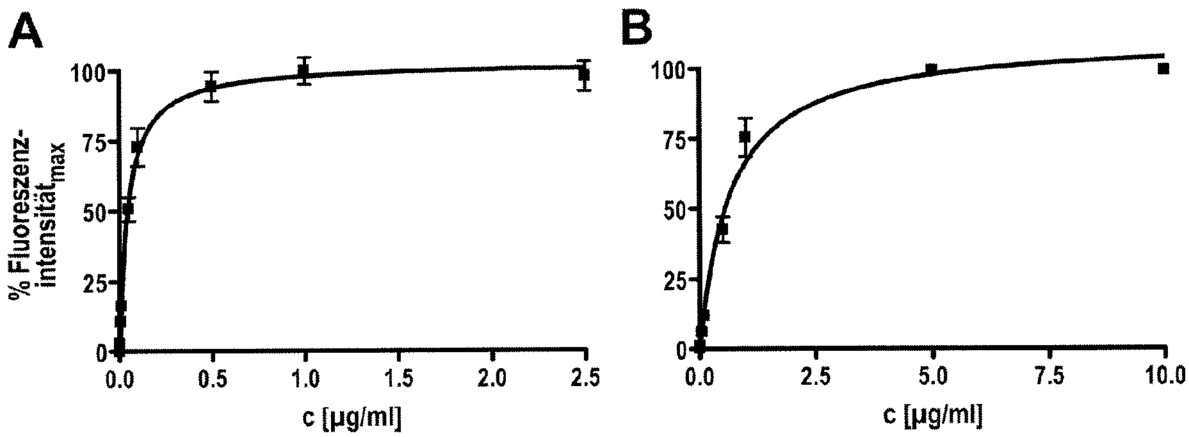


Fig.25

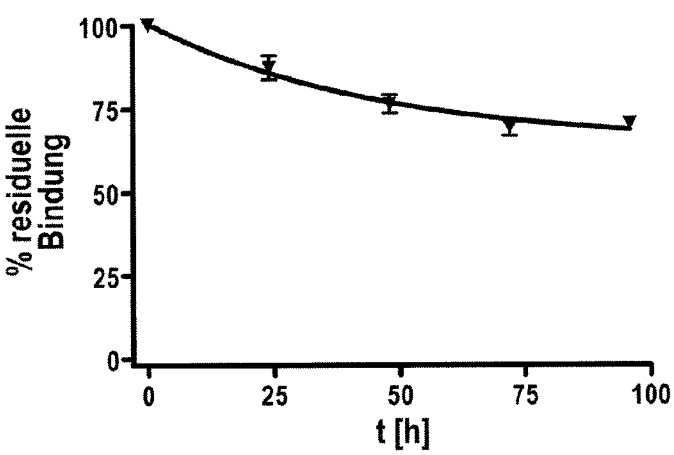


Fig.26

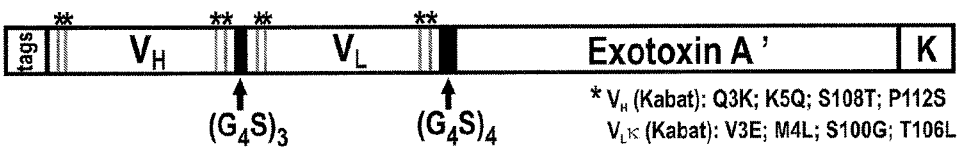
tags
VH
VL
Exotoxin A '
K
(G4S)3
(G4S)4
* VH (Kabat): Q3K; K5Q; S108T; P112S
VLκ (Kabat): V3E; M4L; S100G; T106L

Fig.27

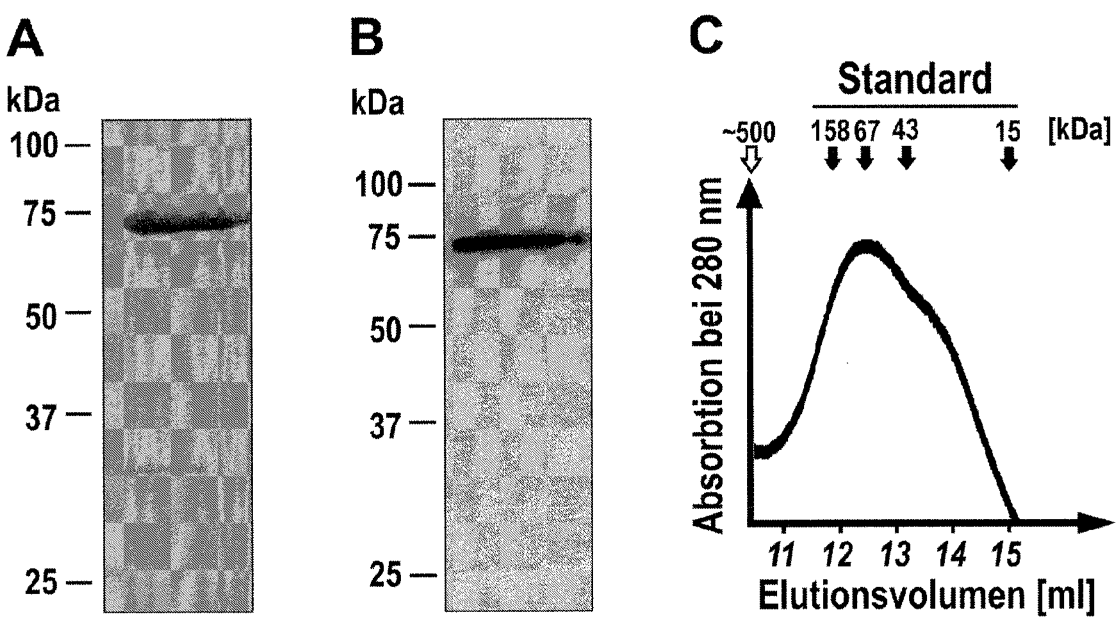
A
kDa
100
75
50
37
25
B
kDa
100
75
50
37
25
C
Standard
~500
158
67
43
15
[kDa]
Absorbtion bei 280 nm
11
12
13
14
15
Elutionsvolumen [ml]

Fig.28

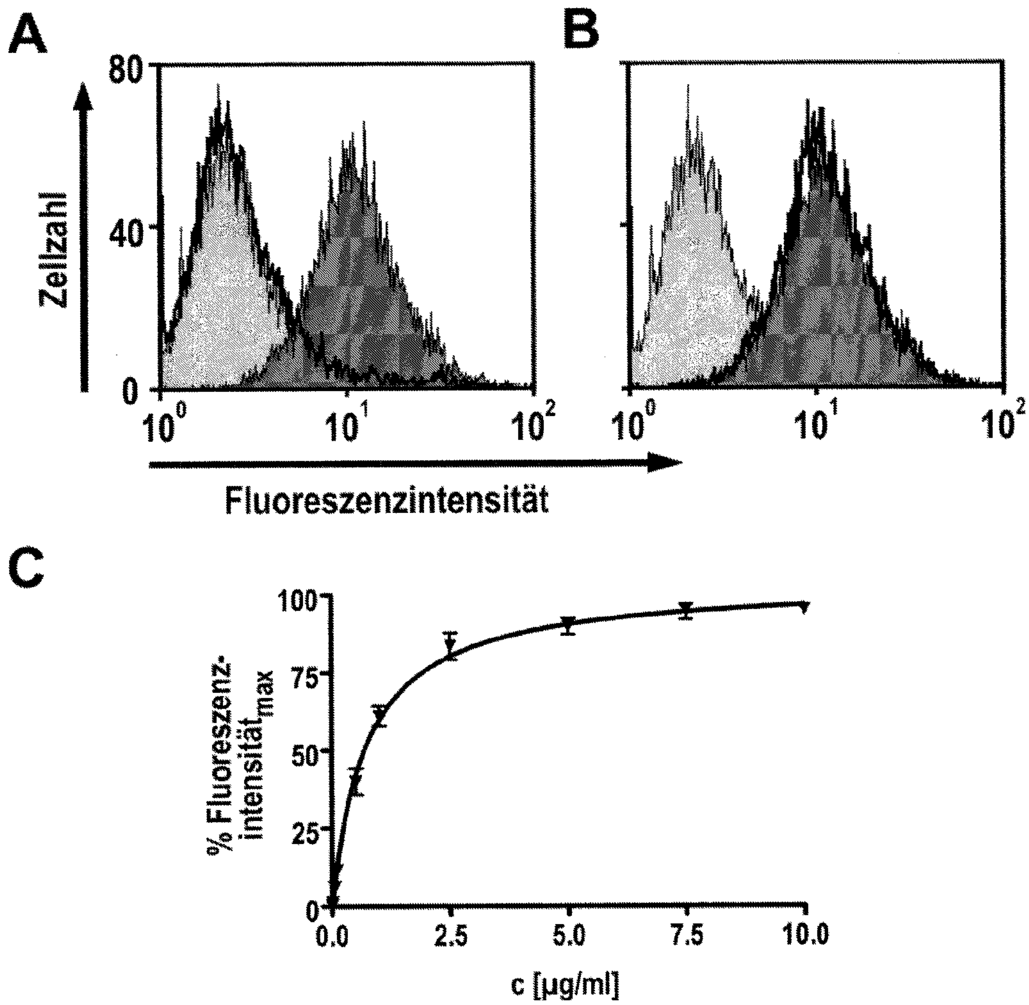
A
B
80
40
0
Zellzahl
$10^0$
$10^1$
$10^2$
$10^0$
$10^1$
$10^2$
Fluoreszenzintensität
C
100
75
50
25
0
% Fluoreszenz-intensität$_{max}$
0.0
2.5
5.0
7.5
10.0
c [µg/ml]

Fig.29

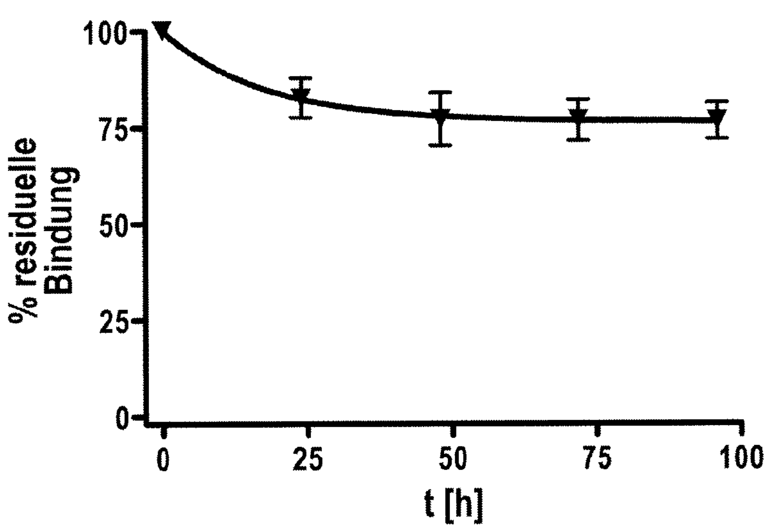
100
75
50
25
0
% residuelle Bindung
0
25
50
75
100
t [h]

Fig.30

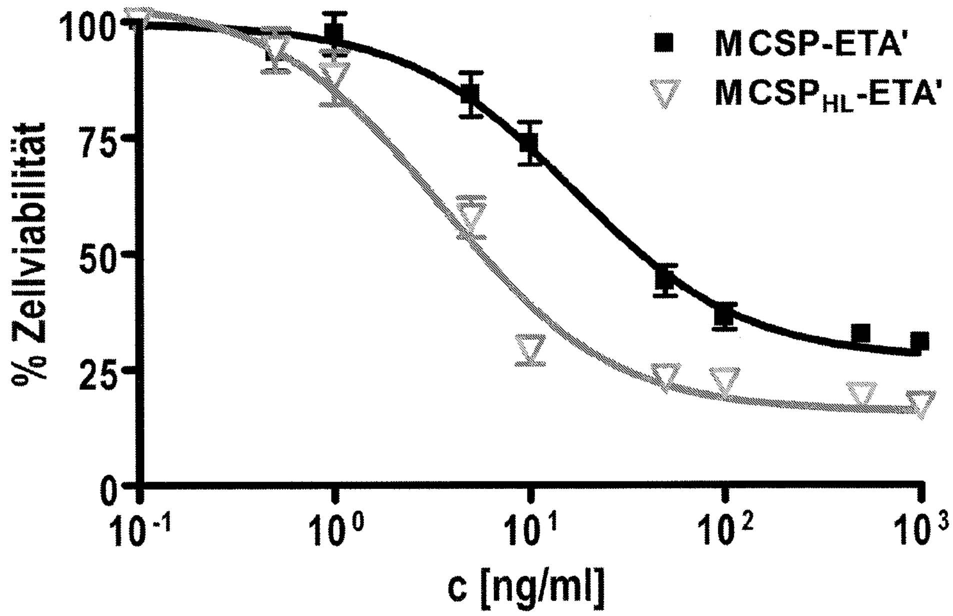
100
75
50
25
0
% Zellviabilität
MCSP-ETA'
MCSP$_{HL}$-ETA'
$10^{-1}$
$10^{0}$
$10^{1}$
$10^{2}$
$10^{3}$
c [ng/ml]

Fig.31

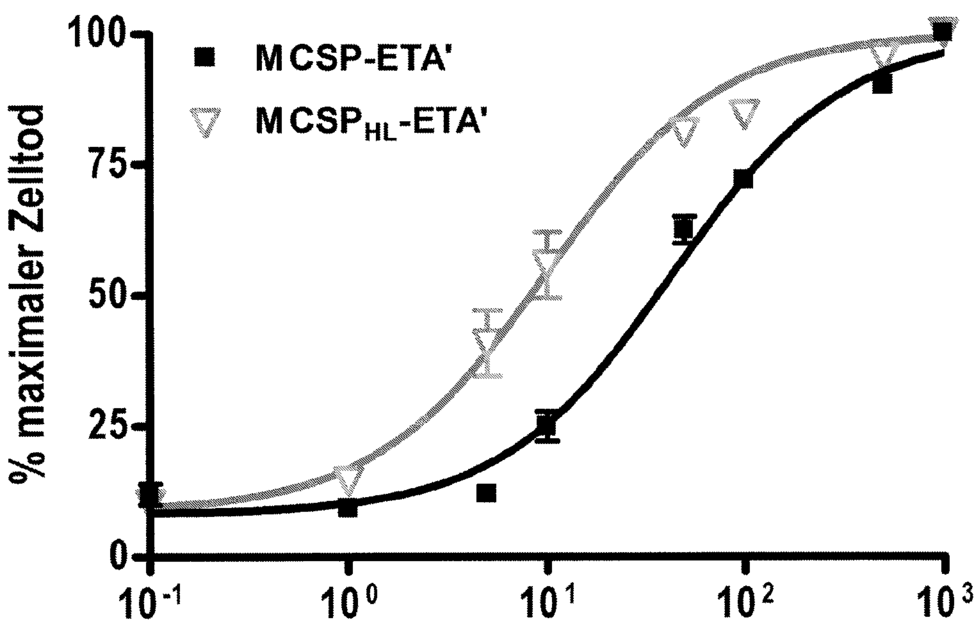
100
75
50
25
0
% maximaler Zelltod
MCSP-ETA'
MCSP$_{HL}$-ETA'
$10^{-1}$
$10^{0}$
$10^{1}$
$10^{2}$
$10^{3}$
c [ng/ml]

Fig.32

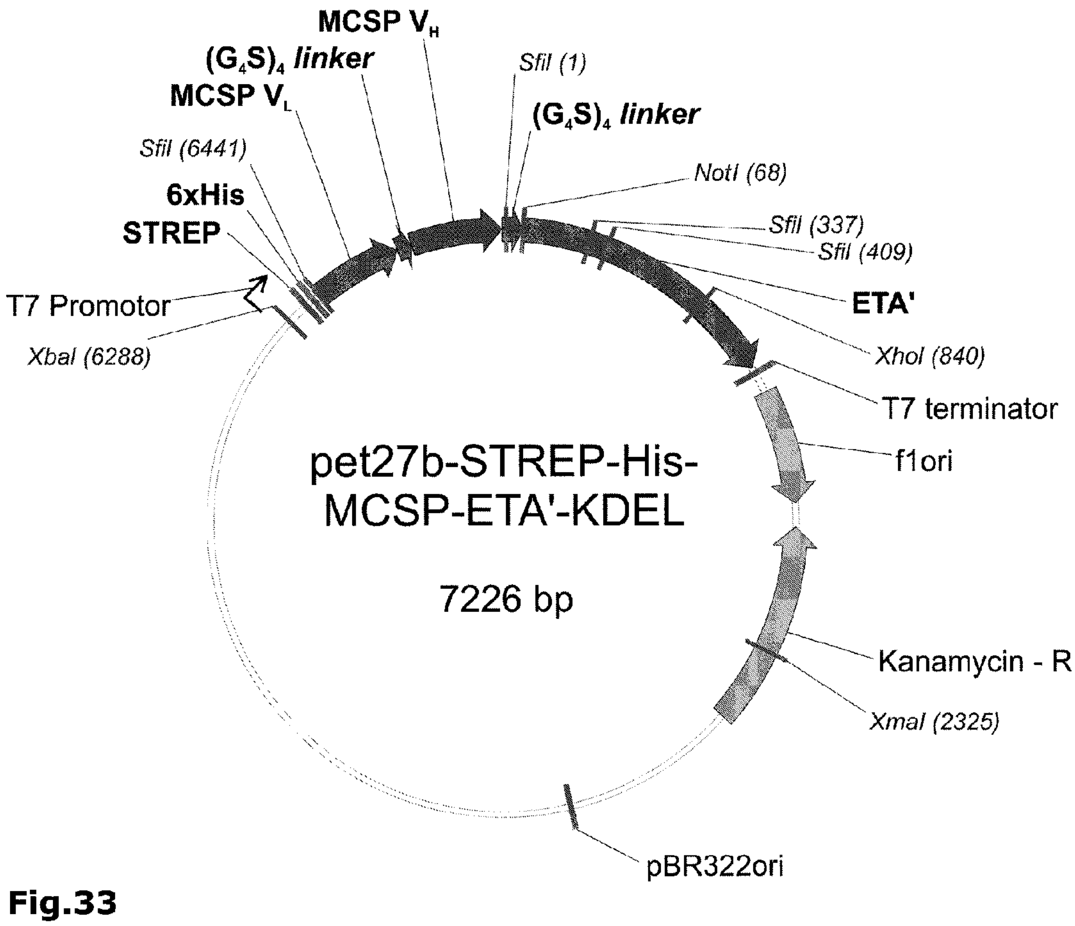
MCSP $V_H$
$(G_4S)_4$ linker
MCSP $V_L$
SfiI (6441)
6xHis
STREP
T7 Promotor
XbaI (6288)
SfiI (1)
$(G_4S)_4$ linker
NotI (68)
SfiI (337)
SfiI (409)
ETA'
XhoI (840)
T7 terminator
f1ori
pet27b-STREP-His-
MCSP-ETA'-KDEL
7226 bp
Kanamycin - R
XmaI (2325)
pBR322ori

Fig.33

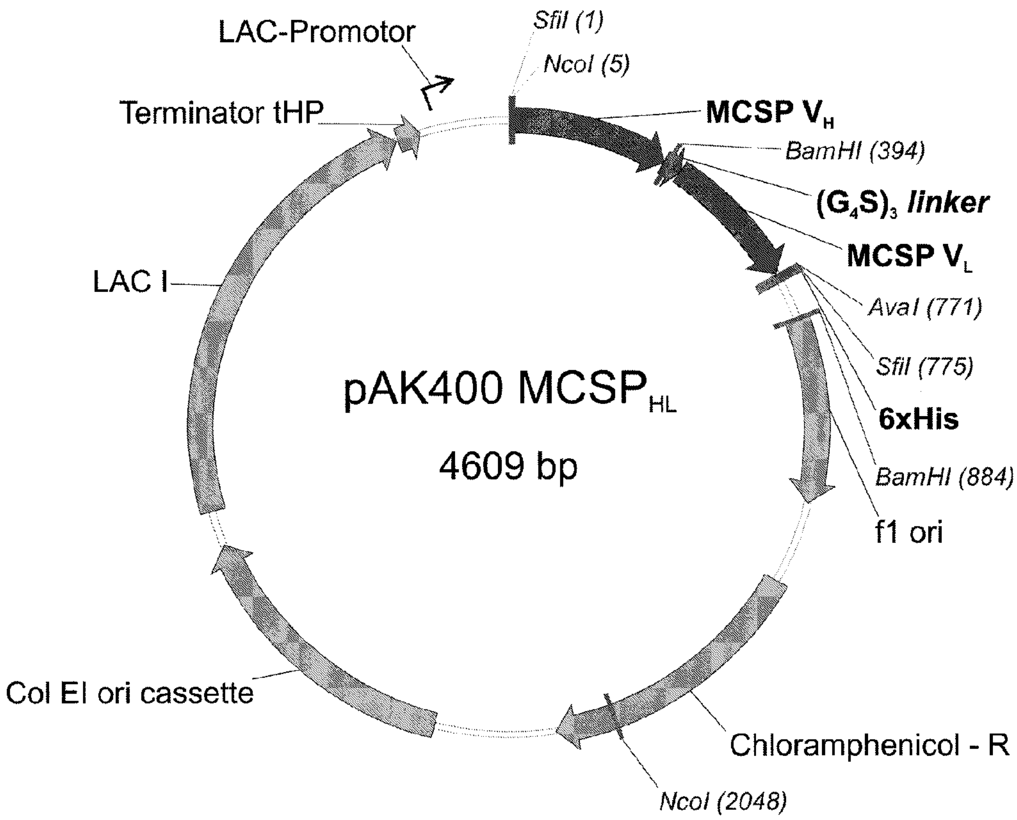
LAC-Promotor
SfiI (1)
NcoI (5)
Terminator tHP
MCSP $V_H$
BamHI (394)
$(G_4S)_3$ linker
MCSP $V_L$
LAC I
AvaI (771)
SfiI (775)
pAK400 $MCSP_{HL}$
4609 bp
6xHis
BamHI (884)
f1 ori
Col EI ori cassette
Chloramphenicol - R
NcoI (2048)

**Fig.34**

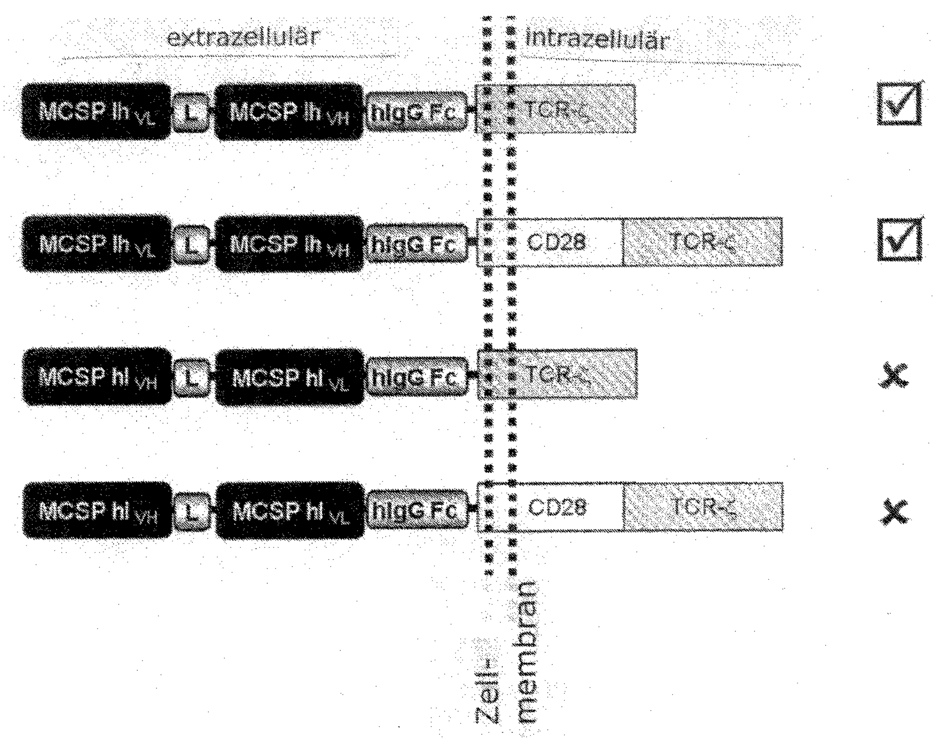
extrazellulär
intrazellulär
MCSP lh VL
L
MCSP lh VH
hIgG Fc
TCR-ζ
CD28
MCSP hl VH
MCSP hl VL
Zell-
membran

**Fig.35**

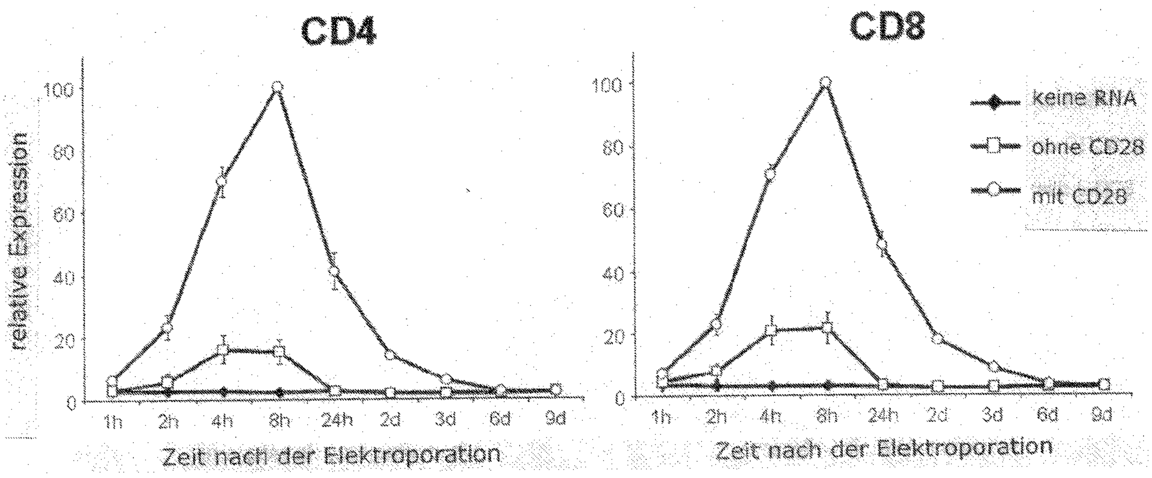
CD4
CD8
relative Expression
100
80
60
40
20
0
1h
2h
4h
8h
24h
2d
3d
6d
9d
Zeit nach der Elektroporation
keine RNA
ohne CD28
mit CD28

**Fig.36**

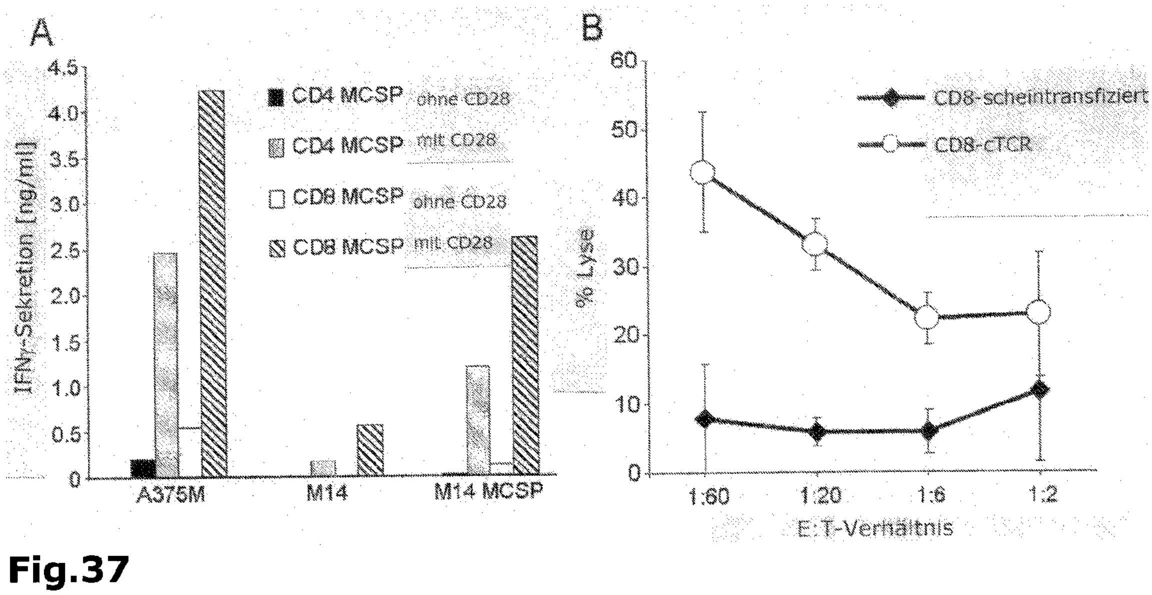
A
B
IFNγ-Sekretion [ng/ml]
4.5
4.0
3.5
3.0
2.5
2.0
1.5
1.0
0.5
0
CD4 MCSP ohne CD28
CD4 MCSP mit CD28
CD8 MCSP ohne CD28
CD8 MCSP mit CD28
A375M
M14
M14 MCSP
% Lyse
60
50
40
30
20
10
0
CD8-scheintransfiziert
CD8-cTCR
1:60
1:20
1:6
1:2
E:T-Verhältnis

**Fig.37**

Europäisches Patentamt
European Patent Office
Office européen des brevets

# EUROPÄISCHER RECHERCHENBERICHT

Nummer der Anmeldung
EP 09 16 2200

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | GROSSE-HOVEST L ET AL: "A recombinant bispecific single-chain antibody induces targeted, supra-agonistic CD28-stimulation and tumor cell killing" EUROPEAN JOURNAL OF IMMUNOLOGY, WILEY - V C H VERLAG GMBH & CO. KGAA, DE, Bd. 33, Nr. 5, 20. Mai 2003 (2003-05-20), Seiten 1334-1340, XP002250206 ISSN: 0014-2980 * Seite 1338, linke Spalte, Absatz 3 * * Seite 1338, linke Spalte, Absatz 1 * * Seite 1338, linke Spalte, Absatz 3 - rechte Spalte, Absatz 1 * * Zusammenfassung * | 1-5,8-15 | INV. C07K16/30 A61K39/395 A61P35/00 C07K16/46 |
| Y | ----- | 6,7 | |
| X | DATABASE EMBL [Online] 11. November 2004 (2004-11-11), "Synthetic construct for bispecific single chain antibody against human CD40 and HMWG, clone r40M" XP002549942 gefunden im EBI accession no. EMBL:AJ853736 Database accession no. AJ853736 * das ganze Dokument * ----- | 1-5,8 | RECHERCHIERTE SACHGEBIETE (IPC): C07K A61K |
| X | DATABASE EMBL [Online] 2. September 2002 (2002-09-02), "Synthetic construct for anti-CD28 and anti-HMWG ScFv antibody, clone r28M" XP002549943 gefunden im EBI accession no. EMBL:AJ507107 Database accession no. AJ507107 * das ganze Dokument * ----- -/-- | 1-5,8 | |

Der vorliegende Recherchenbericht wurde für alle Patentansprüche erstellt

3

| Recherchenort | Abschlußdatum der Recherche | Prüfer |
|---|---|---|
| München | 16. Oktober 2009 | Irion, Andrea |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 09 16 2200

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| X | DATABASE EMBL [Online] 11. November 2004 (2004-11-11), "Synthetic construct for bispecific single chain antibody against human CD3 and HMWG, clone r3M" XP002549944 gefunden im EBI accession no. EMBL:AJ853735 Database accession no. AJ853735 * das ganze Dokument * ----- | 1-5,8 | |
| X | DATABASE EMBL [Online] 3. März 2003 (2003-03-03), "Synthetic construct for anti-CD95 and anti-HMWG ScFv antibody, clone r95M" XP002549945 gefunden im EBI accession no. EMBL:AJ544530 Database accession no. AJ544530 * das ganze Dokument * ----- | 1-5,8 | RECHERCHIERTE SACHGEBIETE (IPC) |
| X | WO 03/042231 A (JUNG GUNDRAM [DE]; JUNG GUDRAM [DE] JUNG GUNDRAM [DE]) 22. Mai 2003 (2003-05-22) * das ganze Dokument * | 1-5,8-15 | |
| Y | * Seite 13, Zeile 11 - Seite 14, Zeile 6 * ----- | 6,7 | |
| | -/-- | | |

3

Der vorliegende Recherchenbericht wurde für alle Patentansprüche erstellt

| Recherchenort | Abschlußdatum der Recherche | Prüfer |
|---|---|---|
| München | 16. Oktober 2009 | Irion, Andrea |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 09 16 2200

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| Y | SCHWENKERT MICHAEL ET AL: "A single chain immunotoxin, targeting the melanoma-associated chondroitin sulfate proteoglycan, is a potent inducer of apoptosis in cultured human melanoma cells"<br>MELANOMA RESEARCH,<br>Bd. 18, Nr. 2, April 2008 (2008-04), Seiten 73-84, XP008106327<br>ISSN: 0960-8931<br>* das ganze Dokument *<br>----- | 6,7 | |
| Y | TENG MICHELE W L ET AL: "Immunotherapy of cancer using systemically delivered gene-modified human T lymphocytes"<br>HUMAN GENE THERAPY,<br>Bd. 15, Nr. 7, Juli 2004 (2004-07), Seiten 699-708, XP002549940<br>ISSN: 1043-0342<br>* das ganze Dokument *<br>----- | 6,7 | |
| Y | ESHHAR Z: "The T-body approach: Redirecting T cells with antibody specificity"<br>HANDBOOK OF EXPERIMENTAL PHARMACOLOGY SPRINGER-VERLAG BERLIN, HEIDELBERGER PLATZ 3, D-14197 BERLIN, GERMANY SERIES : HANDBOOK OF EXPERIMENTAL PHARMACOLOGY (ISSN 0171-2004(PRINT)), 2008, Seiten 329-342, XP009106856<br>ISSN: 978-3-540-73258-7(H)<br>* das ganze Dokument *<br>----- | 6,7 | RECHERCHIERTE SACHGEBIETE (IPC) |
| Y | WO 2007/085470 A (UNIV FRIEDRICH ALEXANDER ER [DE]; FEY GEORG H [DE]; PEIPP MATTHIAS [DE) 2. August 2007 (2007-08-02)<br>* das ganze Dokument *<br>-----<br>-/-- | 6,7 | |

Der vorliegende Recherchenbericht wurde für alle Patentansprüche erstellt

3

| Recherchenort | Abschlußdatum der Recherche | Prüfer |
|---|---|---|
| München | 16. Oktober 2009 | Irion, Andrea |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..........................................................
& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 09 16 2200

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | GROSSE-HOVEST LUDGER ET AL: "Supraagonistic, bispecific single-chain antibody purified from the serum of cloned, transgenic cows induces T-cell-mediated killing of glioblastoma cells in vitro and in vivo" INTERNATIONAL JOURNAL OF CANCER, Bd. 117, Nr. 6, Dezember 2005 (2005-12), Seiten 1060-1064, XP002549948 ISSN: 0020-7136 * das ganze Dokument * ----- | 1-15 | |
| | | | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| München | 16. Oktober 2009 | Irion, Andrea |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

3

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 09 16 2200

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

16-10-2009

| Im Recherchenbericht angeführtes Patentdokument | Datum der Veröffentlichung | Mitglied(er) der Patentfamilie | Datum der Veröffentlichung |
|---|---|---|---|
| WO 03042231 A | 22-05-2003 | AT 364633 T | 15-07-2007 |
| | | AU 2002342896 A1 | 26-05-2003 |
| | | CA 2505342 A1 | 22-05-2003 |
| | | DE 10156482 A1 | 28-05-2003 |
| | | EP 1444268 A2 | 11-08-2004 |
| | | ES 2289156 T3 | 01-02-2008 |
| | | US 2005244416 A1 | 03-11-2005 |
| WO 2007085470 A | 02-08-2007 | US 2007178103 A1 | 02-08-2007 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 20032004

### In der Beschreibung aufgeführte Nicht-Patentliteratur


- **Armstrong, BK. ; Kricker, A.** *Cancer Surv.,* 1994, vol. 19-20, 219-240
- **Garbe, C. ; Blum, A.** *Skin Pharmacol. Appl. Skin Physiol.,* 2001, vol. 14, 280-290
- **Breslow, A.** *Ann. Surg.,* 1970, vol. 172, 902-908
- **Garbe, C.** *Recent Results Cancer Res.,* 2002, vol. 160, 205-215
- **Balch, CM. et al.** *J. Clin. Oncol.,* 2001, vol. 19, 3635-3648
- **Helmbach, H. et al.** *Recent Results Cancer Res.,* 2003, vol. 161, 93-110
- **Avril, MF. et al.** *J. Clin. Oncol.,* 2004, vol. 22, 1118-1125
- **Quirt, I. et al.** *Oncologist,* 2007, vol. 12, 1114-1123
- **Atallah, E. ; Flaherty, L.** *Curr. Treat Options Oncol.,* 2005, vol. 6, 185-193
- **Allen, TM.** *Nat. Rev. Cancer,* 2002, vol. 2, 750-763
- **Kohler, G. ; Milstein, C.** *Nature,* 1975, vol. 256, 495-497
- **Arteaga, CL.** *Breast Cancer Res.,* 2003, vol. 5, 96-100
- **Mirick, GR et al.** *Q. J. Nucl. Med. Mol. Imaging,* 2004, vol. 48, 251-257
- **Morrison, SL. et al.** *Proc. Natl. Acad. Sci. U S A,* 1984, vol. 81, 6851-6855
- **Jenes, PT et al.** *Nature,* 1986, vol. 321, 522-525
- **Qu, Z. et al.** *Methods,* 2005, vol. 36, 84-95
- **Marks, JD. et al.** *J. Mol. Biol.,* 1991, vol. 222, 581-597
- **Hoogenboom, HR.** *Nat. Biotechnol.,* 2005, vol. 23, 1105-1116
- **Markham, A. ; Lamb, HM.** *Drugs,* 2000, vol. 59, 1341-1359
- **Navarro-Sarabia, F. et al.** *J. Rheumatol.,* 2006, vol. 33, 1075-1081
- **Ferrara, N.** *Oncology,* 2005, vol. 69 (3), 11-16
- **Harries, M. ; Smith, I.** *Endocr. Relat. Cancer,* 2002, vol. 9, 75-85
- **Trauth, BC. et al.** *Science,* 1989, vol. 245, 301-305
- **Vuist, WM. et al.** *Blood,* 1994, vol. 83, 899-906
- **Davis, TA. et al.** *Blood,* 1998, vol. 92, 1184-1190
- **Smith, MR.** *Oncogene,* 2003, vol. 22, 7359-7368
- **Voso, MT. et al.** *Haematologica,* 2002, vol. 87, 918-925
- **Shan, D. et al.** *Blood,* 1998, vol. 91, 1644-1652
- **Benoit, NE. ; Wade, WF.** *Immunopharmacology,* 1996, vol. 35, 129-139
- **Meng, R. et al.** *Clin. Cancer Res.,* 2004, vol. 10, 1274-1281
- **Vitale, C. et al.** *Proc. Natl. Acad. Sci. U S A,* 1999, vol. 96, 15091-15096
- **Dechant, M. et al.** *Semin. Oncol.,* 2003, vol. 30, 465-475
- **Di Gaetano, N. et al.** *J. Immunol.,* 2003, vol. 171, 1581-1587
- **Clynes, RA. et al.** *Nat. Med.,* 2000, vol. 6, 443-446
- **Goldenberg, DM. et al.** *N. Engl. J. Med.,* 1978, vol. 298, 1384-1386
- **Witzig, TE. et al.** *J. Clin. Oncol.,* 2002, vol. 20, 2453-2463
- **Davis, TA. et al.** *Clin. Cancer Res.,* 2004, vol. 10, 7792-7798
- **Milenic, DE et al.** *Nat. Rev. Drug Discov.,* 2004, vol. 3, 488-499
- **Aarts, F. et al.** *Cancer Biother. Radiopharm.,* 2008, vol. 23, 92-107
- **Sharkey, RM. ; Goldenberg, DM.** *CA. Cancer J. Clin.,* 2006, vol. 56, 226-243
- **Bennett, JM. et al.** *Blood,* 2005, vol. 105, 4576-4582
- **Gillies, SD. et al.** *Blood,* 2005, vol. 105, 3972-3978
- **Bremer, E. et al.** *Int. J. Cancer,* 2004, vol. 109, 281-290
- **Bremer, E. et al.** *Cancer Res.,* 2005, vol. 65, 3380-3388
- **Dubowchik, G. M. et al.** *Nat. Biotechnol.,* 2005, vol. 23, 1137-1146

- **Trail, PA. et al.** *Science,* 1993, vol. 261, 212-215
- **Mosure, K. W. et al.** *Cancer Chemother. Pharmacol.,* 1997, vol. 40, 251-258
- **Ajani, JA. et al.** *Cancer J.,* 2000, vol. 6, 78-81
- **Bernstein, ID.** *Leukemia,* 2000, vol. 14, 474-475
- **Hamann, PR. et al.** *Bioconjug. Chem.,* 2002, vol. 13, 47-58
- **Bross, PF. et al.** *Clin. Cancer Res.,* 2001, vol. 7, 1490-1496
- **Favaloro, EJ.** *Immunol. Cell. Biol.,* 1993, vol. 71, 571-581
- **Wadleigh, M. et al.** *Blood,* 2003, vol. 102, 1578-1582
- **Jedema, I. et al.** *Leukemia,* 2004, vol. 18, 316-325
- **Schwemmlein, M. et al.** *Br. J. Haematol.,* 2006, vol. 133, 141-151
- **Yamaizumi, M. et al.** *Cell,* 1978, vol. 15, 245-250
- **Doronina, S. O. et al.** *Nat. Biotechnol.,* 2003, vol. 21, 778-784
- **Hartley, MR. ; Lord, JM.** *Biochim. Biophys. Acta,* 2004, vol. 1701, 1-14
- **Naglich, JG. et al.** *Cell,* 1992, vol. 69, 1051-1061
- **Kounnas, M. Z. et al.** *J. Biol. Chem.,* 1992, vol. 267, 12420-12423
- **Lord, J. M. et al.** *Cell Microbiol.,* 1999, vol. 1, 85-91
- **Endo, Y. ; Tsurugi, K.** *J. Biol. Chem.,* 1988, vol. 263, 8735-8739
- **Ippoliti, R. et al.** *FEBS Lett.,* 1992, vol. 298, 145-148
- **Hudak, KA. et al.** *J. Biol. Chem.,* 1999, vol. 274, 3859-3864
- **Van Ness, BG et al.** *J. Biol. Chem.,* 1980, vol. 255, 10710-10716
- **Pastan, I. ; FitzGerald, D.** *J. Biol. Chem.,* 1989, vol. 264, 15157-15160
- **Allured, VS. et al.** *Proc. Natl. Acad. Sci. U S A,* 1986, vol. 83, 1320-1324
- **Hwang, J. et al.** *Cell,* 1987, vol. 48, 129-136
- **Gordon, VM. et al.** *Infect. Immun.,* 1995, vol. 63, 82-87
- **Hessler, JL. ; Kreitman, RJ.** *Biochemistry,* 1997, vol. 36, 14577-14582
- **Munro, S. ; Pelham, HR.** *Cell,* 1987, vol. 48, 899-907
- **Lord, JM. et al.** *Cell. Microbiol.,* 1999, vol. 1, 85-91
- **Andersson, Y. et al.** *Int. J. Cancer,* 2004, vol. 112, 475-483
- **Morland, BJ. et al.** *Br. J. Cancer,* 1994, vol. 69, 279-285
- **Myers, DE et al.** *J. Immunol. Methods,* 1991, vol. 136, 221-237
- **Scott, CF. Jr. et al.** *J. Natl. Cancer Inst.,* 1987, vol. 79, 1163-1172
- **Krolick, KA. et al.** *J. Exp. Med.,* 1982, vol. 155, 1797-1809
- **FitzGerald, DJ. et al.** *J. Clin. Invest.,* 1984, vol. 74, 966-971
- **Thorpe, PE. et al.** *Eur. J. Biochem.,* 1985, vol. 147, 197-206
- **Amlot, PL. et al.** *Blood,* 1993, vol. 82, 2624-2633
- **Stone, MJ. et al.** *Blood,* 1996, vol. 88, 1188-1197
- **Messmann, RA. et al.** *Clin. Cancer Res.,* 2000, vol. 6, 1302-1313
- **Baluna, R. ; Vitetta, ES.** *J. Immunother.,* 1999, vol. 22, 41-47
- **Amlot, PL et al.** *Blood,* vol. 82, 2624-2633
- **Thorpe, SJ. et al.** *Scand. J. Immunol.,* vol. 57, 85-92
- **Bross, PF. et al.** *Cancer Res.,* vol. 7, 1490-1496
- **Hall, PD. et al.** *Leukemia,* 1999, vol. 13, 629-633
- **Sampson, JH. et al.** *J. Neurooncol.,* 2003, vol. 65, 27-35
- **Parney, IF. et al.** *J. Neurosurg.,* 2005, vol. 102, 267-275
- **Foss, F. et al.** *Blood,* 2005, vol. 106, 454-457
- **Peipp, M. et al.** *Cancer Res.,* 2002, vol. 62, 2848-2855
- **Schwemmlein, M. et al.** *Leukemia,* 2007, vol. 21, 1405-1412
- **Barth, S. et al.** *Int. J. Cancer,* 2000, vol. 86, 718-724
- **Barth, S. et al.** *Blood,* 2000, vol. 95, 3909-3914
- **Tur, MK et al.** *Cancer Res.,* 2003, vol. 63, 8414-8419
- **Bruell, D. et al.** *Int. J. Mol. Med.,* 2005, vol. 15, 305-313
- **Di Paolo, C. et al.** *Clin. Cancer Res.,* 2003, vol. 9, 2837-2848
- **Singh, R. et al.** *Mol. Cancer Ther.,* 2007, vol. 6, 562-569
- **Posey, JA. et al.** *Clin. Cancer Res.,* 2002, vol. 8, 3092-3099
- **Azemar, M. et al.** *Breast Cancer Res. Treat.,* 2003, vol. 82, 155-164
- **von Minckwitz, G. et al.** *Breast Cancer Res.,* 2005, vol. 7, R617-626
- **Kreitman, RJ. et al.** *Blood,* 1999, vol. 94, 3340-3348
- **Kreitman, RJ. et al.** *J. Clin. Oncol.,* 2000, vol. 18, 1622-1636
- **Kreitman, RJ. et al.** *J. Clin. Oncol.,* 2005, vol. 23, 6719-6729

- **Kreitman, RJ. et al.** *N. Engl. J. Med.,* 2001, vol. 345, 241-247
- **Pastan, I. et al.** *Nat.,* 2006, vol. 6, 559-565
- **Onda, M. et al.** *J. Immunol.,* 2006, vol. 177, 8822-8834
- **Ohno, N. et al.** *Leuk. Lymphoma.,* 2002, vol. 43, 885-888
- **Schwenkert, M. et al.** *Melanoma Res.,* 2008, vol. 18, 73-84
- **Wilson, BS. et al.** *Int. J. Cancer,* 1981, vol. 28, 293-300
- **Bumol, TF. ; Reisfeld, RA.** *Proc. Natl. Acad. Sci. U S A,* 1982, vol. 79, 1245-1249
- **Reisfeld, RA. ; Cheresh, DA.** *Adv. Immunol.,* 1987, vol. 40, 323-377
- **Campoli, MR. et al.** *Crit. Rev. Immunol.,* 2004, vol. 24, 267-296
- **Schneider, S. et al.** *J. Neurosci.,* 2001, vol. 21, 920-933
- **Stegmuller, J. et al.** *J. Neurocytol.,* 2002, vol. 31, 497-505
- **Wilson, BS. et al.** *Cancer Immunol. Immunother.,* 1983, vol. 14, 196-201
- **Pluschke, G. et al.** *Proc. Natl. Acad. Sci. U S A,* 1996, vol. 93, 9710-9715
- **Timpl, R. et al.** *Matrix Biol.,* 2000, vol. 19, 309-317
- **Stallcup, WB. ; Dahlin-Huppe, K.** *J. Cell. Sci.,* 2001, vol. 114, 2315-2325
- **Liu, D. ; Sy, MS.** *J. Exp. Med.,* 1996, vol. 183, 1987-1994
- **Bernfield, M. et al.** *Annu. Rev. Biochem.,* 1999, vol. 68, 729-777
- **Barritt, DS. et al.** *J. Cell Biochem.,* 2000, vol. 79, 213-224
- **Staub, E. et al.** *FEBS Lett.,* 2002, vol. 527, 114-118
- **Grako, KA. et al.** *J. Cell Sci.,* 1999, vol. 112 (6), 905-915
- **Ferrone, S. et al.** *Pharmacol. Ther.,* 1993, vol. 57, 259-290
- **Schlingemann, RO et al.** *Am. J. Pathol.,* 1990, vol. 136, 1393-1405
- **Midwood, KS. ; Salter, DM.** *Osteoarthritis Cartilage,* 1998, vol. 6, 297-305
- **Tordsson, JM. et al.** *Cancer Immunol. Immunother.,* 2000, vol. 48, 691-702
- **Petrini, S. et al.** *Mol. Cell. Neurosi.,* 2003, vol. 23, 219-231
- **Challier, JC. et al.** *Cell Mol. Biol.,* 2001, vol. 47
- **Natali, PG. et al.** *J. Cutan. Pathol.,* 1983, vol. 10, 225-237
- **Kageshita, T. et al.** *Cancer Res.,* 1991, vol. 51, 1726-1732
- **Kageshita, T. et al.** *Cancer Res.,* 1993, vol. 53, 2830-2833
- **Chekenya, M. et al.** *Int. J. Dev. Neurosci.,* 1999, vol. 17, 421-435
- **Shoshan, Y. et al.** *Proc. Natl. Acad. Sci. U S A,* 1999, vol. 96, 10361-10366
- **Behm, FG. et al.** *Blood,* 1996, vol. 87, 1134-1139
- **Smith, FO. et al.** *Blood,* 1996, vol. 87, 1123-1133
- **de Vries, JE. et al.** *Int. J. Cancer,* 1986, vol. 38, 465-473
- **Ranson, M. ; Andronicos, NM.** *Front Biosci.,* 2003, vol. 8, 294-304
- **Iida, J. et al.** *J. Biol. Chem.,* 2001, vol. 276, 18786-18794
- **Garrigues, HJ. et al.** *J. Cell Biol.,* 1986, vol. 103, 1699-1710
- **Yang, J. et al.** *J. Cell. Biol.,* 2004, vol. 165, 881-891
- **Smalley, KS.** *Int. J. Cancer,* 2003, vol. 104, 527-532
- **Erfurt, C. et al.** *J. Immunol.,* 2007, vol. 178, 7703-7709
- **Grosse-Hovest, L. et al.** *Eur. J. Immunol.,* 2003, vol. 33, 1334-1340
- **Suntharalingam, G. et al.** *N. Engl. J. Med.,* 2006, vol. 355, 1018-1028
- **Ozerdem, U.** *Ophthalmic Res.,* 2006, vol. 38, 251-254
- **Ozerdem, U.** *Prostate,* 2006, vol. 66, 294-304
- **Bumol, TF. et al.** *Proc. Natl. Acad. Sci. U S A,* 1983, vol. 80, 529-533
- **Sivam, G. et al.** *Cancer Res.,* 1987, vol. 47, 3169-3173
- **Bender, H. et al.** *Hybridoma,* 1997, vol. 16, 65-68
- **Wang, B. et al.** *Proc. Natl. Acad. Sci. U S A,* 1999, vol. 96, 1627-1632
- **Imai, K. et al.** *Scand. J. Immunol.,* 1981, vol. 14, 369-377
- **Imai, K et al.** *Cell. Immunol.,* 1982, vol. 72, 239-247
- **Ghose, T. et al.** *Cancer Immunol. Immunother.,* 1991, vol. 34, 90-96
- **Schrappe, M. et al.** *Cancer Res.,* 1992, vol. 52, 3838-3844
- **Spitler, LE. et al.** *Cancer Res.,* 1987, vol. 47, 1717-1723
- **Matsui, M. et al.** *Jpn. J Cancer Res.,* 1985, vol. 76, 119-123
- **Allen, BJ. et al.** *Cancer Biol. Ther.,* 2005, vol. 4, 1318-1324
- **Hjortland, GO. et al.** *J. Neurosurg.,* 2004, vol. 100, 320-327
- **Huston, JS. e.** *Proc. Natl. Acad. Sci. U S A,* 1988, vol. 85, 5879-5883
- **Clay, T.M. et al.** *J. Immunol.,* 1999, vol. 175, 5799

- **Willemsen, R.A. et al.** *Gene Ther.,* 2000, vol. 7, 1369
- **Stanislawski, T et al.** *Nat. Immunol.,* 2001, vol. 2, 962
- **Morgan, R. A. et al.** *J. Immunol.,* 2003, vol. 171, 3287
- **Schaft, N. et al.** *J. Immunol.,* 2003, vol. 170, 2186
- **Roszkowski, J.J. et al.** *Cancer Res.,* 2005, vol. 65, 1570
- **Cohen, C.J. et al.** *J. Immunol.,* 2005, vol. 175, 5799
- **Zhao, Y. et al.** *J. Immunol.,* 2005, vol. 174, 4415
- **Schaft, N. et al.** *Cancer Immunol. Immunother.,* 2006, vol. 55, 1132
- **Morgan, R. A. et al.** *Science,* 2006, vol. 314, 126
- **Zhao, Y. et al.** *Mol. Ther.,* 2006, vol. 13, 151
- **Zhao et al.** *J. Immunol.,* 2005, vol. 174, 4415
- **Morgan R. A. et al.** *Science,* 2006, vol. 314, 126
- **Debets, R. et al.** *Trends Immunol.,* 2002, vol. 23, 435
- **Xue, S. et al.** *Clin. Exp. Immunol.,* 2005, vol. 139, 167
- **Zhang, T. et al.** *Cancer Gene Ther.,* 2004, vol. 11, 487
- **Schaft et al.** *J. Immunol.,* 2003, vol. 170, 2186
- **Stanislawksi, T. et al.** *Nat. Immunol.,* 2001, vol. 2, 962
- **Krebber, A. et al.** *J. Immunol. Methods,* 1997, vol. 201, 35-55
- **Desplancq, D. et al.** *Protein Eng.,* 1994, vol. 7, 1027-1033
- **Huston, JS. et al.** *Proc. Natl. Acad. Sci. U S A,* 1988, vol. 85, 5879-5883
- **Bird, RE. et al.** *Science,* 1988, vol. 242, 423-426
- **Pastan, I. et al.** *Nat. Rev. Cancer,* 2006, vol. 6, 559-565
- **Kreitman, RJ.** *Aaps. J.,* 2006, vol. 8, E532-551
- **Ho, M. et al.** *J. Biol. Chem.,* 2005, vol. 280, 607-617
- **Bang, S. et al.** *Clin. Cancer Res.,* 2005, vol. 11, 1545-1550
- **Fang, D. et al.** *Cancer Res.,* 2005, vol. 65, 9328-9337
- **Hrusak, O. ; Porwit-MacDonald, A.** *Leukemia,* 2002, vol. 16, 1233-1258
- **Somervaille, TC. ; Cleary, ML.** *Cancer Cell,* 2006, vol. 10, 257-268
- **Ghali, L. et al.** *J. Invest. Dermatol.,* 2004, vol. 122, 433-442
- **Fang, D. et al.** *Cancer Res.,* vol. 65, 9328-9337
- **Cartron, G. et al.** *Blood,* 2002, vol. 99, 754-758
- **Weng, WK. ; Levy, R.** *J. Clin. Oncol.,* 2003, vol. 21, 3940-3947
- **Koene, HR. et al.** *Blood,* 1997, vol. 90, 1109-1114
- **Baluna, R. ; Vitetta, ES.** *J. Immunother.,* 1999, vol. 22, 41-47
- **Smallshaw, JE. et al.** *Nat. Biotechnol.,* 2003, vol. 21, 387-391
- **Amlot, PL et al.** *Blood,* 1993, vol. 82, 2624-2633
- **Longo, DL. et al.** *Cancer J.,* 2000, vol. 6, 146-150
- **Tsutsumi, Y. et al.** *Proc. Natl. Acad. Sci. U S A,* 2000, vol. 97, 8548-8553
- *J. Immunol.,* 2006, vol. 177, 8822-8834
- **Kreitman, R.J. et al.** *N. Engl. J. Med.,* 2001, vol. 345, 241-247
- **Tur, MK. et al.** *Cancer Res.,* 2003, vol. 63, 8414-8419
- **van Der Velden, VH. et al.** *Blood,* 2001, vol. 97, 3197-3204
- **Jackson, ME. et al.** *J. Cell. Sci.,* 1999, vol. 112, 467-475
- **Ciechomska, I. et al.** *Int. J. Cancer,* 2005, vol. 117, 59-67
- **Mosieniak, G. et al.** *J. Neurochem.,* 1997, vol. 68, 1142-1149
- **Ito, C. et al.** *Blood,* 1998, vol. 91, 1001-1007
- **Crompton, M. et al.** *Biochem. J.,* 1988, vol. 255, 357-360
- **Zunino, SJ. ; Storms, DH.** *Cancer Lett.,* 2006, vol. 240, 123-134
- **Gothel, SF. ; Marahiel, MA.** *Cell Mol. Life Sci.,* 1999, vol. 55, 423-436
- **Hamilton, GS. ; Steiner, JP.** *J Med. Chem.,* 1998, vol. 41, 5119-5143
- **Sambrook, J. ; Russel, DW.** Molecular Cloning: A Laboratory Manual. Cold Spring Harbour Laboratory Press, 2001
- **Nicoletti, I. et al.** *J. Immunol. Methods,* 1991, vol. 139, 271-279
- **Vermes, I. et al.** *J. Immunol. Methods,* 1995, vol. 184, 39-51
- **Bruenke, J. et al.** *Br. J. Haematol.,* 2005, vol. 130, 218-228
- **Peipp, M. et al.** *J. Immunol. Methods,* 2004, vol. 285, 265-280
- **Morgan, AC. Jr. et al.** *Hybridoma,* 1981, vol. 1, 27-36
- **Seetharam, S. et al.** *J. Biol. Chem.,* 1991, vol. 266, 17376-17381
- **Arakawa, T. ; Timasheff, SN.** *Biophys. J.,* 1985, vol. 47, 411-414
- **Barth, S. et al.** *Appl. Environ. Microbiol.,* 2000, vol. 66, 1572-1579

- **Roscoe, DM. et al.** *J. Immunol.,* 1997, vol. 27, 1459-1468
- **Hombach, A. et al.** *J. Immonol,* 2001, vol. 167, 6123-31
- *Gut,* 2006, vol. 55, 1156-64

SEQUENCE LISTING

<110> SCHULER, Gerold

<120> MSCP-gerichtete scFv-Antikörperfragmente und deren Verwendung

<130> 091219ep

<160> 29

<170> PatentIn version 3.3

<210> 1
<211> 121
<212> PRT
<213> Artificial

<220>
<223> MCSP-VH chain

<400> 1

```
Gln Val Gln Leu Lys Gln Ser Gly Pro Glu Leu Val Lys Pro Gly Ala
1               5                   10                  15

Ser Val Lys Ile Ser Cys Lys Ala Ser Gly Tyr Ala Phe Ser Arg Ser
            20                  25                  30

Trp Met Asn Trp Val Lys Gln Arg Pro Gly Gln Gly Leu Glu Trp Ile
        35                  40                  45

Gly Arg Ile Tyr Pro Gly Asp Gly Asp Thr Asn Tyr Asn Gly Lys Phe
    50                  55                  60

Lys Gly Lys Ala Thr Leu Thr Ala Asp Lys Ser Ser Ser Thr Ala Tyr
65                  70                  75                  80

Met Gln Val Ser Ser Leu Thr Ser Val Asp Ser Ala Val Tyr Phe Cys
                85                  90                  95

Ala Arg Gly Asn Thr Val Val Val Pro Tyr Thr Met Asp Tyr Trp Gly
            100                 105                 110

Gln Gly Thr Ser Val Thr Val Pro Ser
        115                 120
```

<210> 2
<211> 121
<212> PRT
<213> Artificial

<220>
<223> MCSP-VH chain(modified)

<400> 2

Gln Val Lys Leu Gln Gln Ser Gly Pro Glu Leu Val Lys Pro Gly Ala
1               5                   10                  15

Ser Val Lys Ile Ser Cys Lys Ala Ser Gly Tyr Ala Phe Ser Arg Ser
            20                  25                  30

Trp Met Asn Trp Val Lys Gln Arg Pro Gly Gln Gly Leu Glu Trp Ile
        35                  40                  45

Gly Arg Ile Tyr Pro Gly Asp Gly Asp Thr Asn Tyr Asn Gly Lys Phe
    50                  55                  60

Lys Gly Lys Ala Thr Leu Thr Ala Asp Lys Ser Ser Ser Thr Ala Tyr
65                  70                  75                  80

Met Gln Val Ser Ser Leu Thr Ser Val Asp Ser Ala Val Tyr Phe Cys
                85                  90                  95

Ala Arg Gly Asn Thr Val Val Val Pro Tyr Thr Met Asp Tyr Trp Gly
            100                 105                 110

Gln Gly Thr Thr Val Thr Val Ser Ser
        115                 120

<210> 3
<211> 112
<212> PRT
<213> Artificial

<220>
<223> MCSP-VL chain

<400> 3

Asp Ile Val Met Thr Gln Ser Pro Ala Ser Leu Ala Val Ser Leu Gly
1               5                   10                  15

Gln Arg Ala Thr Ile Ser Cys Arg Ala Ser Glu Ser Val Asp Ser Tyr
            20                  25                  30

Gly Asn Ser Phe Met His Trp Tyr Gln Gln Lys Pro Gly Gln Pro Pro
        35                  40                  45

Lys Leu Leu Ile Tyr Leu Ala Ser Asn Leu Glu Ser Gly Val Pro Ala
    50                  55                  60

Arg Phe Ser Gly Ser Gly Ser Arg Thr Asp Phe Thr Leu Thr Ile Asp
65                  70                  75                  80

Pro Val Glu Ala Asp Asp Ala Ala Thr Tyr Tyr Cys Gln Gln Asn Asn
85 90 95

Glu Asp Pro Leu Thr Phe Gly Ser Gly Thr Lys Leu Glu Thr Lys Arg
100 105 110

<210> 4
<211> 112
<212> PRT
<213> Artificial

<220>
<223> MCSP-VL chain(modified)

<400> 4

Asp Ile Glu Leu Thr Gln Ser Pro Ala Ser Leu Ala Val Ser Leu Gly
1 5 10 15

Gln Arg Ala Thr Ile Ser Cys Arg Ala Ser Glu Ser Val Asp Ser Tyr
20 25 30

Gly Asn Ser Phe Met His Trp Tyr Gln Gln Lys Pro Gly Gln Pro Pro
35 40 45

Lys Leu Leu Ile Tyr Leu Ala Ser Asn Leu Glu Ser Gly Val Pro Ala
50 55 60

Arg Phe Ser Gly Ser Gly Ser Arg Thr Asp Phe Thr Leu Thr Ile Asp
65 70 75 80

Pro Val Glu Ala Asp Asp Ala Ala Thr Tyr Tyr Cys Gln Gln Asn Asn
85 90 95

Glu Asp Pro Leu Thr Phe Gly Gly Gly Thr Lys Leu Glu Leu Lys Arg
100 105 110

<210> 5
<211> 265
<212> PRT
<213> Artificial

<220>
<223> scFvMCSPHL

<400> 5

Met Ala Asp Tyr Lys Gln Val Lys Leu Gln Gln Ser Gly Pro Glu Leu
1 5 10 15

Val Lys Pro Gly Ala Ser Val Lys Ile Ser Cys Lys Ala Ser Gly Tyr
20 25 30

```
Ala Phe Ser Arg Ser Trp Met Asn Trp Val Lys Gln Arg Pro Gly Gln
        35                  40                  45

Gly Leu Glu Trp Ile Gly Arg Ile Tyr Pro Gly Asp Gly Asp Thr Asn
    50                  55                  60

Tyr Asn Gly Lys Phe Lys Gly Lys Ala Thr Leu Thr Ala Asp Lys Ser
65                  70                  75                  80

Ser Ser Thr Ala Tyr Met Gln Val Ser Ser Leu Thr Ser Val Asp Ser
                85                  90                  95

Ala Val Tyr Phe Cys Ala Arg Gly Asn Thr Val Val Val Pro Tyr Thr
            100                 105                 110

Met Asp Tyr Trp Gly Gln Gly Thr Thr Val Thr Val Ser Ser Gly Gly
        115                 120                 125

Gly Gly Ser Gly Gly Gly Gly Ser Gly Gly Gly Gly Ser Asp Ile Glu
    130                 135                 140

Leu Thr Gln Ser Pro Ala Ser Leu Ala Val Ser Leu Gly Gln Arg Ala
145                 150                 155                 160

Thr Ile Ser Cys Arg Ala Ser Glu Ser Val Asp Ser Tyr Gly Asn Ser
                165                 170                 175

Phe Met His Trp Tyr Gln Gln Lys Pro Gly Gln Pro Pro Lys Leu Leu
            180                 185                 190

Ile Tyr Leu Ala Ser Asn Leu Glu Ser Gly Val Pro Ala Arg Phe Ser
        195                 200                 205

Gly Ser Gly Ser Arg Thr Asp Phe Thr Leu Thr Ile Asp Pro Val Glu
    210                 215                 220

Ala Asp Asp Ala Ala Thr Tyr Tyr Cys Gln Gln Asn Asn Glu Asp Pro
225                 230                 235                 240

Leu Thr Phe Gly Gly Gly Thr Lys Leu Glu Leu Lys Arg Ser Ala Ser
                245                 250                 255

Gly Ala Asp His His His His His His
            260                 265
```

<210> 6
<211> 269

<212> PRT
<213> Artificial

<220>
<223> scFvMCSP

<400> 6

```
Met Ala Asp Tyr Lys Asp Ile Val Met Thr Gln Ser Pro Ala Ser Leu
1               5                   10                  15

Ala Val Ser Leu Gly Gln Arg Ala Thr Ile Ser Cys Arg Ala Ser Glu
            20                  25                  30

Ser Val Asp Ser Tyr Gly Asn Ser Phe Met His Trp Tyr Gln Gln Lys
        35                  40                  45

Pro Gly Gln Pro Pro Lys Leu Leu Ile Tyr Leu Ala Ser Asn Leu Glu
    50                  55                  60

Ser Gly Val Pro Ala Arg Phe Ser Gly Ser Gly Ser Arg Thr Asp Phe
65                  70                  75                  80

Thr Leu Thr Ile Asp Pro Val Glu Ala Asp Asp Ala Ala Thr Tyr Tyr
                85                  90                  95

Cys Gln Gln Asn Asn Glu Asp Pro Leu Thr Phe Gly Ser Gly Thr Lys
            100                 105                 110

Leu Glu Thr Lys Arg Gly Gly Gly Gly Ser Gly Gly Gly Gly Ser Gly
        115                 120                 125

Gly Gly Gly Ser Gly Gly Gly Gly Ser Gln Val Gln Leu Lys Gln Ser
    130                 135                 140

Gly Pro Glu Leu Val Lys Pro Gly Ala Ser Val Lys Ile Ser Cys Lys
145                 150                 155                 160

Ala Ser Gly Tyr Ala Phe Ser Arg Ser Trp Met Asn Trp Val Lys Gln
                165                 170                 175

Arg Pro Gly Gln Gly Leu Glu Trp Ile Gly Arg Ile Tyr Pro Gly Asp
            180                 185                 190

Gly Asp Thr Asn Tyr Asn Gly Lys Phe Lys Gly Lys Ala Thr Leu Thr
        195                 200                 205

Ala Asp Lys Ser Ser Ser Thr Ala Tyr Met Gln Val Ser Ser Leu Thr
    210                 215                 220
```

```
Ser Val Asp Ser Ala Val Tyr Phe Cys Ala Arg Gly Asn Thr Val Val
225                 230                 235                 240

Val Pro Tyr Thr Met Asp Tyr Trp Gly Gln Gly Thr Ser Val Thr Val
                245                 250                 255

Pro Ser Ala Ser Gly Ala Asp His His His His His His
            260                 265
```

<210>  7
<211>  666
<212>  PRT
<213>  Artificial

<220>
<223>  MCSPHL-ETA

<400>  7

```
Trp Ser His Pro Gln Phe Glu Lys Ile Glu Gly Arg His His His His
1               5                   10                  15

His His Gly Ala Gln Pro Ala Met Ala Asp Tyr Lys Gln Val Lys Leu
            20                  25                  30

Gln Gln Ser Gly Pro Glu Leu Val Lys Pro Gly Ala Ser Val Lys Ile
        35                  40                  45

Ser Cys Lys Ala Ser Gly Tyr Ala Phe Ser Arg Ser Trp Met Asn Trp
    50                  55                  60

Val Lys Gln Arg Pro Gly Gln Gly Leu Glu Trp Ile Gly Arg Ile Tyr
65                  70                  75                  80

Pro Gly Asp Gly Asp Thr Asn Tyr Asn Gly Lys Phe Lys Gly Lys Ala
                85                  90                  95

Thr Leu Thr Ala Asp Lys Ser Ser Ser Thr Ala Tyr Met Gln Val Ser
            100                 105                 110

Ser Leu Thr Ser Val Asp Ser Ala Val Tyr Phe Cys Ala Arg Gly Asn
        115                 120                 125

Thr Val Val Val Pro Tyr Thr Met Asp Tyr Trp Gly Gln Gly Thr Thr
    130                 135                 140

Val Thr Val Ser Ser Gly Gly Gly Gly Ser Gly Gly Gly Gly Ser Gly
145                 150                 155                 160
```

Gly Gly Gly Ser Asp Ile Glu Leu Thr Gln Ser Pro Ala Ser Leu Ala
165 170 175

Val Ser Leu Gly Gln Arg Ala Thr Ile Ser Cys Arg Ala Ser Glu Ser
180 185 190

Val Asp Ser Tyr Gly Asn Ser Phe Met His Trp Tyr Gln Gln Lys Pro
195 200 205

Gly Gln Pro Pro Lys Leu Leu Ile Tyr Leu Ala Ser Asn Leu Glu Ser
210 215 220

Gly Val Pro Ala Arg Phe Ser Gly Ser Gly Ser Arg Thr Asp Phe Thr
225 230 235 240

Leu Thr Ile Asp Pro Val Glu Ala Asp Asp Ala Ala Thr Tyr Tyr Cys
245 250 255

Gln Gln Asn Asn Glu Asp Pro Leu Thr Phe Gly Gly Gly Thr Lys Leu
260 265 270

Glu Leu Lys Arg Ser Ala Ser Gly Ala Gly Gly Gly Gly Ser Gly Gly
275 280 285

Gly Gly Ser Gly Gly Gly Gly Ser Gly Gly Gly Gly Ser Ala Ala Ala
290 295 300

Leu Glu Gly Gly Ser Leu Ala Ala Leu Thr Ala His Gln Ala Cys His
305 310 315 320

Leu Pro Leu Glu Thr Phe Thr Arg His Arg Gln Pro Arg Gly Trp Glu
325 330 335

Gln Leu Glu Gln Cys Gly Tyr Pro Val Gln Arg Leu Val Ala Leu Tyr
340 345 350

Leu Ala Ala Arg Leu Ser Trp Asn Gln Val Asp Gln Val Ile Arg Asn
355 360 365

Ala Leu Ala Ser Pro Gly Ser Gly Gly Asp Leu Gly Glu Ala Ile Arg
370 375 380

Glu Gln Pro Glu Gln Ala Arg Leu Ala Leu Thr Leu Ala Ala Ala Glu
385 390 395 400

Ser Glu Arg Phe Val Arg Gln Gly Thr Gly Asn Asp Glu Ala Gly Ala
405 410 415

```
Ala Asn Ala Asp Val Val Ser Leu Thr Cys Pro Val Ala Ala Gly Glu
            420                 425                 430

Cys Ala Gly Pro Ala Asp Ser Gly Asp Ala Leu Leu Glu Arg Asn Tyr
        435                 440                 445

Pro Thr Gly Ala Glu Phe Leu Gly Asp Gly Gly Asp Val Ser Phe Ser
    450                 455                 460

Thr Arg Gly Thr Gln Asn Trp Thr Val Glu Arg Leu Leu Gln Ala His
465                 470                 475                 480

Arg Gln Leu Glu Glu Arg Gly Tyr Val Phe Val Gly Tyr His Gly Thr
                485                 490                 495

Phe Leu Glu Ala Ala Gln Ser Ile Val Phe Gly Gly Val Arg Ala Arg
            500                 505                 510

Ser Gln Asp Leu Asp Ala Ile Trp Arg Gly Phe Tyr Ile Ala Gly Asp
        515                 520                 525

Pro Ala Leu Ala Tyr Gly Tyr Ala Gln Asp Gln Glu Pro Asp Ala Arg
    530                 535                 540

Gly Arg Ile Arg Asn Gly Ala Leu Leu Arg Val Tyr Val Pro Arg Ser
545                 550                 555                 560

Ser Leu Pro Gly Phe Tyr Arg Thr Gly Leu Thr Leu Ala Ala Pro Glu
                565                 570                 575

Ala Ala Gly Glu Val Glu Arg Leu Ile Gly His Pro Leu Pro Leu Arg
            580                 585                 590

Leu Asp Ala Ile Thr Gly Pro Glu Glu Glu Gly Gly Arg Leu Glu Thr
        595                 600                 605

Ile Leu Gly Trp Pro Leu Ala Glu Arg Thr Val Val Ile Pro Ser Ala
    610                 615                 620

Ile Pro Thr Asp Pro Arg Asn Val Gly Gly Asp Leu Asp Pro Ser Ser
625                 630                 635                 640

Ile Pro Asp Lys Glu Gln Ala Ile Ser Ala Leu Pro Asp Tyr Ala Ser
                645                 650                 655

Gln Pro Gly Lys Pro Pro Lys Asp Glu Leu
            660                 665
```

<210> 8
<211> 670
<212> PRT
<213> Artificial

<220>
<223> MCSP-ETA

<400> 8

```
Trp Ser His Pro Gln Phe Glu Lys Ile Glu Gly Arg His His His His
1               5                   10                  15

His His Gly Ala Gln Pro Ala Met Ala Asp Tyr Lys Asp Ile Val Met
            20                  25                  30

Thr Gln Ser Pro Ala Ser Leu Ala Val Ser Leu Gly Gln Arg Ala Thr
        35                  40                  45

Ile Ser Cys Arg Ala Ser Glu Ser Val Asp Ser Tyr Gly Asn Ser Phe
    50                  55                  60

Met His Trp Tyr Gln Gln Lys Pro Gly Gln Pro Pro Lys Leu Leu Ile
65                  70                  75                  80

Tyr Leu Ala Ser Asn Leu Glu Ser Gly Val Pro Ala Arg Phe Ser Gly
                85                  90                  95

Ser Gly Ser Arg Thr Asp Phe Thr Leu Thr Ile Asp Pro Val Glu Ala
            100                 105                 110

Asp Asp Ala Ala Thr Tyr Tyr Cys Gln Gln Asn Asn Glu Asp Pro Leu
        115                 120                 125

Thr Phe Gly Ser Gly Thr Lys Leu Glu Thr Lys Arg Gly Gly Gly Gly
    130                 135                 140

Ser Gly Gly Gly Gly Ser Gly Gly Gly Gly Ser Gly Gly Gly Gly Ser
145                 150                 155                 160

Gln Val Gln Leu Lys Gln Ser Gly Pro Glu Leu Val Lys Pro Gly Ala
                165                 170                 175

Ser Val Lys Ile Ser Cys Lys Ala Ser Gly Tyr Ala Phe Ser Arg Ser
            180                 185                 190

Trp Met Asn Trp Val Lys Gln Arg Pro Gly Gln Gly Leu Glu Trp Ile
        195                 200                 205
```

```
Gly Arg Ile Tyr Pro Gly Asp Gly Asp Thr Asn Tyr Asn Gly Lys Phe
    210                 215                 220

Lys Gly Lys Ala Thr Leu Thr Ala Asp Lys Ser Ser Ser Thr Ala Tyr
225                 230                 235                 240

Met Gln Val Ser Ser Leu Thr Ser Val Asp Ser Ala Val Tyr Phe Cys
                245                 250                 255

Ala Arg Gly Asn Thr Val Val Val Pro Tyr Thr Met Asp Tyr Trp Gly
            260                 265                 270

Gln Gly Thr Ser Val Thr Val Pro Ser Ala Ser Gly Ala Gly Gly Gly
        275                 280                 285

Gly Ser Gly Gly Gly Gly Ser Gly Gly Gly Gly Ser Gly Gly Gly Gly
    290                 295                 300

Ser Ala Ala Ala Leu Glu Gly Gly Ser Leu Ala Ala Leu Thr Ala His
305                 310                 315                 320

Gln Ala Cys His Leu Pro Leu Glu Thr Phe Thr Arg His Arg Gln Pro
                325                 330                 335

Arg Gly Trp Glu Gln Leu Glu Gln Cys Gly Tyr Pro Val Gln Arg Leu
            340                 345                 350

Val Ala Leu Tyr Leu Ala Ala Arg Leu Ser Trp Asn Gln Val Asp Gln
        355                 360                 365

Val Ile Arg Asn Ala Leu Ala Ser Pro Gly Ser Gly Gly Asp Leu Gly
    370                 375                 380

Glu Ala Ile Arg Glu Gln Pro Glu Gln Ala Arg Leu Ala Leu Thr Leu
385                 390                 395                 400

Ala Ala Ala Glu Ser Glu Arg Phe Val Arg Gln Gly Thr Gly Asn Asp
                405                 410                 415

Glu Ala Gly Ala Ala Asn Ala Asp Val Val Ser Leu Thr Cys Pro Val
            420                 425                 430

Ala Ala Gly Glu Cys Ala Gly Pro Ala Asp Ser Gly Asp Ala Leu Leu
        435                 440                 445

Glu Arg Asn Tyr Pro Thr Gly Ala Glu Phe Leu Gly Asp Gly Gly Asp
    450                 455                 460
```

```
Val Ser Phe Ser Thr Arg Gly Thr Gln Asn Trp Thr Val Glu Arg Leu
465                 470                 475                 480

Leu Gln Ala His Arg Gln Leu Glu Glu Arg Gly Tyr Val Phe Val Gly
                485                 490                 495

Tyr His Gly Thr Phe Leu Glu Ala Ala Gln Ser Ile Val Phe Gly Gly
            500                 505                 510

Val Arg Ala Arg Ser Gln Asp Leu Asp Ala Ile Trp Arg Gly Phe Tyr
        515                 520                 525

Ile Ala Gly Asp Pro Ala Leu Ala Tyr Gly Tyr Ala Gln Asp Gln Glu
    530                 535                 540

Pro Asp Ala Arg Gly Arg Ile Arg Asn Gly Ala Leu Leu Arg Val Tyr
545                 550                 555                 560

Val Pro Arg Ser Ser Leu Pro Gly Phe Tyr Arg Thr Gly Leu Thr Leu
                565                 570                 575

Ala Ala Pro Glu Ala Ala Gly Glu Val Glu Arg Leu Ile Gly His Pro
            580                 585                 590

Leu Pro Leu Arg Leu Asp Ala Ile Thr Gly Pro Glu Glu Glu Gly Gly
        595                 600                 605

Arg Leu Glu Thr Ile Leu Gly Trp Pro Leu Ala Glu Arg Thr Val Val
    610                 615                 620

Ile Pro Ser Ala Ile Pro Thr Asp Pro Arg Asn Val Gly Gly Asp Leu
625                 630                 635                 640

Asp Pro Ser Ser Ile Pro Asp Lys Glu Gln Ala Ile Ser Ala Leu Pro
                645                 650                 655

Asp Tyr Ala Ser Gln Pro Gly Lys Pro Pro Lys Asp Glu Leu
            660                 665                 670

<210>  9
<211>  78
<212>  DNA
<213>  Artificial

<220>
<223>  primer

<400>  9
```

aaaggatccg gtggtggcgg cagcggtggt ggcggcagcg atattgaact gacgcaatct 60

ccagcttctt tggctgtg 78

<210> 10
<211> 95
<212> DNA
<213> Artificial

<220>
<223> primer

<400> 10
tttggccccc gaggccgaac gtttcagttc cagcttggtc ccgccgccaa acgtcagagg 60

gtcttcatta ttttgttgac agtaataggt tgcag 95

<210> 11
<211> 61
<212> DNA
<213> Artificial

<220>
<223> primer

<400> 11
aaaccatggc cgattataaa caggtgaaac tgcagcagtc tggacctgag ctggtgaagc 60

c 61

<210> 12
<211> 62
<212> DNA
<213> Artificial

<220>
<223> primer

<400> 12
tttggatcca ccgccacccg aactcacggt cacggtggtt ccttgacccc agtagtccat 60

ag 62

<210> 13
<211> 18
<212> DNA
<213> Artificial

<220>
<223> primer

<400> 13
agtggcagtg gatctagg 18

<210> 14
<211> 17
<212> DNA
<213> Artificial

<220>
<223>  primer

<400>  14
gtaggctgtg ctggagg                                                    17

<210>  15
<211>  19
<212>  DNA
<213>  Artificial

<220>
<223>  primer

<400>  15
gtcgcactgg ctggtttcg                                                  19

<210>  16
<211>  19
<212>  DNA
<213>  Artificial

<220>
<223>  primer

<400>  16
gtagcggtaa acggcagac                                                  19

<210>  17
<211>  19
<212>  DNA
<213>  Artificial

<220>
<223>  primer

<400>  17
cacaggaaac agctatgac                                                  19

<210>  18
<211>  18
<212>  DNA
<213>  Artificial

<220>
<223>  primer

<400>  18
gacgcagtag cggtaaac                                                   18

<210>  19
<211>  18
<212>  DNA
<213>  Artificial

<220>
<223>  primer

```
<400>  19
gcctacggca gccgctgg                                                   18


<210>  20
<211>  19
<212>  DNA
<213>  Artificial

<220>
<223>  primer

<400>  20
ttcacttcac aggtcaagc                                                  19


<210>  21
<211>  19
<212>  DNA
<213>  Artificial

<220>
<223>  primer

<400>  21
tatagttcct cctttcagc                                                  19


<210>  22
<211>  2108
<212>  DNA
<213>  Artificial

<220>
<223>  MCSPhl CD28/TCRZeta


<220>
<221>  CDS
<222>  (12)..(2108)

<400>  22
tctagactgc c atg gat ttt cag gtg cag att ttc agc ttc ctg cta atc      50
             Met Asp Phe Gln Val Gln Ile Phe Ser Phe Leu Leu Ile
             1               5                   10

agt gcc tca gtc ata atg tct aga atg gcc gat tat aaa cag gtg aaa       98
Ser Ala Ser Val Ile Met Ser Arg Met Ala Asp Tyr Lys Gln Val Lys
    15                  20                  25

ctg cag cag tct gga cct gag ctg gtg aag cct ggg gcc tca gtg aag      146
Leu Gln Gln Ser Gly Pro Glu Leu Val Lys Pro Gly Ala Ser Val Lys
30                  35                  40                  45

att tcc tgc aaa gct tct ggc tac gca ttc agt agg tct tgg atg aac      194
Ile Ser Cys Lys Ala Ser Gly Tyr Ala Phe Ser Arg Ser Trp Met Asn
                50                  55                  60

tgg gtg aag cag agg cct gga cag ggt ctt gag tgg att gga cgg att      242
Trp Val Lys Gln Arg Pro Gly Gln Gly Leu Glu Trp Ile Gly Arg Ile
            65                  70                  75
```

```
tat cct gga gat gga gat act aac tac aat ggg aag ttc aag ggc aag      290
Tyr Pro Gly Asp Gly Asp Thr Asn Tyr Asn Gly Lys Phe Lys Gly Lys
        80                  85                  90

gcc aca ctg act gca gac aaa tcc tcc agc aca gcc tac atg cag gtc      338
Ala Thr Leu Thr Ala Asp Lys Ser Ser Ser Thr Ala Tyr Met Gln Val
    95                  100                 105

agc agc ctg acc tct gtg gac tct gcg gtc tat ttc tgt gca aga ggg      386
Ser Ser Leu Thr Ser Val Asp Ser Ala Val Tyr Phe Cys Ala Arg Gly
110                 115                 120                 125

aat acg gta gta gtt ccc tat act atg gac tac tgg ggt caa gga acc      434
Asn Thr Val Val Val Pro Tyr Thr Met Asp Tyr Trp Gly Gln Gly Thr
                130                 135                 140

acc gtg acc gtg agt tcg ggt ggc ggt gga tcc ggt ggt ggc ggc agc      482
Thr Val Thr Val Ser Ser Gly Gly Gly Gly Ser Gly Gly Gly Gly Ser
            145                 150                 155

ggt ggt ggc ggc agc gat att gaa ctg acg caa tct cca gct tct ttg      530
Gly Gly Gly Gly Ser Asp Ile Glu Leu Thr Gln Ser Pro Ala Ser Leu
        160                 165                 170

gct gtg tct cta ggg cag agg gcc acc ata tcc tgc aga gcc agt gaa      578
Ala Val Ser Leu Gly Gln Arg Ala Thr Ile Ser Cys Arg Ala Ser Glu
    175                 180                 185

agt gtt gat agt tat ggc aat agt ttt atg cac tgg tac cag cag aaa      626
Ser Val Asp Ser Tyr Gly Asn Ser Phe Met His Trp Tyr Gln Gln Lys
190                 195                 200                 205

cca gga cag cca ccc aaa ctc ctc atc tat ctt gca tcc aac cta gaa      674
Pro Gly Gln Pro Pro Lys Leu Leu Ile Tyr Leu Ala Ser Asn Leu Glu
                210                 215                 220

tct ggg gtc cct gcc agg ttc agt ggc agt gga tct agg aca gac ttc      722
Ser Gly Val Pro Ala Arg Phe Ser Gly Ser Gly Ser Arg Thr Asp Phe
            225                 230                 235

acc ctc acc att gat cct gtg gag gct gat gat gct gca acc tat tac      770
Thr Leu Thr Ile Asp Pro Val Glu Ala Asp Asp Ala Ala Thr Tyr Tyr
        240                 245                 250

tgt caa caa aat aat gaa gac cct ctg acg ttt ggc ggc ggg acc aag      818
Cys Gln Gln Asn Asn Glu Asp Pro Leu Thr Phe Gly Gly Gly Thr Lys
    255                 260                 265

ctg gaa ctg aaa cgt tcg gcc tcg ggg gcc gat ccc atg gag ccc aaa      866
Leu Glu Leu Lys Arg Ser Ala Ser Gly Ala Asp Pro Met Glu Pro Lys
270                 275                 280                 285

tct cct gac aaa act cac aca tgc cca ccg tgc cca gca cct gaa ctc      914
Ser Pro Asp Lys Thr His Thr Cys Pro Pro Cys Pro Ala Pro Glu Leu
                290                 295                 300

ctg ggg gga ccg tca gtc ttc ctc ttc ccc cca aaa ccc aag gac acc      962
Leu Gly Gly Pro Ser Val Phe Leu Phe Pro Pro Lys Pro Lys Asp Thr
            305                 310                 315

ctc atg atc tcc cgg acc cct gag gtc aca tgc gtg gtg gtg gac gtg     1010
Leu Met Ile Ser Arg Thr Pro Glu Val Thr Cys Val Val Val Asp Val
        320                 325                 330
```

```
agc cac gaa gac cct gag gtc aag ttc aac tgg tac gtg gac ggc gtg      1058
Ser His Glu Asp Pro Glu Val Lys Phe Asn Trp Tyr Val Asp Gly Val
    335                 340                 345

gag gtg cat aat gcc aag aca aag ccg cgg gag gag cag tac aac agc      1106
Glu Val His Asn Ala Lys Thr Lys Pro Arg Glu Glu Gln Tyr Asn Ser
350                 355                 360                 365

acg tac cgt gtg gtc agc gtc ctc acc gtc ctg cac cag gac tgg ctg      1154
Thr Tyr Arg Val Val Ser Val Leu Thr Val Leu His Gln Asp Trp Leu
                370                 375                 380

aat ggc aag gag tac aag tgc aag gtc tcc aac aaa gcc ctc cca gcc      1202
Asn Gly Lys Glu Tyr Lys Cys Lys Val Ser Asn Lys Ala Leu Pro Ala
            385                 390                 395

ccc atc gag aaa acc atc tcc aaa gcc aaa ggg cag ccc cga gaa cca      1250
Pro Ile Glu Lys Thr Ile Ser Lys Ala Lys Gly Gln Pro Arg Glu Pro
        400                 405                 410

cag gtg tac acc ctg ccc cca tcc cgg gat gag ctg acc aag aac cag      1298
Gln Val Tyr Thr Leu Pro Pro Ser Arg Asp Glu Leu Thr Lys Asn Gln
    415                 420                 425

gtc agc gtg acc tgc ctg gtc aaa ggc ttc tat ccc agc gac atc gcc      1346
Val Ser Val Thr Cys Leu Val Lys Gly Phe Tyr Pro Ser Asp Ile Ala
430                 435                 440                 445

gtg gag tgg gag agc aat ggg cag ccg gag aac aac tac aag acc acg      1394
Val Glu Trp Glu Ser Asn Gly Gln Pro Glu Asn Asn Tyr Lys Thr Thr
                450                 455                 460

cct ccc gtg ctg gac tcc gac ggc tcc ttc ttc ctc tac agc aag ctc      1442
Pro Pro Val Leu Asp Ser Asp Gly Ser Phe Phe Leu Tyr Ser Lys Leu
            465                 470                 475

acc gtg gac aag agc agg tgg cag cag ggg aac gtc ttc tca tgc tcc      1490
Thr Val Asp Lys Ser Arg Trp Gln Gln Gly Asn Val Phe Ser Cys Ser
        480                 485                 490

gtg atg cat gag gct ctg cac aac cac tac acg cag aag agc ctc tcc      1538
Val Met His Glu Ala Leu His Asn His Tyr Thr Gln Lys Ser Leu Ser
    495                 500                 505

ctg tct ccg ggt aaa aaa gat ccc aaa ttt tgg gtg ctg gtg gtg gtt      1586
Leu Ser Pro Gly Lys Lys Asp Pro Lys Phe Trp Val Leu Val Val Val
510                 515                 520                 525

ggt gga gtc ctg gct tgc tat agc ttg cta gta aca gtg gcc ttt att      1634
Gly Gly Val Leu Ala Cys Tyr Ser Leu Leu Val Thr Val Ala Phe Ile
                530                 535                 540

att ttc tgg gtg agg agt aag agg agc agg ctc ctg cac agt gac tac      1682
Ile Phe Trp Val Arg Ser Lys Arg Ser Arg Leu Leu His Ser Asp Tyr
            545                 550                 555

atg aac atg act ccc cgc cgc ccc ggg ccc acc cgc aag cat tac cag      1730
Met Asn Met Thr Pro Arg Arg Pro Gly Pro Thr Arg Lys His Tyr Gln
        560                 565                 570

ccc tat gcc cca cca cgc gac ttc gca gcc tat cgc tcc ctg aga gtg      1778
Pro Tyr Ala Pro Pro Arg Asp Phe Ala Ala Tyr Arg Ser Leu Arg Val
```

```
    575                 580                 585

aag ttc agc agg agc gca gac gcc ccc gcg tac cag cag ggc cag aac      1826
Lys Phe Ser Arg Ser Ala Asp Ala Pro Ala Tyr Gln Gln Gly Gln Asn
590                 595                 600                 605

cag ctc tat aac gag ctc aat cta gga cga aga gag gag tac gat gtt      1874
Gln Leu Tyr Asn Glu Leu Asn Leu Gly Arg Arg Glu Glu Tyr Asp Val
                610                 615                 620

ttg gac aag aga cgt ggc cgg gac cct gag atg ggg gga aag ccg aga      1922
Leu Asp Lys Arg Arg Gly Arg Asp Pro Glu Met Gly Gly Lys Pro Arg
            625                 630                 635

agg aag aac cct cag gaa ggc ctg tac aat gaa ctg cag aaa gat aag      1970
Arg Lys Asn Pro Gln Glu Gly Leu Tyr Asn Glu Leu Gln Lys Asp Lys
        640                 645                 650

atg gcg gag gcc tac agt gag att ggg atg aaa ggc gag cgc cgg agg      2018
Met Ala Glu Ala Tyr Ser Glu Ile Gly Met Lys Gly Glu Arg Arg Arg
    655                 660                 665

ggc aag ggg cac gat ggc ctt tac cag ggt ctc agt aca gcc acc aag      2066
Gly Lys Gly His Asp Gly Leu Tyr Gln Gly Leu Ser Thr Ala Thr Lys
670                 675                 680                 685

gac acc tac gac gcc ctt cac atg cag gcc ctg ccc cct cgc              2108
Asp Thr Tyr Asp Ala Leu His Met Gln Ala Leu Pro Pro Arg
                690                 695


<210>  23
<211>  699
<212>  PRT
<213>  Artificial

<220>
<223>  Synthetic Construct

<400>  23

Met Asp Phe Gln Val Gln Ile Phe Ser Phe Leu Leu Ile Ser Ala Ser
1               5                   10                  15


Val Ile Met Ser Arg Met Ala Asp Tyr Lys Gln Val Lys Leu Gln Gln
            20                  25                  30


Ser Gly Pro Glu Leu Val Lys Pro Gly Ala Ser Val Lys Ile Ser Cys
        35                  40                  45


Lys Ala Ser Gly Tyr Ala Phe Ser Arg Ser Trp Met Asn Trp Val Lys
    50                  55                  60


Gln Arg Pro Gly Gln Gly Leu Glu Trp Ile Gly Arg Ile Tyr Pro Gly
65                  70                  75                  80


Asp Gly Asp Thr Asn Tyr Asn Gly Lys Phe Lys Gly Lys Ala Thr Leu
                85                  90                  95
```

```
Thr Ala Asp Lys Ser Ser Ser Thr Ala Tyr Met Gln Val Ser Ser Leu
                100                 105                 110

Thr Ser Val Asp Ser Ala Val Tyr Phe Cys Ala Arg Gly Asn Thr Val
            115                 120                 125

Val Val Pro Tyr Thr Met Asp Tyr Trp Gly Gln Gly Thr Thr Val Thr
    130                 135                 140

Val Ser Ser Gly Gly Gly Gly Ser Gly Gly Gly Gly Ser Gly Gly Gly
145                 150                 155                 160

Gly Ser Asp Ile Glu Leu Thr Gln Ser Pro Ala Ser Leu Ala Val Ser
                165                 170                 175

Leu Gly Gln Arg Ala Thr Ile Ser Cys Arg Ala Ser Glu Ser Val Asp
            180                 185                 190

Ser Tyr Gly Asn Ser Phe Met His Trp Tyr Gln Gln Lys Pro Gly Gln
        195                 200                 205

Pro Pro Lys Leu Leu Ile Tyr Leu Ala Ser Asn Leu Glu Ser Gly Val
    210                 215                 220

Pro Ala Arg Phe Ser Gly Ser Gly Ser Arg Thr Asp Phe Thr Leu Thr
225                 230                 235                 240

Ile Asp Pro Val Glu Ala Asp Asp Ala Ala Thr Tyr Tyr Cys Gln Gln
                245                 250                 255

Asn Asn Glu Asp Pro Leu Thr Phe Gly Gly Gly Thr Lys Leu Glu Leu
            260                 265                 270

Lys Arg Ser Ala Ser Gly Ala Asp Pro Met Glu Pro Lys Ser Pro Asp
        275                 280                 285

Lys Thr His Thr Cys Pro Pro Cys Pro Ala Pro Glu Leu Leu Gly Gly
    290                 295                 300

Pro Ser Val Phe Leu Phe Pro Pro Lys Pro Lys Asp Thr Leu Met Ile
305                 310                 315                 320

Ser Arg Thr Pro Glu Val Thr Cys Val Val Val Asp Val Ser His Glu
                325                 330                 335

Asp Pro Glu Val Lys Phe Asn Trp Tyr Val Asp Gly Val Glu Val His
```

```
            340                 345                 350

Asn Ala Lys Thr Lys Pro Arg Glu Glu Gln Tyr Asn Ser Thr Tyr Arg
        355                 360                 365

Val Val Ser Val Leu Thr Val Leu His Gln Asp Trp Leu Asn Gly Lys
    370                 375                 380

Glu Tyr Lys Cys Lys Val Ser Asn Lys Ala Leu Pro Ala Pro Ile Glu
385                 390                 395                 400

Lys Thr Ile Ser Lys Ala Lys Gly Gln Pro Arg Glu Pro Gln Val Tyr
                405                 410                 415

Thr Leu Pro Pro Ser Arg Asp Glu Leu Thr Lys Asn Gln Val Ser Val
            420                 425                 430

Thr Cys Leu Val Lys Gly Phe Tyr Pro Ser Asp Ile Ala Val Glu Trp
        435                 440                 445

Glu Ser Asn Gly Gln Pro Glu Asn Asn Tyr Lys Thr Thr Pro Pro Val
    450                 455                 460

Leu Asp Ser Asp Gly Ser Phe Phe Leu Tyr Ser Lys Leu Thr Val Asp
465                 470                 475                 480

Lys Ser Arg Trp Gln Gln Gly Asn Val Phe Ser Cys Ser Val Met His
                485                 490                 495

Glu Ala Leu His Asn His Tyr Thr Gln Lys Ser Leu Ser Leu Ser Pro
            500                 505                 510

Gly Lys Lys Asp Pro Lys Phe Trp Val Leu Val Val Val Gly Gly Val
        515                 520                 525

Leu Ala Cys Tyr Ser Leu Leu Val Thr Val Ala Phe Ile Ile Phe Trp
    530                 535                 540

Val Arg Ser Lys Arg Ser Arg Leu Leu His Ser Asp Tyr Met Asn Met
545                 550                 555                 560

Thr Pro Arg Arg Pro Gly Pro Thr Arg Lys His Tyr Gln Pro Tyr Ala
                565                 570                 575

Pro Pro Arg Asp Phe Ala Ala Tyr Arg Ser Leu Arg Val Lys Phe Ser
            580                 585                 590
```

```
Arg Ser Ala Asp Ala Pro Ala Tyr Gln Gln Gly Gln Asn Gln Leu Tyr
        595                 600                 605

Asn Glu Leu Asn Leu Gly Arg Arg Glu Glu Tyr Asp Val Leu Asp Lys
    610                 615                 620

Arg Arg Gly Arg Asp Pro Glu Met Gly Gly Lys Pro Arg Arg Lys Asn
625                 630                 635                 640

Pro Gln Glu Gly Leu Tyr Asn Glu Leu Gln Lys Asp Lys Met Ala Glu
                645                 650                 655

Ala Tyr Ser Glu Ile Gly Met Lys Gly Glu Arg Arg Arg Gly Lys Gly
            660                 665                 670

His Asp Gly Leu Tyr Gln Gly Leu Ser Thr Ala Thr Lys Asp Thr Tyr
        675                 680                 685

Asp Ala Leu His Met Gln Ala Leu Pro Pro Arg
    690                 695


<210>  24
<211>  1964
<212>  DNA
<213>  Artificial

<220>
<223>  MCSPhl TCRZeta


<220>
<221>  CDS
<222>  (12)..(1964)

<400>  24
tctagactgc c atg gat ttt cag gtg cag att ttc agc ttc ctg cta atc       50
             Met Asp Phe Gln Val Gln Ile Phe Ser Phe Leu Leu Ile
             1               5                   10

agt gcc tca gtc ata atg tct aga atg gcc gat tat aaa cag gtg aaa       98
Ser Ala Ser Val Ile Met Ser Arg Met Ala Asp Tyr Lys Gln Val Lys
    15                  20                  25

ctg cag cag tct gga cct gag ctg gtg aag cct ggg gcc tca gtg aag      146
Leu Gln Gln Ser Gly Pro Glu Leu Val Lys Pro Gly Ala Ser Val Lys
30                  35                  40                  45

att tcc tgc aaa gct tct ggc tac gca ttc agt agg tct tgg atg aac      194
Ile Ser Cys Lys Ala Ser Gly Tyr Ala Phe Ser Arg Ser Trp Met Asn
                50                  55                  60

tgg gtg aag cag agg cct gga cag ggt ctt gag tgg att gga cgg att      242
Trp Val Lys Gln Arg Pro Gly Gln Gly Leu Glu Trp Ile Gly Arg Ile
            65                  70                  75

tat cct gga gat gga gat act aac tac aat ggg aag ttc aag ggc aag      290
```

```
Tyr Pro Gly Asp Gly Asp Thr Asn Tyr Asn Gly Lys Phe Lys Gly Lys
        80                  85                  90

gcc aca ctg act gca gac aaa tcc tcc agc aca gcc tac atg cag gtc      338
Ala Thr Leu Thr Ala Asp Lys Ser Ser Ser Thr Ala Tyr Met Gln Val
    95                  100                 105

agc agc ctg acc tct gtg gac tct gcg gtc tat ttc tgt gca aga ggg      386
Ser Ser Leu Thr Ser Val Asp Ser Ala Val Tyr Phe Cys Ala Arg Gly
110                 115                 120                 125

aat acg gta gta gtt ccc tat act atg gac tac tgg ggt caa gga acc      434
Asn Thr Val Val Val Pro Tyr Thr Met Asp Tyr Trp Gly Gln Gly Thr
                130                 135                 140

acc gtg acc gtg agt tcg ggt ggc ggt gga tcc ggt ggt ggc ggc agc      482
Thr Val Thr Val Ser Ser Gly Gly Gly Gly Ser Gly Gly Gly Gly Ser
            145                 150                 155

ggt ggt ggc ggc agc gat att gaa ctg acg caa tct cca gct tct ttg      530
Gly Gly Gly Gly Ser Asp Ile Glu Leu Thr Gln Ser Pro Ala Ser Leu
        160                 165                 170

gct gtg tct cta ggg cag agg gcc acc ata tcc tgc aga gcc agt gaa      578
Ala Val Ser Leu Gly Gln Arg Ala Thr Ile Ser Cys Arg Ala Ser Glu
    175                 180                 185

agt gtt gat agt tat ggc aat agt ttt atg cac tgg tac cag cag aaa      626
Ser Val Asp Ser Tyr Gly Asn Ser Phe Met His Trp Tyr Gln Gln Lys
190                 195                 200                 205

cca gga cag cca ccc aaa ctc ctc atc tat ctt gca tcc aac cta gaa      674
Pro Gly Gln Pro Pro Lys Leu Leu Ile Tyr Leu Ala Ser Asn Leu Glu
                210                 215                 220

tct ggg gtc cct gcc agg ttc agt ggc agt gga tct agg aca gac ttc      722
Ser Gly Val Pro Ala Arg Phe Ser Gly Ser Gly Ser Arg Thr Asp Phe
            225                 230                 235

acc ctc acc att gat cct gtg gag gct gat gat gct gca acc tat tac      770
Thr Leu Thr Ile Asp Pro Val Glu Ala Asp Asp Ala Ala Thr Tyr Tyr
        240                 245                 250

tgt caa caa aat aat gaa gac cct ctg acg ttt ggc ggc ggg acc aag      818
Cys Gln Gln Asn Asn Glu Asp Pro Leu Thr Phe Gly Gly Gly Thr Lys
    255                 260                 265

ctg gaa ctg aaa cgt tcg gcc tcg ggg gcc gat ccc atg gag ccc aaa      866
Leu Glu Leu Lys Arg Ser Ala Ser Gly Ala Asp Pro Met Glu Pro Lys
270                 275                 280                 285

tct cct gac aaa act cac aca tgc cca ccg tgc cca gca cct gaa ctc      914
Ser Pro Asp Lys Thr His Thr Cys Pro Pro Cys Pro Ala Pro Glu Leu
                290                 295                 300

ctg ggg gga ccg tca gtc ttc ctc ttc ccc cca aaa ccc aag gac acc      962
Leu Gly Gly Pro Ser Val Phe Leu Phe Pro Pro Lys Pro Lys Asp Thr
            305                 310                 315

ctc atg atc tcc cgg acc cct gag gtc aca tgc gtg gtg gtg gac gtg     1010
Leu Met Ile Ser Arg Thr Pro Glu Val Thr Cys Val Val Val Asp Val
        320                 325                 330
```

```
agc cac gaa gac cct gag gtc aag ttc aac tgg tac gtg gac ggc gtg      1058
Ser His Glu Asp Pro Glu Val Lys Phe Asn Trp Tyr Val Asp Gly Val
    335                 340                 345

gag gtg cat aat gcc aag aca aag ccg cgg gag gag cag tac aac agc      1106
Glu Val His Asn Ala Lys Thr Lys Pro Arg Glu Glu Gln Tyr Asn Ser
350                 355                 360                 365

acg tac cgt gtg gtc agc gtc ctc acc gtc ctg cac cag gac tgg ctg      1154
Thr Tyr Arg Val Val Ser Val Leu Thr Val Leu His Gln Asp Trp Leu
                370                 375                 380

aat ggc aag gag tac aag tgc aag gtc tcc aac aaa gcc ctc cca gcc      1202
Asn Gly Lys Glu Tyr Lys Cys Lys Val Ser Asn Lys Ala Leu Pro Ala
            385                 390                 395

ccc atc gag aaa acc atc tcc aaa gcc aaa ggg cag ccc cga gaa cca      1250
Pro Ile Glu Lys Thr Ile Ser Lys Ala Lys Gly Gln Pro Arg Glu Pro
        400                 405                 410

cag gtg tac acc ctg ccc cca tcc cgg gat gag ctg acc aag aac cag      1298
Gln Val Tyr Thr Leu Pro Pro Ser Arg Asp Glu Leu Thr Lys Asn Gln
    415                 420                 425

gtc agc ctg acc tgc ctg gtc aaa ggc ttc tat ccc agc gac atc gcc      1346
Val Ser Leu Thr Cys Leu Val Lys Gly Phe Tyr Pro Ser Asp Ile Ala
430                 435                 440                 445

gtg gag tgg gag agc aat ggg caa ccg gag aac aac tac aag acc acg      1394
Val Glu Trp Glu Ser Asn Gly Gln Pro Glu Asn Asn Tyr Lys Thr Thr
                450                 455                 460

cct ccc gtg ctg gac tcc gac ggc tcc ttc ttc ctc tac agc aag ctc      1442
Pro Pro Val Leu Asp Ser Asp Gly Ser Phe Phe Leu Tyr Ser Lys Leu
            465                 470                 475

acc gtg gac aag agc agg tgg cag cag ggg aac gtc ttc tca tgc tcc      1490
Thr Val Asp Lys Ser Arg Trp Gln Gln Gly Asn Val Phe Ser Cys Ser
        480                 485                 490

gtg atg cat gag gct ctg cac aac cac tac acg cag aag agc ctc tcc      1538
Val Met His Glu Ala Leu His Asn His Tyr Thr Gln Lys Ser Leu Ser
    495                 500                 505

ctg tct ccg ggt aaa aaa gat ccc aaa ctc tgc tac ctg ctg gat gga      1586
Leu Ser Pro Gly Lys Lys Asp Pro Lys Leu Cys Tyr Leu Leu Asp Gly
510                 515                 520                 525

atc ctc ttc atc tat ggt gtc att ctc act gcc ttg ttc ctg aga gtg      1634
Ile Leu Phe Ile Tyr Gly Val Ile Leu Thr Ala Leu Phe Leu Arg Val
                530                 535                 540

aag ttc agc agg agc gca gac gcc ccc gcg tac cag cag ggc cag aac      1682
Lys Phe Ser Arg Ser Ala Asp Ala Pro Ala Tyr Gln Gln Gly Gln Asn
            545                 550                 555

cag ctc tat aac gag ctc aat cta gga cga aga gag gag tac gat gtt      1730
Gln Leu Tyr Asn Glu Leu Asn Leu Gly Arg Arg Glu Glu Tyr Asp Val
        560                 565                 570

ttg gac aag aga cgt ggc cgg gac cct gag atg ggg gga aag ccg aga      1778
Leu Asp Lys Arg Arg Gly Arg Asp Pro Glu Met Gly Gly Lys Pro Arg
    575                 580                 585
```

```
agg aag aac cct cag gaa ggc ctg tac aat gaa ctg cag aaa gat aag      1826
Arg Lys Asn Pro Gln Glu Gly Leu Tyr Asn Glu Leu Gln Lys Asp Lys
590                 595                 600                 605

atg gcg gag gcc tac agt gag att ggg atg aaa ggc gag cgc cgg agg      1874
Met Ala Glu Ala Tyr Ser Glu Ile Gly Met Lys Gly Glu Arg Arg Arg
                610                 615                 620

ggc aag ggg cac gat ggc ctt tac cag ggt ctc agt aca gcc acc aag      1922
Gly Lys Gly His Asp Gly Leu Tyr Gln Gly Leu Ser Thr Ala Thr Lys
            625                 630                 635

gac acc tac gac gcc ctt cac atg cag gcc ctg ccc cct cgc              1964
Asp Thr Tyr Asp Ala Leu His Met Gln Ala Leu Pro Pro Arg
        640                 645                 650


<210>  25
<211>  651
<212>  PRT
<213>  Artificial

<220>
<223>  Synthetic Construct

<400>  25

Met Asp Phe Gln Val Gln Ile Phe Ser Phe Leu Leu Ile Ser Ala Ser
1               5                   10                  15


Val Ile Met Ser Arg Met Ala Asp Tyr Lys Gln Val Lys Leu Gln Gln
            20                  25                  30


Ser Gly Pro Glu Leu Val Lys Pro Gly Ala Ser Val Lys Ile Ser Cys
        35                  40                  45


Lys Ala Ser Gly Tyr Ala Phe Ser Arg Ser Trp Met Asn Trp Val Lys
    50                  55                  60


Gln Arg Pro Gly Gln Gly Leu Glu Trp Ile Gly Arg Ile Tyr Pro Gly
65                  70                  75                  80


Asp Gly Asp Thr Asn Tyr Asn Gly Lys Phe Lys Gly Lys Ala Thr Leu
                85                  90                  95


Thr Ala Asp Lys Ser Ser Ser Thr Ala Tyr Met Gln Val Ser Ser Leu
            100                 105                 110


Thr Ser Val Asp Ser Ala Val Tyr Phe Cys Ala Arg Gly Asn Thr Val
        115                 120                 125


Val Val Pro Tyr Thr Met Asp Tyr Trp Gly Gln Gly Thr Thr Val Thr
    130                 135                 140
```

Val Ser Ser Gly Gly Gly Gly Ser Gly Gly Gly Gly Ser Gly Gly Gly
145 150 155 160

Gly Ser Asp Ile Glu Leu Thr Gln Ser Pro Ala Ser Leu Ala Val Ser
165 170 175

Leu Gly Gln Arg Ala Thr Ile Ser Cys Arg Ala Ser Glu Ser Val Asp
180 185 190

Ser Tyr Gly Asn Ser Phe Met His Trp Tyr Gln Gln Lys Pro Gly Gln
195 200 205

Pro Pro Lys Leu Leu Ile Tyr Leu Ala Ser Asn Leu Glu Ser Gly Val
210 215 220

Pro Ala Arg Phe Ser Gly Ser Gly Ser Arg Thr Asp Phe Thr Leu Thr
225 230 235 240

Ile Asp Pro Val Glu Ala Asp Asp Ala Ala Thr Tyr Tyr Cys Gln Gln
245 250 255

Asn Asn Glu Asp Pro Leu Thr Phe Gly Gly Gly Thr Lys Leu Glu Leu
260 265 270

Lys Arg Ser Ala Ser Gly Ala Asp Pro Met Glu Pro Lys Ser Pro Asp
275 280 285

Lys Thr His Thr Cys Pro Pro Cys Pro Ala Pro Glu Leu Leu Gly Gly
290 295 300

Pro Ser Val Phe Leu Phe Pro Pro Lys Pro Lys Asp Thr Leu Met Ile
305 310 315 320

Ser Arg Thr Pro Glu Val Thr Cys Val Val Val Asp Val Ser His Glu
325 330 335

Asp Pro Glu Val Lys Phe Asn Trp Tyr Val Asp Gly Val Glu Val His
340 345 350

Asn Ala Lys Thr Lys Pro Arg Glu Glu Gln Tyr Asn Ser Thr Tyr Arg
355 360 365

Val Val Ser Val Leu Thr Val Leu His Gln Asp Trp Leu Asn Gly Lys
370 375 380

Glu Tyr Lys Cys Lys Val Ser Asn Lys Ala Leu Pro Ala Pro Ile Glu
385 390 395 400

```
Lys Thr Ile Ser Lys Ala Lys Gly Gln Pro Arg Glu Pro Gln Val Tyr
                405                 410                 415

Thr Leu Pro Pro Ser Arg Asp Glu Leu Thr Lys Asn Gln Val Ser Leu
            420                 425                 430

Thr Cys Leu Val Lys Gly Phe Tyr Pro Ser Asp Ile Ala Val Glu Trp
        435                 440                 445

Glu Ser Asn Gly Gln Pro Glu Asn Asn Tyr Lys Thr Thr Pro Pro Val
    450                 455                 460

Leu Asp Ser Asp Gly Ser Phe Phe Leu Tyr Ser Lys Leu Thr Val Asp
465                 470                 475                 480

Lys Ser Arg Trp Gln Gln Gly Asn Val Phe Ser Cys Ser Val Met His
                485                 490                 495

Glu Ala Leu His Asn His Tyr Thr Gln Lys Ser Leu Ser Leu Ser Pro
            500                 505                 510

Gly Lys Lys Asp Pro Lys Leu Cys Tyr Leu Leu Asp Gly Ile Leu Phe
        515                 520                 525

Ile Tyr Gly Val Ile Leu Thr Ala Leu Phe Leu Arg Val Lys Phe Ser
    530                 535                 540

Arg Ser Ala Asp Ala Pro Ala Tyr Gln Gln Gly Gln Asn Gln Leu Tyr
545                 550                 555                 560

Asn Glu Leu Asn Leu Gly Arg Arg Glu Glu Tyr Asp Val Leu Asp Lys
                565                 570                 575

Arg Arg Gly Arg Asp Pro Glu Met Gly Gly Lys Pro Arg Arg Lys Asn
            580                 585                 590

Pro Gln Glu Gly Leu Tyr Asn Glu Leu Gln Lys Asp Lys Met Ala Glu
        595                 600                 605

Ala Tyr Ser Glu Ile Gly Met Lys Gly Glu Arg Arg Arg Gly Lys Gly
    610                 615                 620

His Asp Gly Leu Tyr Gln Gly Leu Ser Thr Ala Thr Lys Asp Thr Tyr
625                 630                 635                 640

Asp Ala Leu His Met Gln Ala Leu Pro Pro Arg
```

645 650

<210> 26
<211> 2129
<212> DNA
<213> Artificial

<220>
<223> MCSP CD28/TCRZeta

<220>
<221> CDS
<222> (12)..(2129)

<400> 26
```
tctagactgc c atg gat ttt cag gtg cag att ttc agc ttc ctg cta atc      50
             Met Asp Phe Gln Val Gln Ile Phe Ser Phe Leu Leu Ile
             1               5                   10

agt gcc tca gtc ata atg tct aga atg gcg gac tac aaa gat att gtg       98
Ser Ala Ser Val Ile Met Ser Arg Met Ala Asp Tyr Lys Asp Ile Val
    15                  20                  25

atg acg caa tct cca gct tct ttg gct gtg tct cta ggg cag agg gcc      146
Met Thr Gln Ser Pro Ala Ser Leu Ala Val Ser Leu Gly Gln Arg Ala
30                  35                  40                  45

acc ata tcc tgc aga gcc agt gaa agt gtt gat agt tat ggc aat agt      194
Thr Ile Ser Cys Arg Ala Ser Glu Ser Val Asp Ser Tyr Gly Asn Ser
                50                  55                  60

ttt atg cac tgg tac cag cag aaa cca gga cag cca ccc aaa ctc ctc      242
Phe Met His Trp Tyr Gln Gln Lys Pro Gly Gln Pro Pro Lys Leu Leu
            65                  70                  75

atc tat ctt gca tcc aac cta gaa tct ggg gtc cct gcc agg ttc agt      290
Ile Tyr Leu Ala Ser Asn Leu Glu Ser Gly Val Pro Ala Arg Phe Ser
        80                  85                  90

ggc agt gga tct agg aca gac ttc acc ctc acc att gat cct gtg gag      338
Gly Ser Gly Ser Arg Thr Asp Phe Thr Leu Thr Ile Asp Pro Val Glu
    95                  100                 105

gct gat gat gct gca acc tat tac tgt caa caa aat aat gag gat cct      386
Ala Asp Asp Ala Ala Thr Tyr Tyr Cys Gln Gln Asn Asn Glu Asp Pro
110                 115                 120                 125

ctc acg ttc ggc tcg ggg aca aag ctg gaa aca aaa cgt ggt ggt ggt      434
Leu Thr Phe Gly Ser Gly Thr Lys Leu Glu Thr Lys Arg Gly Gly Gly
                130                 135                 140

ggt tct ggt ggt ggt ggt tct ggc ggc ggc ggc tcc ggt ggt ggt gga      482
Gly Ser Gly Gly Gly Gly Ser Gly Gly Gly Gly Ser Gly Gly Gly Gly
            145                 150                 155

tcc cag gtg cag ctg aag cag tct gga cct gag ctg gtg aag cct ggg      530
Ser Gln Val Gln Leu Lys Gln Ser Gly Pro Glu Leu Val Lys Pro Gly
        160                 165                 170

gcc tca gtg aag att tcc tgc aaa gct tct ggc tac gca ttc agt agg      578
Ala Ser Val Lys Ile Ser Cys Lys Ala Ser Gly Tyr Ala Phe Ser Arg
```

```
    175                 180                 185

tct tgg atg aac tgg gtg aag cag agg cct gga cag ggt ctt gag tgg      626
Ser Trp Met Asn Trp Val Lys Gln Arg Pro Gly Gln Gly Leu Glu Trp
190                 195                 200                 205

att gga cgg att tat cct gga gat gga gat act aac tac aat ggg aag      674
Ile Gly Arg Ile Tyr Pro Gly Asp Gly Asp Thr Asn Tyr Asn Gly Lys
                210                 215                 220

ttc aag ggc aag gcc aca ctg act gca gac aaa tcc tcc agc aca gcc      722
Phe Lys Gly Lys Ala Thr Leu Thr Ala Asp Lys Ser Ser Ser Thr Ala
            225                 230                 235

tac atg cag gtc agc agc ctg acc tct gtg gac tct gcg gtc tat ttc      770
Tyr Met Gln Val Ser Ser Leu Thr Ser Val Asp Ser Ala Val Tyr Phe
        240                 245                 250

tgt gca aga ggg aat acg gta gta gtt ccc tat act atg gac tac tgg      818
Cys Ala Arg Gly Asn Thr Val Val Val Pro Tyr Thr Met Asp Tyr Trp
    255                 260                 265

ggt caa gga acc tca gtc acc gtc ccc tcg gcc tcg ggg gcc gat ccc      866
Gly Gln Gly Thr Ser Val Thr Val Pro Ser Ala Ser Gly Ala Asp Pro
270                 275                 280                 285

atg gag ccc aaa tct cct gac aaa act cac aca tgc cca ccg tgc cca      914
Met Glu Pro Lys Ser Pro Asp Lys Thr His Thr Cys Pro Pro Cys Pro
                290                 295                 300

gca cct gaa ctc ctg ggg gga ccg tca gtc ttc ctc ttc ccc cca aaa      962
Ala Pro Glu Leu Leu Gly Gly Pro Ser Val Phe Leu Phe Pro Pro Lys
            305                 310                 315

ccc aag gac acc ctc atg atc tcc cgg acc cct gag gtc aca tgc gtg     1010
Pro Lys Asp Thr Leu Met Ile Ser Arg Thr Pro Glu Val Thr Cys Val
        320                 325                 330

gtg gtg gac gtg agc cac gaa gac cct gaa gtc aag ttc aac tgg tac     1058
Val Val Asp Val Ser His Glu Asp Pro Glu Val Lys Phe Asn Trp Tyr
    335                 340                 345

gtg gac ggc gtg gac ggc gtg gag gtg cat aat gcc aag aca aag ccg     1106
Val Asp Gly Val Asp Gly Val Glu Val His Asn Ala Lys Thr Lys Pro
350                 355                 360                 365

cgg gag gag cag tac aac agc acg tac cgt gtg gtc agc gtc ctc acc     1154
Arg Glu Glu Gln Tyr Asn Ser Thr Tyr Arg Val Val Ser Val Leu Thr
                370                 375                 380

gtc ctg cac cag gac tgg ctg aat ggc aag gag tac aag tgc aag gtc     1202
Val Leu His Gln Asp Trp Leu Asn Gly Lys Glu Tyr Lys Cys Lys Val
            385                 390                 395

tcc aac aaa gcc ctc cca gcc ccc atc gag aaa acc atc tcc aaa gcc     1250
Ser Asn Lys Ala Leu Pro Ala Pro Ile Glu Lys Thr Ile Ser Lys Ala
        400                 405                 410

aaa ggg cag ccc cga gaa cca cag gtg tac acc ctg ccc cca tcc cgg     1298
Lys Gly Gln Pro Arg Glu Pro Gln Val Tyr Thr Leu Pro Pro Ser Arg
    415                 420                 425

gat gag ctg acc aag aac cag gtc agc ctg acc tgc ctg gtc aaa ggc     1346
```

```
Asp Glu Leu Thr Lys Asn Gln Val Ser Leu Thr Cys Leu Val Lys Gly
430                 435                 440                 445

ttc tat ccc agc gac atc gcc gtg gag tgg gag agc aat ggg cag ccg      1394
Phe Tyr Pro Ser Asp Ile Ala Val Glu Trp Glu Ser Asn Gly Gln Pro
                450                 455                 460

gag aac aac tac aag acc acg cct ccc gtg ctg gac tcc gac ggc tcc      1442
Glu Asn Asn Tyr Lys Thr Thr Pro Pro Val Leu Asp Ser Asp Gly Ser
            465                 470                 475

ttc ttc ctc tac agc aag ctc acc gtg gac aag agc agg tgg cag cag      1490
Phe Phe Leu Tyr Ser Lys Leu Thr Val Asp Lys Ser Arg Trp Gln Gln
        480                 485                 490

ggg aac gtc ttc tca tgc tcc gtg atg cat gag gct ctg cac aac cac      1538
Gly Asn Val Phe Ser Cys Ser Val Met His Glu Ala Leu His Asn His
    495                 500                 505

tac acg cag aag agc ctc tcc ctg tct ccg ggt aaa aaa gat ccc aaa      1586
Tyr Thr Gln Lys Ser Leu Ser Leu Ser Pro Gly Lys Lys Asp Pro Lys
510                 515                 520                 525

ttt tgg gtg ctg gtg gtg gtt ggt gga gtc ctg gct tgc tat agc ttg      1634
Phe Trp Val Leu Val Val Val Gly Gly Val Leu Ala Cys Tyr Ser Leu
                530                 535                 540

cta gta aca gtg gcc ttt att att ttc tgg gtg agg agt aag agg agc      1682
Leu Val Thr Val Ala Phe Ile Ile Phe Trp Val Arg Ser Lys Arg Ser
            545                 550                 555

agg ctc ctg cac agt gac tac atg aac atg act ccc cgc cgc ccc ggg      1730
Arg Leu Leu His Ser Asp Tyr Met Asn Met Thr Pro Arg Arg Pro Gly
        560                 565                 570

ccc acc cgc aag cat tac cag ccc tat gcc cca cca cgc gac ttc gca      1778
Pro Thr Arg Lys His Tyr Gln Pro Tyr Ala Pro Pro Arg Asp Phe Ala
    575                 580                 585

gcc tat cgc tcc ctg aga gtg aag ttc agc agg agc gca gac gcc ccc      1826
Ala Tyr Arg Ser Leu Arg Val Lys Phe Ser Arg Ser Ala Asp Ala Pro
590                 595                 600                 605

gcg tac cag cag ggc cag aac cag ctc tat aac gag ctc aat cta gga      1874
Ala Tyr Gln Gln Gly Gln Asn Gln Leu Tyr Asn Glu Leu Asn Leu Gly
                610                 615                 620

cga aga gag gag tac gat gtt ttg gac aag aga cgt ggc cgg gac cct      1922
Arg Arg Glu Glu Tyr Asp Val Leu Asp Lys Arg Arg Gly Arg Asp Pro
            625                 630                 635

gag atg ggg gga aag ccg aga agg aag aac cct cag gaa ggc ctg tac      1970
Glu Met Gly Gly Lys Pro Arg Arg Lys Asn Pro Gln Glu Gly Leu Tyr
        640                 645                 650

aat gaa ctg cag aaa gat aag atg gcg gag gcc tac agt gag att ggg      2018
Asn Glu Leu Gln Lys Asp Lys Met Ala Glu Ala Tyr Ser Glu Ile Gly
    655                 660                 665

atg aaa ggc gag cgc cgg agg ggc aag ggg cac gat ggc ctt tac cag      2066
Met Lys Gly Glu Arg Arg Arg Gly Lys Gly His Asp Gly Leu Tyr Gln
670                 675                 680                 685
```

```
ggt ctc agt aca gcc acc aag gac acc tac gac gcc ctt cac atg cag      2114
Gly Leu Ser Thr Ala Thr Lys Asp Thr Tyr Asp Ala Leu His Met Gln
                690                 695                 700

gcc ctg ccc cct cgc                                                  2129
Ala Leu Pro Pro Arg
            705


<210>  27
<211>  706
<212>  PRT
<213>  Artificial

<220>
<223>  Synthetic Construct

<400>  27

Met Asp Phe Gln Val Gln Ile Phe Ser Phe Leu Leu Ile Ser Ala Ser
1               5                   10                  15


Val Ile Met Ser Arg Met Ala Asp Tyr Lys Asp Ile Val Met Thr Gln
            20                  25                  30


Ser Pro Ala Ser Leu Ala Val Ser Leu Gly Gln Arg Ala Thr Ile Ser
        35                  40                  45


Cys Arg Ala Ser Glu Ser Val Asp Ser Tyr Gly Asn Ser Phe Met His
    50                  55                  60


Trp Tyr Gln Gln Lys Pro Gly Gln Pro Pro Lys Leu Leu Ile Tyr Leu
65                  70                  75                  80


Ala Ser Asn Leu Glu Ser Gly Val Pro Ala Arg Phe Ser Gly Ser Gly
                85                  90                  95


Ser Arg Thr Asp Phe Thr Leu Thr Ile Asp Pro Val Glu Ala Asp Asp
            100                 105                 110


Ala Ala Thr Tyr Tyr Cys Gln Gln Asn Asn Glu Asp Pro Leu Thr Phe
        115                 120                 125


Gly Ser Gly Thr Lys Leu Glu Thr Lys Arg Gly Gly Gly Gly Ser Gly
    130                 135                 140


Gly Gly Gly Ser Gly Gly Gly Gly Ser Gly Gly Gly Gly Ser Gln Val
145                 150                 155                 160


Gln Leu Lys Gln Ser Gly Pro Glu Leu Val Lys Pro Gly Ala Ser Val
                165                 170                 175
```

```
Lys Ile Ser Cys Lys Ala Ser Gly Tyr Ala Phe Ser Arg Ser Trp Met
                180                 185                 190

Asn Trp Val Lys Gln Arg Pro Gly Gln Gly Leu Glu Trp Ile Gly Arg
            195                 200                 205

Ile Tyr Pro Gly Asp Gly Asp Thr Asn Tyr Asn Gly Lys Phe Lys Gly
    210                 215                 220

Lys Ala Thr Leu Thr Ala Asp Lys Ser Ser Ser Thr Ala Tyr Met Gln
225                 230                 235                 240

Val Ser Ser Leu Thr Ser Val Asp Ser Ala Val Tyr Phe Cys Ala Arg
                245                 250                 255

Gly Asn Thr Val Val Val Pro Tyr Thr Met Asp Tyr Trp Gly Gln Gly
            260                 265                 270

Thr Ser Val Thr Val Pro Ser Ala Ser Gly Ala Asp Pro Met Glu Pro
            275                 280                 285

Lys Ser Pro Asp Lys Thr His Thr Cys Pro Pro Cys Pro Ala Pro Glu
    290                 295                 300

Leu Leu Gly Gly Pro Ser Val Phe Leu Phe Pro Pro Lys Pro Lys Asp
305                 310                 315                 320

Thr Leu Met Ile Ser Arg Thr Pro Glu Val Thr Cys Val Val Val Asp
                325                 330                 335

Val Ser His Glu Asp Pro Glu Val Lys Phe Asn Trp Tyr Val Asp Gly
            340                 345                 350

Val Asp Gly Val Glu Val His Asn Ala Lys Thr Lys Pro Arg Glu Glu
        355                 360                 365

Gln Tyr Asn Ser Thr Tyr Arg Val Val Ser Val Leu Thr Val Leu His
    370                 375                 380

Gln Asp Trp Leu Asn Gly Lys Glu Tyr Lys Cys Lys Val Ser Asn Lys
385                 390                 395                 400

Ala Leu Pro Ala Pro Ile Glu Lys Thr Ile Ser Lys Ala Lys Gly Gln
                405                 410                 415

Pro Arg Glu Pro Gln Val Tyr Thr Leu Pro Pro Ser Arg Asp Glu Leu
            420                 425                 430
```

```
Thr Lys Asn Gln Val Ser Leu Thr Cys Leu Val Lys Gly Phe Tyr Pro
        435                 440                 445

Ser Asp Ile Ala Val Glu Trp Glu Ser Asn Gly Gln Pro Glu Asn Asn
    450                 455                 460

Tyr Lys Thr Thr Pro Pro Val Leu Asp Ser Asp Gly Ser Phe Phe Leu
465                 470                 475                 480

Tyr Ser Lys Leu Thr Val Asp Lys Ser Arg Trp Gln Gln Gly Asn Val
                485                 490                 495

Phe Ser Cys Ser Val Met His Glu Ala Leu His Asn His Tyr Thr Gln
            500                 505                 510

Lys Ser Leu Ser Leu Ser Pro Gly Lys Lys Asp Pro Lys Phe Trp Val
        515                 520                 525

Leu Val Val Val Gly Gly Val Leu Ala Cys Tyr Ser Leu Leu Val Thr
    530                 535                 540

Val Ala Phe Ile Ile Phe Trp Val Arg Ser Lys Arg Ser Arg Leu Leu
545                 550                 555                 560

His Ser Asp Tyr Met Asn Met Thr Pro Arg Arg Pro Gly Pro Thr Arg
                565                 570                 575

Lys His Tyr Gln Pro Tyr Ala Pro Pro Arg Asp Phe Ala Ala Tyr Arg
            580                 585                 590

Ser Leu Arg Val Lys Phe Ser Arg Ser Ala Asp Ala Pro Ala Tyr Gln
        595                 600                 605

Gln Gly Gln Asn Gln Leu Tyr Asn Glu Leu Asn Leu Gly Arg Arg Glu
    610                 615                 620

Glu Tyr Asp Val Leu Asp Lys Arg Arg Gly Arg Asp Pro Glu Met Gly
625                 630                 635                 640

Gly Lys Pro Arg Arg Lys Asn Pro Gln Glu Gly Leu Tyr Asn Glu Leu
                645                 650                 655

Gln Lys Asp Lys Met Ala Glu Ala Tyr Ser Glu Ile Gly Met Lys Gly
            660                 665                 670

Glu Arg Arg Arg Gly Lys Gly His Asp Gly Leu Tyr Gln Gly Leu Ser
        675                 680                 685
```

```
Thr Ala Thr Lys Asp Thr Tyr Asp Ala Leu His Met Gln Ala Leu Pro
    690                 695                 700

Pro Arg
705


<210>  28
<211>  1973
<212>  DNA
<213>  Artificial

<220>
<223>  MCSP TCRZeta


<220>
<221>  CDS
<222>  (12)..(1973)

<400>  28
tctagactgc c atg gat ttt cag gtg cag att ttc agc ttc ctg cta atc      50
             Met Asp Phe Gln Val Gln Ile Phe Ser Phe Leu Leu Ile
             1               5                   10

agt gcc tca gtc ata atg tct aga atg gcg gac tac aaa gat att gtg       98
Ser Ala Ser Val Ile Met Ser Arg Met Ala Asp Tyr Lys Asp Ile Val
    15                  20                  25

atg acg caa tct cca gct tct ttg gct gtg tct cta ggg cag agg gcc      146
Met Thr Gln Ser Pro Ala Ser Leu Ala Val Ser Leu Gly Gln Arg Ala
30                  35                  40                  45

acc ata tcc tgc aga gcc agt gaa agt gtt gat agt tat ggc aat agt      194
Thr Ile Ser Cys Arg Ala Ser Glu Ser Val Asp Ser Tyr Gly Asn Ser
                50                  55                  60

ttt atg cac tgg tac cag cag aaa cca gga cag cca ccc aaa ctc ctc      242
Phe Met His Trp Tyr Gln Gln Lys Pro Gly Gln Pro Pro Lys Leu Leu
            65                  70                  75

atc tat ctt gca tcc aac cta gaa tct ggg gtc cct gcc agg ttc agt      290
Ile Tyr Leu Ala Ser Asn Leu Glu Ser Gly Val Pro Ala Arg Phe Ser
        80                  85                  90

ggc agt gga tct agg aca gac ttc acc ctc acc att gat cct gtg gag      338
Gly Ser Gly Ser Arg Thr Asp Phe Thr Leu Thr Ile Asp Pro Val Glu
    95                  100                 105

gct gat gat gct gca acc tat tac tgt caa caa aat aat gag gat cct      386
Ala Asp Asp Ala Ala Thr Tyr Tyr Cys Gln Gln Asn Asn Glu Asp Pro
110                 115                 120                 125

ctc acg ttc ggc tcg ggg aca aag ctg gaa aca aaa cgt ggt ggt ggt      434
Leu Thr Phe Gly Ser Gly Thr Lys Leu Glu Thr Lys Arg Gly Gly Gly
                130                 135                 140

ggt tct ggt ggt ggt ggt tct ggc ggc ggc ggc tcc ggt ggt ggt gga      482
Gly Ser Gly Gly Gly Gly Ser Gly Gly Gly Gly Ser Gly Gly Gly Gly
            145                 150                 155
```

```
tcc cag gtg cag ctg aag cag tct gga cct gag ctg gtg aag cct ggg      530
Ser Gln Val Gln Leu Lys Gln Ser Gly Pro Glu Leu Val Lys Pro Gly
        160                 165                 170

gcc tca gtg aag att tcc tgc aaa gct tct ggc tac gca ttc agt agg      578
Ala Ser Val Lys Ile Ser Cys Lys Ala Ser Gly Tyr Ala Phe Ser Arg
    175                 180                 185

tct tgg atg aac tgg gtg aag cag agg cct gga cag ggt ctt gag tgg      626
Ser Trp Met Asn Trp Val Lys Gln Arg Pro Gly Gln Gly Leu Glu Trp
190                 195                 200                 205

att gga cgg att tat cct gga gat gga gat act aac tac aat ggg aag      674
Ile Gly Arg Ile Tyr Pro Gly Asp Gly Asp Thr Asn Tyr Asn Gly Lys
                210                 215                 220

ttc aag ggc aag gcc aca ctg act gca gac aaa tcc tcc agc aca gcc      722
Phe Lys Gly Lys Ala Thr Leu Thr Ala Asp Lys Ser Ser Ser Thr Ala
            225                 230                 235

tac atg cag gtc agc agc ctg acc tct gtg gac tct gcg gtc tat ttc      770
Tyr Met Gln Val Ser Ser Leu Thr Ser Val Asp Ser Ala Val Tyr Phe
        240                 245                 250

tgt gca aga ggg aat acg gta gta gtt ccc tat act atg gac tac tgg      818
Cys Ala Arg Gly Asn Thr Val Val Val Pro Tyr Thr Met Asp Tyr Trp
    255                 260                 265

ggt caa gga acc tca gtc acc gtc ccc tcg gcc tcg ggg gcc gat ccc      866
Gly Gln Gly Thr Ser Val Thr Val Pro Ser Ala Ser Gly Ala Asp Pro
270                 275                 280                 285

atg gag ccc aaa tct cct gac aaa act cac aca tgc cca ccg tgc cca      914
Met Glu Pro Lys Ser Pro Asp Lys Thr His Thr Cys Pro Pro Cys Pro
                290                 295                 300

gca cct gaa ctc ctg ggg gga ccg tca gtc ttc ctc ttc ccc cca aaa      962
Ala Pro Glu Leu Leu Gly Gly Pro Ser Val Phe Leu Phe Pro Pro Lys
            305                 310                 315

ccc aag gac acc ctc atg atc tcc cgg acc cct gag gtc aca tgc gtg     1010
Pro Lys Asp Thr Leu Met Ile Ser Arg Thr Pro Glu Val Thr Cys Val
        320                 325                 330

gtg gac gtg agc cac gaa gac cct gag gtc aag ttc aac tgg tac gtg     1058
Val Asp Val Ser His Glu Asp Pro Glu Val Lys Phe Asn Trp Tyr Val
    335                 340                 345

gac ggc gtg gag gtg cat aat gcc aag aca aag ccg cgg gag gag cag     1106
Asp Gly Val Glu Val His Asn Ala Lys Thr Lys Pro Arg Glu Glu Gln
350                 355                 360                 365

tac aac agc acg tac cgt gtg gtc agc gtc ctc acc gtc ctg cac cag     1154
Tyr Asn Ser Thr Tyr Arg Val Val Ser Val Leu Thr Val Leu His Gln
                370                 375                 380

gac tgg ctg aat ggc aag gag tac aag tgc aag gtc tcc aac aaa gcc     1202
Asp Trp Leu Asn Gly Lys Glu Tyr Lys Cys Lys Val Ser Asn Lys Ala
            385                 390                 395

ctc cca gcc ccc atc gag aaa acc atc tcc aaa gcc aaa ggg cag ccc     1250
Leu Pro Ala Pro Ile Glu Lys Thr Ile Ser Lys Ala Lys Gly Gln Pro
```

```
        400                 405                 410

cga gaa cca cag gtg tac acc ctg ccc cca tcc cgg gat gag ctg acc      1298
Arg Glu Pro Gln Val Tyr Thr Leu Pro Pro Ser Arg Asp Glu Leu Thr
    415                 420                 425

aag aac cag gtc agc ctg acc tgc ctg gtc aaa ggc ttc tat ccc agc      1346
Lys Asn Gln Val Ser Leu Thr Cys Leu Val Lys Gly Phe Tyr Pro Ser
430                 435                 440                 445

gac atc gcc gtg gag tgg gag agc aat ggg caa ccg gag aac aac tac      1394
Asp Ile Ala Val Glu Trp Glu Ser Asn Gly Gln Pro Glu Asn Asn Tyr
                450                 455                 460

aag acc acg cct ccc gtg ctg gac tcc gac ggc tcc ttc ttc ctc tac      1442
Lys Thr Thr Pro Pro Val Leu Asp Ser Asp Gly Ser Phe Phe Leu Tyr
            465                 470                 475

agc aag ctc acc gtg gac aag agc agg tgg cag cag ggg aac gtc ttc      1490
Ser Lys Leu Thr Val Asp Lys Ser Arg Trp Gln Gln Gly Asn Val Phe
        480                 485                 490

tca tgc tcc gtg atg cat gag gct ctg cac aac cac tac acg cag aag      1538
Ser Cys Ser Val Met His Glu Ala Leu His Asn His Tyr Thr Gln Lys
    495                 500                 505

agc ctc tcc ctg tct ccg ggt aaa aaa gat ccc aaa ctc tgc tac ctg      1586
Ser Leu Ser Leu Ser Pro Gly Lys Lys Asp Pro Lys Leu Cys Tyr Leu
510                 515                 520                 525

ctg gat gga atc ctc ttc atc tat ggt gtc att ctc act gcc ttg ttc      1634
Leu Asp Gly Ile Leu Phe Ile Tyr Gly Val Ile Leu Thr Ala Leu Phe
                530                 535                 540

ctg aga gtg aag ttc agc agg agc gca gac gcc ccc gcg tac cag cag      1682
Leu Arg Val Lys Phe Ser Arg Ser Ala Asp Ala Pro Ala Tyr Gln Gln
            545                 550                 555

ggc cag aac cag ctc tat aac gag ctc aat cta gga cga aga gag gag      1730
Gly Gln Asn Gln Leu Tyr Asn Glu Leu Asn Leu Gly Arg Arg Glu Glu
        560                 565                 570

tac gat gtt ttg gac aag aga cgt ggc cgg gac cct gag atg ggg gga      1778
Tyr Asp Val Leu Asp Lys Arg Arg Gly Arg Asp Pro Glu Met Gly Gly
    575                 580                 585

aag ccg aga agg aag aac cct cag gaa ggc ctg tac aat gaa ctg cag      1826
Lys Pro Arg Arg Lys Asn Pro Gln Glu Gly Leu Tyr Asn Glu Leu Gln
590                 595                 600                 605

aaa gat aag atg gcg gag gcc tac agt gag att ggg atg aaa ggc gag      1874
Lys Asp Lys Met Ala Glu Ala Tyr Ser Glu Ile Gly Met Lys Gly Glu
                610                 615                 620

cgc cgg agg ggc aag ggg cac gat ggc ctt tac cag ggt ctc agt aca      1922
Arg Arg Arg Gly Lys Gly His Asp Gly Leu Tyr Gln Gly Leu Ser Thr
            625                 630                 635

gcc acc aag gac acc tac gac gcc ctt cac atg cag gcc ctg ccc cct      1970
Ala Thr Lys Asp Thr Tyr Asp Ala Leu His Met Gln Ala Leu Pro Pro
        640                 645                 650

cgc                                                                  1973
```

Arg

<210> 29
<211> 654
<212> PRT
<213> Artificial

<220>
<223> Synthetic Construct

<400> 29

Met Asp Phe Gln Val Gln Ile Phe Ser Phe Leu Leu Ile Ser Ala Ser
1 5 10 15

Val Ile Met Ser Arg Met Ala Asp Tyr Lys Asp Ile Val Met Thr Gln
20 25 30

Ser Pro Ala Ser Leu Ala Val Ser Leu Gly Gln Arg Ala Thr Ile Ser
35 40 45

Cys Arg Ala Ser Glu Ser Val Asp Ser Tyr Gly Asn Ser Phe Met His
50 55 60

Trp Tyr Gln Gln Lys Pro Gly Gln Pro Pro Lys Leu Leu Ile Tyr Leu
65 70 75 80

Ala Ser Asn Leu Glu Ser Gly Val Pro Ala Arg Phe Ser Gly Ser Gly
85 90 95

Ser Arg Thr Asp Phe Thr Leu Thr Ile Asp Pro Val Glu Ala Asp Asp
100 105 110

Ala Ala Thr Tyr Tyr Cys Gln Gln Asn Asn Glu Asp Pro Leu Thr Phe
115 120 125

Gly Ser Gly Thr Lys Leu Glu Thr Lys Arg Gly Gly Gly Gly Ser Gly
130 135 140

Gly Gly Gly Ser Gly Gly Gly Gly Ser Gly Gly Gly Gly Ser Gln Val
145 150 155 160

Gln Leu Lys Gln Ser Gly Pro Glu Leu Val Lys Pro Gly Ala Ser Val
165 170 175

Lys Ile Ser Cys Lys Ala Ser Gly Tyr Ala Phe Ser Arg Ser Trp Met
180 185 190

Asn Trp Val Lys Gln Arg Pro Gly Gln Gly Leu Glu Trp Ile Gly Arg

```
        195                 200                 205

Ile Tyr Pro Gly Asp Gly Asp Thr Asn Tyr Asn Gly Lys Phe Lys Gly
    210                 215                 220

Lys Ala Thr Leu Thr Ala Asp Lys Ser Ser Ser Thr Ala Tyr Met Gln
225                 230                 235                 240

Val Ser Ser Leu Thr Ser Val Asp Ser Ala Val Tyr Phe Cys Ala Arg
                245                 250                 255

Gly Asn Thr Val Val Val Pro Tyr Thr Met Asp Tyr Trp Gly Gln Gly
            260                 265                 270

Thr Ser Val Thr Val Pro Ser Ala Ser Gly Ala Asp Pro Met Glu Pro
        275                 280                 285

Lys Ser Pro Asp Lys Thr His Thr Cys Pro Pro Cys Pro Ala Pro Glu
    290                 295                 300

Leu Leu Gly Gly Pro Ser Val Phe Leu Phe Pro Pro Lys Pro Lys Asp
305                 310                 315                 320

Thr Leu Met Ile Ser Arg Thr Pro Glu Val Thr Cys Val Val Asp Val
                325                 330                 335

Ser His Glu Asp Pro Glu Val Lys Phe Asn Trp Tyr Val Asp Gly Val
            340                 345                 350

Glu Val His Asn Ala Lys Thr Lys Pro Arg Glu Glu Gln Tyr Asn Ser
        355                 360                 365

Thr Tyr Arg Val Val Ser Val Leu Thr Val Leu His Gln Asp Trp Leu
    370                 375                 380

Asn Gly Lys Glu Tyr Lys Cys Lys Val Ser Asn Lys Ala Leu Pro Ala
385                 390                 395                 400

Pro Ile Glu Lys Thr Ile Ser Lys Ala Lys Gly Gln Pro Arg Glu Pro
                405                 410                 415

Gln Val Tyr Thr Leu Pro Pro Ser Arg Asp Glu Leu Thr Lys Asn Gln
            420                 425                 430

Val Ser Leu Thr Cys Leu Val Lys Gly Phe Tyr Pro Ser Asp Ile Ala
        435                 440                 445
```

```
Val Glu Trp Glu Ser Asn Gly Gln Pro Glu Asn Asn Tyr Lys Thr Thr
    450                 455                 460

Pro Pro Val Leu Asp Ser Asp Gly Ser Phe Phe Leu Tyr Ser Lys Leu
465                 470                 475                 480

Thr Val Asp Lys Ser Arg Trp Gln Gln Gly Asn Val Phe Ser Cys Ser
                485                 490                 495

Val Met His Glu Ala Leu His Asn His Tyr Thr Gln Lys Ser Leu Ser
            500                 505                 510

Leu Ser Pro Gly Lys Lys Asp Pro Lys Leu Cys Tyr Leu Leu Asp Gly
        515                 520                 525

Ile Leu Phe Ile Tyr Gly Val Ile Leu Thr Ala Leu Phe Leu Arg Val
    530                 535                 540

Lys Phe Ser Arg Ser Ala Asp Ala Pro Ala Tyr Gln Gln Gly Gln Asn
545                 550                 555                 560

Gln Leu Tyr Asn Glu Leu Asn Leu Gly Arg Arg Glu Glu Tyr Asp Val
                565                 570                 575

Leu Asp Lys Arg Arg Gly Arg Asp Pro Glu Met Gly Gly Lys Pro Arg
            580                 585                 590

Arg Lys Asn Pro Gln Glu Gly Leu Tyr Asn Glu Leu Gln Lys Asp Lys
        595                 600                 605

Met Ala Glu Ala Tyr Ser Glu Ile Gly Met Lys Gly Glu Arg Arg Arg
    610                 615                 620

Gly Lys Gly His Asp Gly Leu Tyr Gln Gly Leu Ser Thr Ala Thr Lys
625                 630                 635                 640

Asp Thr Tyr Asp Ala Leu His Met Gln Ala Leu Pro Pro Arg
                645                 650
```